# EUROPEAN PATENT APPLICATION

(11) **EP 3 492 482 A1**
(43) Date of publication of application: **05.06.2019**
(21) Application number: 17204817.5
(22) Date of filing: 30.11.2017
(51) Int. Cl.: C07H 15/04, C07H 15/08, C07H 15/18, C07H 15/26, A61K 39/108, A61K 47/64

(54) **VACCINE AGAINST KLEBSIELLA PNEUMONIAE**

(71) Applicant: Vaxxilon AG, 4153 Reinach (CH)
(72) Inventor: PEREIRA, Claney Lebev, 12203 Berlin (DE)
(74) Representative: Arth, Hans-Lothar

(57) **Abstract**

The present invention relates to a synthetic saccharide of general formula **(I)** that is related to *Klebsiella pneumoniae* serotype O1, O2, O2ac, and O8 O-polysaccharide and carbapenem-resistant *Klebsiella pneumoniae* ST258 O-polysaccharide and conjugate thereof. Said synthetic saccharide, said conjugate and pharmaceutical composition containing said synthetic saccharide or said conjugate are useful for prevention and/or treatment of diseases associated with *Klebsiella pneumoniae.* Furthermore, the synthetic saccharide of general formula **(I)** is useful as marker in immunological assays for detection of antibodies against *Klebsiella pneumoniae* bacteria.

## Description

### Field of the invention

The present invention relates to a synthetic saccharide of general formula (**I**) that is related to *Klebsiella pneumoniae* serotype O1, O2, O2ac, and O8 O-polysaccharide and carbapanem-resistant *Klebsiella pneumoniae* ST258 O-polysaccharide and conjugate thereof. Said synthetic saccharide, said conjugate and pharmaceutical composition containing said synthetic saccharide or said conjugate are useful for prevention and/or treatment of diseases associated with *Klebsiella pneumoniae.* Furthermore, the synthetic saccharide of general formula (**I**) is useful as marker in immunological assays for detection of antibodies against *Klebsiella pneumoniae* bacteria.

### Background of the invention

*Klebsiella pneumoniae* is a gram-negative, facultative anaerobic, rod-shaped bacterium colonizing mostly of the respiratory and urinary tracts and causing *K*. *pneumoniae* infections (KPIs). KPI is the main cause of nosocomial infections, primarily affecting immunocompromised patients. In the last ten years, infections caused by *K*. *pneumoniae* are becoming an important challenge in health-care settings due to the emergence of strains resistant to almost all available antimicrobial agents and their worldwide dissemination. Infections caused by *Klebsiella pneumoniae* are responsible of high rates of morbidity and mortality. Thus, prevention of infections caused by *K*. *pneumoniae* is highly desirable, and vaccination of risk groups is the most cost-efficient and the most powerful means.

Like most bacteria, *K*. *pneumoniae* usually develop capsules composed of complex polysaccharides on the bacterial surface, which are highly immunogenic and nontoxic. In comparison with proteins, carbohydrates are evolutionarily more stable and have been exploited in a series of commonly employed vaccines. When covalently connected to a carrier protein, oligosaccharide antigens can elicit long lasting, T-cell-dependent protection. *K*. *pneumoniae* typically expresses both, lipopolysaccharide (LPS) and capsular polysaccharide (CPS, K-antigen), which contribute to the virulence of this species. LPS is a main surface antigen built of the O-specific polysaccharide (O-PS) containing different number of oligosaccharide repeating units (RU), core oligosaccharide and lipid A. O-PS structures (O-antigens) define O-serotypes of *Klebsiella* strains. Variability of *K*. *pneumoniae* O-antigens is currently limited to 9 major O-serotypes: O1, O2, O2ac, O3, O4, O5, O7, O8, 012 and a few subtypes within these serogroups such as subtypes O2a, O2ab, O2ae, O2aeh, and O2afg of serotype O2. *Klebsiella pneumoniae* has also been classified serologically into numerous capsular (K) types. Therefore, various *K*. *pneumoniae* strains having different K antigens belong to a specific O-antigen serotype. For example, numerous K-serotypes of *Klebsiella pneumoniae* strains belonging to O1-antigen serotype have been identified (Infection and Immunity, 1983, p.56-61). Most popular K-serotypes of *Klebsiella pneumoniae* strains belonging to O1-antigen serotype are O1:K1, O1:K2, O1:K7, O1:K8, O1:K10, O1:K12, O1:K16, O1:K19, O1:K21, O1:K22, O:K27, O:K34, O1:K42, O1:K45, O1:K55, O1:K57, O1:K62, O1:K65, O1:K66, O1:K69 and O1:K70.
Recently, carbapenem resistant *Klebsiella pneumoniae* (CRKP) has emerged and spread globally. Carbapenem resistant *K*. *pneumoniae* (CRKP) is a major health concern due to the very limited treatment options. Such CRKP has usually carbapenemases that are able to cleave most beta-lactam type antibiotics. A specific lineage termed sequence type (ST) 258 has been shown to be responsible for the majority of KPC-producing *Klebsiella* infections. It is also known that CRKP ST258 strains have different capsular polysaccharide (CPS).
Lipopolysaccharide (LPS) and capsular polysaccharide (CPS), two surface components of Klebsiella pneumonia are mainly discussed as candidates for an anti-Klebsiella vaccine. CPS has been proven to be highly immunogenic. However, the serious disadvantage of Klebsiella CPS vaccine is the great number of K-types (more 80 different antigens). In the utilization of LPS antigens in Klebsiella vaccines, the adverse toxic reactions caused mainly by the lipid A of LPS present a great drawback of active immunization with LPS-containing vaccines.
Since O-antigens are far less variable than CPS, *Klebsiella pneumoniae* LPS O-antigens without core oligosaccharide and lipid A can be potential target antigens for immunotherapy.

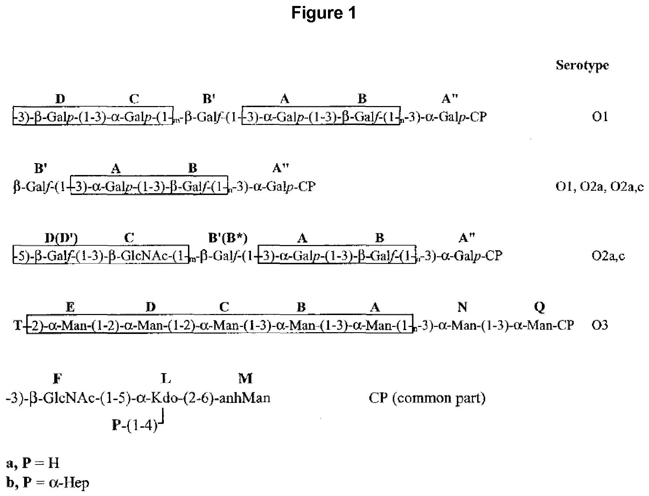

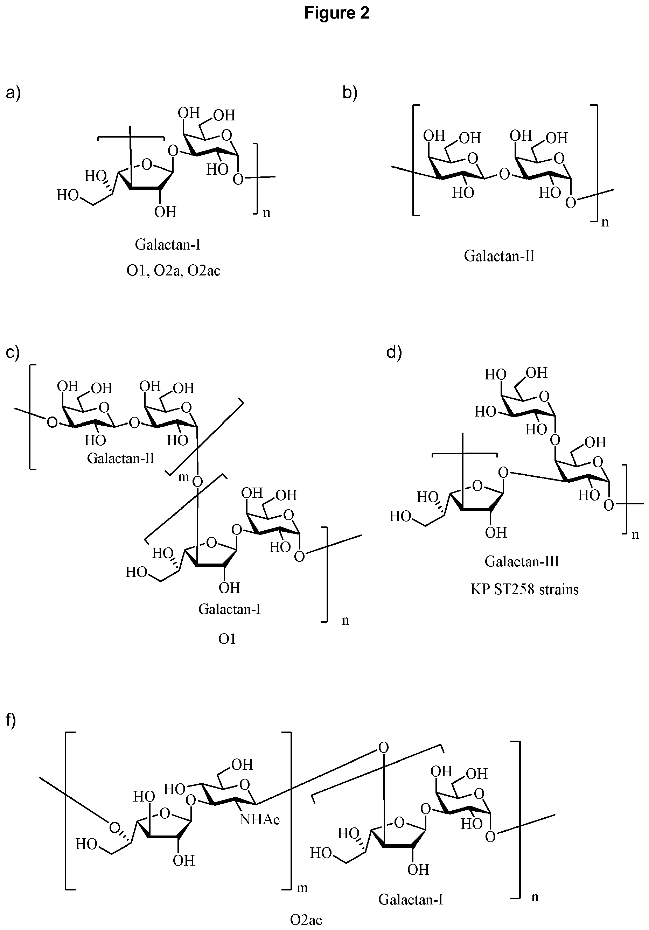

The repeating units of the O-antigens, i.e. O-polysaccharides of *K*. *pneumoniae* were elucidated (Journal of Bacteriology, 1996, p.5205-5214; The Journal of Biological Chemistry, 2002, 277 (28), pp.25070-25081) (see **Figures 1** and **2**).
The common structure of the O-polysaccharide (OPS) of *K*. *pneumoniae serotype* O1, O2a, O2ab, O2ac consists of a disaccharide repeating unit:

→ 3)-β-D-Gal*f*-(1→3)-α-D-Gal*p*-(1→ (galactan I).

The common structure of the O-polysaccharide (OPS) of *K*. *pneumoniae serotypes* O1, and O8 consists of a disaccharide repeating unit:

→ 3)-β-D-Gal*p*-(1→3)-α-D-Gal*p*-(1→ (galactan II)

The repeating unit of the O-polysaccharide of *K*. *pneumoniae serotype* O1 consists of:

[→3)-β-D-Gal*p*-(1→3)-α-D-Gal*p*-(1→]ₘ-[→3)-β-D-Gal*f*-(1→3)-α-D-Gal*p*-(1→]ₙ.

The repeating unit of the O-polysaccharide of *K. pneumoniae serotype* O2a consists of:

→ 3)-β-D-Gal*f*-(1→3)-α-D-Gal*p*-(1→.

The repeating unit of the O-polysaccharide of *K. pneumoniae serotype* O2ac consists of:

[→5)-β-D-Gal*f*-(1→3)-β-D-GlcNAc-(1→]ₘ-[→3)-β-D-Gal*f*-(1→3)-α-D-Gal*p*-(1→]ₙ.

The repeating unit of the O-polysaccharide of *K. pneumoniae serotypes* O2ae and O2aeh consists of: 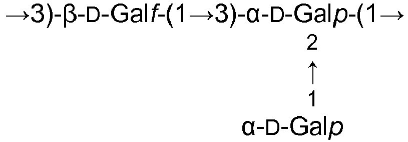 The repeating unit of the O-polysaccharide of *K. pneumoniae serotype* O2afg, and carbapenem resistant *K. pneumoniae (CRKP) ST258 strains* consists of: 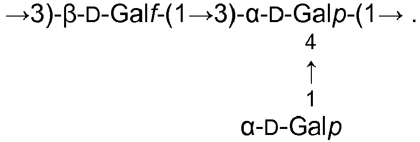 The repeating unit of the O-polysaccharide of *K. pneumoniae serotype* O8 consists of a pentasaccharide:

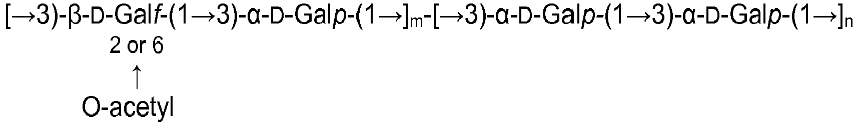

It is the objective of the present invention to provide a well-defined synthetic saccharide of general formula (**I**) that is related *Klebsiella pneumoniae* O-polysaccharide and contains a protective immunogenic O-antigen epitope i.e. an O-antigen epitope that elicits antibodies which protect against diseases caused by *Klebsiella pneumoniae.* Said saccharide can be conjugated to an immunogenic carrier to provide a conjugate and pharmaceutical composition thereof that are useful for prevention and/or treatment of diseases associated with *Klebsiella pneumoniae.* Furthermore, the synthetic saccharide of general formula (**I**) is useful as marker in immunological assays for detection of antibodies against *Klebsiella pneumoniae* bacteria.

The objective of the present invention is solved by the teaching of the independent claims. Further advantageous features, aspects and details of the invention are evident from the dependent claims, the description, the figures, and the examples of the present application.

### Description of the invention

### Definitions

The term "linker" as used herein encompasses molecular fragments capable of connecting the reducing-end monosaccharide of a saccharide with an immunogenic carrier or a solid support, optionally by binding to at least one interconnecting molecule. Thus, the function of the linker *per se* or together with the interconnecting molecule is to establish, keep and/or bridge a special distance between the reducing-end monosaccharide and an immunogenic carrier or a solid support. More specifically, one extremity of the linker is connected to the exocyclic oxygen atom at the anomeric center of the reducing-end monosaccharide and the other extremity is connected *via* the nitrogen atom with the interconnecting molecule, or directly with the immunogenic carrier or the solid support.

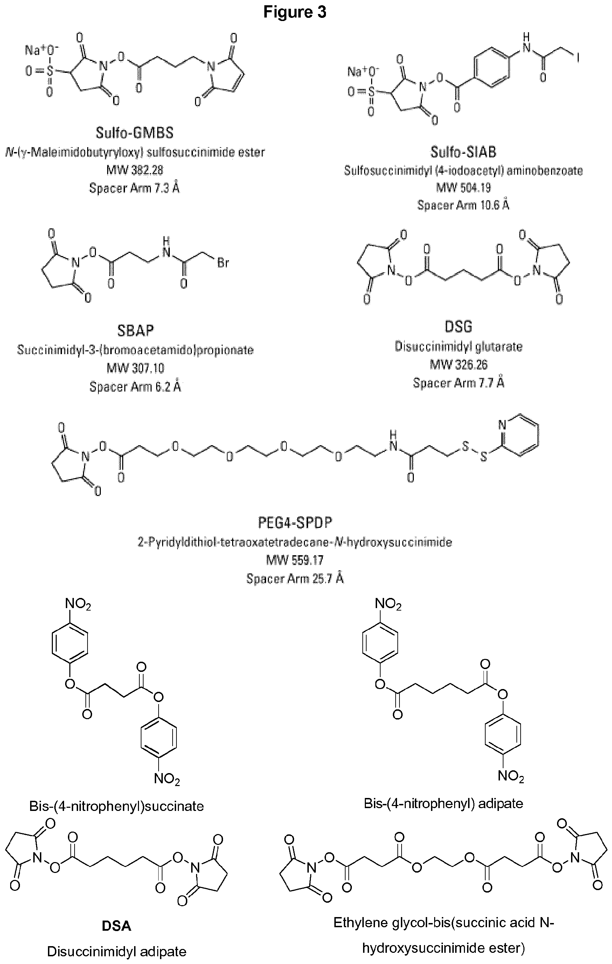

As used herein, the term "interconnecting molecule" refers to a bifunctional molecule containing functional group X and functional group Y, wherein functional group X is capable of reacting with the terminal amino group on the linker L and the functional group Y is capable of reacting with a functionality present on an immunogenic carrier or on a solid support. **Figure 3** displays examples of commercially available interconnecting molecules, but does not restrict the interconnecting molecules that can be used according to the present invention to the examples displayed herein.

The term "adjuvant" as used herein refers to an immunological adjuvant i.e. a material used in a vaccine composition that modifies or augments the effects of said vaccine by enhancing the immune response to a given antigen contained in the vaccine without being antigenically related to it. For the person skilled in the art, classically recognized examples of adjuvants include:
- mineral-containing compositions, including calcium salts and aluminium salts (or mixtures thereof). Calcium salts include calcium phosphate. Aluminium salts include hydroxides, phosphates, sulfates, etc., with the salts taking any suitable form (e.g. gel, crystalline, amorphous, etc.). Adsorption to these salts is preferred. The mineral containing compositions may also be formulated as a particle of metal salt. The adjuvants known as aluminium hydroxide and aluminium phosphate may be also used. The invention can use any of the "hydroxide" or "phosphate" adjuvants that are in general used as adjuvants. The adjuvants known as "aluminium hydroxide" are typically aluminium oxyhydroxide salts, which are usually at least partially crystalline. The adjuvants known as "aluminium phosphate" are typically aluminium hydroxyphosphates, often also containing a small amount of sulfate (i. e. aluminium hydroxyphosphate sulfate). They may be obtained by precipitation, and the reaction conditions and concentrations during precipitation influence the degree of substitution of phosphate for hydroxyl in the salt. Mixtures of both an aluminium hydroxide and an aluminium phosphate can be employed in the formulation according to the present invention;

- saponins, which are a heterologous group of sterol glycosides and triterpenoid glycosides that are found in the bark, leaves, stems, roots and even flowers of a wide range of plant species. Saponins from the bark of the *Quillaia saponaria,* Molina tree have been widely studied as adjuvants. Saponins can also be commercially obtained from Smilax ornata (sarsaprilla), Gypsophilla paniculata (brides veil), and Saponaria oficianalis (soap root). Saponin adjuvant formulations include purified formulations, such as QS21, as well as lipid formulations, such as ISCOMs. Saponin compositions have been purified using HPLC and RP-HPLC. Specific purified fractions using these techniques have been identified, including QS 7, QS 17, QS 18, QS2 1, QH-A, QH-B and QH-C. Saponin formulations may also comprise a sterol, such as cholesterol. Combinations of saponins and cholesterols can be used to form unique particles called immunostimulating complexes (ISCOMs). ISCOMs generally include a phospholipid such as phosphatidylethanolamine or phosphatidylcholine. Any known saponin can be used in ISCOMs. Preferably, the ISCOM includes one or more of QuilA, QHA & QHC;
- microparticles (i.e. a particle of 100 nm to 150 pm in diameter, more preferably 200 nm to 30 pm in diameter, or 500 nm to 10 pm in diameter) formed from materials that are biodegradable and non-toxic. Such non-toxic and biodegradable materials include, but are not restricted to poly(α-hydroxy acid), polyhydroxybutyric acid, polyorthoester, polyanhydride, polycaprolactone;
- CD1d ligands, such as an α-glycosylceramide, phytosphingosine-containing α-glycosylceramides, OCH, KRN7000 [(2*S*,3*S*,4*R*)-1-*O*-(α-D-galactopyranosyl)-2-(*N-*hexacosanoylamino)-1,3,4-octadecanetriol], CRONY- 101, 3"-sulfo-galactosyl-ceramide;
- immunostimulatory oligonucleotides, such CpG motif containing ones (a dinucleotide sequence containing an unmethylated cytosine residue linked by a phosphate bond to a guanosine residue), or Cpl motif containing ones (a dinucleotide sequence containing cytosine linked to inosine), or a double-stranded RNA, or an oligonucleotide containing a palindromic sequence, or an oligonucleotide containing a poly(dG) sequence. Immunostimulatory oligonucleotides can include nucleotide modifications/analogs such as phosphorothioate modifications and can be double-stranded or (except for RNA) single-stranded;
- compounds containing lipids linked to a phosphate-containing acyclic backbone, such as the TLR4 antagonist E5564;
- oil emulsions (e.g. Freund's adjuvant).

Theoretically, each molecule or substance that is able to favor or amplify a particular situation in the cascade of immunological events, ultimately leading to a more pronounced immunological response, can be defined as an adjuvant.
In principle, through the use of adjuvants in vaccine formulations, one can:
- direct and optimize immune responses that are appropriate or desirable for the vaccine;
- enable mucosal delivery of vaccines, i.e. administration that results in contact of the vaccine with a mucosal surface such as buccal or gastric or lung epithelium and the associated lymphoid tissue;
- promote cell-mediated immune responses;
- enhance the immunogenicity of weaker immunogens, such as highly purified or recombinant antigens;
- reduce the amount of antigen or the frequency of immunization required to provide protective immunity; and
- improve the efficacy of vaccines in individuals with reduced or weakened immune responses, such as newborns, the aged, and immunocompromised vaccine recipients.
Although little is known about their mode of action, it is currently believed that adjuvants augment immune responses by one of the following mechanisms:
- increasing the biological or immunologic half-life of antigens;
- improving antigen delivery to antigen-presenting cells (APCs), as well as antigen processing and presentation by the APCs e.g., by enabling antigen to cross endosomal membranes into the cytosol after ingestion of antigen-adjuvant complexes by APC;
- mimicking danger inducing signals from stressed or damaged cells, which serve to initiate an immune response;
- inducing the production of immunomodulatory cytokines;
- biasing the immune response towards a specific subset of the immune system; and - blocking the rapid dispersal of the antigen challenge.

Saccharides are known by the person skilled in the art as TI-2 (T cell independent-2) antigens and poor immunogens. Therefore, to produce a saccharide-based vaccine, said saccharides are conjugated to an immunogenic carrier to provide a conjugate, which presents an increased immunogenicity in comparison with the saccharide. In this context the term "immunogenic carrier" is defined as a structure, which is conjugated to the saccharide to form a conjugate that presents an increased immunity in comparison with the saccharide *per se.* Thus, the conjugation of the saccharides to the immunogenic carrier, preferably protein carrier, has as effect the stimulation of the immune response against said saccharide, without inducing an immune response against the said immunogenic carrier.

Hence, the present invention is directed to a saccharide of general formula **(I)** 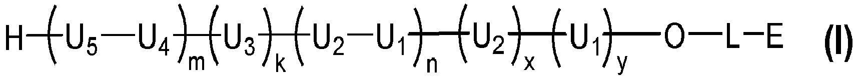 wherein
**U₁** represents 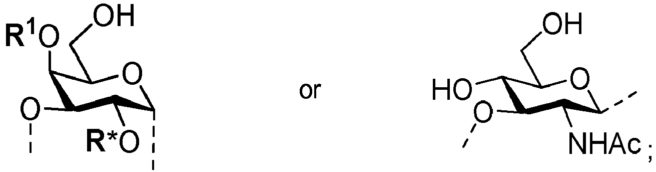
**U₂** represents 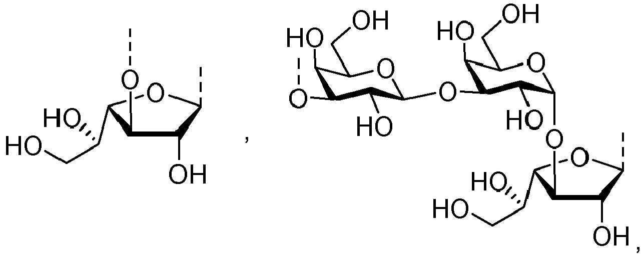 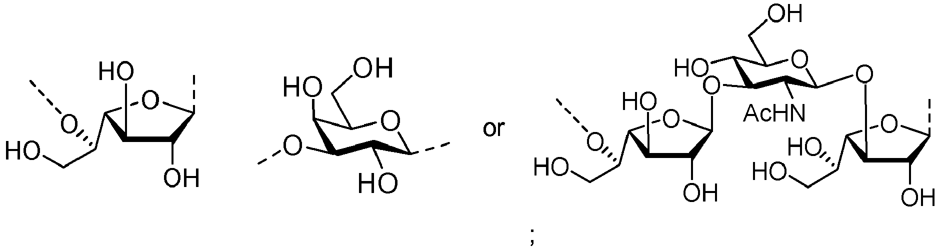
**U₃** represents 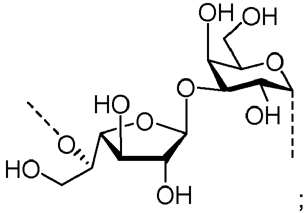
**U₄** represents 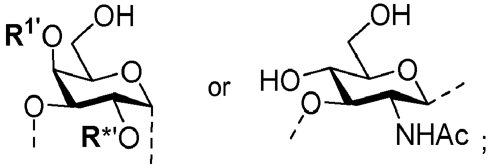
**U₅** represents a covalent bond or 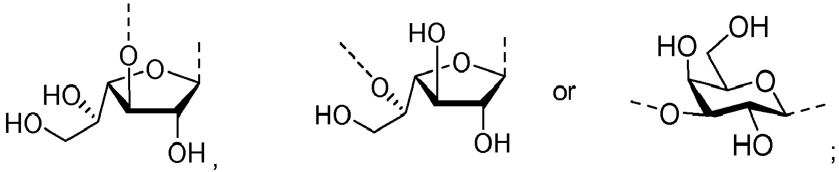
**U₆** represents 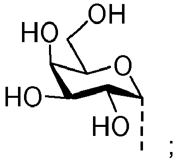
**R¹**, **R^{1'}**, **R^{*}** and **R*'** represent independently from each other -H or **U₆**, wherein **R¹** and **R^{*}** cannot be simultaneously -**U₆** and **R^{1'}** and **R^{*'}** cannot be simultaneously **-U₆**,
**L** represents a linker;
**E** represents -NH₂, -N₃, -CN, -O-NH₂, -CH=CH₂, -C=CH, -Br, -Cl, -I, -CO₂R', -CONH-NH₂, -OH, -SH, or -SAc;
**R'** represents -H, -Me, -Et, 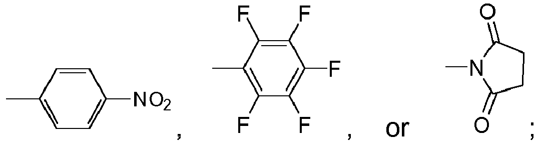
**n** is an integer from 1 to 20;
**m** is an integer from 0 to 20;
**k** is an integer selected from 0 to 10;
**x** and **y** are independently of each other the integer 0 or 1;
   and
when **U₁** and **U₂** are monosaccharides and **n** is 1, **m, x,** and **y** are not 0 at the same time;
or anomers, hydrates, or pharmaceutically acceptable salts thereof.

The linker L preferably contains between 2 and 40 carbon atoms (including the carbon atoms of optional side chains), more preferably between 2 and 30, more preferably between 2 and 20, more preferably between 2 and 14, more preferably between 2 and 12, and still more preferably between 2 and 10 carbon atoms.

The shortest atom chain between the oxygen atom (i.e. the oxygen of -O-L-NH₂) and the NH₂-group consists preferably of 2 to 14 atoms, more preferably of 2 to 12 atoms, more preferably of 2 to 10 atoms, more preferably of 2 to 8 atoms. In case the shortest chain (which is the shortest possible connection between the oxygen at the anomeric center and the NH₂-group) consists of 2 to 6 atoms, these are preferably carbon atoms. In case the shortest chain consists of 4 to 8 atoms, the chain may contain 1 or 2 heteroatoms selected from O, N and S. In case the shortest chain consists of 9 to 14 atoms, the chain may contain 1, 2, 3, or 4 heteroatoms selected from O, N and S.

It is also preferred that the linker -L-, or the shortest chain is fully or partially fluorinated. The linker -L- may contain a 3-membered or a 4-membered or a 5-membered or a 6-membered saturated carbocycle or a 5-membered partly unsaturated (and not aromatic) carbocycle or a 4-membered or a 5-membered or a 6-membered saturated oxygen heterocycle or a 4-membered or a 5-membered or a 6-membered saturated nitrogen heterocycle or a 6-membered aromatic carbocycle.

The linker -L- may also contain amide (-NH-CO-, -CO-NH-) and/or urea (-NH-CO-NH-) residues and preferably only one amide or urea residue. The linker may also contain substituents and preferably two substituents such as R¹⁰ and R¹¹ or four substituents such as R¹⁰, R¹¹, R¹⁵ and R¹⁴, which have the meanings as defined herein and which are preferably selected from: -F, -Cl, -CH₃, -C₂H₅, -C₃H₇, -C₅H₉, -C₆H₁₃, -OCH₃, -OC₂H₅, -CH₂F, -CHF₂, -CF₃, -C(O)-NH₂, -SCH₃, -SC₂H₅, -NHC(O)CH₃, -N(CH₃)₂, and -N(C₂H₅)₂.

In case the linker -L- is fluorinated, more than two substituents -F are preferred.

Preferably the linker -L- is selected from: -CH₂-, -(CH₂)₂-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -CF₂-, -(CF₂)₂-, -(CF₂)₃-, -(CF₂)₄-, -(CF₂)₅-, -(CF₂)₆-, -(CF₂)₇-, -(CF₂)₈-, -(CF₂)₉-, -(CF₂)₁₀-, -(CH₂)₂-O-(CH₂)₂-, -CH₂-O-(CH₂)₃-, -(CH₂)₃-O-CH₂-, -CH₂-O-(CH₂)₂-, -(CH₂)₂-O-CH₂-, -(CH₂)₃-O-(CH₂)₂-, -(CH₂)₂-O-(CH₂)₃-, -(CH₂)₄-O-CH₂-, -CH₂-O-(CH₂)₄-, -L^{a}-, -L^{a}-L^{e}-, -L^{a}-L^{b}-L^{e}-, -L^{a}-L^{b}-L^{d}-L^{c}-L^{e}-, -L^{a}-L^{d}-L^{e}-;
wherein
-L^{a}- is selected from: -(CH₂)ₒ-, -(CF₂)ₒ-, -(CH₂-CH₂-O)ₒ-C₂H₄-, -(CH₂-CH₂-O)ₒ-CH₂-, -(CR¹⁰R¹¹)ₒ-, 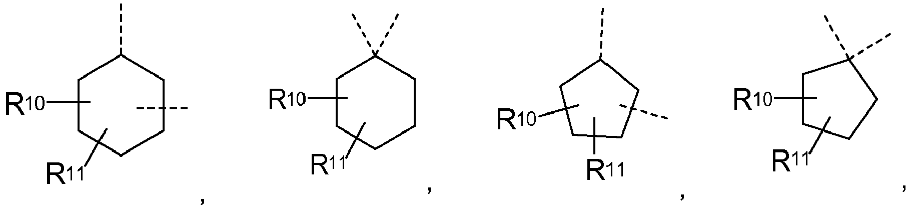 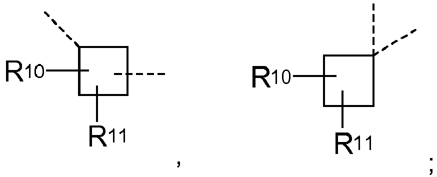 -L^{b}- and -L^{c}- are independently of each other selected from: -O-, -NH-C(O)-NH-, -NH-C(S)-NH-, -NH-C(O)-, -C(O)-NH-, -NH-C(O)-O-, -NR⁹-, -NR¹⁸-, -SO₂-, 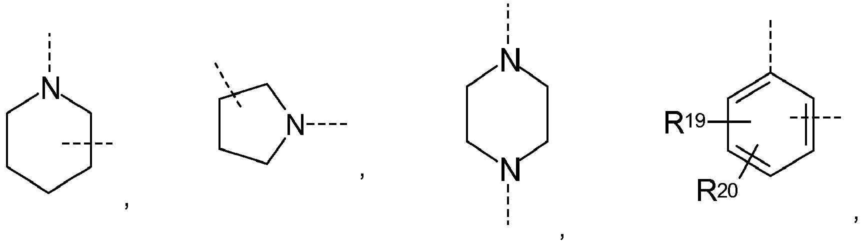 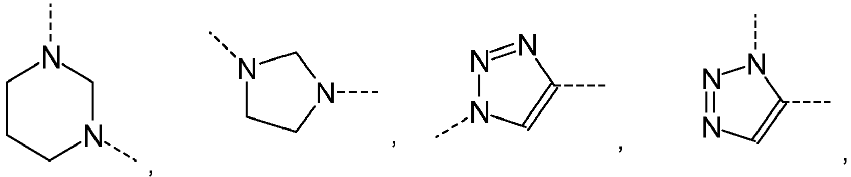 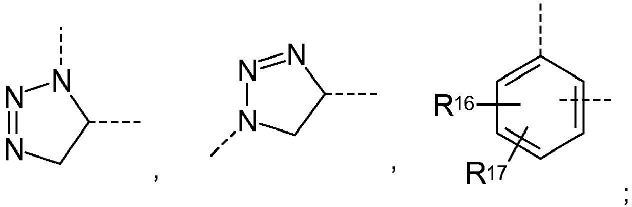 -L^{d}- represents -(CH₂)_{q}-, -(CF₂)_{q}-, -(CR¹²R¹³)_{q}-, -(CH₂-CH₂-O)_{q}-C₂H₄-, -(CH₂-CH₂-O)_{q}-CH₂-, 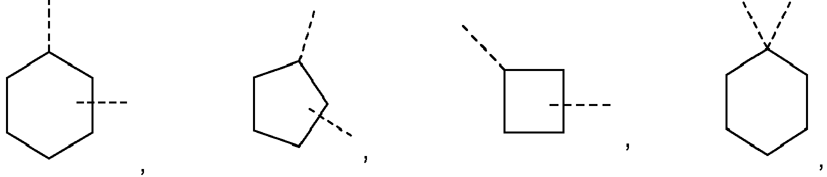 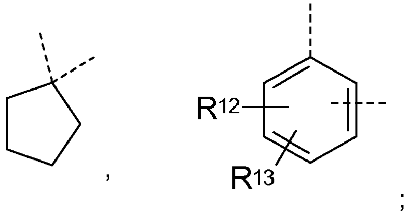 -L^{e}- is selected from: -(CH₂)ₚ₁-, -(CF₂)ₚ₁-, -C₂H₄-(O-CH₂-CH₂)ₚ₁-, -CH₂-(O-CH₂-CH₂)ₚ₁-, -(CH₂)ₚ₁-O-(CH₂)ₚ₂-, -(CR¹⁴R¹⁵)ₚ₁-, -(CR¹⁴R¹⁵)ₚ₁-O-(CR²¹R²²)ₚ₂-, 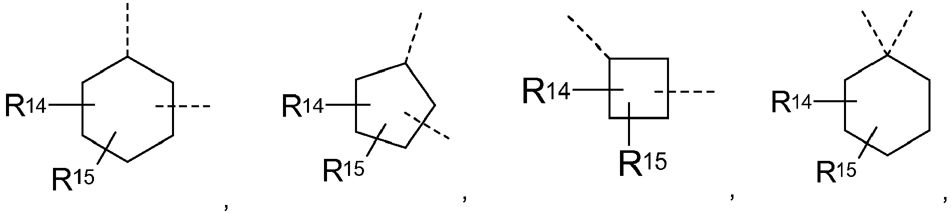 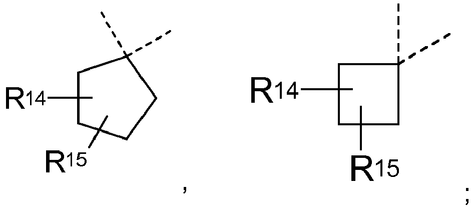
R⁹ and R¹⁸ are independently of each other selected from: -CH₃, -C₂H₅, -C₃H₇ and -C(O)CH₃;
R¹⁰, R¹¹, R¹², R¹³, R¹⁴, R¹⁵, R¹⁶, R¹⁷, R¹⁹, R²⁰, R²¹ and R²² are independently of each other selected from: -H, -F, -Cl, -CH₃, -C₂H₅, -C₃H₇, -C₅H₉, -C₆H₁₃, -OCH₃, -OC₂H₅, -CH₂F, -CHF₂, -CF₃, -C(O)-NH₂, -SCH₃, -SC₂H₅, -NHC(O)CH₃, -N(CH₃)₂ and -N(C₂H₅)₂;
o, q, p1 and p2 are independently of each other an integer selected from 1, 2, 3, 4, 5, 6, 7, 8, 9, and 10.

More preferred, **-L-** represents **-L^{a}-, -L^{a}-L^{e}-, -L^{a}-L^{b}-L^{e}-,** or **-L^{a}-L^{d}-L^{e}-; -L^{a}-** represents -(CH₂)ₒ-, -(CH₂-CH₂-O)ₒ-C₂H₄-, or -(CH₂-CH₂-O)ₒ-CH₂; **-L^{b}-** represents -O-;
**-L^{d}-** represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂-;
**-L^{e}-** represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂-; and
**o, q, p1** and **p2** are independently of each other an integer selected from 1, 2, 3, 4, 5, and 6, with the proviso that L is not -C₃H₆- if **-E** is -NH₂.

Still more preferably, **-L-E** represents **-L^{a}-E, -L^{a}-L^{e}-E, -L^{a}-L^{b}-L^{e}-E,** or **-L^{a}-L^{d}-L^{e}-E;**
**-L^{a}-** represents -(CH₂)ₒ-, -(CH₂-CH₂-O)ₒ-C₂H₄-, or -(CH₂-CH₂-O)ₒ-CH₂; **-L^{b}-** represents -O-;
**-L^{d}-** represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂-;
**-L^{e}-** represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂-; and
**-E** represents -NH₂, -N₃, -O-NH₂, -CH=CH₂, -C=CH, -Br, -Cl, -I, -COOH, -COOCH₃, -COOC₂H₅, -CO₂R', -CONH-NH₂, -OH, or -SH;
**R'** represents 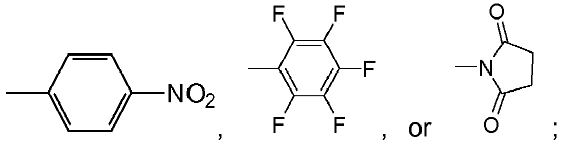
**o, q, p1** and **p2** are independently of each other an integer selected from 1, 2, 3, 4, 5, and 6, with the proviso that -L-E is not -C₃H₆-NH₂.

Still most preferred, the saccharide of the formula (I) has the residue -O-**L-E** selected from the group consisting of: 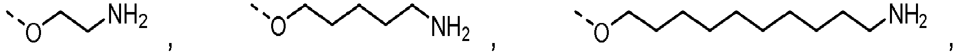 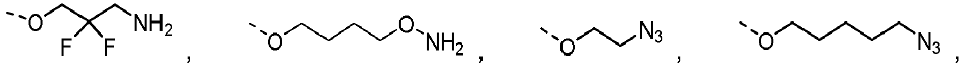 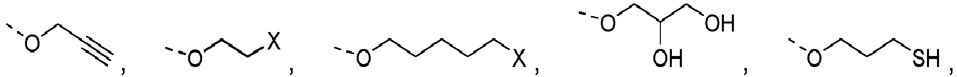 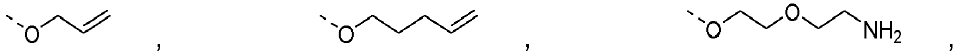 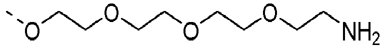 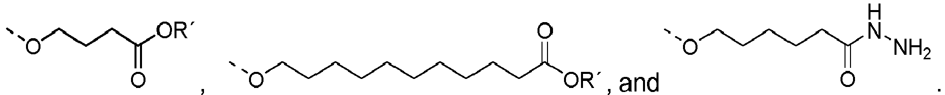 wherein R' represents -H, -Me, -Et, 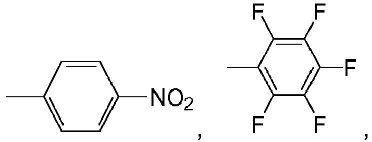 or 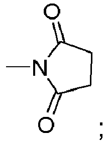 X represents -Br, -Cl, -I, -CO₂H, or -SAc.

Most preferred, the saccharide of the formula (I) has the residue -O-**L-E** selected from the group consisting of: 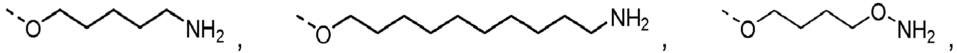 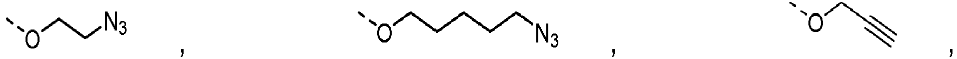 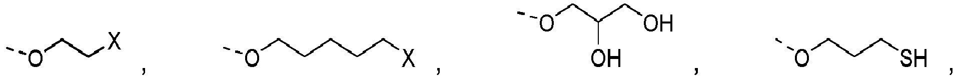 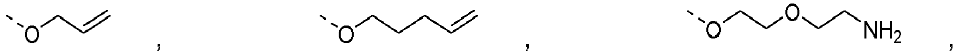 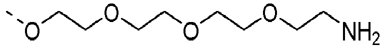 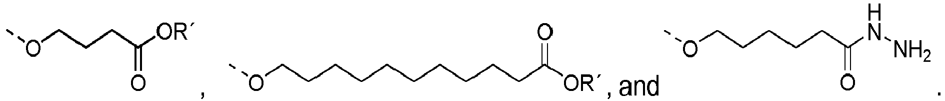 wherein R' represents -H, -Me, -Et, 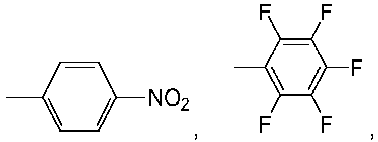 or 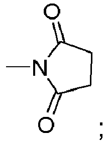 X represents -Br, -Cl, -I, -CO₂H, or -SAc.

The anomers of saccharides of the present invention mean the α/β-anomers at C-1-postion to which the group -O-**L-E** is bounded. It is clear for the skilled person in the art of carbohydrate chemistry that the stereochemistry of the glycosidic bond is defined by the stereochemistry indicated for the anomeric center of the sugar fragment **U₁**, and **U₂** in the general formula (I).

The saccharides of the present invention are hygroscopic and thus can build various hydrates thereof. Preferred, molar ratio of water molecule to the saccharide is in the range of 1 to 20, more preferred, 1 to 10, most preferred, 5-10.
The saccharides of the present invention bear basic and/or acidic substituents and they may form salts with organic or inorganic acids or bases.

Examples of suitable acids for such acid addition salt formation are hydrochloric acid, hydrobromic acid, sulfuric acid, phosphoric acid, acetic acid, citric acid, oxalic acid, malonic acid, salicylic acid, *p*-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, *p*-aminobenzoic acid, *p*-hydroxybenzoic acid, methanesulfonic acid, ethanesulfonic acid, nitrous acid, hydroxyethanesulfonic acid, ethylenesulfonic acid, *p*-toluenesulfonic acid, naphthylsulfonic acid, sulfanilic acid, camphorsulfonic acid, china acid, mandelic acid, *o*-methylmandelic acid, hydrogen-benzenesulfonic acid, picric acid, adipic acid, d-o-tolyltartaric acid, tartronic acid, (*o, m, p*)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

Examples of suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (**I**) with a solution of a base, selected out of the group mentioned above.

Surprisingly, it was found that a saccharide of general formula (**I**) contains an immunogenic protective epitope and is able to induce a protective immune response against *K*. *pneumoniae* bacteria or serotypes O1, O2, O2ac, O8 and carbapanem-resistant *Klebsiella pneumoniae* ST258 in a human and/or animal host. The saccharide of general formula (**I**) elicits antibodies that are cross-reacting with the *K*. *pneumoniae* serotype O1, O2, O2ac, O8 O-polysaccharide as well as carbapanem-resistant *Klebsiella pneumoniae* ST258 O-polysaccharide saccharide, recognize specifically *K*. *pneumoniae* bacteria and opsonize them for killing by phagocytes.

The saccharides of the present invention overcome all the problems associated with the saccharides produced from bacterial sources and conjugates thereof in terms of purity and easiness of production. It is well known that the isolation and purification of pure saccharides of defined length and structure from capsular polysaccharides of pathogenic bacteria is a tedious and sometimes not feasible process. Firstly, the production of capsular polysaccharides requires optimization of the growth conditions. Secondly, depolymerization conditions under which the structural integrity of the constituting monosaccharides is maintained need to be found. Finally, purification conditions enabling the isolation of the pure saccharide of defined length and structure need to be determined. Besides usual contaminants, such as cellular polysaccharides, nucleic acids and proteins, also the undesired saccharides obtained through the depolymerization process, must be excluded. Thus, the production of pure saccharides of defined structure and length from bacterial sources is a tedious, almost impossible process.

Preferred, are synthetic saccharides of general formula (I), 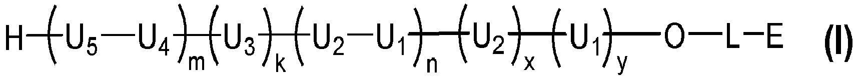 wherein
**U₁** represents 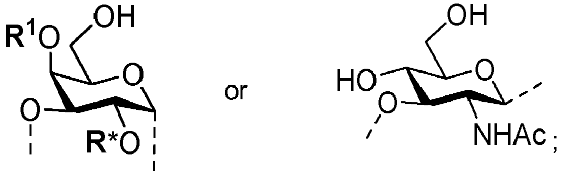
**U₂** represents 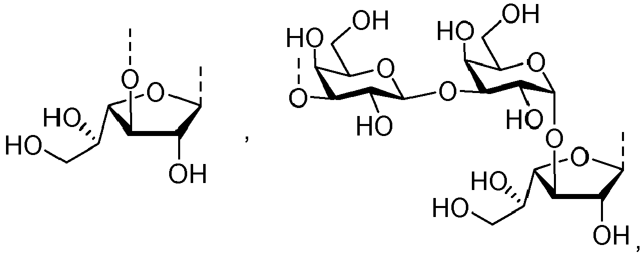 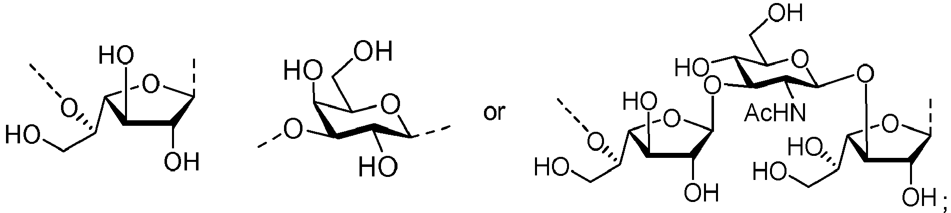
**U₃** represents 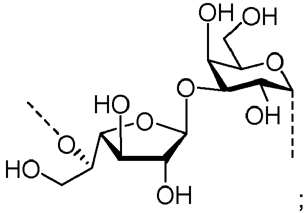
**U₄** represents 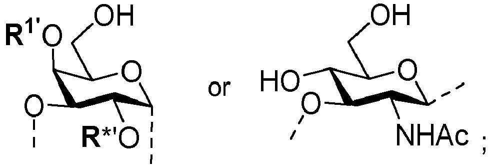
**U₅** represents a covalent bond or 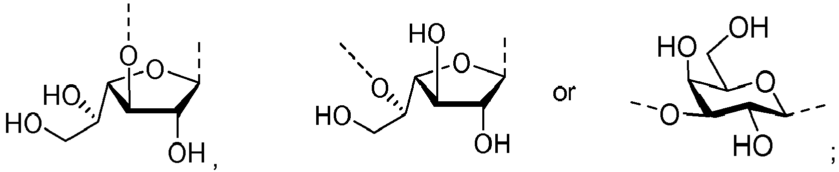
**U₆** represents 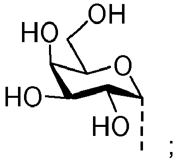
**R¹**, **R^{1'} R^{*}** and **R***' represent independently from each other -H or **U₆**, wherein **R¹** and **R^{*}** cannot be simultaneously -**U₆** and **R^{1'}** and **R^{*'}** cannot be simultaneously **-U₆**,
**L** represents a linker;
**E** represents -NH₂, -N₃, -CN, -O-NH₂, -CH=CH₂, -C=CH, -Br, -Cl, -I, -CO₂R', -CONH-NH₂, -OH, -SH, or -SAc;
**R'** represents -H, -Me, -Et, 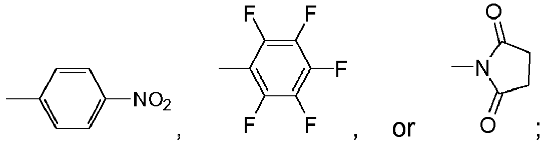
**n** is an integer from 1 to 20;
**m** is an integer from 0 to 20;
**k** is an integer selected from 0 to 10;
**x** and **y** are independently of each other the integer 0 or 1; and
when **U₂-U₁** represents 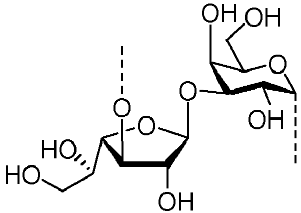
**m** cannot be 0 and **U₅-U₄** cannot be **U₂-U₁**;
when **U₁** and **U₂** are monosaccharides and **n** is 1, **m, x,** and **y** are not 0 at the same time;
or anomers, hydrates, or pharmaceutically acceptable salts thereof.

Preferred, are synthetic saccharides of general formula (**I**), 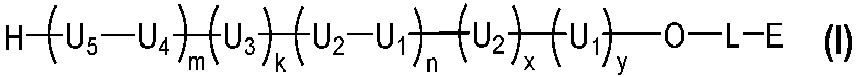 wherein
**U₁** represents 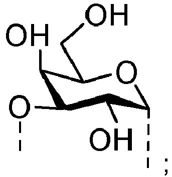
**U₂** represents 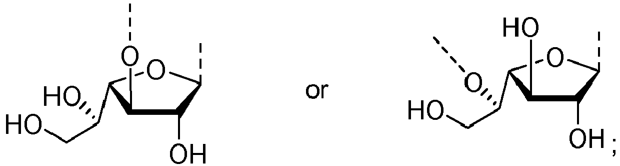
**U₃** represents 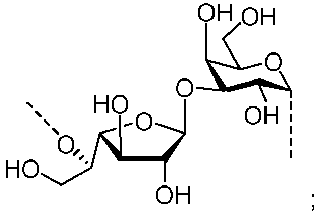
**U₄** represents 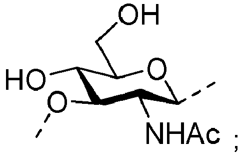
**U₅** represents 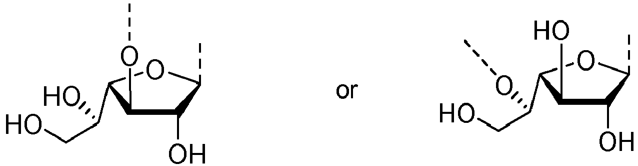
m represents an integer selected from 1 to 10;
k represents an integer selected from 0 to 10;
n represents an integer selected from 1 to 10; and
**x, y, L** and **E** have the meanings as defined herein.

Preferred, are synthetic saccharides of general formula (**I**), 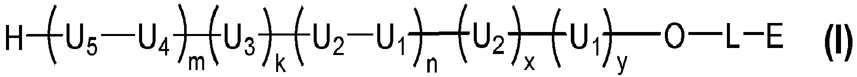 wherein
**U₁** represents 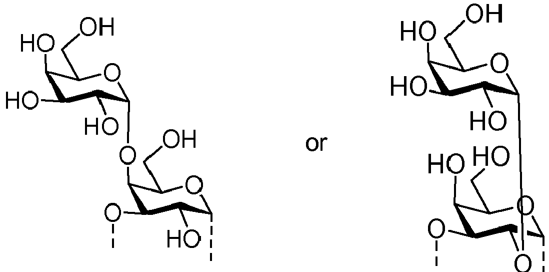
**U₂** represents 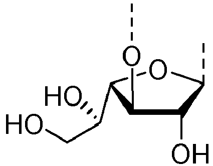
**U₃** represents 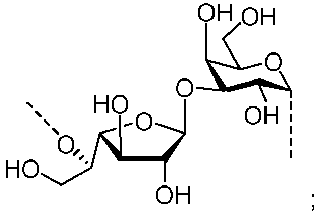
**U₅** represents a covalent bond or 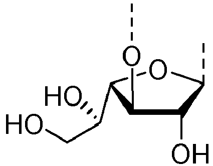
**m, n, k, x, y, L** and **E** have the meanings as defined herein.

Preferred, are synthetic saccharides of general formula (**I**), wherein
**U₁** represents 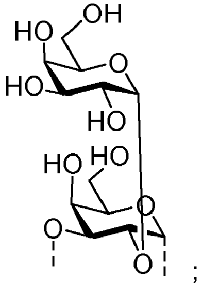
**U₂** represents 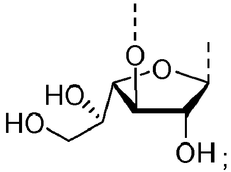
**U₄** represents 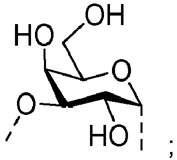
**U₅** represents a covalent bond, or 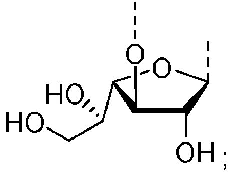
**m** is an integer selected from 0 and 1;
k is 0,
**n, U₃, x, y, L** and **E** have the meanings as defined herein.

Preferred, are synthetic saccharides of general formula (**I**), wherein
**U₁** represents 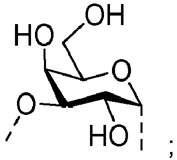
**U₂** represents 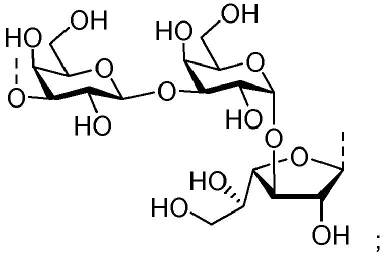
**U₄** represents 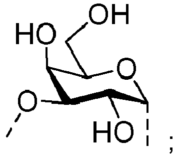
**U₅** represents a covalent bond, or 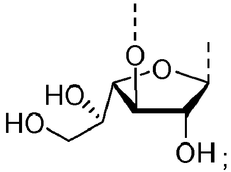
**m** is an integer selected from 0 and 1,
k is 0,
**n, U₃, x, y, L** and **E** have the meanings as defined herein.

Preferred, are synthetic saccharides of general formula (**I**), wherein
**U₁** represents 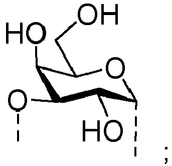
**U₂** represents 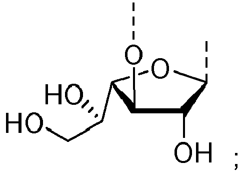
**U₄** represents 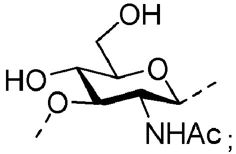
**U₅** represents a covalent bond, or 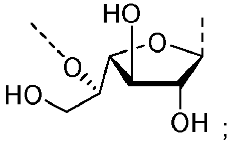
**m** is an integer from 1 to 10,
k is 0,
**n, U₃, x, y, L** and **E** have the meanings as defined in Claim 1.

Preferred are synthetic saccharides of general formula (**I-A**), 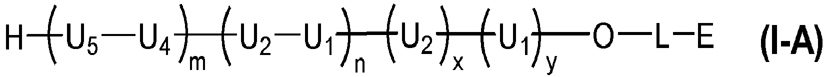 wherein
**U₁** represents 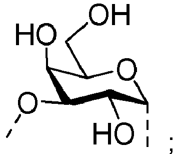
**U₂** represents 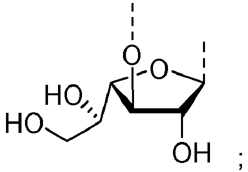
**U₄** represents 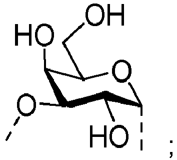
**U₅** represents a covalent bond or 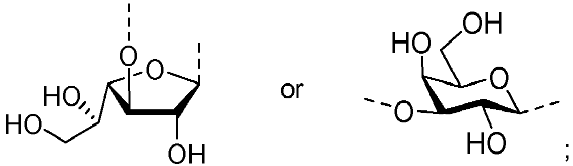
**L, E, m, n, x**, and **y** have the same meanings as defined herein,
or anomers, hydrates, or pharmaceutically acceptable salt of these saccharides.
As defined above, **U₁** and **U₂** are monosaccharides and thus when **n** is 1, **m, x,** and **y** are not 0 at the same time;

Preferred, are synthetic saccharides of general formula (**I-A**), 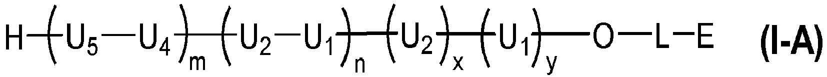 wherein
**U₁** represents 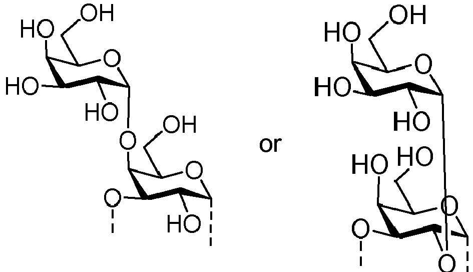
**U₂** represents 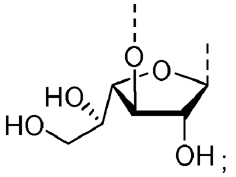
**U₄** represents 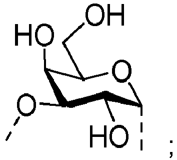
**U₅** represents a covalent bond, or 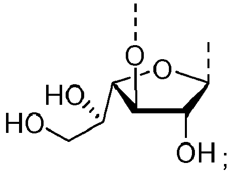
**m** is an integer selected from 0 and 1;
**L, E, n, x,** and **y** have the meanings as defined herein.

Preferred, are synthetic saccharides of general formula (**I-A**), 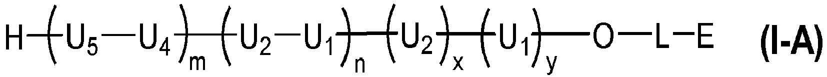 wherein
**U₁** represents 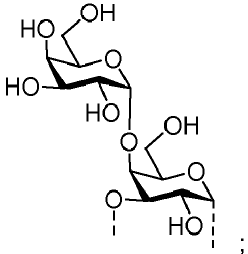
**U₂** represents 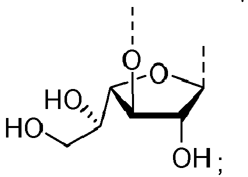
**U₄** represents 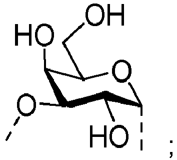
**U₅** represents a covalent bond, or 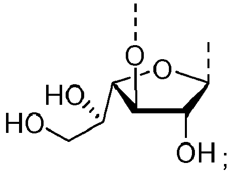
**m** is an integer selected from 0 and 1;
**L, E, n, x,** and **y** have the meanings as defined herein.

Preferred, are synthetic saccharides of general formula (**I-A**),
wherein
**U₁** represents 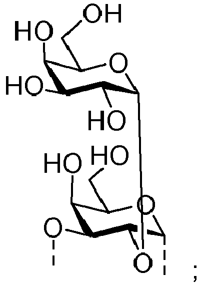
**U₂** represents 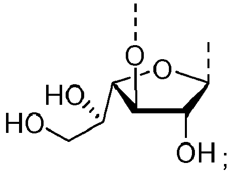
**U₄** represents 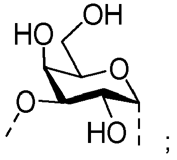
**U₅** represents a covalent bond, or 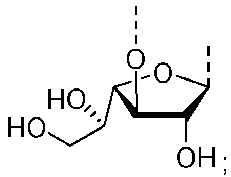
**m** is an integer selected from 0 and 1;
**L, E, n, x,** and **y** have the meanings as defined herein.

Preferred, are synthetic saccharides of general formula (**I-A**), wherein
**U₁** represents 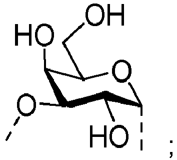
**U₂** represents 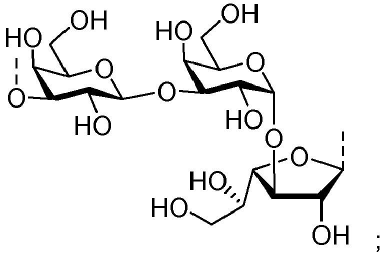
**U₄** represents 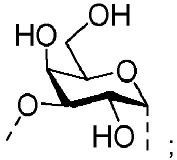
**U₅** represents a covalent bond, or 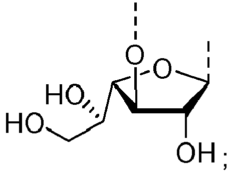
**m** is an integer selected from 0 and 1;
**L, E, n, x,** and **y** have the meanings as defined herein.

Preferred, are synthetic saccharides of general formula (**I-A**), wherein
**U₁** represents 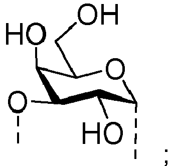
**U₂** represents 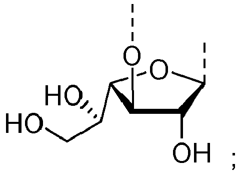
**U₄** represents 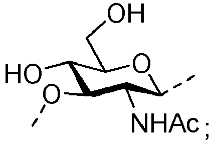
**U₅** represents a covalent bond, or 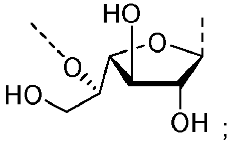
**m** is an integer from 1 to 10;
**L, E, n, x,** and **y** have the same meanings as defined herein.

Preferred, are synthetic saccharides of general formula (**I-A**), wherein
**U₁** represents 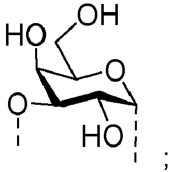
**U₂** represents 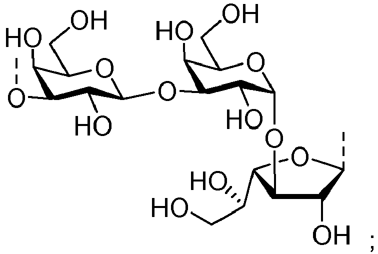
**m, x,** and **y** are 0;
**L, E,** and **n** have the same meanings as defined herein.

Preferred, are synthetic saccharides of general formula **(I-A),** wherein
**U₁** represents 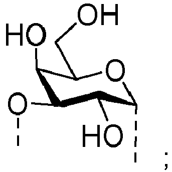
**U₂** represents 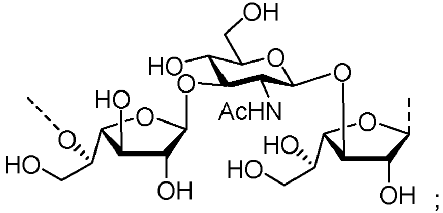
**m, x,** and **y** are 0;
**L, E,** and **n** have the same meanings as defined herein.

Preferred, are synthetic saccharides of general formula (**I-A**), wherein
**U₁** and **U₄** represent 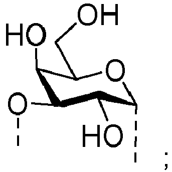
**U₂** and **U₅** represent 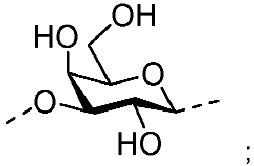
**m** is an integer from 1 to 10.
**L, E, n, x,** and **y** have the same meanings as defined herein.
As defined above, **U₁** and **U₂** are monosaccharides and thus when **n** is 1, **m, x,** and **y** are not 0 at the same time;

More preferred, the saccharide of general formula (**I-A**), wherein **n** is an integer from 1 to 10.

Preferred, are also synthetic saccharides of general formula (**I-B**), 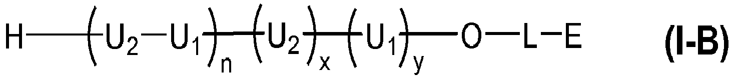 wherein
**U₁** represents 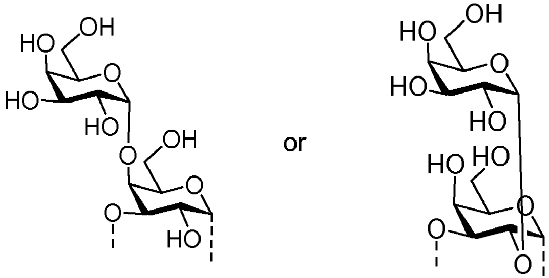
**U₂** represents 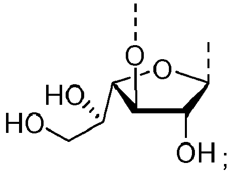
n is an integer selected from 1, 2, 3, 4, 5, 6, 7, 8 and 9,
x and y are 1, and
**L** and **E** have the meanings as defined herein.

Hence, within the scope of the present invention falls also a synthetic saccharide of any one of formulae (**II-1**) - (**II-17**): 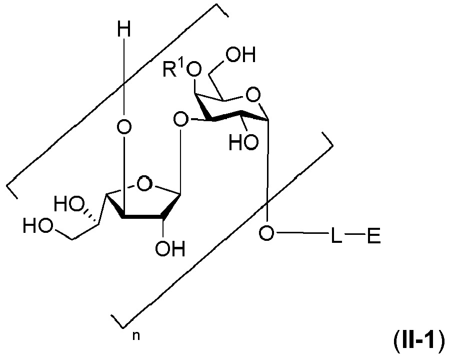 wherein **n, R¹**, **m, L** and **E** have the same meanings as defined above when **R¹** is -H, **n** is an integer from 2 to 20, preferably, **n** is an integer from 2 to 12; 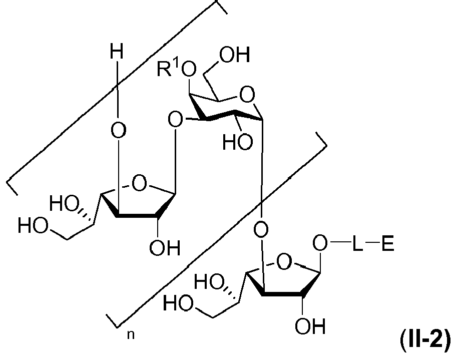 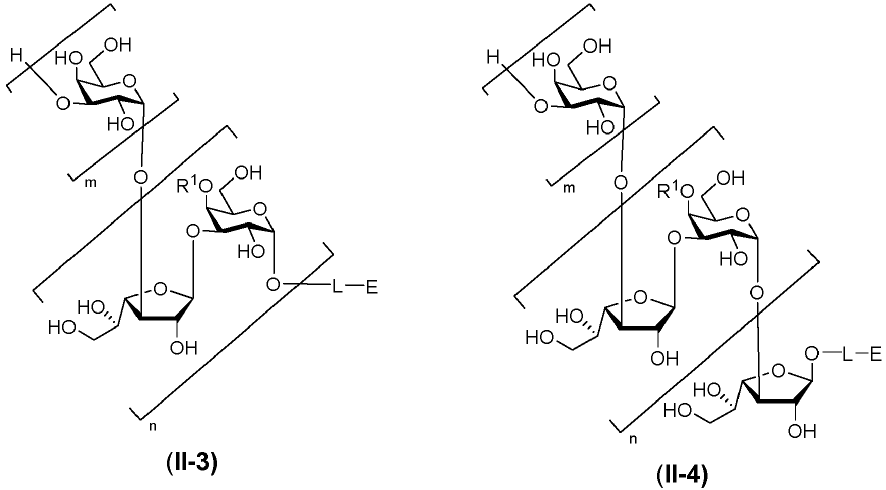 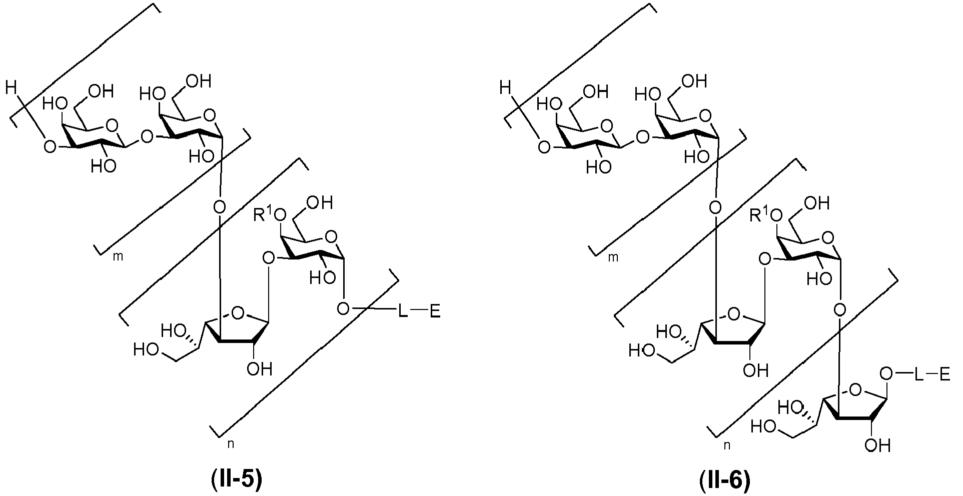 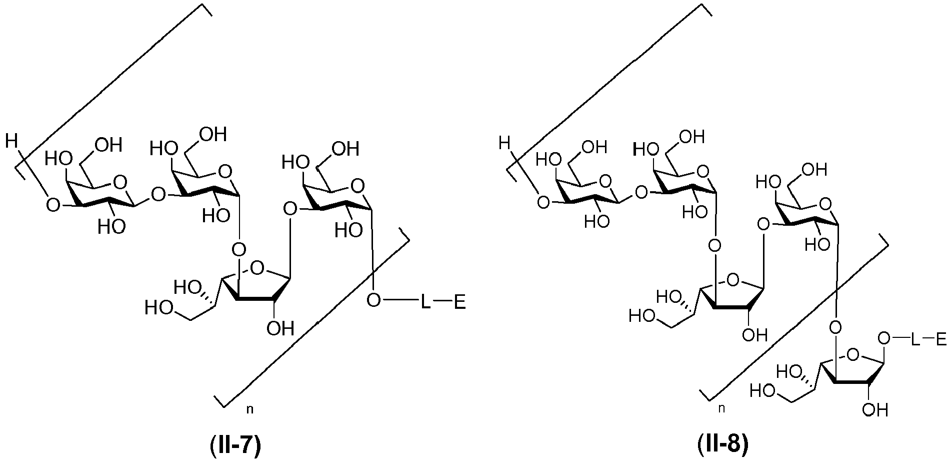 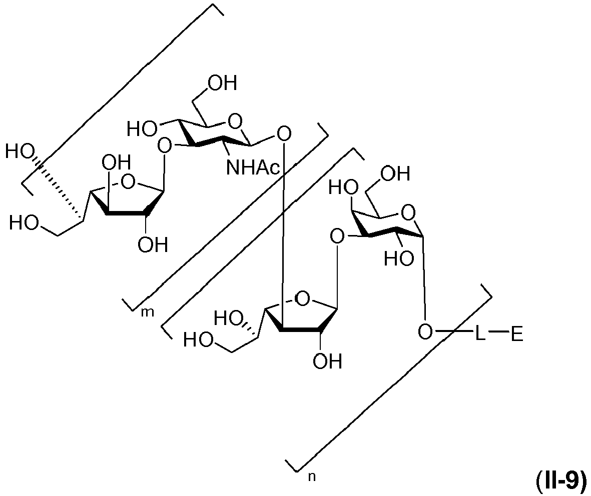 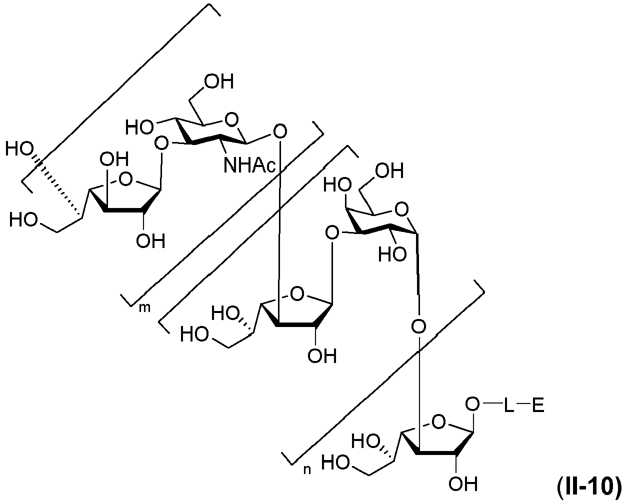 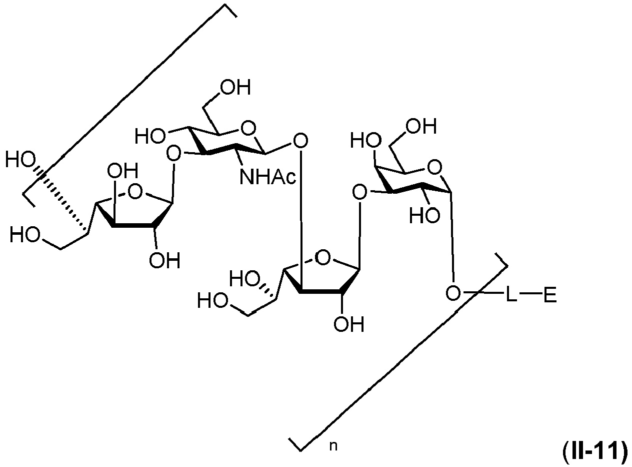 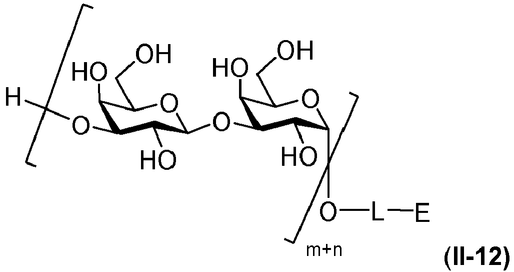 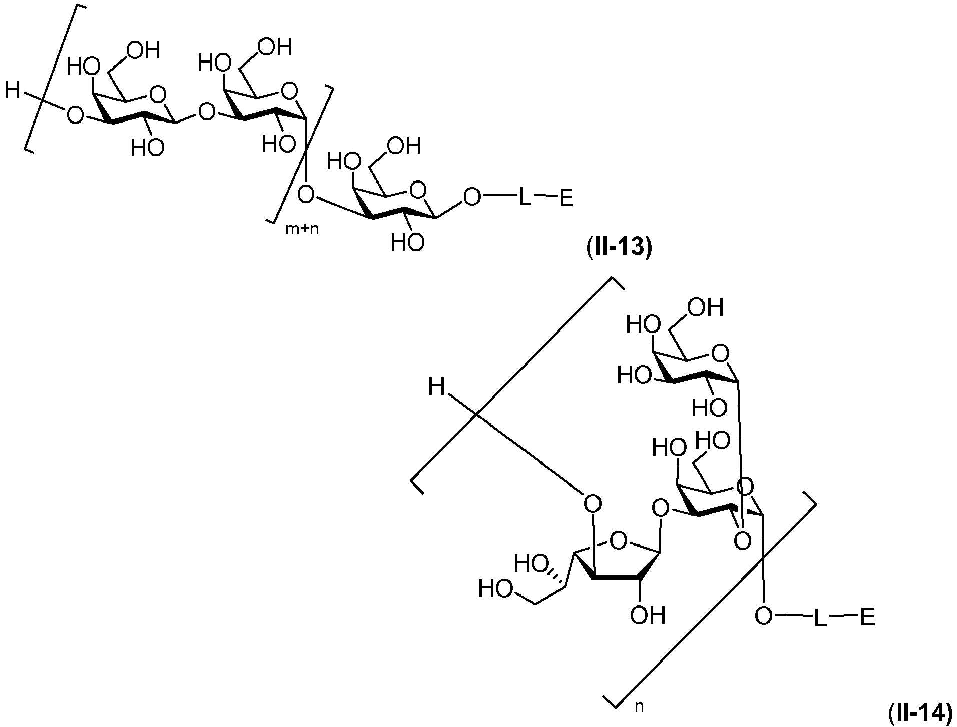 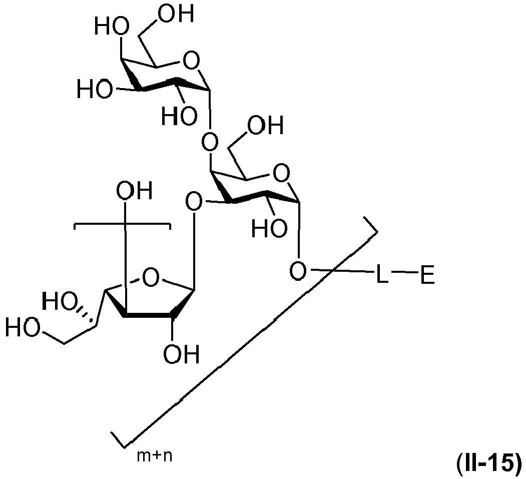 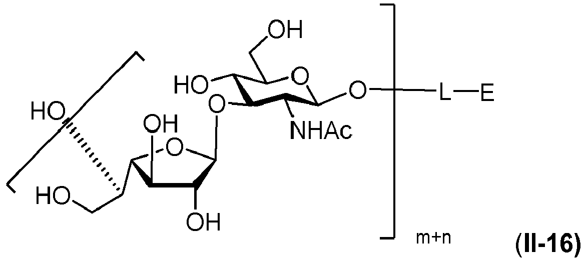 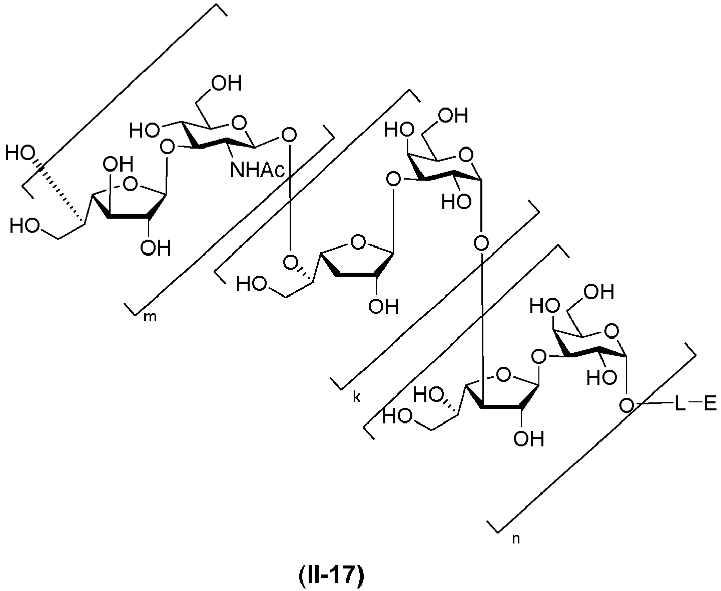 wherein **R¹**, **m, n, k, L** and **E** have the same meanings as defined above, preferably, **n** and **m** is an integer from 1 to 10 and preferably k is an integer from 0 to 10.

Preferably, the linker **-L-** represents in the general formulae (**I**), (**I-A**), (**I-B**) and (**II-1**)-(**II-17**)
**-L^{a}-, -L^{a}-L^{e}-, -L^{a}-L^{b}-L^{e}-,** or **-L^{a}-L^{d}-L^{e}-;** wherein
**-L^{a}-** represents -(CH₂)ₒ-, -(CH₂-CH₂-O)ₒ-C₂H₄-, or -(CH₂-CH₂-O)ₒ-CH₂;
**-L^{b}-** represents -O-;
**-L^{d}-** represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂-;
**-L^{e}-** represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂-; and
**o, q, p1** and **p2** are independently of each other an integer selected from 1, 2, 3, 4, 5, and 6, with the proviso that **L** is not -C₃H₆- if -**E** is -NH₂.

Still more preferably, **-L-E** represents in the general formulae (**I**), (**I-A**), (**I-B**) and (**II-1**)-(**II-17**)
**-L^{a}-E, -L^{a}-L^{e}-E, -L^{a}-L^{b}-L^{e}-E,** or **-L^{a}-L^{d}-L^{e}-E;** wherein
**-L^{a}-** represents -(CH₂)ₒ-, -(CH₂-CH₂-O)ₒ-C₂H₄-, or -(CH₂-CH₂-O)ₒ-CH₂;
**-L^{b}-** represents -O-;
**-L^{d}-** represents -(CH₂)_{q}-, -(CH(OH))_{q}-, -(CF₂)_{q}-, -(CH₂-CH₂-O)_{q}-C₂H₄-, or
-(CH₂-CH₂-O)_{q}-CH₂-;
**-L^{e}-** represents -(CH₂)ₚ₁-, -(CF₂)ₚ₁-, -C₂H₄-(O-CH₂-CH₂)ₚ₁-,
-CH₂-(O-CH₂-CH₂)ₚ₁- or -(CH₂)ₚ₁-O-(CH₂)ₚ₂-; and
**-E** represents -NH₂, -N₃, -O-NH₂, -CH=CH₂, -C≡CH, -Br, -Cl, -I, -COOH, -COOCH₃, -COOC₂H₅, -CO₂R', -CONH-NH₂, -OH, or -SH;
**R'** represents 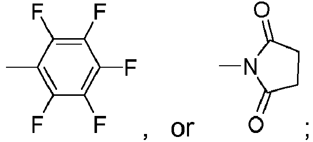 **o, q, p1** and **p2** are independently of each other an integer selected from 1, 2, 3, 4, 5, and 6, with the proviso that -L-E is not -C₃H₆-NH₂.

Still most preferred, the saccharide of the formula (**I**), (**I-A**), (**I-B**) and (**II-1**)-(**II-17**) has the residue -O-**L-E** selected from the group consisting of: 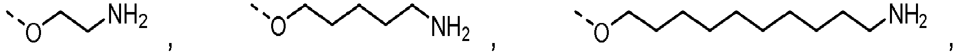 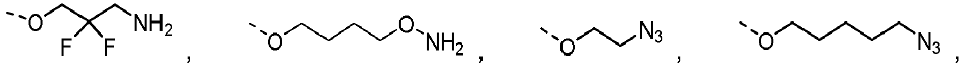 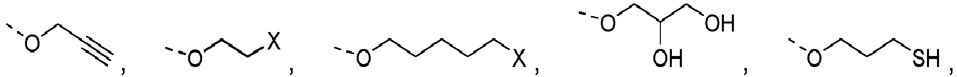 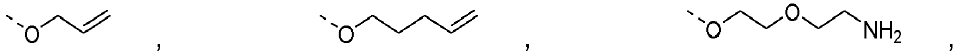 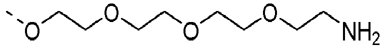 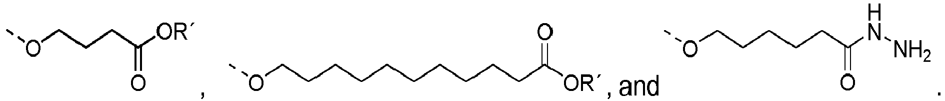 wherein R' represents -H, -Me, -Et, 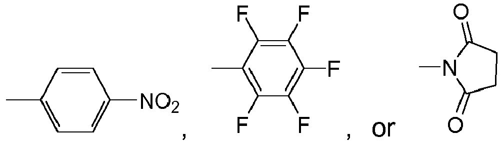 ;
X represents -Br, -Cl, -I, -CO₂H, or-SAc;

Most preferred, the saccharide of the formula (**I**), (**I-A**), (**I-B**) or (**II-1**)-(**II-17**) has the residue -O-**L-E** selected from the group consisting of: 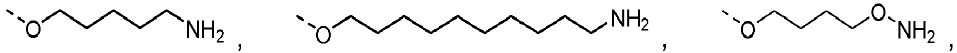 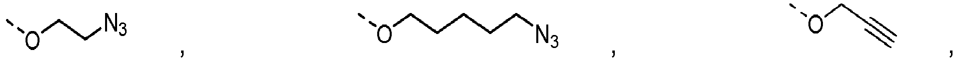 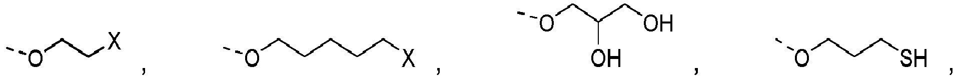 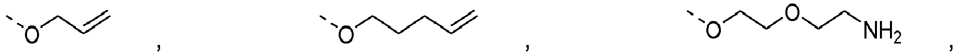 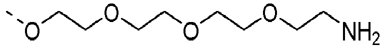 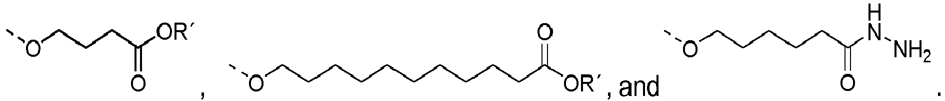 wherein R' represents -H, -Me, -Et, 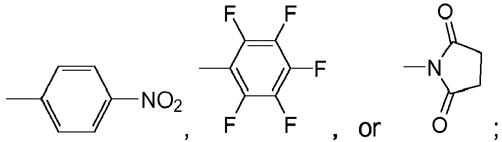 X represents -Br, -Cl, -I, .-CO₂H, or -SAc.

In the most preferred embodiment, -L- represents -(CH₂)ₒ- and o is an integer selected from 4, 5 and 6. Hence, an especially preferred synthetic saccharide is a saccharide of any one of general formulae (**I**), (**I-A**), (**I-B**) and (**II-1**)-(**II-17**), wherein -L- represents -(CH₂)ₒ- and o is an integer selected from 4, 5 and 6.

In yet another preferred embodiment, the saccharide according to the present invention is selected from the group consisting of: compounds **A-01 - A-140, B-01 - B-140, C-01 - C-70, D-01 - D-70, E-01 - E-70, F-01 - F-530, G-01 - G-350, H-01 - H-70, J-01 - J-70, K-01 - K-350, L-01 - L-112, M-01 - M-70, N-01 - N-70, O-01 - O-70, P-01 - P-70** and **Q-1 - Q-700.**

Most preferred, the saccharide according to the present invention is selected from the group consisting of: compounds **A-01 - A-07, A-11 - A17, A-21 - A-27, A-31 - A-37, A-41 - A-47, A-51 - A-57, A-61 - A-67, A-71 - A-77, A-81 - A-87, A-91 - A-97, A-101** - **A-107, A-111 - A-117, A-121 - A-127, A-131 - A-137, F-01, F-19, F-27, F-31, F-36, F-54, F-62, F-66, F-71, F-89, F-97, F-101, F-106, F-124, F-132, F-136, F-141, F-159, F-167, F-171, F-176, F-194, F-202, F-206, F-211, F-229, F-237, F-241, F-246, F-264, F-299, F-281, F-272, F-276, F-307, F-311, F-316, F-334, F-342, F-346, F-351, F-414, F-417, F-421, F-426, F-444, F-452, F-456, F-461, F-479, F-487, F-491, F-496, F-514, F-522, F-526, K-01, K-06, K-11, K-26, K-31, K-36, K-51, K-56, K-61, K-76, K-81, K-86, K-101, K-106, K-111, K-126, K-131, K-136, K-151, K-156, K-161, K-176, K-181, K-186, K-201, K-206, K-211, K-226, K-231, K-236, K-251, K-256, K-261, K-276, K-281, K-286, K-301, K-306, K-311, K-326, K-331, K-336, O-01, O-02, O-03, O-06, O-07, O-08, O-11, O-12, O-13, O-16, O-17, O-18, O-21, O-22, O-23, O-26, O-27, O-28, O-31, O-32, O-33, O-36, O-37, O-38, O41, O-42, O-43, O-46, O-47, O-48, O-51, O-52, O-53, O-56, O-57, O-58, O-61, O-62, O-63, O-66, O-67, O-88, P-01 - P-03, P-06 - P-08, P-11 - P-13, P-16 - P-18, P-21 - P-23, P-26 - P-28, P-31 - P-33, P-36 - P-38, P-41 - P-43, P-46 - P-48, P-51 - P-53, P-56 - P-58, P-61 - P-63, P-66 - P-68, Q-1, Q-26, Q-101, Q-151, Q-251, Q-301, Q-351, Q-376, Q-451, Q-501, Q-551, Q-601** and **Q-651.**

**Table 1**

| | | | | | |
|---|---|---|---|---|---|
| 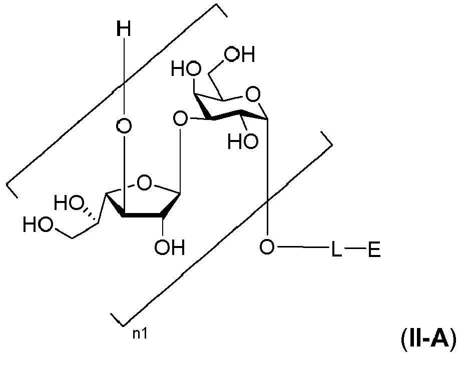 | | | | | |

| **-O-L-E** | n1 | **Compound No.** | **-O-L-E** | n1 | **Compound No.** |
|---|---|---|---|---|---|
| 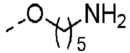 | 2 | **A-01** | 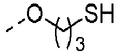 | 2 | **A-71** |
| | 3 | **A-02** | | 3 | **A-82** |
| | 4 | **A-03** | | 4 | **A-73** |
| | 5 | **A-04** | | 5 | **A-74** |
| | 6 | **A-05** | | 6 | **A-75** |
| | 7 | **A-06** | | 7 | **A-76** |
| | 8 | **A-07** | | 8 | **A-77** |
| | 9 | **A-08** | | 9 | **A-78** |
| | 10 | **A-09** | | 10 | **A-79** |
| | 11 | **A-10** | | 11 | **A-80** |
| 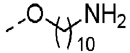 | 2 | **A-11** | 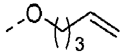 | 2 | **A-81** |
| | 3 | **A-12** | | 3 | **A-82** |
| | 4 | **A-13** | | 4 | **A-83** |
| | 5 | **A-14** | | 5 | **A-84** |
| | 6 | **A-15** | | 6 | **A-85** |
| | 7 | **A-16** | | 7 | **A-86** |
| | 8 | **A-17** | | 8 | **A-87** |
| | 9 | **A-18** | | 9 | **A-88** |
| | 10 | **A-19** | | 10 | **A-89** |
| | 11 | **A-20** | | 11 | **A-90** |
| 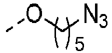 | 2 | **A-21** | 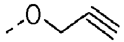 | 2 | **A-91** |
| | 3 | **A-22** | | 3 | **A-92** |
| | 4 | **A-23** | | 4 | **A-93** |
| | 5 | **A-24** | | 5 | **A-94** |
| | 6 | **A-25** | | 6 | **A-95** |
| | 7 | **A-26** | | 7 | **A-96** |
| | 8 | **A-27** | | 8 | **A-97** |
| | 9 | **A-28** | | 9 | **A-98** |
| | 10 | **A-29** | | 10 | **A-99** |
| | 11 | **A-30** | | 11 | **A-100** |
| 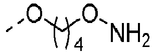 | 2 | **A-31** | 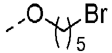 | 2 | **A-101** |
| | 3 | **A-32** | | 3 | **A-102** |
| | 4 | **A-33** | | 4 | **A-103** |
| | 5 | **A-34** | | 5 | **A-104** |
| | 6 | **A-35** | | 6 | **A-105** |
| | 7 | **A-36** | | 7 | **A-106** |
| | 8 | **A-37** | | 8 | **A-107** |
| | 9 | **A-38** | | 9 | **A-108** |
| | 10 | **A-39** | | 10 | **A-109** |
| | 11 | **A-40** | | 11 | **A-110** |
| 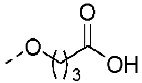 | 2 | **A-41** | 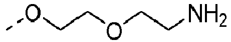 | 2 | **A-111** |
| | 3 | **A-42** | | 3 | **A-112** |
| | 4 | **A-43** | | 4 | **A-113** |
| | 5 | **A-44** | | 5 | **A-114** |
| | 6 | **A-45** | | 6 | **A-115** |
| | 7 | **A-46** | | 7 | **A-116** |
| | 8 | **A-47** | | 8 | **A-117** |
| | 9 | **A-48** | | 9 | **A-118** |
| | 10 | **A-49** | | 10 | **A-119** |
| | 11 | **A-50** | | 11 | **A-120** |
| 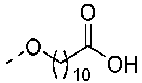 | 2 | **A-51** | 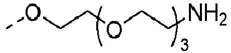 | 2 | **A-121** |
| | 3 | **A-52** | | 3 | **A-122** |
| | 4 | **A-53** | | 4 | **A-123** |
| | 5 | **A-54** | | 5 | **A-124** |
| | 6 | **A-55** | | 6 | **A-125** |
| | 7 | **A-56** | | 7 | **A-126** |
| | 8 | **A-57** | | 8 | **A-127** |
| | 9 | **A-58** | | 9 | **A-128** |
| | 10 | **A-59** | | 10 | **A-129** |
| | 11 | **A-60** | | 11 | **A-130** |
| 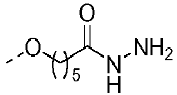 | 2 | **A-61** | 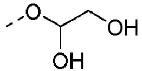 | 2 | **A-131** |
| | 3 | **A-62** | | 3 | **A-132** |
| | 4 | **A-63** | | 4 | **A-133** |
| | 5 | **A-64** | | 5 | **A-134** |
| | 6 | **A-65** | | 6 | **A-135** |
| | 7 | **A-66** | | 7 | **A-136** |
| | 8 | **A-67** | | 8 | **A-137** |
| | 9 | **A-68** | | 9 | **A-138** |
| | 10 | **A-69** | | 10 | **A-139** |
| | 11 | **A-70** | | 11 | **A-140** |

**Table 2**

| | | | | | |
|---|---|---|---|---|---|
| 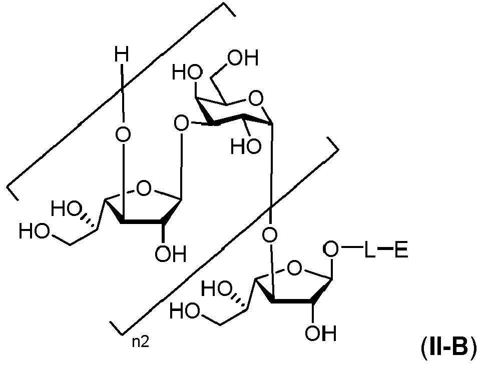 | | | | | |

| **-O-L-E** | n2 | **Compound No.** | **-O-L-E** | n2 | **Compound No.** |
|---|---|---|---|---|---|
| 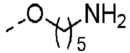 | 1 | **B-01** | 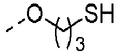 | 1 | **B-71** |
| | 2 | **B-02** | | 2 | **B-82** |
| | 3 | **B-03** | | 3 | **B-73** |
| | 4 | **B-04** | | 4 | **B-74** |
| | 5 | **B-05** | | 5 | **B-75** |
| | 6 | **B-06** | | 6 | **B-76** |
| | 7 | **B-07** | | 7 | **B-77** |
| | 8 | **B-08** | | 8 | **B-78** |
| | 9 | **B-09** | | 9 | **B-79** |
| | 10 | **B-10** | | 10 | **B-80** |
| 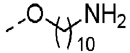 | 1 | **B-11** | 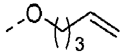 | 1 | **B-81** |
| | 2 | **B-12** | | 2 | **B-82** |
| | 3 | **B-13** | | 3 | **B-83** |
| | 4 | **B-14** | | 4 | **B-84** |
| | 5 | **B-15** | | 5 | **B-85** |
| | 6 | **B-16** | | 6 | **B-86** |
| | 7 | **B-17** | | 7 | **B-87** |
| | 8 | **B-18** | | 8 | **B-88** |
| | 9 | **B-19** | | 9 | **B-89** |
| | 10 | **B-20** | | 10 | **B-90** |
| 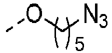 | 1 | **B-21** | 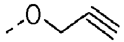 | 1 | **B-91** |
| | 2 | **B-22** | | 2 | **B-92** |
| | 3 | **B-23** | | 3 | **B-93** |
| | 4 | **B-24** | | 4 | **B-94** |
| | 5 | **B-25** | | 5 | **B-95** |
| | 6 | **B-26** | | 6 | **B-96** |
| | 7 | **B-27** | | 7 | **B-97** |
| | 8 | **B-28** | | 8 | **B-98** |
| | 9 | **B-29** | | 9 | **B-99** |
| | 10 | **B-30** | | 10 | **B-100** |
| 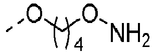 | 1 | **B-31** | 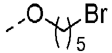 | 1 | **B-101** |
| | 2 | **B-32** | | 2 | **B-102** |
| | 3 | **B-33** | | 3 | **B-103** |
| | 4 | **B-34** | | 4 | **B-104** |
| | 5 | **B-35** | | 5 | **B-105** |
| | 6 | **B-36** | | 6 | **B-106** |
| | 7 | **B-37** | | 7 | **B-107** |
| | 8 | **B-38** | | 8 | **B-108** |
| | 9 | **B-39** | | 9 | **B-109** |
| | 10 | **B-40** | | 10 | **B-110** |
| 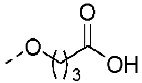 | 1 | **B-41** | 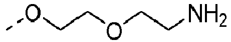 | 1 | **B-111** |
| | 2 | **B-42** | | 2 | **B-112** |
| | 3 | **B-43** | | 3 | **B-113** |
| | 4 | **B-44** | | 4 | **B-114** |
| | 5 | **B-45** | | 5 | **B-115** |
| | 6 | **B-46** | | 6 | **B-116** |
| | 7 | **B-47** | | 7 | **B-117** |
| | 8 | **B-48** | | 8 | **B-118** |
| | 9 | **B-49** | | 9 | **B-119** |
| | 10 | **B-50** | | 10 | **B-120** |
| 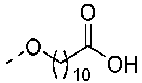 | 1 | **B-51** | 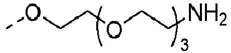 | 1 | **B-121** |
| | 2 | **B-52** | | 2 | **B-122** |
| | 3 | **B-53** | | 3 | **B-123** |
| | 4 | **B-54** | | 4 | **B-124** |
| | 5 | **B-55** | | 5 | **B-125** |
| | 6 | **B-56** | | 6 | **B-126** |
| | 7 | **B-57** | | 7 | **B-127** |
| | 8 | **B-58** | | 8 | **B-128** |
| | 9 | **B-59** | | 9 | **B-129** |
| | 10 | **B-60** | | 10 | **B-130** |
| 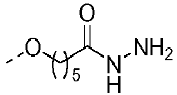 | 1 | **B-61** | 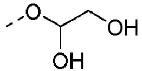 | 1 | **B-131** |
| | 2 | **B-62** | | 2 | **B-132** |
| | 3 | **B-63** | | 3 | **B-133** |
| | 4 | **B-64** | | 4 | **B-134** |
| | 5 | **B-65** | | 5 | **B-135** |
| | 6 | **B-66** | | 6 | **B-136** |
| | 7 | **B-67** | | 7 | **B-137** |
| | 8 | **B-68** | | 8 | **B-138** |
| | 9 | **B-69** | | 9 | **B-139** |
| | 10 | **B-70** | | 10 | **B-140** |

**Table 3**

| | | | | | |
|---|---|---|---|---|---|
| 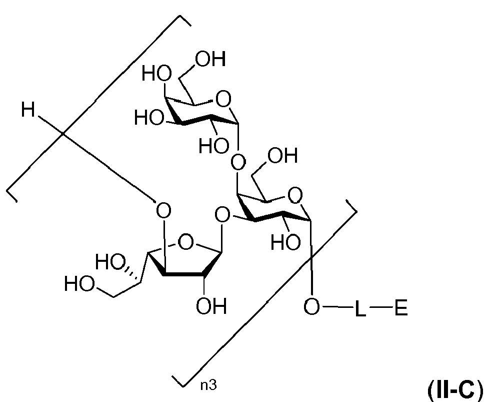 | | | | | |

| **-O-L-E** | n3 | **Compound No.** | **-O-L-E** | n3 | **Compound No.** |
|---|---|---|---|---|---|
| 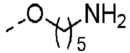 | 1 | **C-01** | 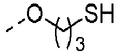 | 1 | **C-36** |
| | 2 | **C-02** | | 2 | **C-37** |
| | 3 | **C-03** | | 3 | **C-38** |
| | 4 | **C-04** | | 4 | **C-39** |
| | 5 | **C-05** | | 5 | **C-40** |
| 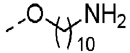 | 1 | **C-06** | 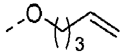 | 1 | **C-41** |
| | 2 | **C-07** | | 2 | **C-42** |
| | 3 | **C-08** | | 3 | **C-43** |
| | 4 | **C-09** | | 4 | **C-44** |
| | 5 | **C-10** | | 5 | **C-45** |
| 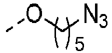 | 1 | **C-11** | 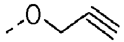 | 1 | **C-46** |
| | 2 | **C-12** | | 2 | **C-47** |
| | 3 | **C-13** | | 3 | **C-48** |
| | 4 | **C-14** | | 4 | **C-49** |
| | 5 | **C-15** | | 5 | **C-50** |
| 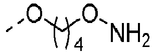 | 1 | **C-16** | 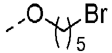 | 1 | **C-51** |
| | 2 | **C-17** | | 2 | **C-52** |
| | 3 | **C-18** | | 3 | **C-53** |
| | 4 | **C-19** | | 4 | **C-54** |
| | 5 | **C-20** | | 5 | **C-55** |
| 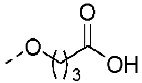 | 1 | **C-21** | 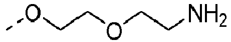 | 1 | **C-56** |
| | 2 | **C-22** | | 2 | **C-57** |
| | 3 | **C-23** | | 3 | **C-58** |
| | 4 | **C-24** | | 4 | **C-59** |
| | 5 | **C-25** | | 5 | **C-60** |
| 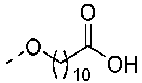 | 1 | **C-26** | 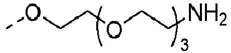 | 1 | **C-61** |
| | 2 | **C-27** | | 2 | **C-62** |
| | 3 | **C-28** | | 3 | **C-63** |
| | 4 | **C-29** | | 4 | **C-64** |
| | 5 | **C-30** | | 5 | **C-65** |
| 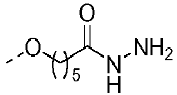 | 1 | **C-31** | 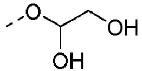 | 1 | **C-66** |
| | 2 | **C-32** | | 2 | **C-67** |
| | 3 | **C-33** | | 3 | **C-68** |
| | 4 | **C-34** | | 4 | **C-69** |
| | 5 | **C-35** | | 5 | **C-70** |

**Table 4**

| | | | | | |
|---|---|---|---|---|---|
| 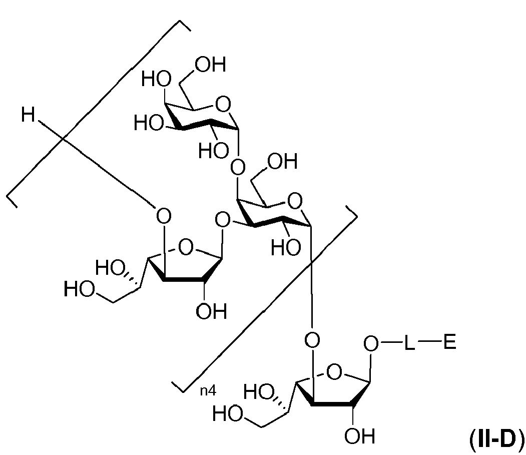 | | | | | |

| **-O-L-E** | n4 | **Compound No.** | **-O-L-E** | n4 | **Compound No.** |
|---|---|---|---|---|---|
| 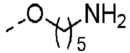 | 1 | **D-01** | 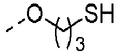 | 1 | **D-36** |
| | 2 | **D-02** | | 2 | **D-37** |
| | 3 | **D-03** | | 3 | **D-38** |
| | 4 | **D-04** | | 4 | **D-39** |
| | 5 | **D-05** | | 5 | **D-40** |
| 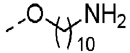 | 1 | **D-06** | 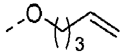 | 1 | **D-41** |
| | 2 | **D-07** | | 2 | **D-42** |
| | 3 | **D-08** | | 3 | **D-43** |
| | 4 | **D-09** | | 4 | **D-44** |
| | 5 | **D-10** | | 5 | **D-45** |
| 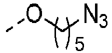 | 1 | **D-11** | 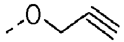 | 1 | **D-46** |
| | 2 | **D-12** | | 2 | **D-47** |
| | 3 | **D-13** | | 3 | **D-48** |
| | 4 | **D-14** | | 4 | **D-49** |
| | 5 | **D-15** | | 5 | **D-50** |
| 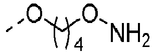 | 1 | **D-16** | 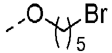 | 1 | **D-51** |
| | 2 | **D-17** | | 2 | **D-52** |
| | 3 | **D-18** | | 3 | **D-53** |
| | 4 | **D-19** | | 4 | **D-54** |
| | 5 | **D-20** | | 5 | **D-55** |
| 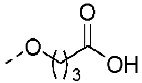 | 1 | **D-21** | 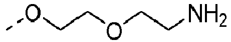 | 1 | **D-56** |
| | 2 | **D-22** | | 2 | **D-57** |
| | 3 | **D-23** | | 3 | **D-58** |
| | 4 | **D-24** | | 4 | **D-59** |
| | 5 | **D-25** | | 5 | **D-60** |
| 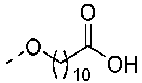 | 1 | **D-26** | 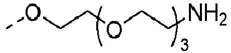 | 1 | **D-61** |
| | 2 | **D-27** | | 2 | **D-62** |
| | 3 | **D-28** | | 3 | **D-63** |
| | 4 | **D-29** | | 4 | **D-64** |
| | 5 | **D-30** | | 5 | **D-65** |
| 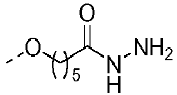 | 1 | **D-31** | 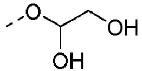 | 1 | **D-66** |
| | 2 | **D-32** | | 2 | **D-67** |
| | 3 | **D-33** | | 3 | **D-68** |
| | 4 | **D-34** | | 4 | **D-69** |
| | 5 | **D-35** | | 5 | **D-70** |

**Table 5**

| | | | | | |
|---|---|---|---|---|---|
| 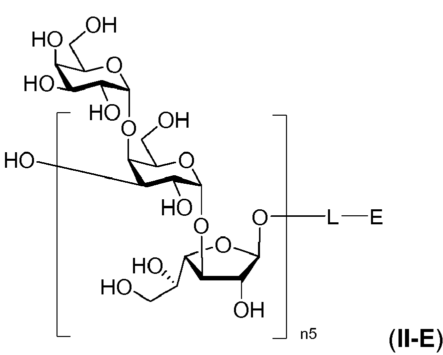 | | | | | |

| **-O-L-E** | n5 | **Compound No.** | **-O-L-E** | n5 | **Compound No.** |
|---|---|---|---|---|---|
| 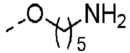 | 1 | **E-01** | 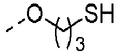 | 1 | **E-36** |
| | 2 | **E-02** | | 2 | **E-37** |
| | 3 | **E-03** | | 3 | **E-38** |
| | 4 | **E-04** | | 4 | **E-39** |
| | 5 | **E-05** | | 5 | **E-40** |
| 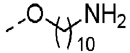 | 1 | **E-06** | 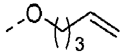 | 1 | **E-41** |
| | 2 | **E-07** | | 2 | **E-42** |
| | 3 | **E-08** | | 3 | **E-43** |
| | 4 | **E-09** | | 4 | **E-44** |
| | 5 | **E-10** | | 5 | **E-45** |
| 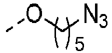 | 1 | **E-11** | 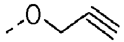 | 1 | **E-46** |
| | 2 | **E-12** | | 2 | **E-47** |
| | 3 | **E-13** | | 3 | **E-48** |
| | 4 | **E-14** | | 4 | **E-49** |
| | 5 | **E-15** | | 5 | **E-50** |
| 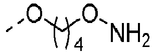 | 1 | **E-16** | 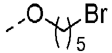 | 1 | **E-51** |
| | 2 | **E-17** | | 2 | **E-52** |
| | 3 | **E-18** | | 3 | **E-53** |
| | 4 | **E-19** | | 4 | **E-54** |
| | 5 | **E-20** | | 5 | **E-55** |
| 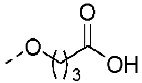 | 1 | **E-21** | 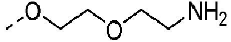 | 1 | **E-56** |
| | 2 | **E-22** | | 2 | **E-57** |
| | 3 | **E-23** | | 3 | **E-58** |
| | 4 | **E-24** | | 4 | **E-59** |
| | 5 | **E-25** | | 5 | **E-60** |
| 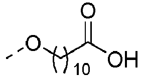 | 1 | **E-26** | 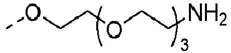 | 1 | **E-61** |
| | 2 | **E-27** | | 2 | **E-62** |
| | 3 | **E-28** | | 3 | **E-63** |
| | 4 | **E-29** | | 4 | **E-64** |
| | 5 | **E-30** | | 5 | **E-65** |
| 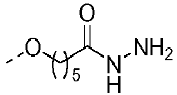 | 1 | **E-31** | 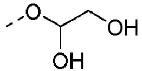 | 1 | **E-66** |
| | 2 | **E-32** | | 2 | **E-67** |
| | 3 | **E-33** | | 3 | **E-68** |
| | 4 | **E-34** | | 4 | **E-69** |
| | 5 | **E-35** | | 5 | **E-70** |

**Table 6**

| | | | | | | | |
|---|---|---|---|---|---|---|---|
| 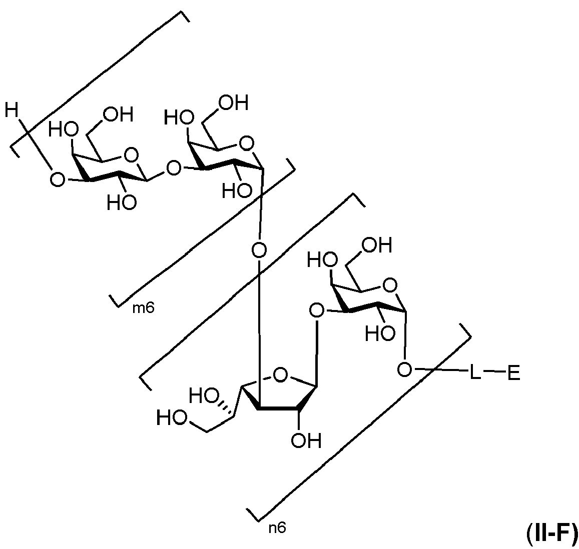 | | | | | | | |

| **-O-L-E** | m6 | n6 | **Compound No.** | **-O-L-E** | m6 | n6 | **Compound No.** |
|---|---|---|---|---|---|---|---|
| 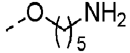 | 1 | 1 | **F-01** | 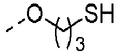 | 1 | 1 | **F-246** |
| | | 2 | **F-02** | | | 2 | **F-247** |
| | | 3 | **F-03** | | | 3 | **F-248** |
| | | 4 | **F-04** | | | 4 | **F-249** |
| | | 5 | **F-05** | | | 5 | **F-250** |
| 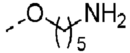 | 2 | 1 | **F-06** | 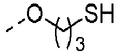 | 2 | 1 | **F-251** |
| | | 2 | **F-07** | | | 2 | **F-252** |
| | | 3 | **F-08** | | | 3 | **F-253** |
| | | 4 | **F-09** | | | 4 | **F-254** |
| | | 5 | **F-10** | | | 5 | **F-255** |
| 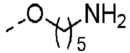 | 3 | 1 | **F-11** | 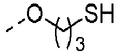 | 3 | 1 | **F-256** |
| | | 2 | **F-12** | | | 2 | **F-257** |
| | | 3 | **F-13** | | | 3 | **F-258** |
| | | 4 | **F-14** | | | 4 | **F-259** |
| | | 5 | **F-15** | | | 5 | **F-260** |
| 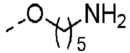 | 4 | 1 | **F-16** | 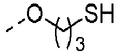 | 4 | 1 | **F-261** |
| | | 2 | **F-17** | | | 2 | **F-262** |
| | | 3 | **F-18** | | | 3 | **F-263** |
| | | 4 | **F-19** | | | 4 | **F-264** |
| | | 5 | **F-20** | | | 5 | **F-265** |
| 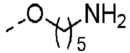 | 5 | 1 | **F-21** | 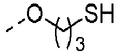 | 5 | 1 | **F-266** |
| | | 2 | **F-22** | | | 2 | **F-267** |
| | | 3 | **F-23** | | | 3 | **F-268** |
| | | 4 | **F-24** | | | 4 | **F-269** |
| | | 5 | **F-25** | | | 5 | **F-270** |
| 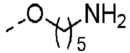 | 6 | 1 | **F-26** | 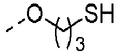 | 6 | 1 | **F-271** |
| | | 2 | **F-27** | | | 2 | **F-272** |
| | | 3 | **F-28** | | | 3 | **F-273** |
| | | 4 | **F-29** | | | 4 | **F-274** |
| | | 5 | **F-30** | | | 5 | **F-275** |
| 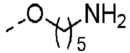 | 7 | 1 | **F-31** | 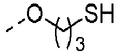 | 7 | 1 | **F-276** |
| | | 2 | **F-32** | | | 2 | **F-277** |
| | | 3 | **F-33** | | | 3 | **F-278** |
| | | 4 | **F-34** | | | 4 | **F-279** |
| | | 5 | **F-35** | | | 5 | **F-280** |
| 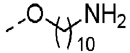 | 1 | 1 | **F-36** | 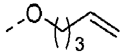 | 1 | 1 | **F-281** |
| | | 2 | **F-37** | | | 2 | **F-282** |
| | | 3 | **F-38** | | | 3 | **F-283** |
| | | 4 | **F-39** | | | 4 | **F-284** |
| | | 5 | **F-40** | | | 5 | **F-285** |
| 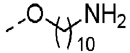 | 2 | 1 | **F-41** | 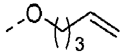 | 2 | 1 | **F-286** |
| | | 2 | **F-42** | | | 2 | **F-287** |
| | | 3 | **F-43** | | | 3 | **F-288** |
| | | 4 | **F-44** | | | 4 | **F-289** |
| | | 5 | **F-45** | | | 5 | **F-290** |
| 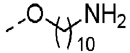 | 3 | 1 | **F-46** | 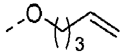 | 3 | 1 | **F-291** |
| | | 2 | **F-47** | | | 2 | **F-292** |
| | | 3 | **F-48** | | | 3 | **F-293** |
| | | 4 | **F-49** | | | 4 | **F-294** |
| | | 5 | **F-50** | | | 5 | **F-295** |
| 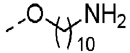 | 4 | 1 | **F-51** | 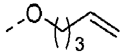 | 4 | 1 | **F-296** |
| | | 2 | **F-52** | | | 2 | **F-297** |
| | | 3 | **F-53** | | | 3 | **F-298** |
| | | 4 | **F-54** | | | 4 | **F-299** |
| | | 5 | **F-55** | | | 5 | **F-300** |
| 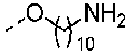 | 5 | 1 | **F-56** | 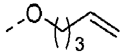 | 5 | 1 | **F-301** |
| | | 2 | **F-57** | | | 2 | **F-302** |
| | | 3 | **F-58** | | | 3 | **F-303** |
| | | 4 | **F-59** | | | 4 | **F-304** |
| | | 5 | **F-60** | | | 5 | **F-305** |
| 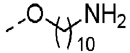 | 6 | 1 | **F-61** | 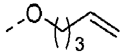 | 6 | 1 | **F-306** |
| | | 2 | **F-62** | | | 2 | **F-307** |
| | | 3 | **F-63** | | | 3 | **F-308** |
| | | 4 | **F-64** | | | 4 | **F-309** |
| | | 5 | **F-65** | | | 5 | **F-310** |
| 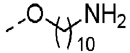 | 7 | 1 | **F-66** | 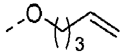 | 7 | 1 | **F-311** |
| | | 2 | **F-67** | | | 2 | **F-312** |
| | | 3 | **F-68** | | | 3 | **F-313** |
| | | 4 | **F-69** | | | 4 | **F-314** |
| | | 5 | **F-70** | | | 5 | **F-315** |
| 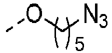 | 1 | 1 | **F-71** | 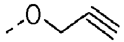 | 1 | 1 | **F-316** |
| | | 2 | **F-72** | | | 2 | **F-317** |
| | | 3 | **F-73** | | | 3 | **F-318** |
| | | 4 | **F-74** | | | 4 | **F-319** |
| | | 5 | **F-75** | | | 5 | **F-320** |
| 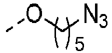 | 2 | 1 | **F-76** | 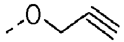 | 2 | 1 | **F-321** |
| | | 2 | **F-77** | | | 2 | **F-322** |
| | | 3 | **F-78** | | | 3 | **F-323** |
| | | 4 | **F-79** | | | 4 | **F-324** |
| | | 5 | **F-80** | | | 5 | **F-325** |
| 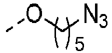 | 3 | 1 | **F-81** | 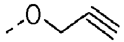 | 3 | 1 | **F-326** |
| | | 2 | **F-82** | | | 2 | **F-327** |
| | | 3 | **F-83** | | | 3 | **F-328** |
| | | 4 | **F-84** | | | 4 | **F-329** |
| | | 5 | **F-85** | | | 5 | **F-330** |
| 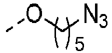 | 4 | 1 | **F-86** | 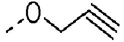 | 4 | 1 | **F-331** |
| | | 2 | **F-87** | | | 2 | **F-332** |
| | | 3 | **F-88** | | | 3 | **F-333** |
| | | 4 | **F-89** | | | 4 | **F-334** |
| | | 5 | **F-90** | | | 5 | **F-335** |
| 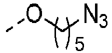 | 5 | 1 | **F-91** | 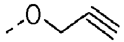 | 5 | 1 | **F-336** |
| | | 2 | **F-92** | | | 2 | **F-337** |
| | | 3 | **F-93** | | | 3 | **F-338** |
| | | 4 | **F-94** | | | 4 | **F-339** |
| | | 5 | **F-95** | | | 5 | **F-340** |
| 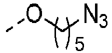 | 6 | 1 | **F-96** | 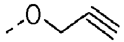 | 6 | 1 | **F-341** |
| | | 2 | **F-97** | | | 2 | **F-342** |
| | | 3 | **F-98** | | | 3 | **F-343** |
| | | 4 | **F-99** | | | 4 | **F-344** |
| | | 5 | **F-100** | | | 5 | **F-345** |
| 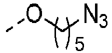 | 7 | 1 | **F-101** | 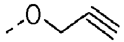 | 7 | 1 | **F-346** |
| | | 2 | **F-102** | | | 2 | **F-347** |
| | | 3 | **F-103** | | | 3 | **F-348** |
| | | 4 | **F-104** | | | 4 | **F-349** |
| | | 5 | **F-105** | | | 5 | **F-350** |
| 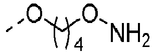 | 1 | 1 | **F-106** | 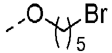 | 1 | 1 | **F-351** |
| | | 2 | **F-107** | | | 2 | **F-352** |
| | | 3 | **F-108** | | | 3 | **F-353** |
| | | 4 | **F-109** | | | 4 | **F-354** |
| | | 5 | **F-110** | | | 5 | **F-355** |
| 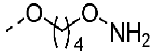 | 2 | 1 | **F-111** | 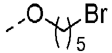 | 2 | 1 | **F-356** |
| | | 2 | **F-112** | | | 2 | **F-357** |
| | | 3 | **F-113** | | | 3 | **F-358** |
| | | 4 | **F-114** | | | 4 | **F-359** |
| | | 5 | **F-115** | | | 5 | **F-400** |
| 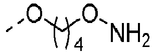 | 3 | 1 | **F-116** | 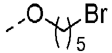 | 3 | 1 | **F-401** |
| | | 2 | **F-117** | | | 2 | **F-402** |
| | | 3 | **F-118** | | | 3 | **F-403** |
| | | 4 | **F-119** | | | 4 | **F-404** |
| | | 5 | **F-120** | | | 5 | **F-405** |
| 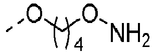 | 4 | 1 | **F-121** | 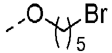 | 4 | 1 | **F-406** |
| | | 2 | **F-122** | | | 2 | **F-407** |
| | | 3 | **F-123** | | | 3 | **F-408** |
| | | 4 | **F-124** | | | 4 | **F-409** |
| | | 5 | **F-125** | | | 5 | **F-410** |
| 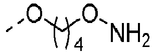 | 5 | 1 | **F-126** | 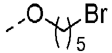 | 5 | 1 | **F-411** |
| | | 2 | **F-127** | | | 2 | **F-412** |
| | | 3 | **F-128** | | | 3 | **F-413** |
| | | 4 | **F-129** | | | 4 | **F-414** |
| | | 5 | **F-130** | | | 5 | **F-415** |
| 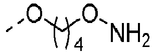 | 6 | 1 | **F-131** | 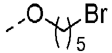 | 6 | 1 | **F-416** |
| | | 2 | **F-132** | | | 2 | **F-417** |
| | | 3 | **F-133** | | | 3 | **F-418** |
| | | 4 | **F-134** | | | 4 | **F-419** |
| | | 5 | **F-135** | | | 5 | **F-420** |
| 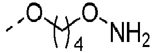 | 7 | 1 | **F-136** | 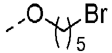 | 7 | 1 | **F-421** |
| | | 2 | **F-137** | | | 2 | **F-422** |
| | | 3 | **F-138** | | | 3 | **F-423** |
| | | 4 | **F-139** | | | 4 | **F-424** |
| | | 5 | **F-140** | | | 5 | **F-425** |
| 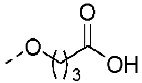 | 1 | 1 | **F-141** | 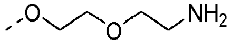 | 1 | 1 | **F-426** |
| | | 2 | **F-142** | | | 2 | **F-427** |
| | | 3 | **F-143** | | | 3 | **F-428** |
| | | 4 | **F-144** | | | 4 | **F-429** |
| | | 5 | **F-145** | | | 5 | **F-430** |
| 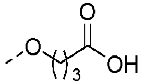 | 2 | 1 | **F-146** | 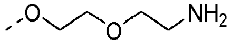 | 2 | 1 | **F-431** |
| | | 2 | **F-147** | | | 2 | **F-432** |
| | | 3 | **F-148** | | | 3 | **F-433** |
| | | 4 | **F-149** | | | 4 | **F-434** |
| | | 5 | **F-150** | | | 5 | **F-435** |
| 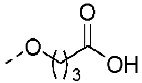 | 3 | 1 | **F-151** | 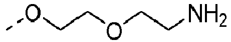 | 3 | 1 | **F-436** |
| | | 2 | **F-152** | | | 2 | **F-437** |
| | | 3 | **F-153** | | | 3 | **F-438** |
| | | 4 | **F-154** | | | 4 | **F-439** |
| | | 5 | **F-155** | | | 5 | **F-440** |
| 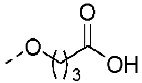 | 4 | 1 | **F-156** | 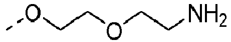 | 4 | 1 | **F-441** |
| | | 2 | **F-157** | | | 2 | **F-442** |
| | | 3 | **F-158** | | | 3 | **F-443** |
| | | 4 | **F-159** | | | 4 | **F-444** |
| | | 5 | **F-160** | | | 5 | **F-445** |
| 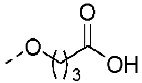 | 5 | 1 | **F-161** | 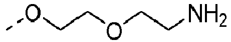 | 5 | 1 | **F-446** |
| | | 2 | **F-162** | | | 2 | **F-447** |
| | | 3 | **F-163** | | | 3 | **F-448** |
| | | 4 | **F-164** | | | 4 | **F-449** |
| | | 5 | **F-165** | | | 5 | **F-450** |
| 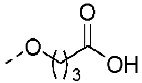 | 6 | 1 | **F-166** | 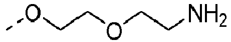 | 6 | 1 | **F-451** |
| | | 2 | **F-167** | | | 2 | **F-452** |
| | | 3 | **F-168** | | | 3 | **F-453** |
| | | 4 | **F-169** | | | 4 | **F-454** |
| | | 5 | **F-170** | | | 5 | **F-455** |
| 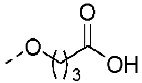 | 7 | 1 | **F-171** | 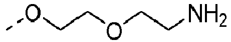 | 7 | 1 | **F-456** |
| | | 2 | **F-172** | | | 2 | **F-457** |
| | | 3 | **F-173** | | | 3 | **F-458** |
| | | 4 | **F-174** | | | 4 | **F-459** |
| | | 5 | **F-175** | | | 5 | **F-460** |
| 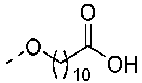 | 1 | 1 | **F-176** | 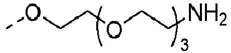 | 1 | 1 | **F-461** |
| | | 2 | **F-177** | | | 2 | **F-462** |
| | | 3 | **F-178** | | | 3 | **F-463** |
| | | 4 | **F-179** | | | 4 | **F-464** |
| | | 5 | **F-180** | | | 5 | **F-465** |
| 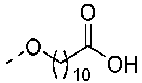 | 2 | 1 | **F-181** | 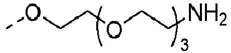 | 2 | 1 | **F-466** |
| | | 2 | **F-182** | | | 2 | **F-467** |
| | | 3 | **F-183** | | | 3 | **F-468** |
| | | 4 | **F-184** | | | 4 | **F-469** |
| | | 5 | **F-185** | | | 5 | **F-470** |
| 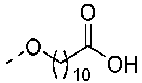 | 3 | 1 | **F-186** | 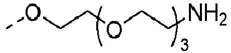 | 3 | 1 | **F-471** |
| | | 2 | **F-187** | | | 2 | **F-472** |
| | | 3 | **F-188** | | | 3 | **F-473** |
| | | 4 | **F-189** | | | 4 | **F-474** |
| | | 5 | **F-190** | | | 5 | **F-475** |
| 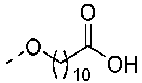 | 4 | 1 | **F-191** | 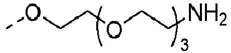 | 4 | 1 | **F-476** |
| | | 2 | **F-192** | | | 2 | **F-477** |
| | | 3 | **F-193** | | | 3 | **F-478** |
| | | 4 | **F-194** | | | 4 | **F-479** |
| | | 5 | **F-195** | | | 5 | **F-480** |
| 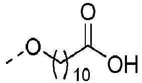 | 5 | 1 | **F-196** | 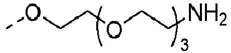 | 5 | 1 | **F-481** |
| | | 2 | **F-197** | | | 2 | **F-482** |
| | | 3 | **F-198** | | | 3 | **F-483** |
| | | 4 | **F-199** | | | 4 | **F-484** |
| | | 5 | **F-200** | | | 5 | **F-485** |
| 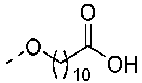 | 6 | 1 | **F-201** | 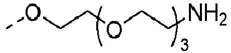 | 6 | 1 | **F-486** |
| | | 2 | **F-202** | | | 2 | **F-487** |
| | | 3 | **F-203** | | | 3 | **F-488** |
| | | 4 | **F-204** | | | 4 | **F-489** |
| | | 5 | **F-205** | | | 5 | **F-490** |
| 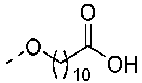 | 7 | 1 | **F-206** | 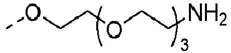 | 7 | 1 | **F-491** |
| | | 2 | **F-207** | | | 2 | **F-492** |
| | | 3 | **F-208** | | | 3 | **F-493** |
| | | 4 | **F-209** | | | 4 | **F-494** |
| | | 5 | **F-210** | | | 5 | **F-495** |
| 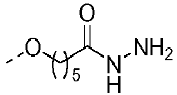 | 1 | 1 | **F-211** | 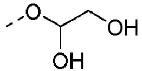 | 1 | 1 | **F-496** |
| | | 2 | **F-212** | | | 2 | **F-497** |
| | | 3 | **F-213** | | | 3 | **F-498** |
| | | 4 | **F-214** | | | 4 | **F-499** |
| | | 5 | **F-215** | | | 5 | **F-500** |
| 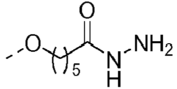 | 2 | 1 | **F-216** | 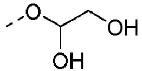 | 2 | 1 | **F-501** |
| | | 2 | **F-217** | | | 2 | **F-502** |
| | | 3 | **F-218** | | | 3 | **F-503** |
| | | 4 | **F-219** | | | 4 | **F-504** |
| | | 5 | **F-220** | | | 5 | **F-505** |
| 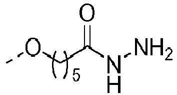 | 3 | 1 | **F-221** | 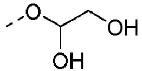 | 3 | 1 | **F-506** |
| | | 2 | **F-222** | | | 2 | **F-507** |
| | | 3 | **F-223** | | | 3 | **F-508** |
| | | 4 | **F-224** | | | 4 | **F-509** |
| | | 5 | **F-225** | | | 5 | **F-510** |
| 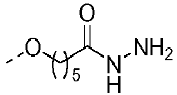 | 4 | 1 | **F-226** | 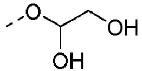 | 4 | 1 | **F-511** |
| | | 2 | **F-227** | | | 2 | **F-512** |
| | | 3 | **F-228** | | | 3 | **F-513** |
| | | 4 | **F-229** | | | 4 | **F-514** |
| | | 5 | **F-230** | | | 5 | **F-515** |
| 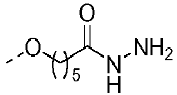 | 5 | 1 | **F-231** | 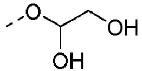 | 5 | 1 | **F-516** |
| | | 2 | **F-232** | | | 2 | **F-517** |
| | | 3 | **F-233** | | | 3 | **F-518** |
| | | 4 | **F-234** | | | 4 | **F-519** |
| | | 5 | **F-235** | | | 5 | **F-520** |
| 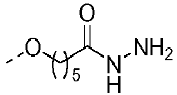 | 6 | 1 | **F-236** | 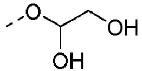 | 6 | 1 | **F-521** |
| | | 2 | **F-237** | | | 2 | **F-522** |
| | | 3 | **F-238** | | | 3 | **F-523** |
| | | 4 | **F-239** | | | 4 | **F-524** |
| | | 5 | **F-240** | | | 5 | **F-525** |
| 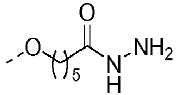 | 7 | 1 | **F-241** | 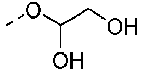 | 7 | 1 | **F-526** |
| | | 2 | **F-242** | | | 2 | **F-527** |
| | | 3 | **F-243** | | | 3 | **F-528** |
| | | 4 | **F-244** | | | 4 | **F-529** |
| | | 5 | **F-245** | | | 5 | **F-530** |

**Table 7**

| | | | | | | | |
|---|---|---|---|---|---|---|---|
| 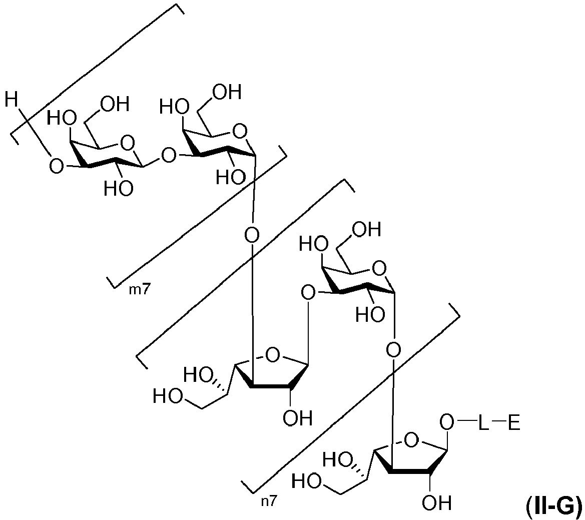 | | | | | | | |

| **-O-L-E** | m7 | n7 | **Compound No.** | **-O-L-E** | m7 | n7 | **Compound No.** |
|---|---|---|---|---|---|---|---|
| 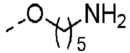 | 1 | 1 | **G-01** | 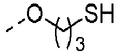 | 1 | 1 | **G-176** |
| | | 2 | **G-02** | | | 2 | **G-177** |
| | | 3 | **G-03** | | | 3 | **G-178** |
| | | 4 | **G-04** | | | 4 | **G-179** |
| | | 5 | **G-05** | | | 5 | **G-180** |
| 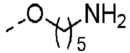 | 2 | 1 | **G-06** | 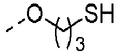 | 2 | 1 | **G-181** |
| | | 2 | **G-07** | | | 2 | **G-182** |
| | | 3 | **G-08** | | | 3 | **G-183** |
| | | 4 | **G-09** | | | 4 | **G-184** |
| | | 5 | **G-10** | | | 5 | **G-185** |
| 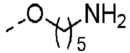 | 3 | 1 | **G-11** | 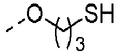 | 3 | 1 | **G-186** |
| | | 2 | **G-12** | | | 2 | **G-187** |
| | | 3 | **G-13** | | | 3 | **G-188** |
| | | 4 | **G-14** | | | 4 | **G-189** |
| | | 5 | **G-15** | | | 5 | **G-190** |
| 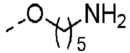 | 4 | 1 | **G-16** | 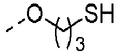 | 4 | 1 | **G-191** |
| | | 2 | **G-17** | | | 2 | **G-192** |
| | | 3 | **G-18** | | | 3 | **G-193** |
| | | 4 | **G-19** | | | 4 | **G-194** |
| | | 5 | **G-20** | | | 5 | **G-195** |
| 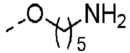 | 5 | 1 | **G-21** | 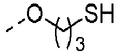 | 5 | 1 | **G-196** |
| | | 2 | **G-22** | | | 2 | **G-197** |
| | | 3 | **G-23** | | | 3 | **G-198** |
| | | 4 | **G-24** | | | 4 | **G-199** |
| | | 5 | **G-25** | | | 5 | **G-200** |
| 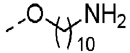 | 1 | 1 | **G-26** | 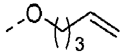 | 1 | 1 | **G-201** |
| | | 2 | **G-27** | | | 2 | **G-202** |
| | | 3 | **G-28** | | | 3 | **G-203** |
| | | 4 | **G-29** | | | 4 | **G-204** |
| | | 5 | **G-30** | | | 5 | **G-205** |
| 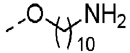 | 2 | 1 | **G-31** | 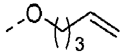 | 2 | 1 | **G-206** |
| | | 2 | **G-32** | | | 2 | **G-207** |
| | | 3 | **G-33** | | | 3 | **G-208** |
| | | 4 | **G-34** | | | 4 | **G-209** |
| | | 5 | **G-35** | | | 5 | **G-210** |
| 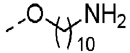 | 3 | 1 | **G-36** | 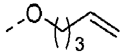 | 3 | 1 | **G-211** |
| | | 2 | **G-37** | | | 2 | **G-212** |
| | | 3 | **G-38** | | | 3 | **G-213** |
| | | 4 | **G-39** | | | 4 | **G-214** |
| | | 5 | **G-40** | | | 5 | **G-215** |
| 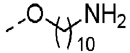 | 4 | 1 | **G-41** | 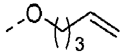 | 4 | 1 | **G-216** |
| | | 2 | **G-42** | | | 2 | **G-217** |
| | | 3 | **G-43** | | | 3 | **G-218** |
| | | 4 | **G-44** | | | 4 | **G-219** |
| | | 5 | **G-45** | | | 5 | **G-220** |
| 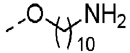 | 5 | 1 | **G-46** | 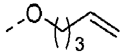 | 5 | 1 | **G-221** |
| | | 2 | **G-47** | | | 2 | **G-222** |
| | | 3 | **G-48** | | | 3 | **G-223** |
| | | 4 | **G-49** | | | 4 | **G-224** |
| | | 5 | **G-50** | | | 5 | **G-225** |
| 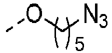 | 1 | 1 | **G-51** | 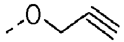 | 1 | 1 | **G-226** |
| | | 2 | **G-52** | | | 2 | **G-227** |
| | | 3 | **G-53** | | | 3 | **G-228** |
| | | 4 | **G-54** | | | 4 | **G-229** |
| | | 5 | **G-55** | | | 5 | **G-230** |
| 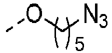 | 2 | 1 | **G-56** | 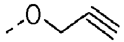 | 2 | 1 | **G-231** |
| | | 2 | **G-57** | | | 2 | **G-232** |
| | | 3 | **G-58** | | | 3 | **G-233** |
| | | 4 | **G-59** | | | 4 | **G-234** |
| | | 5 | **G-60** | | | 5 | **G-235** |
| 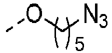 | 3 | 1 | **G-61** | 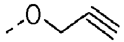 | 3 | 1 | **G-236** |
| | | 2 | **G-62** | | | 2 | **G-237** |
| | | 3 | **G-63** | | | 3 | **G-238** |
| | | 4 | **G-64** | | | 4 | **G-239** |
| | | 5 | **G-65** | | | 5 | **G-240** |
| 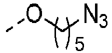 | 4 | 1 | **G-66** | 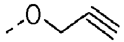 | 4 | 1 | **G-241** |
| | | 2 | **G-67** | | | 2 | **G-242** |
| | | 3 | **G-68** | | | 3 | **G-243** |
| | | 4 | **G-69** | | | 4 | **G-244** |
| | | 5 | **G-70** | | | 5 | **G-245** |
| 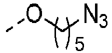 | 5 | 1 | **G-71** | 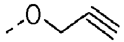 | 5 | 1 | **G-246** |
| | | 2 | **G-72** | | | 2 | **G-247** |
| | | 3 | **G-73** | | | 3 | **G-248** |
| | | 4 | **G-74** | | | 4 | **G-249** |
| | | 5 | **G-75** | | | 5 | **G-250** |
| 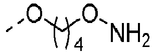 | 1 | 1 | **G-76** | 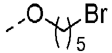 | 1 | 1 | **G-251** |
| | | 2 | **G-77** | | | 2 | **G-252** |
| | | 3 | **G-78** | | | 3 | **G-253** |
| | | 4 | **G-79** | | | 4 | **G-254** |
| | | 5 | **G-80** | | | 5 | **G-255** |
| 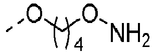 | 2 | 1 | **G-81** | 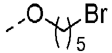 | 2 | 1 | **G-256** |
| | | 2 | **G-82** | | | 2 | **G-257** |
| | | 3 | **G-83** | | | 3 | **G-258** |
| | | 4 | **G-84** | | | 4 | **G-259** |
| | | 5 | **G-85** | | | 5 | **G-260** |
| 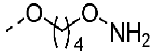 | 3 | 1 | **G-86** | 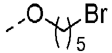 | 3 | 1 | **G-261** |
| | | 2 | **G-87** | | | 2 | **G-262** |
| | | 3 | **G-88** | | | 3 | **G-263** |
| | | 4 | **G-89** | | | 4 | **G-264** |
| | | 5 | **G-90** | | | 5 | **G-265** |
| 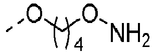 | 4 | 1 | **G-91** | 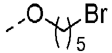 | 4 | 1 | **G-266** |
| | | 2 | **G-92** | | | 2 | **G-267** |
| | | 3 | **G-93** | | | 3 | **G-268** |
| | | 4 | **G-94** | | | 4 | **G-269** |
| | | 5 | **G-95** | | | 5 | **G-270** |
| 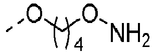 | 5 | 1 | **G-96** | 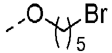 | 5 | 1 | **G-271** |
| | | 2 | **G-97** | | | 2 | **G-272** |
| | | 3 | **G-98** | | | 3 | **G-273** |
| | | 4 | **G-99** | | | 4 | **G-274** |
| | | 5 | **G-100** | | | 5 | **G-275** |
| 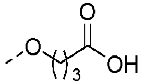 | 1 | 1 | **G-101** | 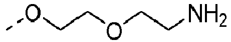 | 1 | 1 | **G-276** |
| | | 2 | **G-102** | | | 2 | **G-277** |
| | | 3 | **G-103** | | | 3 | **G-278** |
| | | 4 | **G-104** | | | 4 | **G-279** |
| | | 5 | **G-105** | | | 5 | **G-280** |
| 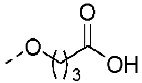 | 2 | 1 | **G-106** | 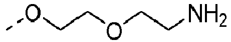 | 2 | 1 | **G-281** |
| | | 2 | **G-107** | | | 2 | **G-282** |
| | | 3 | **G-108** | | | 3 | **G-283** |
| | | 4 | **G-109** | | | 4 | **G-284** |
| | | 5 | **G-110** | | | 5 | **G-285** |
| 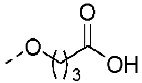 | 3 | 1 | **G-111** | 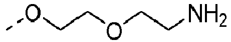 | 3 | 1 | **G-286** |
| | | 2 | **G-112** | | | 2 | **G-287** |
| | | 3 | **G-113** | | | 3 | **G-288** |
| | | 4 | **G-114** | | | 4 | **G-289** |
| | | 5 | **G-115** | | | 5 | **G-290** |
| 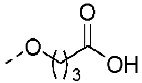 | 4 | 1 | **G-116** | 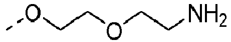 | 4 | 1 | **G-291** |
| | | 2 | **G-117** | | | 2 | **G-292** |
| | | 3 | **G-118** | | | 3 | **G-293** |
| | | 4 | **G-119** | | | 4 | **G-294** |
| | | 5 | **G-120** | | | 5 | **G-295** |
| 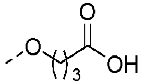 | 5 | 1 | **G-121** | 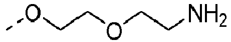 | 5 | 1 | **G-296** |
| | | 2 | **G-122** | | | 2 | **G-297** |
| | | 3 | **G-123** | | | 3 | **G-298** |
| | | 4 | **G-124** | | | 4 | **G-299** |
| | | 5 | **G-125** | | | 5 | **G-300** |
| 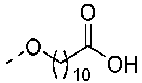 | 1 | 1 | **G-126** | 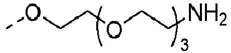 | 1 | 1 | **G-301** |
| | | 2 | **G-127** | | | 2 | **G-302** |
| | | 3 | **G-128** | | | 3 | **G-303** |
| | | 4 | **G-129** | | | 4 | **G-304** |
| | | 5 | **G-130** | | | 5 | **G-305** |
| 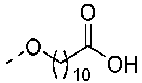 | 2 | 1 | **G-131** | 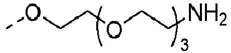 | 2 | 1 | **G-306** |
| | | 2 | **G-132** | | | 2 | **G-307** |
| | | 3 | **G-133** | | | 3 | **G-308** |
| | | 4 | **G-134** | | | 4 | **G-309** |
| | | 5 | **G-135** | | | 5 | **G-310** |
| 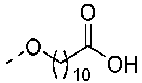 | 3 | 1 | **G-136** | 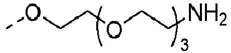 | 3 | 1 | **G-311** |
| | | 2 | **G-137** | | | 2 | **G-312** |
| | | 3 | **G-138** | | | 3 | **G-313** |
| | | 4 | **G-139** | | | 4 | **G-314** |
| | | 5 | **G-140** | | | 5 | **G-315** |
| 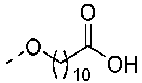 | 4 | 1 | **G-141** | 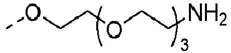 | 4 | 1 | **G-316** |
| | | 2 | **G-142** | | | 2 | **G-317** |
| | | 3 | **G-143** | | | 3 | **G-318** |
| | | 4 | **G-144** | | | 4 | **G-319** |
| | | 5 | **G-145** | | | 5 | **G-320** |
| 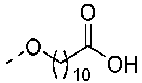 | 5 | 1 | **G-146** | 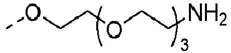 | 5 | 1 | **G-321** |
| | | 2 | **G-147** | | | 2 | **G-322** |
| | | 3 | **G-148** | | | 3 | **G-323** |
| | | 4 | **G-149** | | | 4 | **G-324** |
| | | 5 | **G-150** | | | 5 | **G-325** |
| 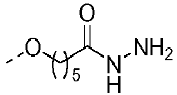 | 1 | 1 | **G-151** | 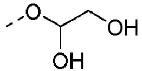 | 1 | 1 | **G-326** |
| | | 2 | **G-152** | | | 2 | **G-327** |
| | | 3 | **G-153** | | | 3 | **G-328** |
| | | 4 | **G-154** | | | 4 | **G-329** |
| | | 5 | **G-155** | | | 5 | **G-330** |
| 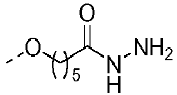 | 2 | 1 | **G-156** | 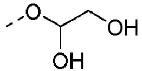 | 2 | 1 | **G-331** |
| | | 2 | **G-157** | | | 2 | **G-332** |
| | | 3 | **G-158** | | | 3 | **G-333** |
| | | 4 | **G-159** | | | 4 | **G-334** |
| | | 5 | **G-160** | | | 5 | **G-335** |
| 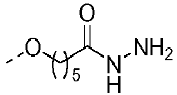 | 3 | 1 | **G-161** | 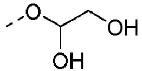 | 3 | 1 | **G-336** |
| | | 2 | **G-162** | | | 2 | **G-337** |
| | | 3 | **G-163** | | | 3 | **G-338** |
| | | 4 | **G-164** | | | 4 | **G-339** |
| | | 5 | **G-165** | | | 5 | **G-340** |
| 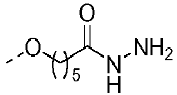 | 4 | 1 | **G-166** | 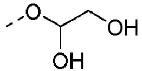 | 4 | 1 | **G-341** |
| | | 2 | **G-167** | | | 2 | **G-342** |
| | | 3 | **G-168** | | | 3 | **G-343** |
| | | 4 | **G-169** | | | 4 | **G-344** |
| | | 5 | **G-170** | | | 5 | **G-345** |
| 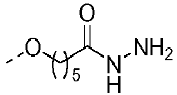 | 5 | 1 | **G-171** | 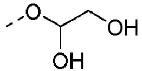 | 5 | 1 | **G-346** |
| | | 2 | **G-172** | | | 2 | **G-347** |
| | | 3 | **G-173** | | | 3 | **G-348** |
| | | 4 | **G-174** | | | 4 | **G-349** |
| | | 5 | **G-175** | | | 5 | **G-350** |

**Table 8**

| | | | | | |
|---|---|---|---|---|---|
| 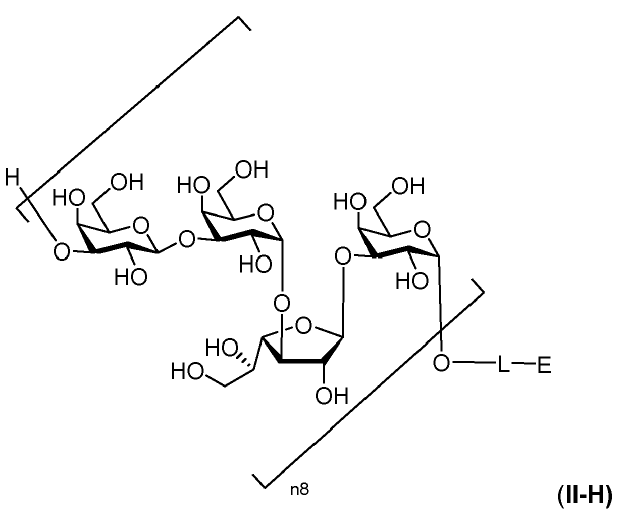 | | | | | |

| **-O-L-E** | n8 | **Compound No.** | **-O-L-E** | n8 | **Compound No.** |
|---|---|---|---|---|---|
| 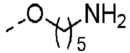 | 1 | **H-01** | 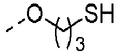 | 1 | **H-36** |
| | 2 | **H-02** | | 2 | **H-37** |
| | 3 | **H-03** | | 3 | **H-38** |
| | 4 | **H-04** | | 4 | **H-39** |
| | 5 | **H-05** | | 5 | **H-40** |
| 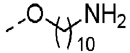 | 1 | **H-06** | 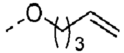 | 1 | **H-41** |
| | 2 | **H-07** | | 2 | **H-42** |
| | 3 | **H-08** | | 3 | **H-43** |
| | 4 | **H-09** | | 4 | **H-44** |
| | 5 | **H-10** | | 5 | **H-45** |
| 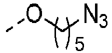 | 1 | **H-11** | 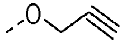 | 1 | **H-46** |
| | 2 | **H-12** | | 2 | **H-47** |
| | 3 | **H-13** | | 3 | **H-48** |
| | 4 | **H-14** | | 4 | **H-49** |
| | 5 | **H-15** | | 5 | **H-50** |
| 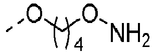 | 1 | **H-16** | 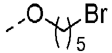 | 1 | **H-51** |
| | 2 | **H-17** | | 2 | **H-52** |
| | 3 | **H-18** | | 3 | **H-53** |
| | 4 | **H-19** | | 4 | **H-54** |
| | 5 | **H-20** | | 5 | **H-55** |
| 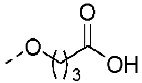 | 1 | **H-21** | 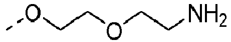 | 1 | **H-56** |
| | 2 | **H-22** | | 2 | **H-57** |
| | 3 | **H-23** | | 3 | **H-58** |
| | 4 | **H-24** | | 4 | **H-59** |
| | 5 | **H-25** | | 5 | **H-60** |
| 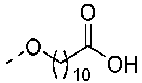 | 1 | **H-26** | 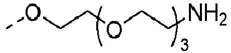 | 1 | **H-61** |
| | 2 | **H-27** | | 2 | **H-62** |
| | 3 | **H-28** | | 3 | **H-63** |
| | 4 | **H-29** | | 4 | **H-64** |
| | 5 | **H-30** | | 5 | **H-65** |
| 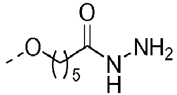 | 1 | **H-31** | 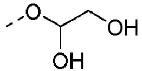 | 1 | **H-66** |
| | 2 | **H-32** | | 2 | **H-67** |
| | 3 | **H-33** | | 3 | **H-68** |
| | 4 | **H-34** | | 4 | **H-69** |
| | 5 | **H-35** | | 5 | **H-70** |

**Table 9**

| | | | | | |
|---|---|---|---|---|---|
| 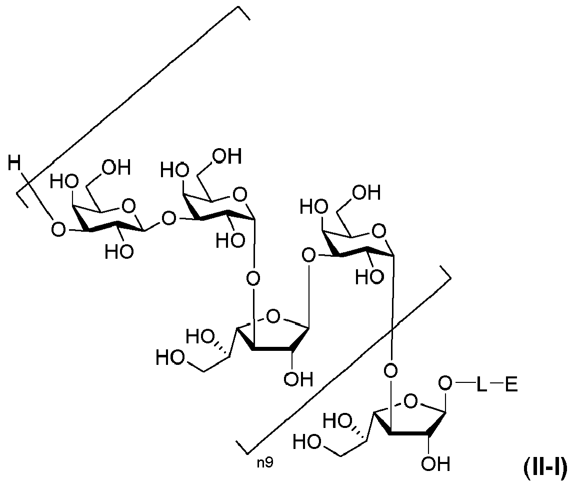 | | | | | |

| **-O-L-E** | n9 | **Compound No.** | **-O-L-E** | n9 | **Compound No.** |
|---|---|---|---|---|---|
| 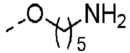 | 1 | **J-01** | 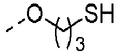 | 1 | **J-36** |
| | 2 | **J-02** | | 2 | **J-37** |
| | 3 | **J-03** | | 3 | **J-38** |
| | 4 | **J-04** | | 4 | **J-39** |
| | 5 | **J-05** | | 5 | **J-40** |
| 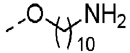 | 1 | **J-06** | 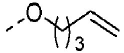 | 1 | **J-41** |
| | 2 | **J-07** | | 2 | **J-42** |
| | 3 | **J-08** | | 3 | **J-43** |
| | 4 | **J-09** | | 4 | **J-44** |
| | 5 | **J-10** | | 5 | **J-45** |
| 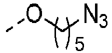 | 1 | **J-11** | 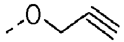 | 1 | **J-46** |
| | 2 | **J-12** | | 2 | **J-47** |
| | 3 | **J-13** | | 3 | **J-48** |
| | 4 | **J-14** | | 4 | **J-49** |
| | 5 | **J-15** | | 5 | **J-50** |
| 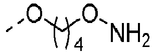 | 1 | **J-16** | 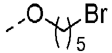 | 1 | **J-51** |
| | 2 | **J-17** | | 2 | **J-52** |
| | 3 | **J-18** | | 3 | **J-53** |
| | 4 | **J-19** | | 4 | **J-54** |
| | 5 | **J-20** | | 5 | **J-55** |
| 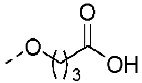 | 1 | **J-21** | 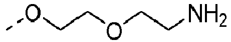 | 1 | **J-56** |
| | 2 | **J-22** | | 2 | **J-57** |
| | 3 | **J-23** | | 3 | **J-58** |
| | 4 | **J-24** | | 4 | **J-59** |
| | 5 | **J-25** | | 5 | **J-60** |
| 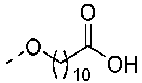 | 1 | **J-26** | 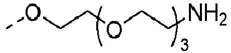 | 1 | **J-61** |
| | 2 | **J-27** | | 2 | **J-62** |
| | 3 | **J-28** | | 3 | **J-63** |
| | 4 | **J-29** | | 4 | **J-64** |
| | 5 | **J-30** | | 5 | **J-65** |
| 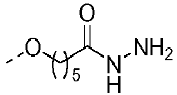 | 1 | **J-31** | 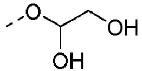 | 1 | **J-66** |
| | 2 | **J-32** | | 2 | **J-67** |
| | 3 | **J-33** | | 3 | **J-68** |
| | 4 | **J-34** | | 4 | **J-69** |
| | 5 | **J-35** | | 5 | **J-70** |

**Table 10**

| | | | | | | | |
|---|---|---|---|---|---|---|---|
| 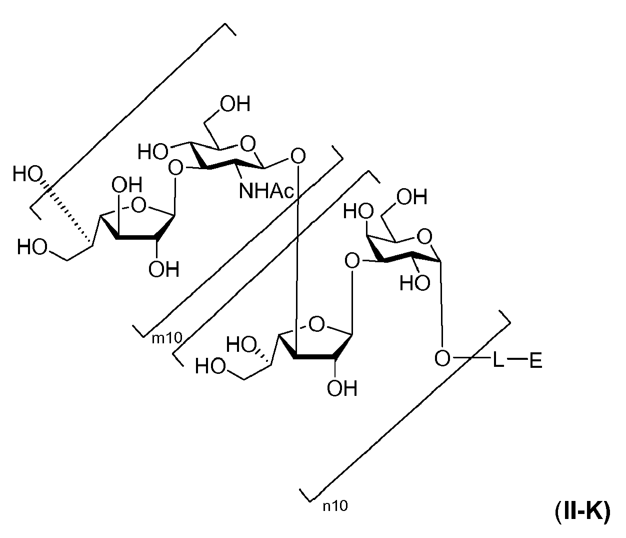 | | | | | | | |

| **-O-L-E** | m10 | n10 | **Compound No.** | **-O-L-E** | m10 | n10 | **Compound No.** |
|---|---|---|---|---|---|---|---|
| 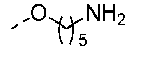 | 1 | 1 | **K-01** | 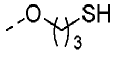 | 1 | 1 | **K-176** |
| | | 2 | **K-02** | | | 2 | **K-177** |
| | | 3 | **K-03** | | | 3 | **K-178** |
| | | 4 | **K-04** | | | 4 | **K-179** |
| | | 5 | **K-05** | | | 5 | **K-180** |
| 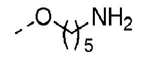 | 2 | 1 | **K-06** | 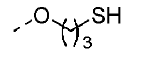 | 2 | 1 | **K-181** |
| | | 2 | **K-07** | | | 2 | **K-182** |
| | | 3 | **K-08** | | | 3 | **K-183** |
| | | 4 | **K-09** | | | 4 | **K-184** |
| | | 5 | **K-10** | | | 5 | **K-185** |
| 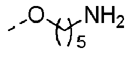 | 3 | 1 | **K-11** | 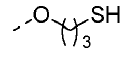 | 3 | 1 | **K-186** |
| | | 2 | **K-12** | | | 2 | **K-187** |
| | | 3 | **K-13** | | | 3 | **K-188** |
| | | 4 | **K-14** | | | 4 | **K-189** |
| | | 5 | **K-15** | | | 5 | **K-190** |
| 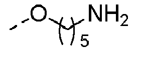 | 4 | 1 | **K-16** | 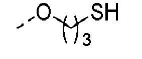 | 4 | 1 | **K-191** |
| | | 2 | **K-17** | | | 2 | **K-192** |
| | | 3 | **K-18** | | | 3 | **K-193** |
| | | 4 | **K-19** | | | 4 | **K-194** |
| | | 5 | **K-20** | | | 5 | **K-195** |
| 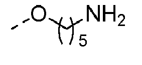 | 5 | 1 | **K-21** | 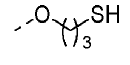 | 5 | 1 | **K-196** |
| | | 2 | **K-22** | | | 2 | **K-197** |
| | | 3 | **K-23** | | | 3 | **K-198** |
| | | 4 | **K-24** | | | 4 | **K-199** |
| | | 5 | **K-25** | | | 5 | **K-200** |
| 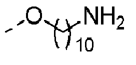 | 1 | 1 | **K-26** | 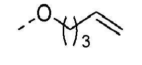 | 1 | 1 | **K-201** |
| | | 2 | **K-27** | | | 2 | **K-202** |
| | | 3 | **K-28** | | | 3 | **K-203** |
| | | 4 | **K-29** | | | 4 | **K-204** |
| | | 5 | **K-30** | | | 5 | **K-205** |
| 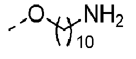 | 2 | 1 | **K-31** | 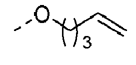 | 2 | 1 | **K-206** |
| | | 2 | **K-32** | | | 2 | **K-207** |
| | | 3 | **K-33** | | | 3 | **K-208** |
| | | 4 | **K-34** | | | 4 | **K-209** |
| | | 5 | **K-35** | | | 5 | **K-210** |
| 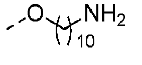 | 3 | 1 | **K-36** | 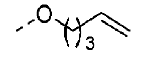 | 3 | 1 | **K-211** |
| | | 2 | **K-37** | | | 2 | **K-212** |
| | | 3 | **K-38** | | | 3 | **K-213** |
| | | 4 | **K-39** | | | 4 | **K-214** |
| | | 5 | **K-40** | | | 5 | **K-215** |
| 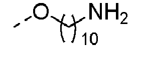 | 4 | 1 | **K-41** | 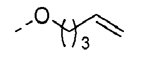 | 4 | 1 | **K-216** |
| | | 2 | **K-42** | | | 2 | **K-217** |
| | | 3 | **K-43** | | | 3 | **K-218** |
| | | 4 | **K-44** | | | 4 | **K-219** |
| | | 5 | **K-45** | | | 5 | **K-220** |
| 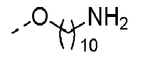 | 5 | 1 | **K-46** | 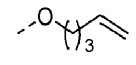 | 5 | 1 | **K-221** |
| | | 2 | **K-47** | | | 2 | **K-222** |
| | | 3 | **K-48** | | | 3 | **K-223** |
| | | 4 | **K-49** | | | 4 | **K-224** |
| | | 5 | **K-50** | | | 5 | **K-225** |
| 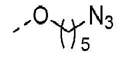 | 1 | 1 | **K-51** | 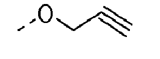 | 1 | 1 | **K-226** |
| | | 2 | **K-52** | | | 2 | **K-227** |
| | | 3 | **K-53** | | | 3 | **K-228** |
| | | 4 | **K-54** | | | 4 | **K-229** |
| | | 5 | **K-55** | | | 5 | **K-230** |
| 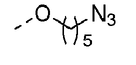 | 2 | 1 | **K-56** | 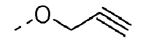 | 2 | 1 | **K-231** |
| | | 2 | **K-57** | | | 2 | **K-232** |
| | | 3 | **K-58** | | | 3 | **K-233** |
| | | 4 | **K-59** | | | 4 | **K-234** |
| | | 5 | **K-60** | | | 5 | **K-235** |
| 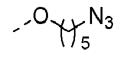 | 3 | 1 | **K-61** | 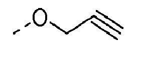 | 3 | 1 | **K-236** |
| | | 2 | **K-62** | | | 2 | **K-237** |
| | | 3 | **K-63** | | | 3 | **K-238** |
| | | 4 | **K-64** | | | 4 | **K-239** |
| | | 5 | **K-65** | | | 5 | **K-240** |
| 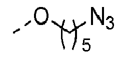 | 4 | 1 | **K-66** | 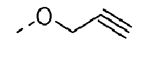 | 4 | 1 | **K-241** |
| | | 2 | **K-67** | | | 2 | **K-242** |
| | | 3 | **K-68** | | | 3 | **K-243** |
| | | 4 | **K-69** | | | 4 | **K-244** |
| | | 5 | **K-70** | | | 5 | **K-245** |
| 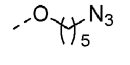 | 5 | 1 | **K-71** | 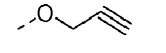 | 5 | 1 | **K-246** |
| | | 2 | **K-72** | | | 2 | **K-247** |
| | | 3 | **K-73** | | | 3 | **K-248** |
| | | 4 | **K-74** | | | 4 | **K-249** |
| | | 5 | **K-75** | | | 5 | **K-250** |
| 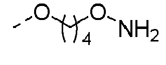 | 1 | 1 | **K-76** | 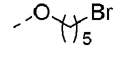 | 1 | 1 | **K-251** |
| | | 2 | **K-77** | | | 2 | **K-252** |
| | | 3 | **K-78** | | | 3 | **K-253** |
| | | 4 | **K-79** | | | 4 | **K-254** |
| | | 5 | **K-80** | | | 5 | **K-255** |
| 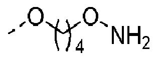 | 2 | 1 | **K-81** | 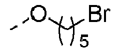 | 2 | 1 | **K-256** |
| | | 2 | **K-82** | | | 2 | **K-257** |
| | | 3 | **K-83** | | | 3 | **K-258** |
| | | 4 | **K-84** | | | 4 | **K-259** |
| | | 5 | **K-85** | | | 5 | **K-260** |
| 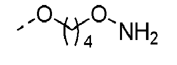 | 3 | 1 | **K-86** | 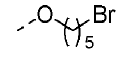 | 3 | 1 | **K-261** |
| | | 2 | **K-87** | | | 2 | **K-262** |
| | | 3 | **K-88** | | | 3 | **K-263** |
| | | 4 | **K-89** | | | 4 | **K-264** |
| | | 5 | **K-90** | | | 5 | **K-265** |
| 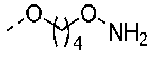 | 4 | 1 | **K-91** | 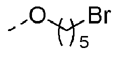 | 4 | 1 | **K-266** |
| | | 2 | **K-92** | | | 2 | **K-267** |
| | | 3 | **K-93** | | | 3 | **K-268** |
| | | 4 | **K-94** | | | 4 | **K-269** |
| | | 5 | **K-95** | | | 5 | **K-270** |
| 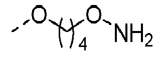 | 5 | 1 | **K-96** | 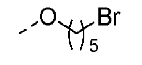 | 5 | 1 | **K-271** |
| | | 2 | **K-97** | | | 2 | **K-272** |
| | | 3 | **K-98** | | | 3 | **K-273** |
| | | 4 | **K-99** | | | 4 | **K-274** |
| | | 5 | **K-100** | | | 5 | **K-275** |
| 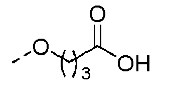 | 1 | 1 | **K-101** | 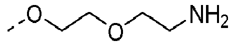 | 1 | 1 | **K-276** |
| | | 2 | **K-102** | | | 2 | **K-277** |
| | | 3 | **K-103** | | | 3 | **K-278** |
| | | 4 | **K-104** | | | 4 | **K-279** |
| | | 5 | **K-105** | | | 5 | **K-280** |
| 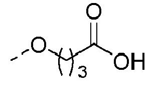 | 2 | 1 | **K-106** | 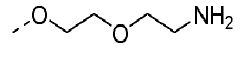 | 2 | 1 | **K-281** |
| | | 2 | **K-107** | | | 2 | **K-282** |
| | | 3 | **K-108** | | | 3 | **K-283** |
| | | 4 | **K-109** | | | 4 | **K-284** |
| | | 5 | **K-110** | | | 5 | **K-285** |
| 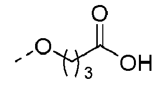 | 3 | 1 | **K-111** | 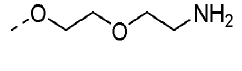 | 3 | 1 | **K-286** |
| | | 2 | **K-112** | | | 2 | **K-287** |
| | | 3 | **K-113** | | | 3 | **K-288** |
| | | 4 | **K-114** | | | 4 | **K-289** |
| | | 5 | **K-115** | | | 5 | **K-290** |
| 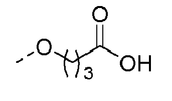 | 4 | 1 | **K-116** | 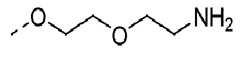 | 4 | 1 | **K-291** |
| | | 2 | **K-117** | | | 2 | **K-292** |
| | | 3 | **K-118** | | | 3 | **K-293** |
| | | 4 | **K-119** | | | 4 | **K-294** |
| | | 5 | **K-120** | | | 5 | **K-295** |
| 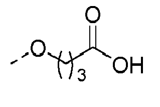 | 5 | 1 | **K-121** | 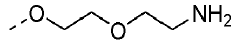 | 5 | 1 | **K-296** |
| | | 2 | **K-122** | | | 2 | **K-297** |
| | | 3 | **K-123** | | | 3 | **K-298** |
| | | 4 | **K-124** | | | 4 | **K-299** |
| | | 5 | **K-125** | | | 5 | **K-300** |
| 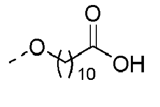 | 1 | 1 | **K-126** | 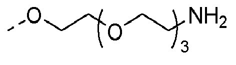 | 1 | 1 | **K-301** |
| | | 2 | **K-127** | | | 2 | **K-302** |
| | | 3 | **K-128** | | | 3 | **K-303** |
| | | 4 | **K-129** | | | 4 | **K-304** |
| | | 5 | **K-130** | | | 5 | **K-305** |
| 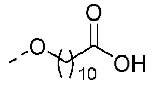 | 2 | 1 | **K-131** | 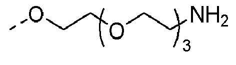 | 2 | 1 | **K-306** |
| | | 2 | **K-132** | | | 2 | **K-307** |
| | | 3 | **K-133** | | | 3 | **K-308** |
| | | 4 | **K-134** | | | 4 | **K-309** |
| | | 5 | **K-135** | | | 5 | **K-310** |
| 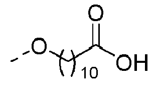 | 3 | 1 | **K-136** | 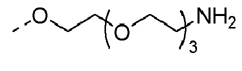 | 3 | 1 | **K-311** |
| | | 2 | **K-137** | | | 2 | **K-312** |
| | | 3 | **K-138** | | | 3 | **K-313** |
| | | 4 | **K-139** | | | 4 | **K-314** |
| | | 5 | **K-140** | | | 5 | **K-315** |
| 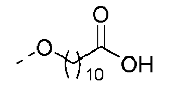 | 4 | 1 | **K-141** | 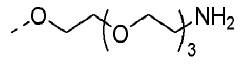 | 4 | 1 | **K-316** |
| | | 2 | **K-142** | | | 2 | **K-317** |
| | | 3 | **K-143** | | | 3 | **K-318** |
| | | 4 | **K-144** | | | 4 | **K-319** |
| | | 5 | **K-145** | | | 5 | **K-320** |
| 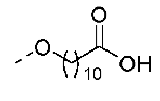 | 5 | 1 | **K-146** | 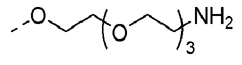 | 5 | 1 | **K-321** |
| | | 2 | **K-147** | | | 2 | **K-322** |
| | | 3 | **K-148** | | | 3 | **K-323** |
| | | 4 | **K-149** | | | 4 | **K-324** |
| | | 5 | **K-150** | | | 5 | **K-325** |
| 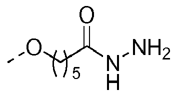 | 1 | 1 | **K-151** | 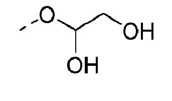 | 1 | 1 | **K-326** |
| | | 2 | **K-152** | | | 2 | **K-327** |
| | | 3 | **K-153** | | | 3 | **K-328** |
| | | 4 | **K-154** | | | 4 | **K-329** |
| | | 5 | **K-155** | | | 5 | **K-330** |
| 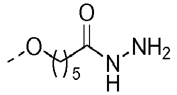 | 2 | 1 | **K-156** | 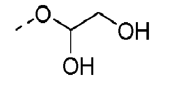 | 2 | 1 | **K-331** |
| | | 2 | **K-157** | | | 2 | **K-332** |
| | | 3 | **K-158** | | | 3 | **K-333** |
| | | 4 | **K-159** | | | 4 | **K-334** |
| | | 5 | **K-160** | | | 5 | **K-335** |
| 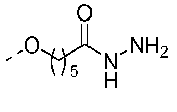 | 3 | 1 | **K-161** | 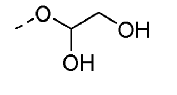 | 3 | 1 | **K-336** |
| | | 2 | **K-162** | | | 2 | **K-337** |
| | | 3 | **K-163** | | | 3 | **K-338** |
| | | 4 | **K-164** | | | 4 | **K-339** |
| | | 5 | **K-165** | | | 5 | **K-340** |
| 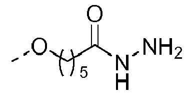 | 4 | 1 | **K-166** | 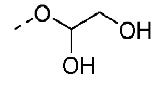 | 4 | 1 | **K-341** |
| | | 2 | **K-167** | | | 2 | **K-342** |
| | | 3 | **K-168** | | | 3 | **K-343** |
| | | 4 | **K-169** | | | 4 | **K-344** |
| | | 5 | **K-170** | | | 5 | **K-345** |
| 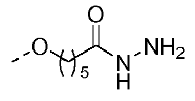 | 5 | 1 | **K-171** | 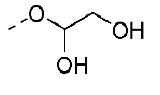 | 5 | 1 | **K-346** |
| | | 2 | **K-172** | | | 2 | **K-347** |
| | | 3 | **K-173** | | | 3 | **K-348** |
| | | 4 | **K-174** | | | 4 | **K-349** |
| | | 5 | **K-175** | | | 5 | **K-350** |

**Table 11**

| | | | | | |
|---|---|---|---|---|---|
| 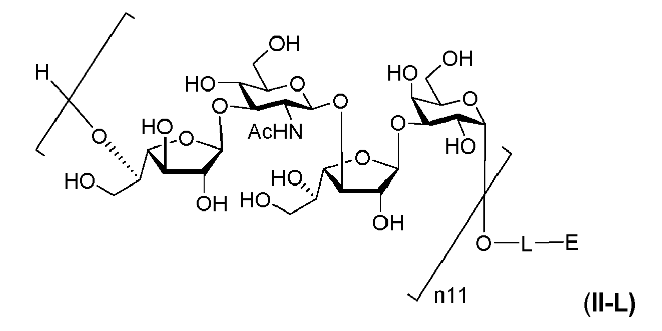 | | | | | |

| **-O-L-E** | n11 | **Compound No.** | **-O-L-E** | n11 | **Compound No.** |
|---|---|---|---|---|---|
| 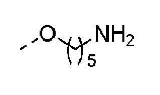 | 1 | **L-01** | 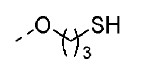 | 1 | **L-57** |
| | 2 | **L-02** | | 2 | **L-58** |
| | 3 | **L-03** | | 3 | **L-59** |
| | 4 | **L-04** | | 4 | **L-60** |
| | 5 | **L-05** | | 5 | **L-61** |
| | 6 | **L-06** | | 6 | **L-62** |
| | 7 | **L-07** | | 7 | **L-63** |
| | 8 | **L-08** | | 8 | **L-64** |
| 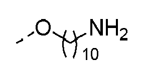 | 1 | **L-09** | 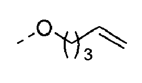 | 1 | **L-65** |
| | 2 | **L-10** | | 2 | **L-66** |
| | 3 | **L-11** | | 3 | **L-67** |
| | 4 | **L-12** | | 4 | **L-68** |
| | 5 | **L-13** | | 5 | **L-69** |
| | 6 | **L-14** | | 6 | **L-70** |
| | 7 | **L-15** | | 7 | **L-71** |
| | 8 | **L-16** | | 8 | **L-72** |
| 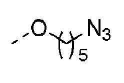 | 1 | **L-17** | 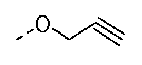 | 1 | **L-73** |
| | 2 | **L-18** | | 2 | **L-74** |
| | 3 | **L-19** | | 3 | **L-75** |
| | 4 | **L-20** | | 4 | **L-76** |
| | 5 | **L-21** | | 5 | **L-77** |
| | 6 | **L-22** | | 6 | **L-78** |
| | 7 | **L-23** | | 7 | **L-79** |
| | 8 | **L-24** | | 8 | **L-80** |
| 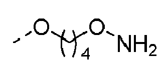 | 1 | **L-25** | 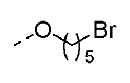 | 1 | **L-81** |
| | 2 | **L-26** | | 2 | **L-82** |
| | 3 | **L-27** | | 3 | **L-83** |
| | 4 | **L-28** | | 4 | **L-84** |
| | 5 | **L-29** | | 5 | **L-85** |
| | 6 | **L-30** | | 6 | **L-86** |
| | 7 | **L-31** | | 7 | **L-87** |
| | 8 | **L-32** | | 8 | **L-88** |
| 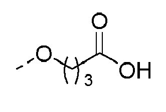 | 1 | **L-33** | 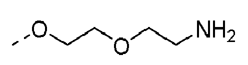 | 1 | **L-89** |
| | 2 | **L-34** | | 2 | **L-90** |
| | 3 | **L-35** | | 3 | **L-91** |
| | 4 | **L-36** | | 4 | **L-92** |
| | 5 | **L-37** | | 5 | **L-93** |
| | 6 | **L-38** | | 6 | **L-94** |
| | 7 | **L-39** | | 7 | **L-95** |
| | 8 | **L-40** | | 8 | **L-96** |
| 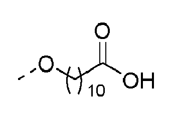 | 1 | **L-41** | 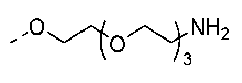 | 1 | **L-97** |
| | 2 | **L-42** | | 2 | **L-98** |
| | 3 | **L-43** | | 3 | **L-99** |
| | 4 | **L-44** | | 4 | **L-100** |
| | 5 | **L-45** | | 5 | **L-101** |
| | 6 | **L-46** | | 6 | **L-102** |
| | 7 | **L-47** | | 7 | **L-103** |
| | 8 | **L-48** | | 8 | **L-104** |
| 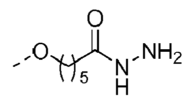 | 1 | **L-49** | 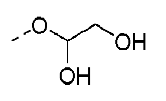 | 1 | **L-105** |
| | 2 | **L-50** | | 2 | **L-106** |
| | 3 | **L-51** | | 3 | **L-107** |
| | 4 | **L-52** | | 4 | **L-108** |
| | 5 | **L-53** | | 5 | **L-109** |
| | 6 | **L-54** | | 6 | **L-110** |
| | 7 | **L-55** | | 7 | **L-111** |
| | 8 | **L-56** | | 8 | **L-112** |

**Table 12**

| | | | | | |
|---|---|---|---|---|---|
| 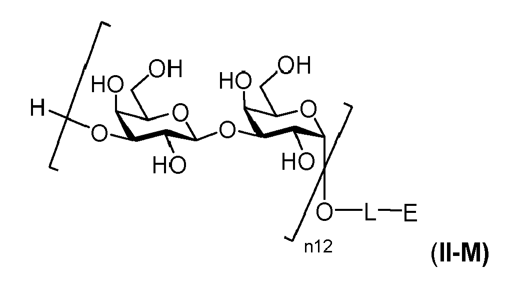 | | | | | |

| **-O-L-E** | n12 | **Compound No.** | **-O-L-E** | n12 | **Compound No.** |
|---|---|---|---|---|---|
| 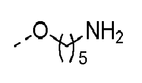 | 2 | **M-01** | 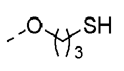 | 1 | **M-36** |
| | 3 | **M-02** | | 2 | **M-37** |
| | 4 | **M-03** | | 3 | **M-38** |
| | 5 | **M-04** | | 4 | **M-39** |
| | 6 | **M-05** | | 5 | **M-40** |
| 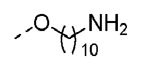 | 2 | **M-06** | 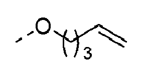 | 1 | **M-41** |
| | 3 | **M-07** | | 2 | **M-42** |
| | 4 | **M-08** | | 3 | **M-43** |
| | 5 | **M-09** | | 4 | **M-44** |
| | 6 | **M-10** | | 5 | **M-45** |
| 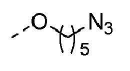 | 2 | **M-11** | 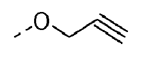 | 1 | **M-46** |
| | 3 | **M-12** | | 2 | **M-47** |
| | 4 | **M-13** | | 3 | **M-48** |
| | 5 | **M-14** | | 4 | **M-49** |
| | 6 | **M-15** | | 6 | **M-50** |
| 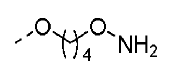 | 2 | **M-16** | 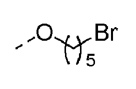 | 2 | **M-51** |
| | 3 | **M-17** | | 3 | **M-52** |
| | 4 | **M-18** | | 4 | **M-53** |
| | 5 | **M-19** | | 5 | **M-54** |
| | 6 | **M-20** | | 6 | **M-55** |
| 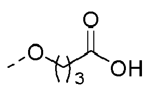 | 2 | **M-21** | 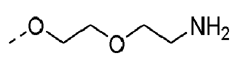 | 2 | **M-56** |
| | 3 | **M-22** | | 3 | **M-57** |
| | 4 | **M-23** | | 4 | **M-58** |
| | 5 | **M-24** | | 5 | **M-59** |
| | 6 | **M-25** | | 6 | **M-60** |
| 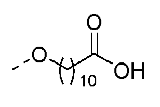 | 2 | **M-26** | 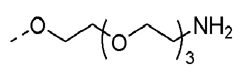 | 2 | **M-61** |
| | 3 | **M-27** | | 3 | **M-62** |
| | 4 | **M-28** | | 4 | **M-63** |
| | 5 | **M-29** | | 5 | **M-64** |
| | 6 | **M-30** | | 6 | **M-65** |
| 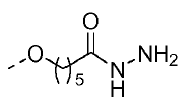 | 2 | **M-31** | 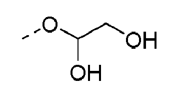 | 2 | **M-66** |
| | 3 | **M-32** | | 3 | **M-67** |
| | 4 | **M-33** | | 4 | **M-68** |
| | 5 | **M-34** | | 5 | **M-69** |
| | 6 | **M-35** | | 6 | **M-70** |

**Table 13**

| | | | | | |
|---|---|---|---|---|---|
| 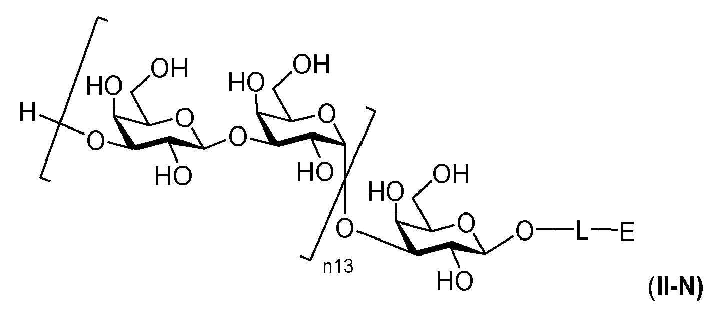 | | | | | |

| **-O-L-E** | n13 | **Compound No.** | **-O-L-E** | n13 | **Compound No.** |
|---|---|---|---|---|---|
| 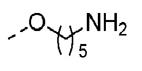 | 1 | **N-01** | 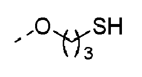 | 1 | **N-36** |
| | 2 | **N-02** | | 2 | **N-37** |
| | 3 | **N-03** | | 3 | **N-38** |
| | 4 | **N-04** | | 4 | **N-39** |
| | 5 | **N-05** | | 5 | **N-40** |
| 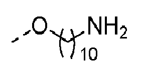 | 1 | **N-06** | 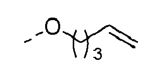 | 1 | **N-41** |
| | 2 | **N-07** | | 2 | **N-42** |
| | 3 | **N-08** | | 3 | **N-43** |
| | 4 | **N-09** | | 4 | **N-44** |
| | 5 | **N-10** | | 5 | **N-45** |
| 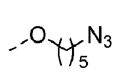 | 1 | **N-11** | 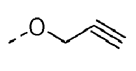 | 1 | **N-46** |
| | 2 | **N-12** | | 2 | **N-47** |
| | 3 | **N-13** | | 3 | **N-48** |
| | 4 | **N-14** | | 4 | **N-49** |
| | 5 | **N-15** | | 5 | **N-50** |
| 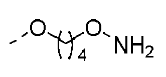 | 1 | **N-16** | 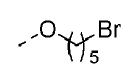 | 1 | **N-51** |
| | 2 | **N-17** | | 2 | **N-52** |
| | 3 | **N-18** | | 3 | **N-53** |
| | 4 | **N-19** | | 4 | **N-54** |
| | 5 | **N-20** | | 5 | **N-55** |
| 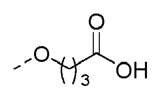 | 1 | **N-21** | 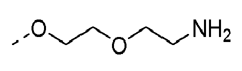 | 1 | **N-56** |
| | 2 | **N-22** | | 2 | **N-57** |
| | 3 | **N-23** | | 3 | **N-58** |
| | 4 | **N-24** | | 4 | **N-59** |
| | 5 | **N-25** | | 5 | **N-60** |
| 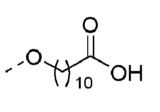 | 1 | **N-26** | 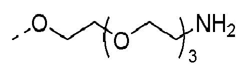 | 1 | **N-61** |
| | 2 | **N-27** | | 2 | **N-62** |
| | 3 | **N-28** | | 3 | **N-63** |
| | 4 | **N-29** | | 4 | **N-64** |
| | 5 | **N-30** | | 5 | **N-65** |
| 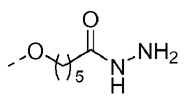 | 1 | **N-31** | 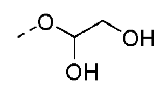 | 1 | **N-66** |
| | 2 | **N-32** | | 2 | **N-67** |
| | 3 | **N-33** | | 3 | **N-68** |
| | 4 | **N-34** | | 4 | **N-69** |
| | 5 | **N-35** | | 5 | **N-70** |

**Table 14**

| | | | | | |
|---|---|---|---|---|---|
| 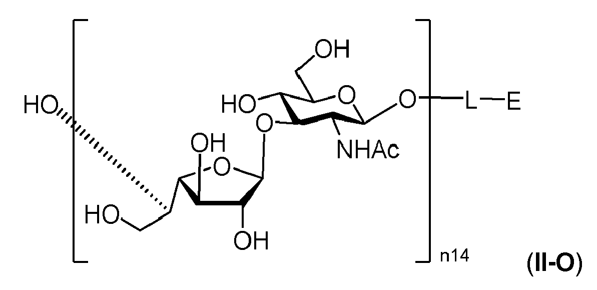 | | | | | |

| **-O-L-E** | n14 | **Compound No.** | **-O-L-E** | n14 | **Compound No.** |
|---|---|---|---|---|---|
| 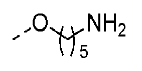 | 2 | **O-01** | 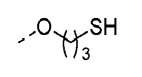 | 2 | **O-36** |
| | 3 | **O-02** | | 3 | **O-37** |
| | 4 | **O-03** | | 4 | **O-38** |
| | 5 | **O-04** | | 5 | **O-39** |
| | 6 | **O-05** | | 6 | **O-40** |
| 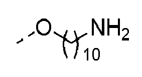 | 2 | **O-06** | 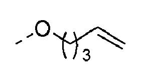 | 2 | **O-41** |
| | 3 | **O-07** | | 3 | **O-42** |
| | 4 | **O-08** | | 4 | **O-43** |
| | 5 | **O-09** | | 5 | **O-44** |
| | 6 | **O-10** | | 6 | **O-45** |
| 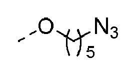 | 2 | **O-11** | 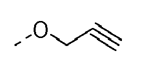 | 2 | **O-46** |
| | 3 | **O-12** | | 3 | **O-47** |
| | 4 | **O-13** | | 4 | **O-48** |
| | 5 | **O-14** | | 5 | **O-49** |
| | 6 | **O-15** | | 6 | **O-50** |
| 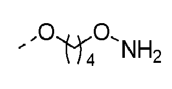 | 2 | **O-16** | 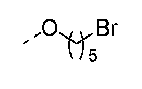 | 2 | **O-51** |
| | 3 | **O-17** | | 3 | **O-52** |
| | 4 | **O-18** | | 4 | **O-53** |
| | 5 | **O-19** | | 5 | **O-54** |
| | 6 | **O-20** | | 6 | **O-55** |
| 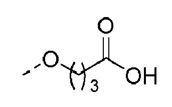 | 2 | **O-21** | 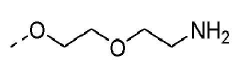 | 2 | **O-56** |
| | 3 | **O-22** | | 3 | **O-57** |
| | 4 | **O-23** | | 4 | **O-58** |
| | 5 | **O-24** | | 5 | **O-59** |
| | 6 | **O-25** | | 6 | **O-60** |
| 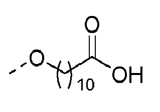 | 2 | **O-26** | 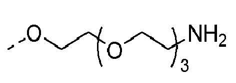 | 2 | **O-61** |
| | 3 | **O-27** | | 3 | **O-62** |
| | 4 | **O-28** | | 4 | **O-63** |
| | 5 | **O-29** | | 5 | **O-64** |
| | 6 | **O-30** | | 6 | **O-65** |
| 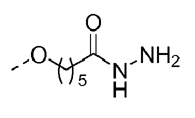 | 2 | **O-31** | 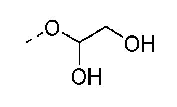 | 2 | **O-66** |
| | 3 | **O-32** | | 3 | **O-67** |
| | 4 | **O-33** | | 4 | **O-68** |
| | 5 | **O-34** | | 5 | **O-69** |
| | 6 | **O-35** | | 6 | **O-70** |

**Table 15**

| | | | | | |
|---|---|---|---|---|---|
| 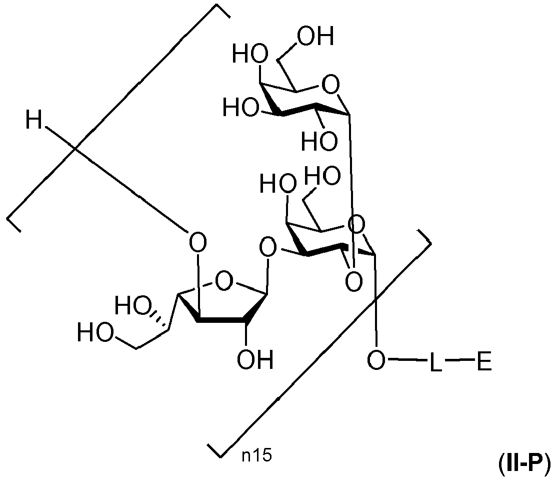 | | | | | |

| **-O-L-E** | n15 | **Compound No.** | **-O-L-E** | n15 | **Compound No.** |
|---|---|---|---|---|---|
| 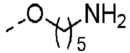 | 1 | **P-01** | 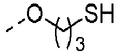 | 1 | **P 36** |
| | 2 | **P-02** | | 2 | **P 37** |
| | 3 | **P-03** | | 3 | **P 38** |
| | 4 | **P-04** | | 4 | **P 39** |
| | 5 | **P-05** | | 5 | **P 40** |
| 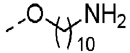 | 1 | **P-06** | 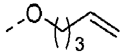 | 1 | **P-41** |
| | 2 | **P-07** | | 2 | **P-42** |
| | 3 | **P-08** | | 3 | **P-43** |
| | 4 | **P-09** | | 4 | **P-44** |
| | 5 | **P-10** | | 5 | **P-45** |
| 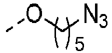 | 1 | **P-11** | 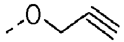 | 1 | **P-46** |
| | 2 | **P-12** | | 2 | **P-47** |
| | 3 | **P-13** | | 3 | **P-48** |
| | 4 | **P-14** | | 4 | **P-49** |
| | 5 | **P-15** | | 5 | **P-50** |
| 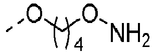 | 1 | **P-16** | 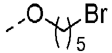 | 1 | **P-51** |
| | 2 | **P-17** | | 2 | **P-52** |
| | 3 | **P-18** | | 3 | **P-53** |
| | 4 | **P-19** | | 4 | **P-54** |
| | 5 | **P-20** | | 5 | **P-55** |
| 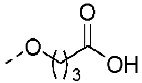 | 1 | **P-21** | 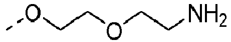 | 1 | **P-56** |
| | 2 | **P-22** | | 2 | **P-57** |
| | 3 | **P-23** | | 3 | **P-58** |
| | 4 | **P-24** | | 4 | **P-59** |
| | 5 | **P-25** | | 5 | **P-60** |
| 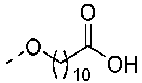 | 1 | **P-26** | 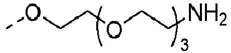 | 1 | **P-61** |
| | 2 | **P-27** | | 2 | **P-62** |
| | 3 | **P-28** | | 3 | **P-63** |
| | 4 | **P-29** | | 4 | **P-64** |
| | 5 | **P-30** | | 5 | **P-65** |
| 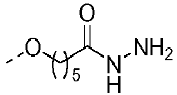 | 1 | **P-31** | 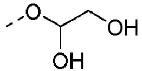 | 1 | **P-66** |
| | 2 | **P-32** | | 2 | **P-67** |
| | 3 | **P-33** | | 3 | **P-68** |
| | 4 | **P-34** | | 4 | **P-69** |
| | 5 | **P-35** | | 5 | **P-70** |

**Table 16**

| | | | | | | | | | |
|---|---|---|---|---|---|---|---|---|---|
| 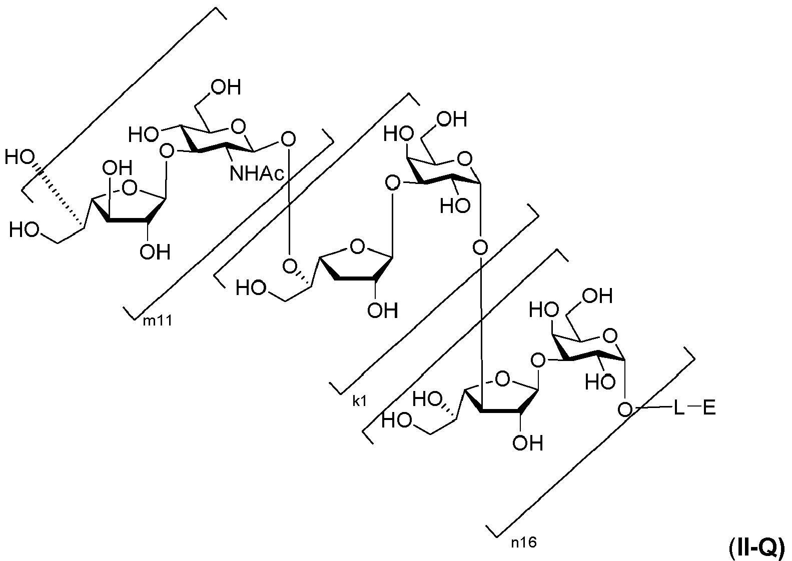 | | | | | | | | | |

| **-O-L-E** | m11 | k1 | n16 | **Compd. No.** | **-O-L-E** | m11 | k1 | n16 | **Compd. No.** |
|---|---|---|---|---|---|---|---|---|---|
| 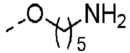 | 1 | 1 | 1 | **Q-1** | 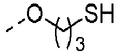 | 1 | 1 | 1 | **Q-351** |
| | | | 2 | **Q-2** | | | | 2 | **Q-352** |
| | | | 3 | **Q-3** | | | | 3 | **Q-353** |
| | | | 4 | **Q-4** | | | | 4 | **Q-354** |
| | | | 5 | **Q-5** | | | | 5 | **Q-355** |
| 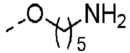 | 2 | 1 | 1 | **Q-6** | 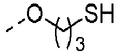 | 2 | 1 | 1 | **Q-356** |
| | | | 2 | **Q-7** | | | | 2 | **Q-357** |
| | | | 3 | **Q-8** | | | | 3 | **Q-358** |
| | | | 4 | **Q-9** | | | | 4 | **Q-359** |
| | | | 5 | **Q-10** | | | | 5 | **Q-360** |
| 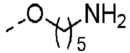 | 3 | 1 | 1 | **Q-11** | 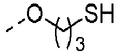 | 3 | 1 | 1 | **Q-361** |
| | | | 2 | **Q-12** | | | | 2 | **Q-362** |
| | | | 3 | **Q-13** | | | | 3 | **Q-363** |
| | | | 4 | **Q-14** | | | | 4 | **Q-364** |
| | | | 5 | **Q-15** | | | | 5 | **Q-365** |
| 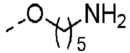 | 4 | 1 | 1 | **Q-16** | 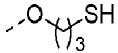 | 4 | 1 | 1 | **Q-366** |
| | | | 2 | **Q-17** | | | | 2 | **Q-367** |
| | | | 3 | **Q-18** | | | | 3 | **Q-368** |
| | | | 4 | **Q-19** | | | | 4 | **Q-369** |
| | | | 5 | **Q-20** | | | | 5 | **Q-370** |
| 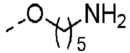 | 5 | 1 | 1 | **Q-21** | 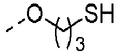 | 5 | 1 | 1 | **Q-371** |
| | | | 2 | **Q-22** | | | | 2 | **Q-372** |
| | | | 3 | **Q-23** | | | | 3 | **Q-373** |
| | | | 4 | **Q-24** | | | | 4 | **Q-374** |
| | | | 5 | **Q-25** | | | | 5 | **Q-375** |
| 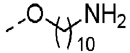 | 1 | 1 | 1 | **Q-26** | 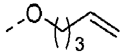 | 1 | 1 | 1 | **Q-376** |
| | | | 2 | **Q-27** | | | | 2 | **Q-377** |
| | | | 3 | **Q-28** | | | | 3 | **Q-378** |
| | | | 4 | **Q-29** | | | | 4 | **Q-379** |
| | | | 5 | **Q-30** | | | | 5 | **Q-380** |
| 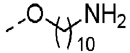 | 2 | 1 | 1 | **Q-31** | 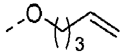 | 2 | 1 | 1 | **Q-381** |
| | | | 2 | **Q-32** | | | | 2 | **Q-382** |
| | | | 3 | **Q-33** | | | | 3 | **Q-383** |
| | | | 4 | **Q-34** | | | | 4 | **Q-384** |
| | | | 5 | **Q-35** | | | | 5 | **Q-385** |
| 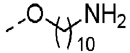 | 3 | 1 | 1 | **Q-36** | 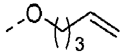 | 3 | 1 | 1 | **Q-386** |
| | | | 2 | **Q-37** | | | | 2 | **Q-387** |
| | | | 3 | **Q-38** | | | | 3 | **Q-388** |
| | | | 4 | **Q-39** | | | | 4 | **Q-389** |
| | | | 5 | **Q-40** | | | | 5 | **Q-390** |
| 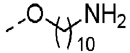 | 4 | 1 | 1 | **Q-41** | 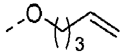 | 4 | 1 | 1 | **Q-391** |
| | | | 2 | **Q-42** | | | | 2 | **Q-392** |
| | | | 3 | **Q-43** | | | | 3 | **Q-393** |
| | | | 4 | **Q-44** | | | | 4 | **Q-394** |
| | | | 5 | **Q-45** | | | | 5 | **Q-395** |
| 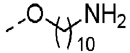 | 5 | 1 | 1 | **Q-46** | 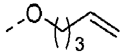 | 5 | 1 | 1 | **Q-396** |
| | | | 2 | **Q-47** | | | | 2 | **Q-397** |
| | | | 3 | **Q-48** | | | | 3 | **Q-398** |
| | | | 4 | **Q-49** | | | | 4 | **Q-399** |
| | | | 5 | **Q-50** | | | | 5 | **Q-400** |
| 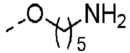 | 1 | 2 | 1 | **Q-51** | 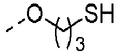 | 1 | 2 | 1 | **Q-401** |
| | | | 2 | **Q-52** | | | | 2 | **Q-402** |
| | | | 3 | **Q-53** | | | | 3 | **Q-403** |
| | | | 4 | **Q-54** | | | | 4 | **Q-404** |
| | | | 5 | **Q-55** | | | | 5 | **Q-405** |
| 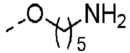 | 2 | 2 | 1 | **Q-56** | 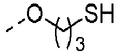 | 2 | 2 | 1 | **Q-406** |
| | | | 2 | **Q-57** | | | | 2 | **Q-407** |
| | | | 3 | **Q-58** | | | | 3 | **Q-408** |
| | | | 4 | **Q-59** | | | | 4 | **Q-409** |
| | | | 5 | **Q-60** | | | | 5 | **Q-410** |
| 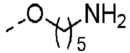 | 3 | 2 | 1 | **Q-61** | 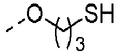 | 3 | 2 | 1 | **Q-411** |
| | | | 2 | **Q-62** | | | | 2 | **Q-412** |
| | | | 3 | **Q-63** | | | | 3 | **Q-413** |
| | | | 4 | **Q-64** | | | | 4 | **Q-414** |
| | | | 5 | **Q-65** | | | | 5 | **Q-415** |
| 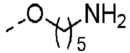 | 4 | 2 | 1 | **Q-66** | 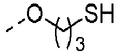 | 4 | 2 | 1 | **Q-416** |
| | | | 2 | **Q-67** | | | | 2 | **Q-417** |
| | | | 3 | **Q-68** | | | | 3 | **Q-418** |
| | | | 4 | **Q-69** | | | | 4 | **Q-419** |
| | | | 5 | **Q-70** | | | | 5 | **Q-420** |
| 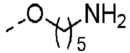 | 5 | 2 | 1 | **Q-71** | 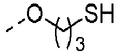 | 5 | 2 | 1 | **Q-421** |
| | | | 2 | **Q-72** | | | | 2 | **Q-422** |
| | | | 3 | **Q-73** | | | | 3 | **Q-423** |
| | | | 4 | **Q-74** | | | | 4 | **Q-424** |
| | | | 5 | **Q-75** | | | | 5 | **Q-425** |
| 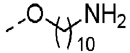 | 1 | 2 | 1 | **Q-76** | 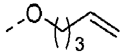 | 1 | 2 | 1 | **Q-426** |
| | | | 2 | **Q-77** | | | | 2 | **Q-427** |
| | | | 3 | **Q-78** | | | | 3 | **Q-428** |
| | | | 4 | **Q-79** | | | | 4 | **Q-429** |
| | | | 5 | **Q-80** | | | | 5 | **Q-430** |
| 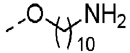 | 2 | 2 | 1 | **Q-81** | 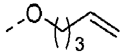 | 2 | 2 | 1 | **Q-431** |
| | | | 2 | **Q-82** | | | | 2 | **Q-432** |
| | | | 3 | **Q-83** | | | | 3 | **Q-433** |
| | | | 4 | **Q-84** | | | | 4 | **Q-434** |
| | | | 5 | **Q-85** | | | | 5 | **Q-435** |
| 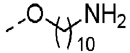 | 3 | 2 | 1 | **Q-86** | 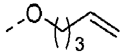 | 3 | 2 | 1 | **Q-436** |
| | | | 2 | **Q-87** | | | | 2 | **Q-437** |
| | | | 3 | **Q-88** | | | | 3 | **Q-438** |
| | | | 4 | **Q-89** | | | | 4 | **Q-439** |
| | | | 5 | **Q-90** | | | | 5 | **Q-440** |
| 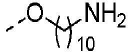 | 4 | 2 | 1 | **Q-91** | 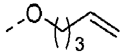 | 4 | 2 | 1 | **Q-441** |
| | | | 2 | **Q-92** | | | | 2 | **Q-442** |
| | | | 3 | **Q-93** | | | | 3 | **Q-443** |
| | | | 4 | **Q-94** | | | | 4 | **Q-444** |
| | | | 5 | **Q-95** | | | | 5 | **Q-445** |
| 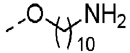 | 5 | 2 | 1 | **Q-96** | 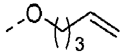 | 5 | 2 | 1 | **Q-446** |
| | | | 2 | **Q-97** | | | | 2 | **Q-447** |
| | | | 3 | **Q-98** | | | | 3 | **Q-448** |
| | | | 4 | **Q-99** | | | | 4 | **Q-449** |
| | | | 5 | **Q-100** | | | | 5 | **Q-450** |
| 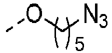 | 1 | 1 | 1 | **Q-101** | 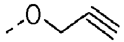 | 1 | 1 | 1 | **Q-451** |
| | | | 2 | **Q-102** | | | | 2 | **Q-452** |
| | | | 3 | **Q-103** | | | | 3 | **Q-453** |
| | | | 4 | **Q-104** | | | | 4 | **Q-454** |
| | | | 5 | **Q-105** | | | | 5 | **Q-455** |
| 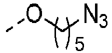 | 2 | 1 | 1 | **Q-106** | 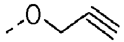 | 2 | 1 | 1 | **Q-456** |
| | | | 2 | **Q-107** | | | | 2 | **Q-457** |
| | | | 3 | **Q-108** | | | | 3 | **Q-458** |
| | | | 4 | **Q-109** | | | | 4 | **Q-459** |
| | | | 5 | **Q-110** | | | | 5 | **Q-460** |
| 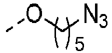 | 3 | 1 | 1 | **Q-111** | 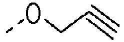 | 3 | 1 | 1 | **Q-461** |
| | | | 2 | **Q-112** | | | | 2 | **Q-462** |
| | | | 3 | **Q-113** | | | | 3 | **Q-463** |
| | | | 4 | **Q-114** | | | | 4 | **Q-464** |
| | | | 5 | **Q-115** | | | | 5 | **Q-465** |
| 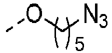 | 4 | 1 | 1 | **Q-116** | 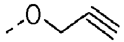 | 4 | 1 | 1 | **Q-466** |
| | | | 2 | **Q-117** | | | | 2 | **Q-467** |
| | | | 3 | **Q-118** | | | | 3 | **Q-468** |
| | | | 4 | **Q-119** | | | | 4 | **Q-469** |
| | | | 5 | **Q-120** | | | | 5 | **Q-470** |
| 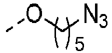 | 5 | 1 | 1 | **Q-121** | 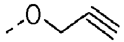 | 5 | 1 | 1 | **Q-471** |
| | | | 2 | **Q-122** | | | | 2 | **Q-472** |
| | | | 3 | **Q-123** | | | | 3 | **Q-473** |
| | | | 4 | **Q-124** | | | | 4 | **Q-474** |
| | | | 5 | **Q-125** | | | | 5 | **Q-475** |
| 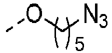 | 1 | 2 | 1 | **Q-126** | 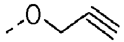 | 1 | 2 | 1 | **Q-476** |
| | | | 2 | **Q-127** | | | | 2 | **Q-477** |
| | | | 3 | **Q-128** | | | | 3 | **Q-478** |
| | | | 4 | **Q-129** | | | | 4 | **Q-479** |
| | | | 5 | **Q-130** | | | | 5 | **Q-480** |
| 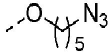 | 2 | 2 | 1 | **Q-131** | 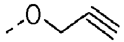 | 2 | 2 | 1 | **Q-481** |
| | | | 2 | **Q-132** | | | | 2 | **Q-482** |
| | | | 3 | **Q-133** | | | | 3 | **Q-483** |
| | | | 4 | **Q-134** | | | | 4 | **Q-484** |
| | | | 5 | **Q-135** | | | | 5 | **Q-485** |
| 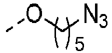 | 3 | 2 | 1 | **Q-136** | 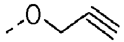 | 3 | 2 | 1 | **Q-486** |
| | | | 2 | **Q-137** | | | | 2 | **Q-487** |
| | | | 3 | **Q-138** | | | | 3 | **Q-488** |
| | | | 4 | **Q-139** | | | | 4 | **Q-489** |
| | | | 5 | **Q-140** | | | | 5 | **Q-490** |
| 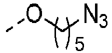 | 4 | 2 | 1 | **Q-141** | 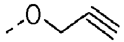 | 4 | 2 | 1 | **Q-491** |
| | | | 2 | **Q-142** | | | | 2 | **Q-492** |
| | | | 3 | **Q-143** | | | | 3 | **Q-493** |
| | | | 4 | **Q-144** | | | | 4 | **Q-494** |
| | | | 5 | **Q-145** | | | | 5 | **Q-495** |
| 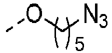 | 5 | 2 | 1 | **Q-146** | 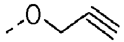 | 5 | 2 | 1 | **Q-496** |
| | | | 2 | **Q-147** | | | | 2 | **Q-497** |
| | | | 3 | **Q-148** | | | | 3 | **Q-498** |
| | | | 4 | **Q-149** | | | | 4 | **Q-499** |
| | | | 5 | **Q-150** | | | | 5 | **Q-500** |
| 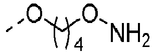 | 1 | 1 | 1 | **Q-151** | 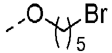 | 1 | 1 | 1 | **Q-501** |
| | | | 2 | **Q-152** | | | | 2 | **Q-502** |
| | | | 3 | **Q-153** | | | | 3 | **Q-503** |
| | | | 4 | **Q-154** | | | | 4 | **Q-504** |
| | | | 5 | **Q-155** | | | | 5 | **Q-505** |
| 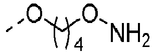 | 2 | 1 | 1 | **Q-156** | 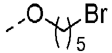 | 2 | 1 | 1 | **Q-506** |
| | | | 2 | **Q-157** | | | | 2 | **Q-507** |
| | | | 3 | **Q-158** | | | | 3 | **Q-508** |
| | | | 4 | **Q-159** | | | | 4 | **Q-509** |
| | | | 5 | **Q-160** | | | | 5 | **Q-510** |
| 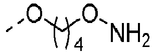 | 3 | 1 | 1 | **Q-161** | 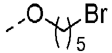 | 3 | 1 | 1 | **Q-511** |
| | | | 2 | **Q-162** | | | | 2 | **Q-512** |
| | | | 3 | **Q-163** | | | | 3 | **Q-513** |
| | | | 4 | **Q-164** | | | | 4 | **Q-514** |
| | | | 5 | **Q-165** | | | | 5 | **Q-515** |
| 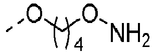 | 4 | 1 | 1 | **Q-166** | 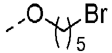 | 4 | 1 | 1 | **Q-516** |
| | | | 2 | **Q-167** | | | | 2 | **Q-517** |
| | | | 3 | **Q-168** | | | | 3 | **Q-518** |
| | | | 4 | **Q-169** | | | | 4 | **Q-519** |
| | | | 5 | **Q-170** | | | | 5 | **Q-520** |
| 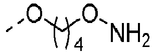 | 5 | 1 | 1 | **Q-171** | 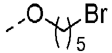 | 5 | 1 | 1 | **Q-521** |
| | | | 2 | **Q-172** | | | | 2 | **Q-522** |
| | | | 3 | **Q-173** | | | | 3 | **Q-523** |
| | | | 4 | **Q-174** | | | | 4 | **Q-524** |
| | | | 5 | **Q-175** | | | | 5 | **Q-525** |
| 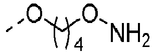 | 1 | 2 | 1 | **Q-176** | 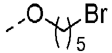 | 1 | 2 | 1 | **Q-526** |
| | | | 2 | **Q-177** | | | | 2 | **Q-527** |
| | | | 3 | **Q-178** | | | | 3 | **Q-528** |
| | | | 4 | **Q-179** | | | | 4 | **Q-529** |
| | | | 5 | **Q-180** | | | | 5 | **Q-530** |
| 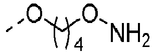 | 2 | 2 | 1 | **Q-181** | 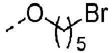 | 2 | 2 | 1 | **Q-531** |
| | | | 2 | **Q-182** | | | | 2 | **Q-532** |
| | | | 3 | **Q-183** | | | | 3 | **Q-533** |
| | | | 4 | **Q-184** | | | | 4 | **Q-534** |
| | | | 5 | **Q-185** | | | | 5 | **Q-535** |
| 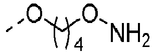 | 3 | 2 | 1 | **Q-186** | 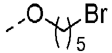 | 3 | 2 | 1 | **Q-536** |
| | | | 2 | **Q-187** | | | | 2 | **Q-537** |
| | | | 3 | **Q-188** | | | | 3 | **Q-538** |
| | | | 4 | **Q-189** | | | | 4 | **Q-539** |
| | | | 5 | **Q-190** | | | | 5 | **Q-540** |
| 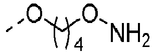 | 4 | 2 | 1 | **Q-191** | 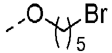 | 4 | 2 | 1 | **Q-541** |
| | | | 2 | **Q-192** | | | | 2 | **Q-542** |
| | | | 3 | **Q-193** | | | | 3 | **Q-543** |
| | | | 4 | **Q-194** | | | | 4 | **Q-544** |
| | | | 5 | **Q-195** | | | | 5 | **Q-545** |
| 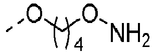 | 5 | 2 | 1 | **Q-196** | 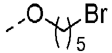 | 5 | 2 | 1 | **Q-546** |
| | | | 2 | **Q-197** | | | | 2 | **Q-547** |
| | | | 3 | **Q-198** | | | | 3 | **Q-548** |
| | | | 4 | **Q-199** | | | | 4 | **Q-549** |
| | | | 5 | **Q-200** | | | | 5 | **Q-550** |
| 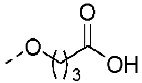 | 1 | 1 | 1 | **Q-201** | 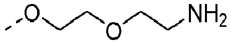 | 1 | 1 | 1 | **Q-551** |
| | | | 2 | **Q-202** | | | | 2 | **Q-552** |
| | | | 3 | **Q-203** | | | | 3 | **Q-553** |
| | | | 4 | **Q-204** | | | | 4 | **Q-554** |
| | | | 5 | **Q-205** | | | | 5 | **Q-555** |
| 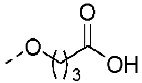 | 2 | 1 | 1 | **Q-206** | 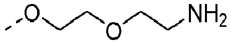 | 2 | 1 | 1 | **Q-556** |
| | | | 2 | **Q-207** | | | | 2 | **Q-557** |
| | | | 3 | **Q-208** | | | | 3 | **Q-558** |
| | | | 4 | **Q-209** | | | | 4 | **Q-559** |
| | | | 5 | **Q-210** | | | | 5 | **Q-560** |
| 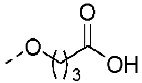 | 3 | 1 | 1 | **Q-211** | 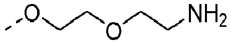 | 3 | 1 | 1 | **Q-561** |
| | | | 2 | **Q-212** | | | | 2 | **Q-562** |
| | | | 3 | **Q-213** | | | | 3 | **Q-563** |
| | | | 4 | **Q-214** | | | | 4 | **Q-564** |
| | | | 5 | **Q-215** | | | | 5 | **Q-565** |
| 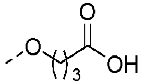 | 4 | 1 | 1 | **Q-216** | 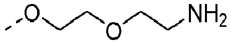 | 4 | 1 | 1 | **Q-566** |
| | | | 2 | **Q-217** | | | | 2 | **Q-567** |
| | | | 3 | **Q-218** | | | | 3 | **Q-568** |
| | | | 4 | **Q-219** | | | | 4 | **Q-569** |
| | | | 5 | **Q-220** | | | | 5 | **Q-570** |
| 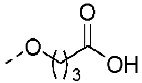 | 5 | 1 | 1 | **Q-221** | 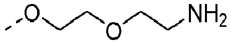 | 5 | 1 | 1 | **Q-571** |
| | | | 2 | **Q-222** | | | | 2 | **Q-572** |
| | | | 3 | **Q-223** | | | | 3 | **Q-573** |
| | | | 4 | **Q-224** | | | | 4 | **Q-574** |
| | | | 5 | **Q-225** | | | | 5 | **Q-575** |
| 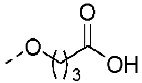 | 1 | 2 | 1 | **Q-226** | 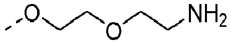 | 1 | 2 | 1 | **Q-576** |
| | | | 2 | **Q-227** | | | | 2 | **Q-577** |
| | | | 3 | **Q-228** | | | | 3 | **Q-578** |
| | | | 4 | **Q-229** | | | | 4 | **Q-579** |
| | | | 5 | **Q-230** | | | | 5 | **Q-580** |
| 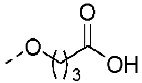 | 2 | 2 | 1 | **Q-231** | 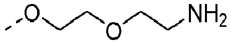 | 2 | 2 | 1 | **Q-581** |
| | | | 2 | **Q-232** | | | | 2 | **Q-582** |
| | | | 3 | **Q-233** | | | | 3 | **Q-583** |
| | | | 4 | **Q-234** | | | | 4 | **Q-584** |
| | | | 5 | **Q-235** | | | | 5 | **Q-585** |
| 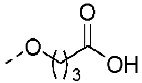 | 3 | 2 | 1 | **Q-236** | 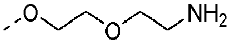 | 3 | 2 | 1 | **Q-586** |
| | | | 2 | **Q-237** | | | | 2 | **Q-587** |
| | | | 3 | **Q-238** | | | | 3 | **Q-588** |
| | | | 4 | **Q-239** | | | | 4 | **Q-589** |
| | | | 5 | **Q-240** | | | | 5 | **Q-590** |
| 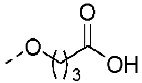 | 4 | 2 | 1 | **Q-241** | 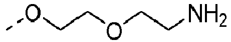 | 4 | 2 | 1 | **Q-591** |
| | | | 2 | **Q-242** | | | | 2 | **Q-592** |
| | | | 3 | **Q-243** | | | | 3 | **Q-593** |
| | | | 4 | **Q-244** | | | | 4 | **Q-594** |
| | | | 5 | **Q-245** | | | | 5 | **Q-595** |
| 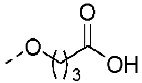 | 5 | 2 | 1 | **Q-246** | 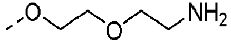 | 5 | 2 | 1 | **Q-596** |
| | | | 2 | **Q-247** | | | | 2 | **Q-597** |
| | | | 3 | **Q-248** | | | | 3 | **Q-598** |
| | | | 4 | **Q-249** | | | | 4 | **Q-599** |
| | | | 5 | **Q-250** | | | | 5 | **Q-600** |
| 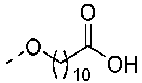 | 1 | 1 | 1 | **Q-251** | 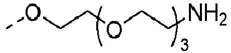 | 1 | 1 | 1 | **Q-601** |
| | | | 2 | **Q-252** | | | | 2 | **Q-602** |
| | | | 3 | **Q-253** | | | | 3 | **Q-603** |
| | | | 4 | **Q-254** | | | | 4 | **Q-604** |
| | | | 5 | **Q-255** | | | | 5 | **Q-605** |
| 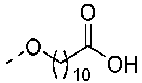 | 2 | 1 | 1 | **Q-256** | 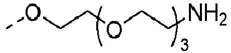 | 2 | 1 | 1 | **Q-606** |
| | | | 2 | **Q-257** | | | | 2 | **Q-607** |
| | | | 3 | **Q-258** | | | | 3 | **Q-608** |
| | | | 4 | **Q-259** | | | | 4 | **Q-609** |
| | | | 5 | **Q-260** | | | | 5 | **Q-610** |
| 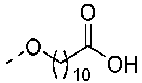 | 3 | 1 | 1 | **Q-261** | 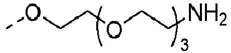 | 3 | 1 | 1 | **Q-611** |
| | | | 2 | **Q-262** | | | | 2 | **Q-612** |
| | | | 3 | **Q-263** | | | | 3 | **Q-613** |
| | | | 4 | **Q-264** | | | | 4 | **Q-614** |
| | | | 5 | **Q-265** | | | | 5 | **Q-615** |
| 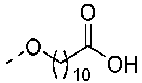 | 4 | 1 | 1 | **Q-266** | 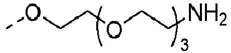 | 4 | 1 | 1 | **Q-616** |
| | | | 2 | **Q-267** | | | | 2 | **Q-617** |
| | | | 3 | **Q-268** | | | | 3 | **Q-618** |
| | | | 4 | **Q-269** | | | | 4 | **Q-619** |
| | | | 5 | **Q-270** | | | | 5 | **Q-620** |
| 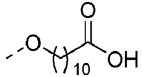 | 5 | 1 | 1 | **Q-271** | 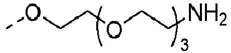 | 5 | 1 | 1 | **Q-621** |
| | | | 2 | **Q-272** | | | | 2 | **Q-622** |
| | | | 3 | **Q-273** | | | | 3 | **Q-623** |
| | | | 4 | **Q-274** | | | | 4 | **Q-624** |
| | | | 5 | **Q-275** | | | | 5 | **Q-625** |
| 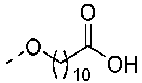 | 1 | 2 | 1 | **Q-276** | 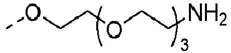 | 1 | 2 | 1 | **Q-626** |
| | | | 2 | **Q-277** | | | | 2 | **Q-627** |
| | | | 3 | **Q-278** | | | | 3 | **Q-628** |
| | | | 4 | **Q-279** | | | | 4 | **Q-629** |
| | | | 5 | **Q-280** | | | | 5 | **Q-630** |
| 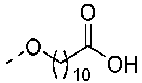 | 2 | 2 | 1 | **Q-281** | 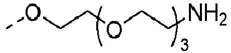 | 2 | 2 | 1 | **Q-631** |
| | | | 2 | **Q-282** | | | | 2 | **Q-632** |
| | | | 3 | **Q-283** | | | | 3 | **Q-633** |
| | | | 4 | **Q-284** | | | | 4 | **Q-634** |
| | | | 5 | **Q-285** | | | | 5 | **Q-635** |
| 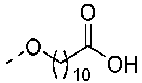 | 3 | 2 | 1 | **Q-286** | 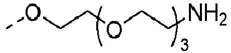 | 3 | 2 | 1 | **Q-636** |
| | | | 2 | **Q-287** | | | | 2 | **Q-637** |
| | | | 3 | **Q-288** | | | | 3 | **Q-638** |
| | | | 4 | **Q-289** | | | | 4 | **Q-639** |
| | | | 5 | **Q-290** | | | | 5 | **Q-640** |
| 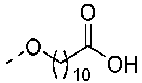 | 4 | 2 | 1 | **Q-291** | 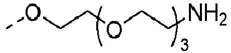 | 4 | 2 | 1 | **Q-641** |
| | | | 2 | **Q-292** | | | | 2 | **Q-642** |
| | | | 3 | **Q-293** | | | | 3 | **Q-643** |
| | | | 4 | **Q-294** | | | | 4 | **Q-644** |
| | | | 5 | **Q-295** | | | | 5 | **Q-645** |
| 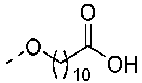 | 5 | 2 | 1 | **Q-296** | 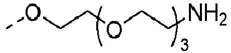 | 5 | 2 | 1 | **Q-646** |
| | | | 2 | **Q-297** | | | | 2 | **Q-647** |
| | | | 3 | **Q-298** | | | | 3 | **Q-648** |
| | | | 4 | **Q-299** | | | | 4 | **Q-649** |
| | | | 5 | **Q-300** | | | | 5 | **Q-650** |
| 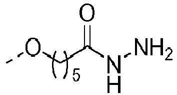 | 1 | 1 | 1 | **Q-301** | 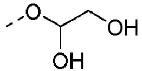 | 1 | 1 | 1 | **Q-651** |
| | | | 2 | **Q-302** | | | | 2 | **Q-652** |
| | | | 3 | **Q-303** | | | | 3 | **Q-653** |
| | | | 4 | **Q-304** | | | | 4 | **Q-654** |
| | | | 5 | **Q-305** | | | | 5 | **Q-655** |
| 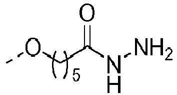 | 2 | 1 | 1 | **Q-306** | 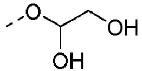 | 2 | 1 | 1 | **Q-656** |
| | | | 2 | **Q-307** | | | | 2 | **Q-657** |
| | | | 3 | **Q-308** | | | | 3 | **Q-658** |
| | | | 4 | **Q-309** | | | | 4 | **Q-659** |
| | | | 5 | **Q-310** | | | | 5 | **Q-660** |
| 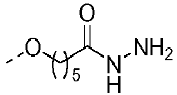 | 3 | 1 | 1 | **Q-311** | 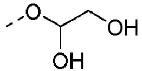 | 3 | 1 | 1 | **Q-661** |
| | | | 2 | **Q-312** | | | | 2 | **Q-662** |
| | | | 3 | **Q-313** | | | | 3 | **Q-663** |
| | | | 4 | **Q-314** | | | | 4 | **Q-664** |
| | | | 5 | **Q-315** | | | | 5 | **Q-665** |
| 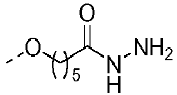 | 4 | 1 | 1 | **Q-316** | 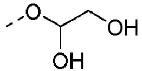 | 4 | 1 | 1 | **Q-666** |
| | | | 2 | **Q-317** | | | | 2 | **Q-667** |
| | | | 3 | **Q-318** | | | | 3 | **Q-668** |
| | | | 4 | **Q-319** | | | | 4 | **Q-669** |
| | | | 5 | **Q-320** | | | | 5 | **Q-670** |
| 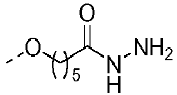 | 5 | 1 | 1 | **Q-321** | 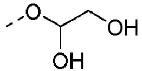 | 5 | 1 | 1 | **Q-671** |
| | | | 2 | **Q-322** | | | | 2 | **Q-672** |
| | | | 3 | **Q-323** | | | | 3 | **Q-673** |
| | | | 4 | **Q-324** | | | | 4 | **Q-674** |
| | | | 5 | **Q-325** | | | | 5 | **Q-675** |
| 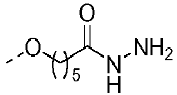 | 1 | 2 | 1 | **Q-326** | 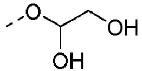 | 1 | 2 | 1 | **Q-676** |
| | | | 2 | **Q-327** | | | | 2 | **Q-677** |
| | | | 3 | **Q-328** | | | | 3 | **Q-678** |
| | | | 4 | **Q-329** | | | | 4 | **Q-679** |
| | | | 5 | **Q-330** | | | | 5 | **Q-680** |
| 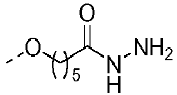 | 2 | 2 | 1 | **Q-331** | 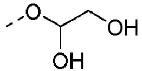 | 2 | 2 | 1 | **Q-681** |
| | | | 2 | **Q-332** | | | | 2 | **Q-682** |
| | | | 3 | **Q-333** | | | | 3 | **Q-683** |
| | | | 4 | **Q-334** | | | | 4 | **Q-684** |
| | | | 5 | **Q-335** | | | | 5 | **Q-685** |
| 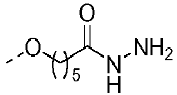 | 3 | 2 | 1 | **Q-336** | 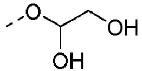 | 3 | 2 | 1 | **Q-686** |
| | | | 2 | **Q-337** | | | | 2 | **Q-687** |
| | | | 3 | **Q-338** | | | | 3 | **Q-688** |
| | | | 4 | **Q-339** | | | | 4 | **Q-689** |
| | | | 5 | **Q-340** | | | | 5 | **Q-690** |
| 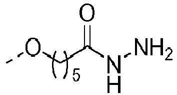 | 4 | 2 | 1 | **Q-341** | 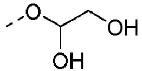 | 4 | 2 | 1 | **Q-691** |
| | | | 2 | **Q-342** | | | | 2 | **Q-692** |
| | | | 3 | **Q-343** | | | | 3 | **Q-693** |
| | | | 4 | **Q-344** | | | | 4 | **Q-694** |
| | | | 5 | **Q-345** | | | | 5 | **Q-695** |
| 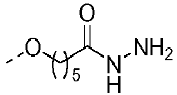 | 5 | 2 | 1 | **Q-346** | 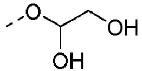 | 5 | 2 | 1 | **Q-696** |
| | | | 2 | **Q-347** | | | | 2 | **Q-697** |
| | | | 3 | **Q-348** | | | | 3 | **Q-698** |
| | | | 4 | **Q-349** | | | | 4 | **Q-699** |
| | | | 5 | **Q-350** | | | | 5 | **Q-700** |

### Chemical synthesis

Another aspect of the present invention is directed to a synthetic method of the general formula **(I)** comprising:
**A1)** providing a disaccharide **D1** 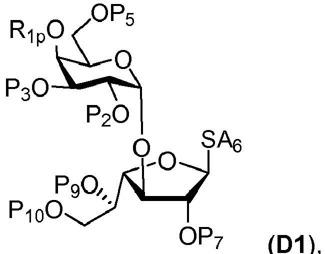 wherein **R_{1P}** is **P₄** or **U₅ₚ**;
   **U₅ₚ** is 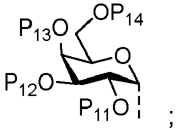
**B1)** reacting **D1** with a disaccharide **D2** 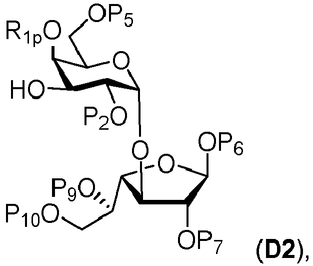 to obtain a saccharide **O1a** 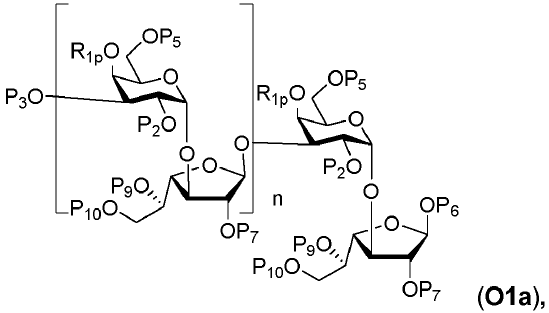 wherein n is 1,
   when n is an integer from 2 to 20,
   then repeating following steps **B2)** and **B3)** for **n-1** times
**B2)** removing the protecting group **P₃** of a saccharide obtained by reacting with the saccharide **D1;**
**B3)** reacting the saccharide **D1** with the saccharide obtained after the step **B2)** to obtain a saccharide **O1a** 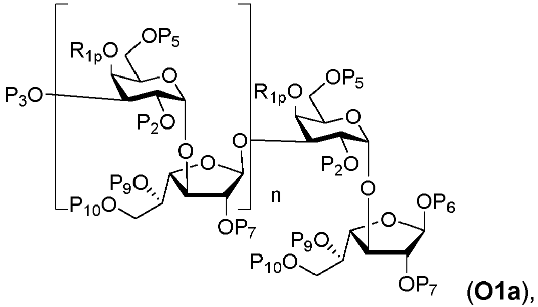 wherein n is an integer from 2 to 20;
**C)** removing a protecting group P₆ of the saccharide **O1a** and introducing an activating group A₆ to obtain a saccharide **O1b** 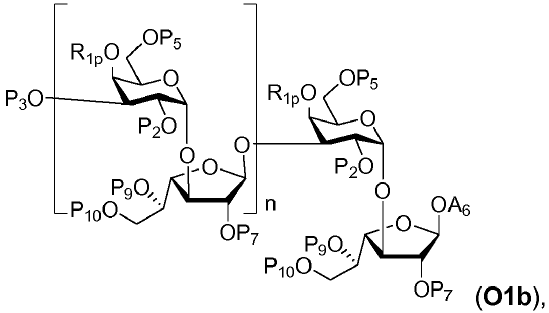
**D1)** converting the activating group A₆ of the saccharide **O1b** by reacting with a reactant HO-L-Eₚ to obtain a saccharide **O1c** 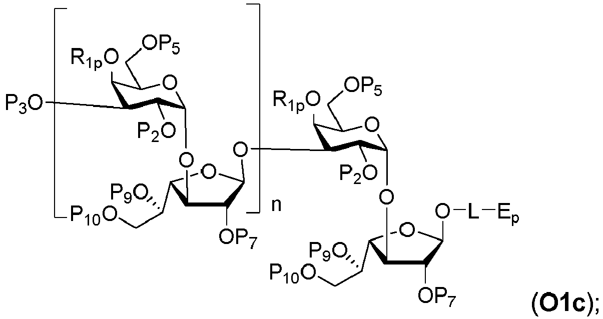 or
**D2)** reacting the saccharide **O1b** with a saccharide **M1** 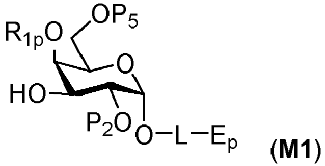 to obtain a saccharide **O2a** 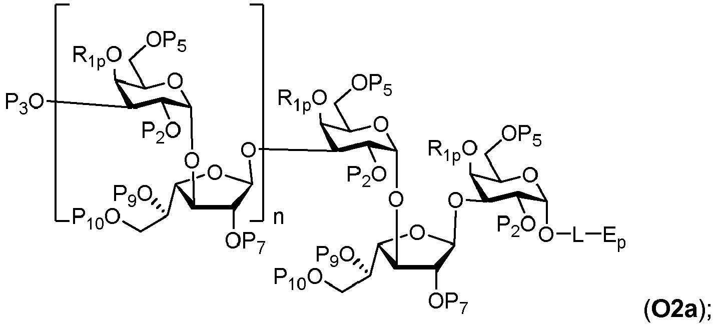 optionally,
**E1)** removing the protecting group P₃ of the saccharide **(O1c)** to obtain a saccharide **O1d,** 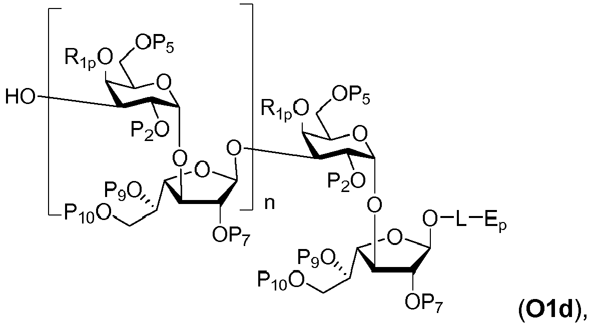 reacting the saccharide **O1d** with a saccharide **M2** 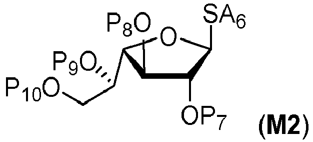 to obtain a saccharide **O3a** 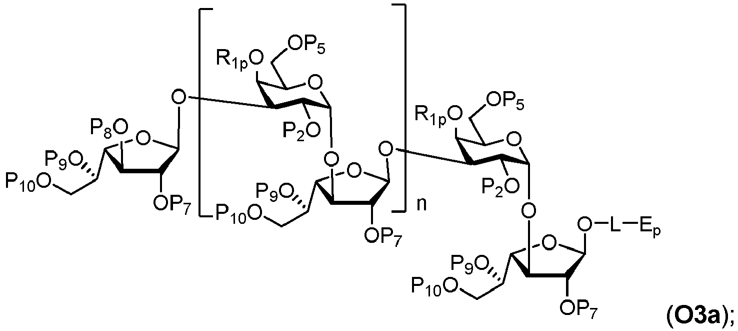 or
**E2)** removing the protecting group P₃ of the saccharide **(O2a)** to obtain a saccharide **O2b,** 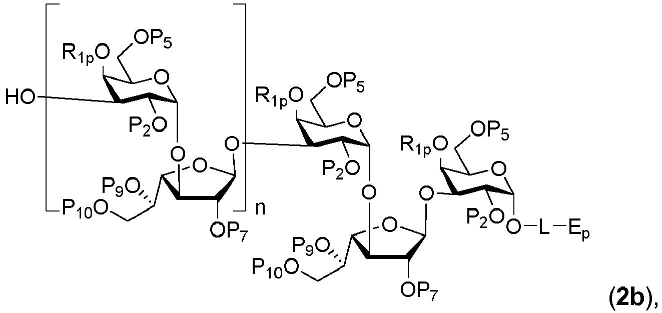 reacting the saccharide **O2b** with a saccharide **M2** 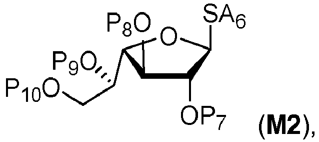 to obtain a saccharide **O4a** 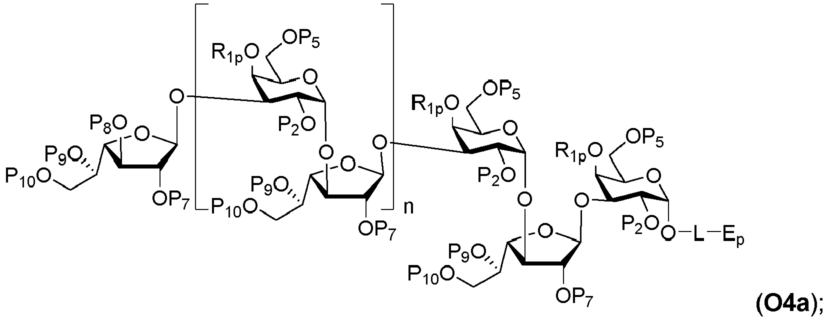 and
**F1)** removing all protecting groups of the saccharide **O1c, O2a, O3a** or **O4a** to obtain a corresponding saccharide of formula **(I-1), (I-2), (I-3),** or **(I-4),** 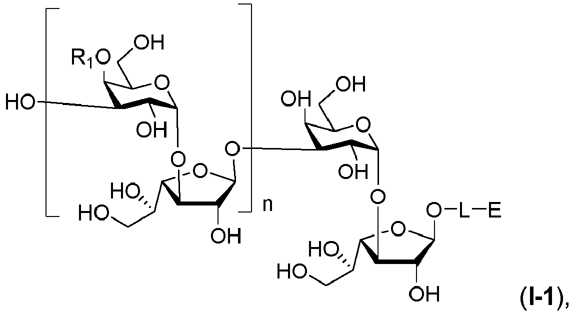 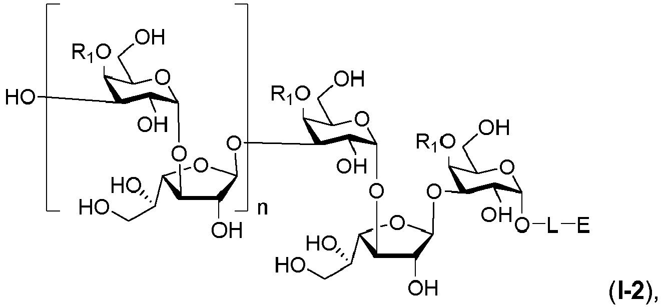 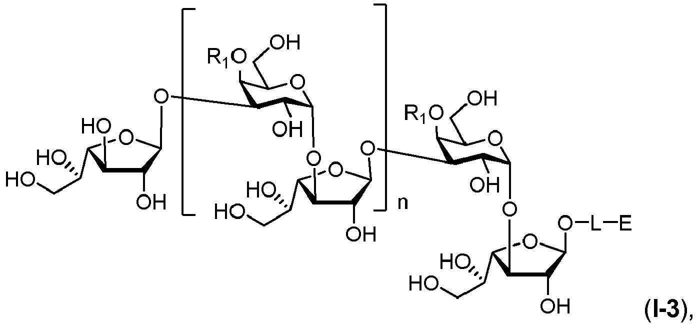 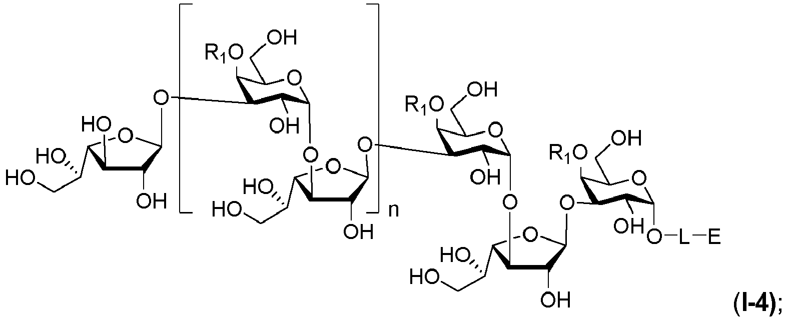 wherein **Eₚ** is a protected end group, **A₆** is an activating group,
   **P₁**, **P₂, P₃, P₅, P₆, P₇, P₈, P₉, P₁₀, P₁₁, P₁₂, P₁₃,** and **P₁₄** represent protecting groups, and **L, E, R₁**, **R₁ₚ** have the same meanings as defined herein.

Preferably **R₁ₚ** is **P₄.**

Therefore, according to the above described synthetic method, following combinations of the steps may be carried out to obtain the inventive synthetic saccharides :
Steps: **A1)** → **B1)** → **C)** → **D1)** → **F1), A1)** → **B1)** → **C)** → **D2)** →**F1**), **A1)** → **B1)** → **C)** → **D1)** → **E1)** → **F1),** or **A1)** → **B1)** → **C)** →**D2)** → **E2)** → **F1).**

Alternatively, a method for synthesis of a saccharide of general formula **(I)** comprising:
**A2)** providing a disaccharide **D6** 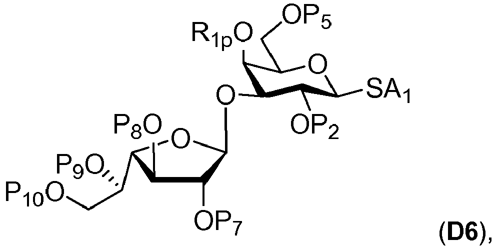 wherein **R_{1P}** is **P₄** or **U₅ₚ**;
   **U₅ₚ** is 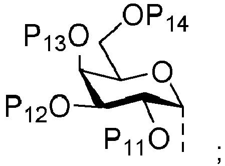
**B1')** reacting the saccharide **D6** with a saccharide **D8** 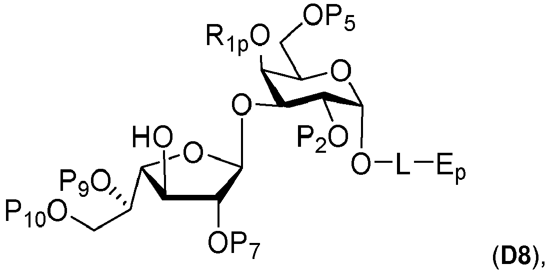 to obtain a saccharide **O2b** 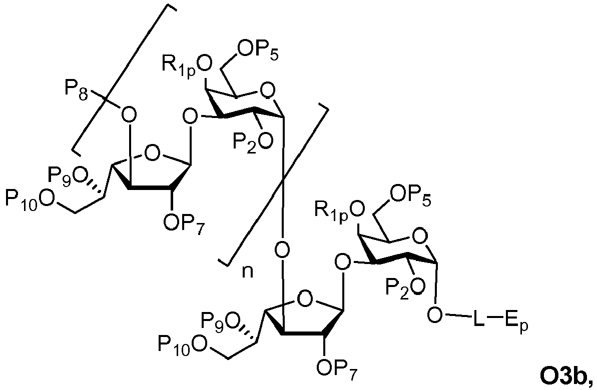 wherein n is 1;
   when n is an integer from 2 to 20,
   then repeating the following steps **B2')** and **B3')** for **n-1** times
**B2')** removing the protecting group P₈ of a saccharide obtained by reacting with the saccharide **D6;**
**B3')** reacting the saccharide **D6** with the saccharide obtained after the step **B2')** to obtain a saccharide **O3b** 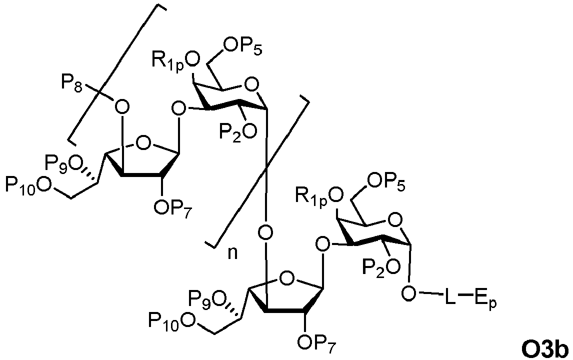 wherein n is an integer from 2 to 20,
   optionally,
**E3)** removing the protecting group P₈ of the saccharide **O3b** to obtain a saccharide **O3c,** 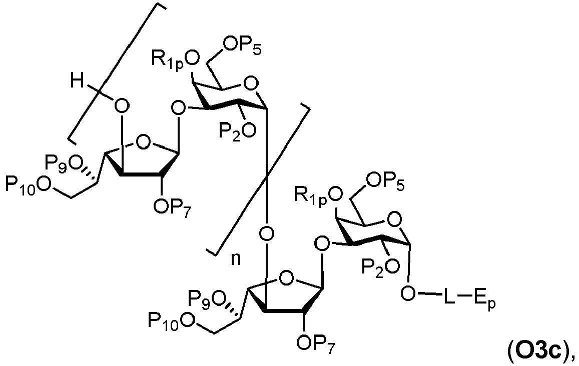 and
**E4)** reacting the saccharide **O3c** with a saccharide **M3** 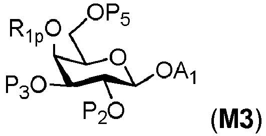 to obtain a saccharide **O4b** 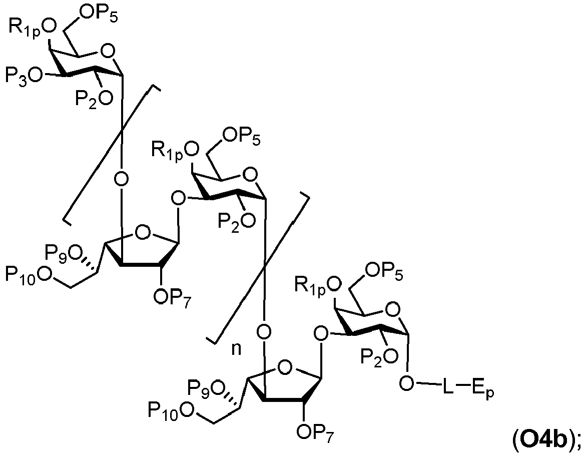 or
**E5)** reacting the saccharide **O3c** with a disaccharide **D4** 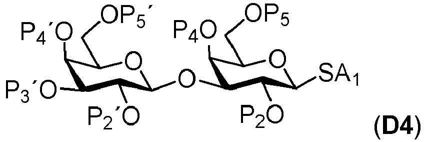 to obtain a saccharide **O5a** 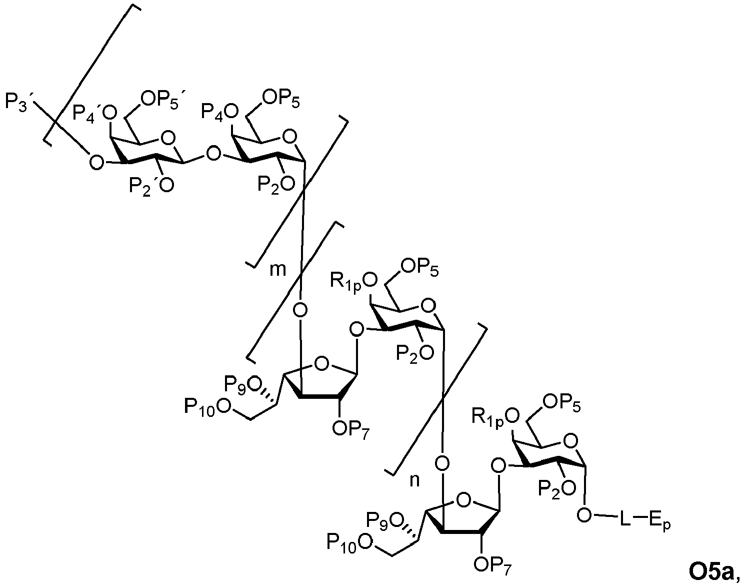 wherein **m** is 1,
   when **m** is an integer from 2 to 20,
   then repeating the following steps **e5)** and **e6)** for **m-1** times
   **e5)** removing the protecting group **P₃'** of a saccharide obtained by reacting with the monosaccharide **D4;**
   **e6)** reacting the saccharide **D4** with the saccharide obtained after the step **e5)** to obtain a saccharide **O5a** 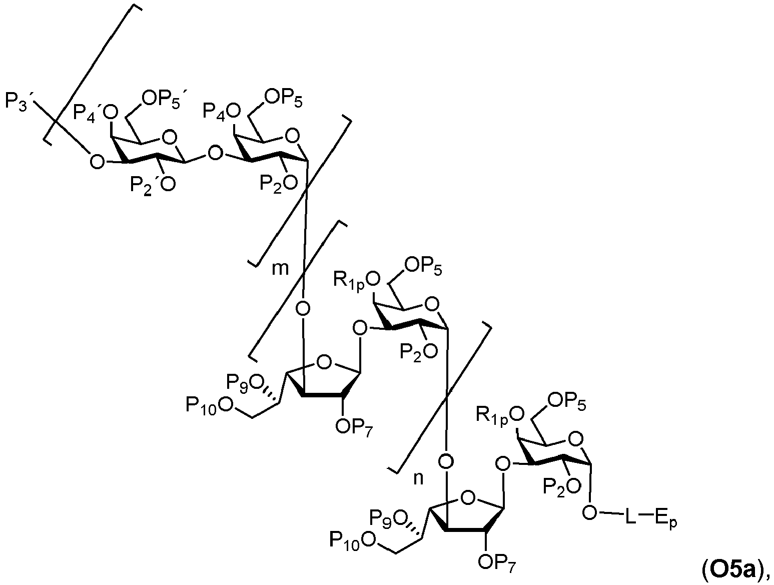 wherein **m** is an integer from 2 to 20;
      or
**E6)** reacting the saccharide **O3c** with a disaccharide **D5** 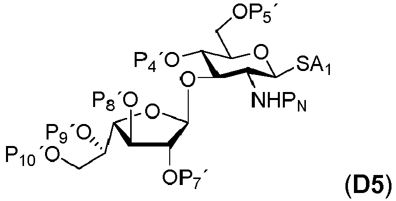 wherein **A₁** represents an activating group
   to obtain a saccharide **O5b** 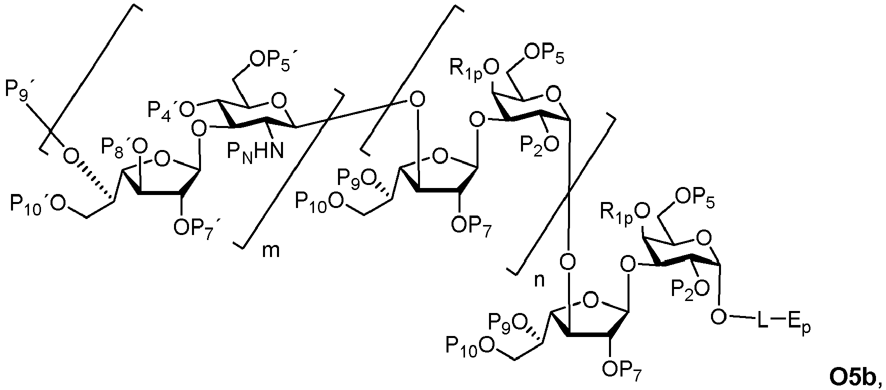 wherein **m** is 1,
   when **m** is an integer from 1 to 20,
   then repeating following steps **e7)** and **e8)** for **m-1** times
   **e7)** removing the protecting group **P₉'** of a saccharide obtained by reacting with the monosaccharide **D5;**
   **e8)** reacting the saccharide **D5** with the saccharide obtained after the step **e7)** to obtain a saccharide **O5b** 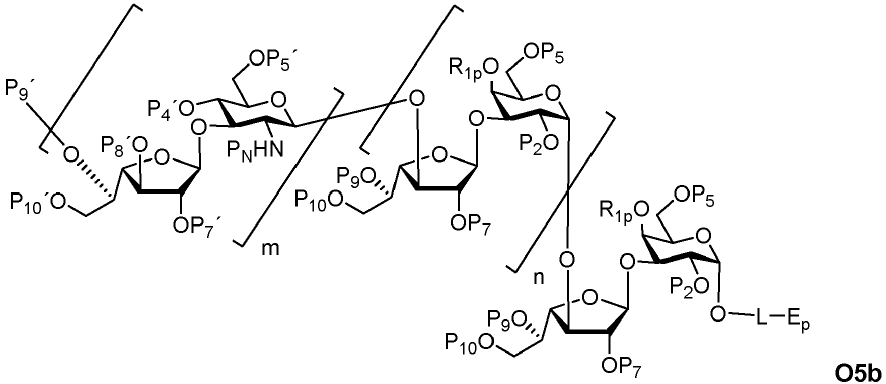 wherein **m** is an integer from 2 to 20;
   **e9)** removing a protecting groups **P_{N}** and converting resulting -NH₂ groups to -NHAc groups to obtain a saccharide **O5c** 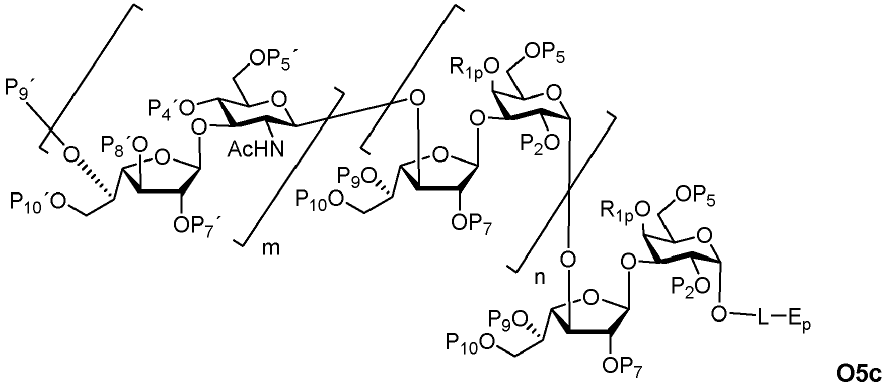
**F2)** removing all protecting groups of the saccharide **O3b, O4b, O5a,** or **O5c,** to obtain a corresponding saccharide of the formula (**I-5**), (**I-6**), **(I-7),** or **(I-8),** 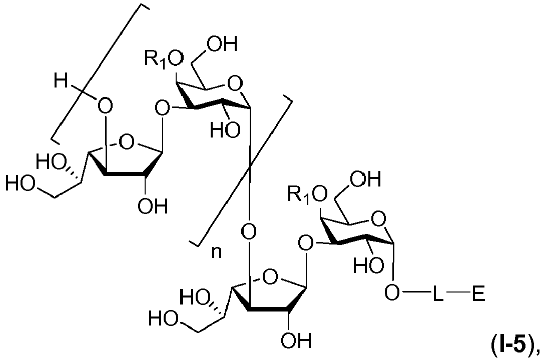 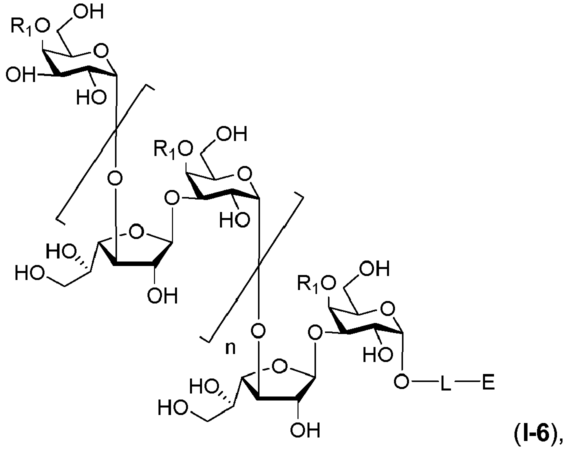 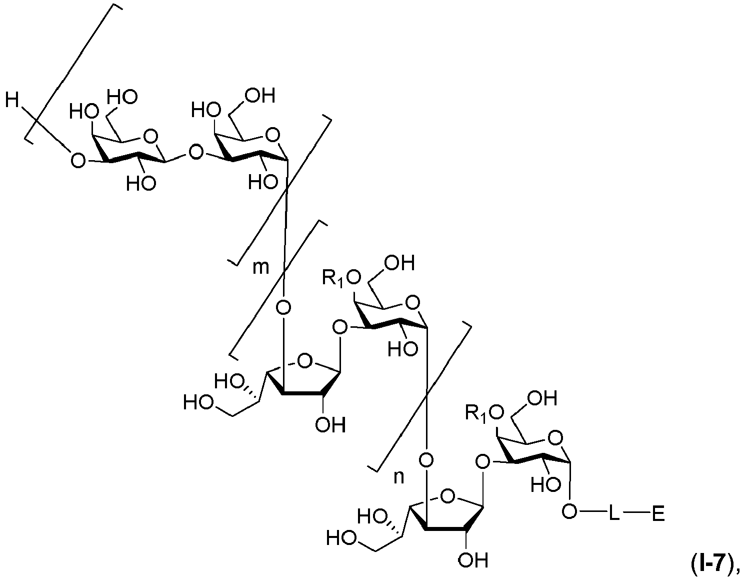 or 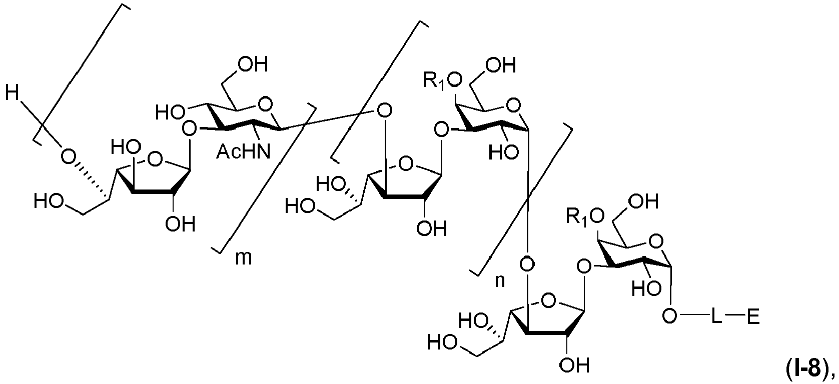 wherein **Eₚ** is a protected end group; **A₁** is an activating group; **P_{N}, P₁, P₂, P₂', P₃, P₃', P₄, P₄', P₅, P₅', P₆, P₇, P₇', P₈, P₈', P₉, P₉', P₁₀, P₁₀', P₁₁, P₁₂, P₁₃**, and **P₁₄** represent protecting groups, and **L, E, R₁, R₁ₚ,** m, and n have the same meanings as defined herein.

Preferably **R₁ₚ** is **P₄,** and **R₁** is H.

Therefore, according to the above described synthetic method, following combinations of the steps may be carried out to obtain the inventive synthetic saccharides :
**Steps: A2)** → **B1')** → **E3)** → **F2), A2)** → **B1')** → **B2')** → **B3')** → **E3)→ F2), A2)** → **B1')** → **B2')** → **B3')** → **E3) → E4)** → **F2), A2)** → **B1')** → **B2')** → **B3')** → **E3) → E5)** → **F2), A2)** → **B1')** → **B2')** → **B3')** → **E3) → E5)** → **F2).**

Optionally, a step **A2')** for preparing the saccharide **D8** can be carried out before the step **B1')** and the step **A2')** is as follows:
**a1)** Providing a monosaccharide **M3** 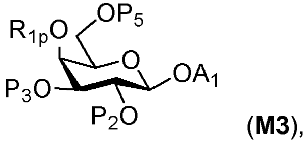 wherein **R_{1P}** is **P₄** or **U₅ₚ;**
   **U₅ₚ** is 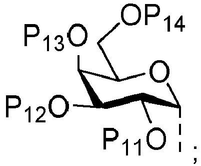
**a2)** converting the activating group **A₁** of the saccharide **M3** by reacting with a reactant HO-L-Eₚ to obtain a saccharide a monosaccharide **(M4)** 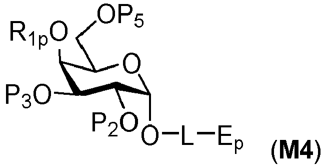
**a3)** removing a protecting group **P₃** of **M4** to obtain a monosaccharide **M5** 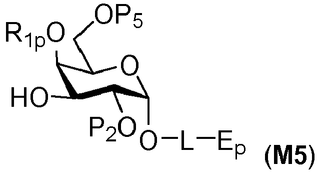
**a4)** reacting the monosaccharide **M5** with the saccharide **M3** 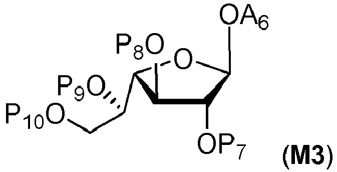 to obtain a saccharide **D7,** 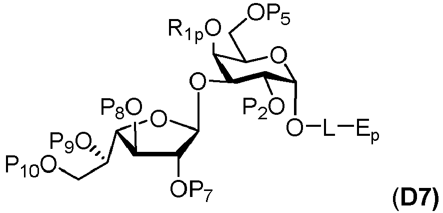
**a5)** removing a protecting group **P₈** of **D7** to obtain a monosaccharide **D8** 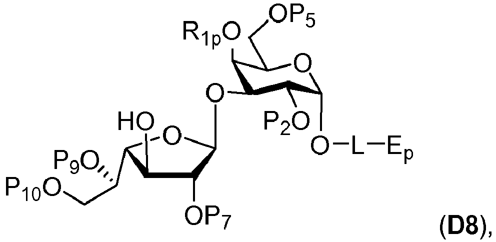 wherein **R_{1P}** is **P₄** or **U₅ₚ;**
   **U₅ₚ** is 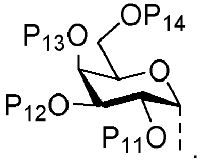 wherein, **A₁** and **A₆** represent activating groups; **Eₚ** is a protected end group;
   **P₁**, **P₂, P₃, P₄, P₅, P₆, P₇, P₈, P₉, P₁₀, P₁₁, P₁₂, P₁₃** and **P₁₄** represent protecting groups, and **L, E, R₁, m,** and n have the same meanings as defined above.

Alternatively, a method for synthesis of a saccharide of general formula (I) comprising:
**A3)** providing a disaccharide **D8** 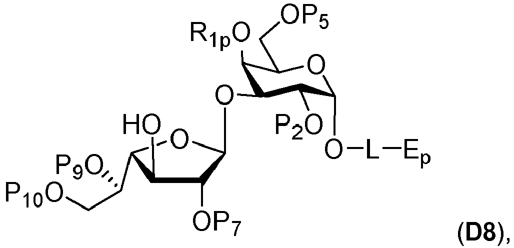 wherein **R_{1P}** is **P₄** or **U₅ₚ;**
   **U₅ₚ** is 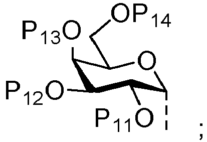
**B1")** reacting the saccharide **D8** with a saccharide **D4** 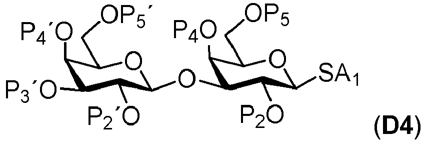 to obtain a saccharide **O3d** 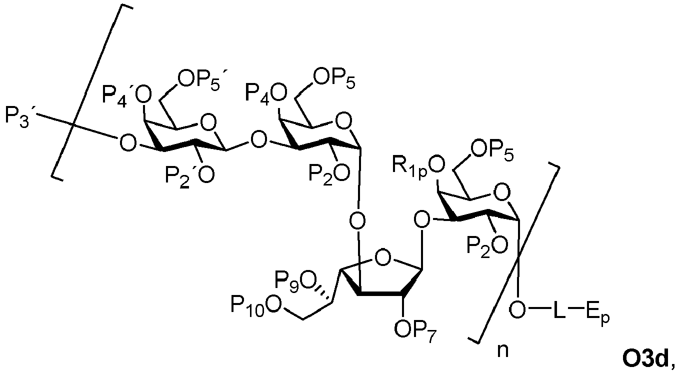 wherein n is 1,
   when **n** is an integer from 2 to 20,
   then repeating the following steps **B2")** - **B5")** for **n-1** times
**B2")** removing the protecting group **P₃'** of a saccharide obtained by reacting with the saccharide **D4;**
**B3")** reacting the saccharide **D6** with the saccharide obtained after the step B2) 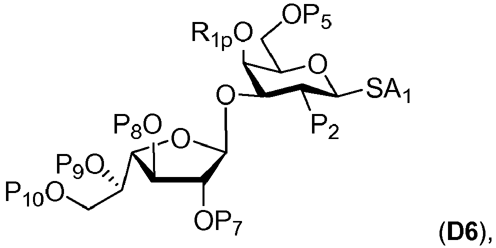
**B4")** removing the protecting group **P₈** of a saccharide obtained by reacting with the saccharide **D6;**
**B5")** reacting the saccharide **D4** with the saccharide obtained after the step B4) 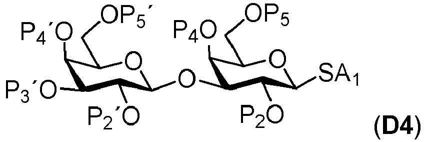 wherein **A₁** represents an activating group
   to obtain a saccharide **O3d** 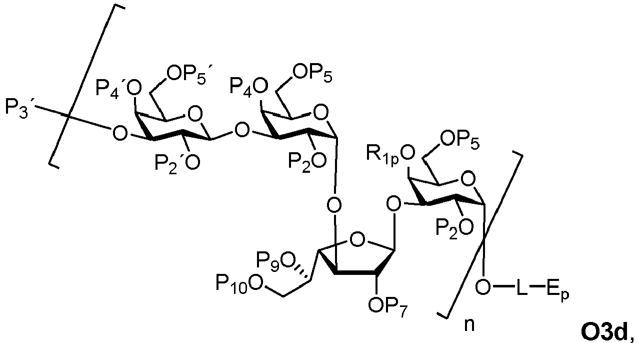 wherein n is an integer from 2 to 20,
   and **Eₚ** is a protected end group; or
**B1''')** reacting the saccharide **D8** with a saccharide **D5** 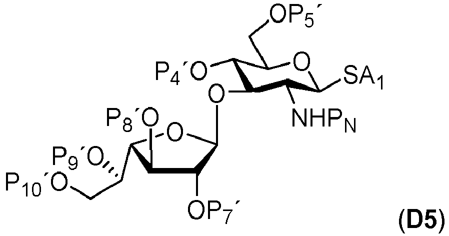 to obtain a saccharide **O3e** 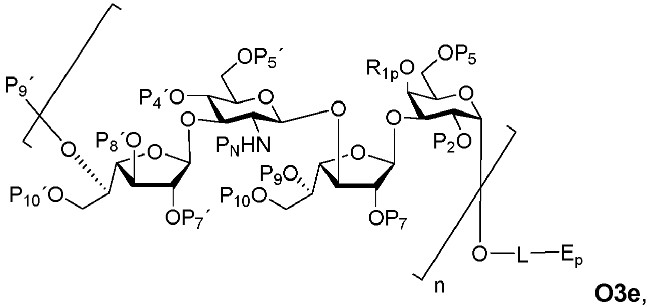 wherein n is 1,
   when n is an integer from 2 to 20,
   then repeating the following steps **B2''')** - **B5''')** for **n-1** times
**B2''')** removing the protecting group **P₉'** of a saccharide obtained by reacting with the saccharide **D4;**
**B3''')** reacting the saccharide **D6** with the saccharide obtained after the step B2) 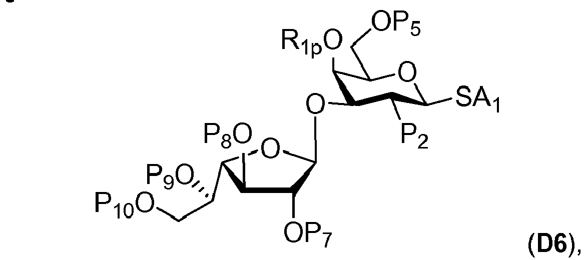
**B4''')** removing the protecting group **P₈** of a saccharide obtained by reacting with the saccharide **D6;**
**B5''')** reacting the saccharide **D5** with the saccharide obtained after the step B4) 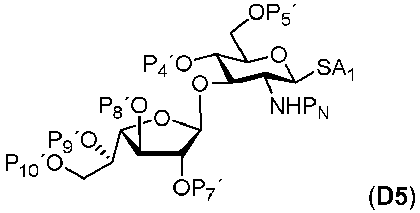 wherein **A₁** represents an activating group
   to obtain a saccharide **O3e** 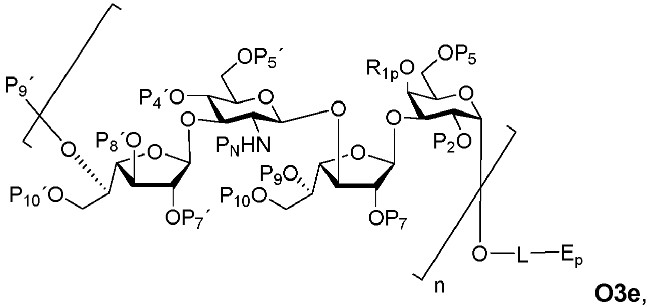 wherein n is an integer from 2 to 20,
   and **Eₚ** is a protected end group;
**B6''')** removing a protecting groups **P_{N}** and converting resulting -NH₂ groups to -NHAc groups to obtain a saccharide **O3f** 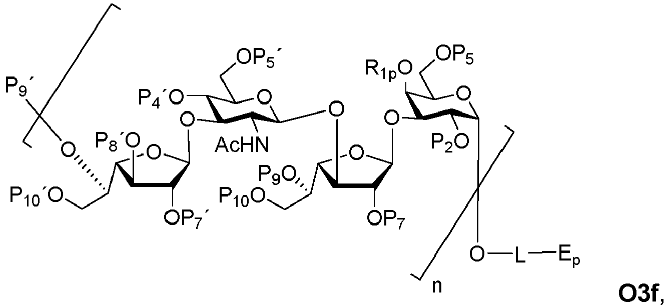
**F3)** removing all protecting groups of the saccharide **O3d** or **O3f** to obtain a corresponding saccharide of the formula (**I-9**) or (**I-10**) 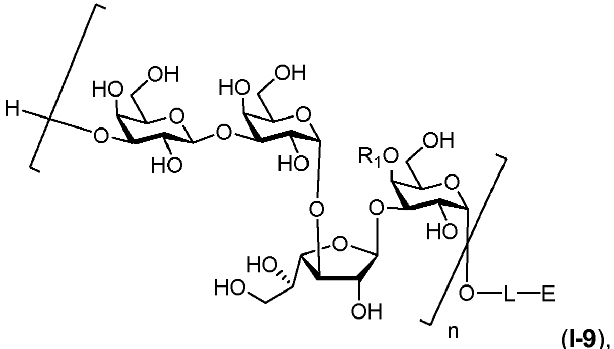 or 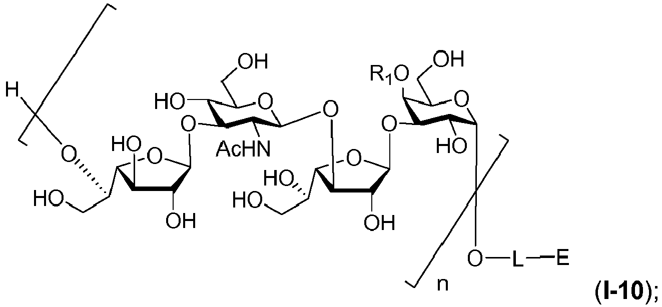 wherein **P_{N}, P₂, P₂', P₃', P₄, P₄', P₅, P₅', P₇, P₇', P₈, P₈', P₉, P₉', P₁₀, P₁₀', P₁₁, P₁₂, P₁₃** and **P₁₄** represent protecting groups, and **L, E, R₁,** m, and n have the same meanings as defined herein.

Preferred, wherein **R₁ₚ** is **P₄,** and **R₁** is H.

Therefore, according to the above described synthetic method, following combinations of the steps may be carried out to obtain the inventive synthetic saccharides:
**Steps: A3)** → **B1**") → **F3), A3)** → **B1**") → **B2")** → **B3")** → **B4")** → **B5")** → **F3)**, **A3)** → **B1''')** → **F3), A3)** → **B1'''**) → **B2''')** → **B3''')** → **B4''')** → **B5''')** → **B6''')** → **F3).**

**P₁, P₂, P₂', P₃, P₃', P₄, P₄', P₅, P₅', P₆, P₇, P₇', P₈, P₈', P₉, P₉', P₁₀, P₁₀', P₁₁, P₁₂, P₁₃** and **P₁₄** represent protecting groups. The term "protecting group" as used herein refers to commonly used groups in organic synthesis, preferably used for protection hydroxyl groups, and thiols. **P_{N}** represents protecting group used for protection amine group.
More preferably, **P₁, P₂', P₃, P₃', P₄, P₄', P₅, P₅', P₆, P₇, P₇', P₈, P₈', P₉, P₉', P₁₀, P₁₀', P₁₁, P₁₂, P₁₃ and P₁₄** are suitable protecting groups for hydroxyl groups, more preferably different suitable protecting groups for hydroxyl groups capable of being removed subsequently one after another by a suitable sequence of deprotection reactions. Preferred protecting groups for hydroxyl groups are acetyl, phenyl, benzyl, isopropylidene, benzylidene, benzoyl, *p*-methoxybenzyl, *p*-methoxybenzylidene, *p-*methoxyphenyl, *p*-bromobenzyledene, *p*-nitrophenyl, allyl, acetyl, isopropyl, *p-*bromobenzyl, dimethoxytrityl, trityl, 2-naphthylmethyl, pivaloyl, triisopropylsilyl, *tert-*butyldimethylsilyl, *tert*-butyldiphenylsilyl, *tert*-butylmethoxphenylsilyl, triethylsilyl, trimethylsilyl, 2-trimethylsilylethoxymethyl, 9-fluorenylmethoxycarbonyl, benzyloxymethyl, methyloxymethyl, *tert*-butyloxymethyl, methoxyethyloxymethyl, levulinoyl and **P_{N}** represents 2,2,2-trichloroethyl carbonyl (Troc) or 9-fluorenylmethyloxycarbonyl (Fmoc).

Specifically, protecting groups **P₁** and **P₆** represent phenyl, protecting groups **P₃** and **P₃'** represent 2-naphthylmethyl, protecting groups **P₂, P₄, P₄', P₅', P₁₁, P₁₂,** and **P₁₄** represent benzyl, p-methoxybenzyl, protecting groups **P₂', P₅, P₇, P₇', P₈, P₈', P₉, P₁₀, P₁₀',** and **P₁₃** are benzoyl, and protecting group **P₆** represents butyldimethylsilyl. Optionally, **OP₄** and **OP₅, OP₄'** and **OP₅'** form a phenyl hemiacetal. **P₉'** is benzoyl or levulinoyl. **P_{N}** is 2,2,2-Trichloroethyl carbonyl (Troc).

Activating groups **A₁** and **A₆** represent -Ph, or -C(CF₃)NPh.

**Eₚ** represents a protected end group. **E** represents -NH₂, -N₃, -CN, -O-NH₂, -CH=CH₂, -C=CH, -Br, -CI, -I, -CO₂R', -CONHNH₂, -SH, or -SAc; and The corresponding protected end group **Eₚ** represents -N(P_{N1})(P_{N2}), -N₃, -CN, -O-N(P_{N1})(P_{N2}), -CH=CH₂, -C=CH, -Br, -CI, -I, -CO₂R', -CONHN(P_{N1})(P_{N2}), -SPₛ, or -SAc;
**P_{N1}, and P_{N2}** are suitable protecting groups for amines and form together with the amine to be protected carbamates or amides. Examples of protecting groups forming carbamates include *tert*-butyloxy carbonyl, 9-fluorenylmethyl carbonyl, allyl carbonyl, trichloroethyl carbonyl and benzyloxy carbonyl. Examples of protecting groups forming amides include acetyl or trichloro acetyl. Preferably, protecting group P¹² represents benzyl and protecting group P¹³ represents benzyloxy carbonyl.
**Pₛ** is suitable protecting group for thiol and selected from phenyl, benzyl, p-methoxybenzyl, p-methoxyphenyl, p-nitrophenyl, and allyl.

It is preferred that the coupling reaction between saccharides in the steps **B1), B1'), B1"), B1'"), B3), B3'), B3"), B3"'), B5"), B5"'), D1), D2), E1), E2), E4), E5), E6), a4), e6), e8)** is performed by activation with NIS/TfOH or TBSOTf, in a mixture of apolar solvent and polar aprotic solvent at a temperature of between -80 °C and -60 °C. Even more preferred is that said reaction is performed by activation with TBSOTf, in a mixture of toluene and diethyl ether at -70 °C.

Preferably, the coupling reaction between the monosaccharide **M5** and the saccharide **M3** in step **a4)** is performed by activation with TBSOTf in an apolar solvent at a temperature of between -10 °C to +10 °C.

The removal of protecting groups P¹, P³, P⁵- P¹³ performed at **steps F1)** - **F3)** involves:
- first cleavage of the base-labile protecting groups by treatment with a base in presence of hydrogen peroxide in a mixture of solvents. Preferably, the base is NaOMe or LiOH; and
- second cleavage of the protecting groups sensitive to hydrogenation by subjecting the compound to hydrogen in presence of a palladium catalyst in a mixture of solvents.

A further aspect according to the present invention refers to an intermediate compound for preparing a saccharide of the general formula (I), wherein the intermediate compound has any one of general formulae **(O1b), (O1c), (O1d), (O2a), (O2b), (O3a), (O3b)**, **(O3c), (O3d), (O3e), (O3f), (O4a), (O4b), (O5a), (O5b)** and **(O5c):** 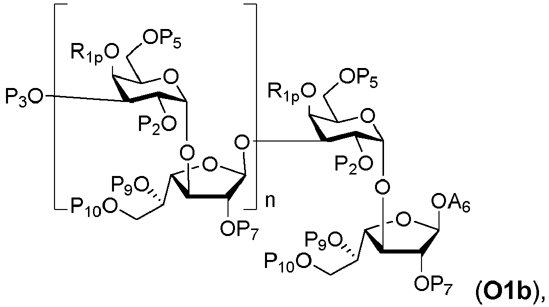 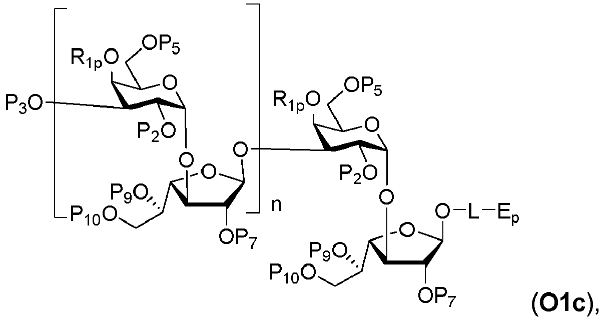 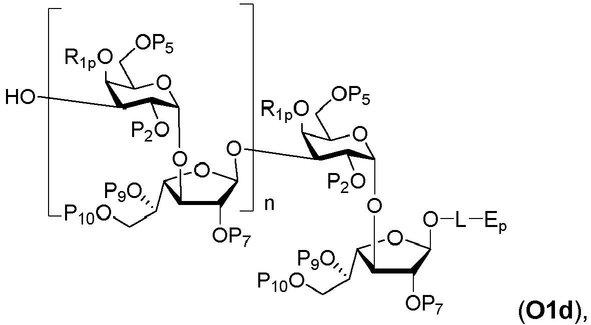 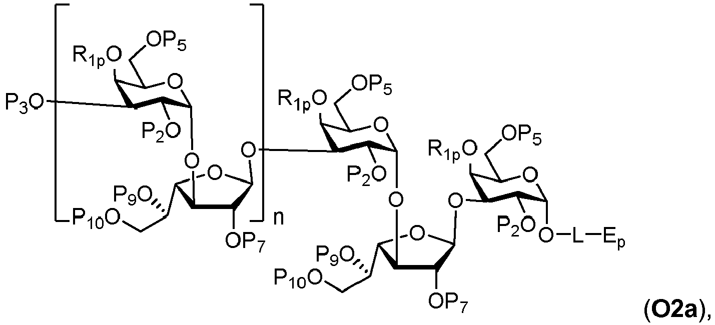 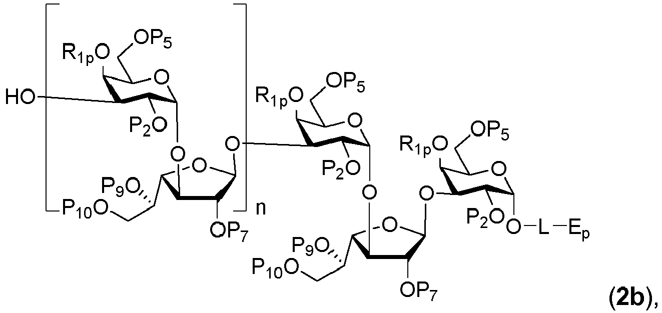 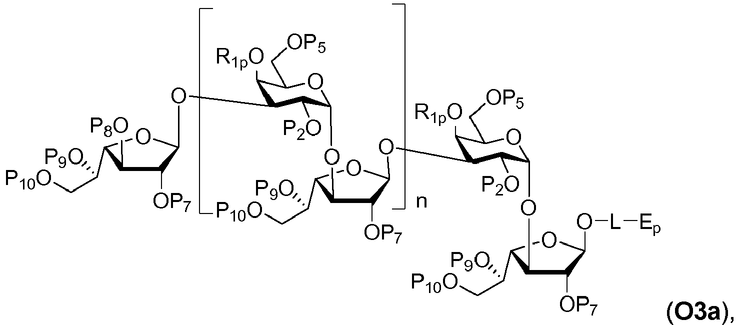 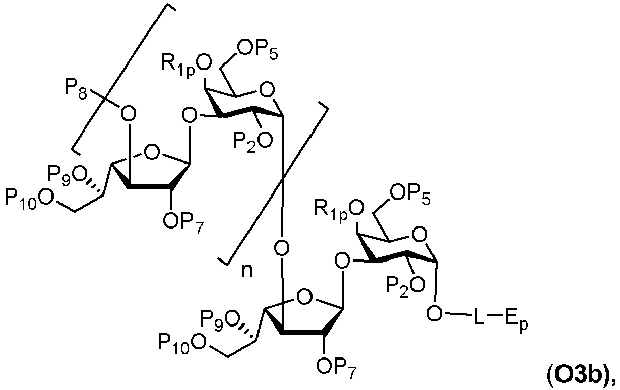 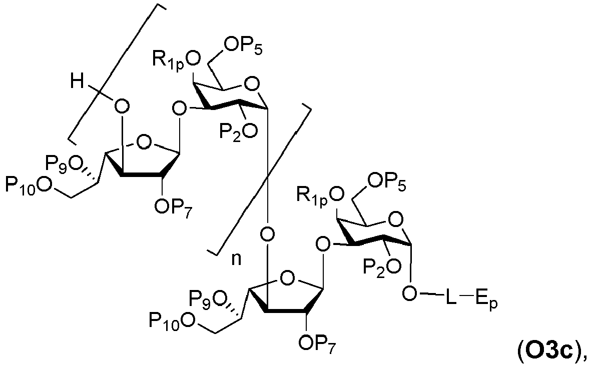 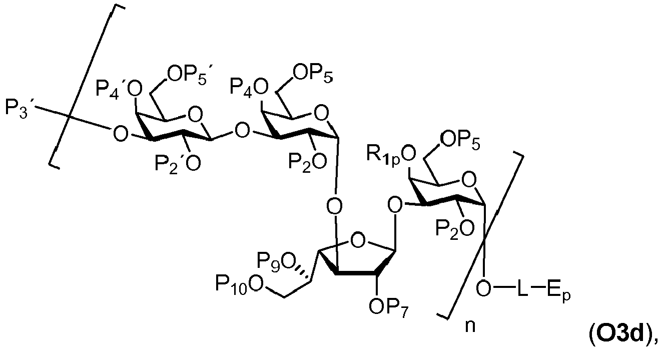 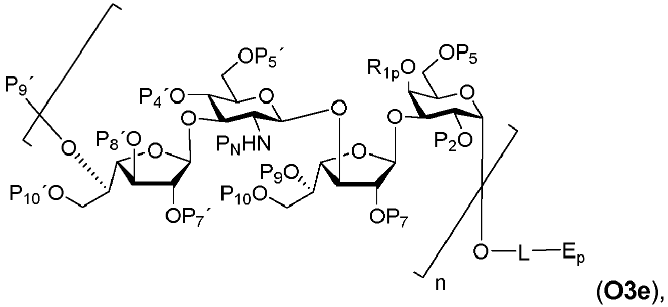 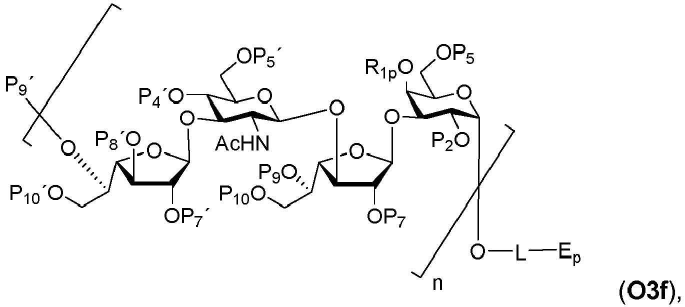 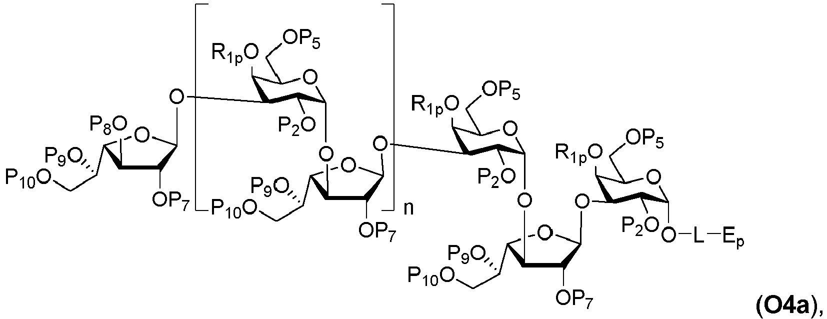 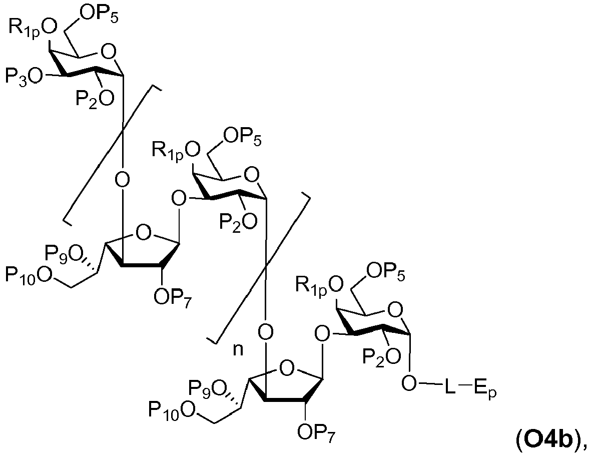 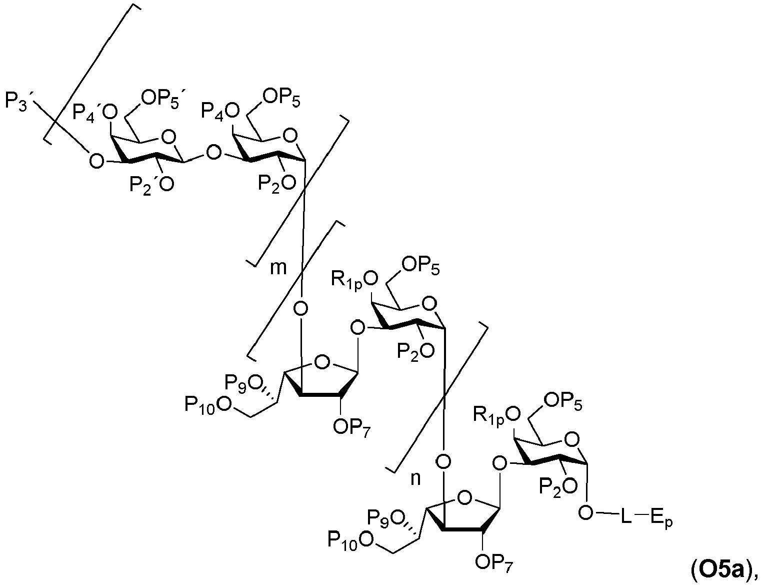 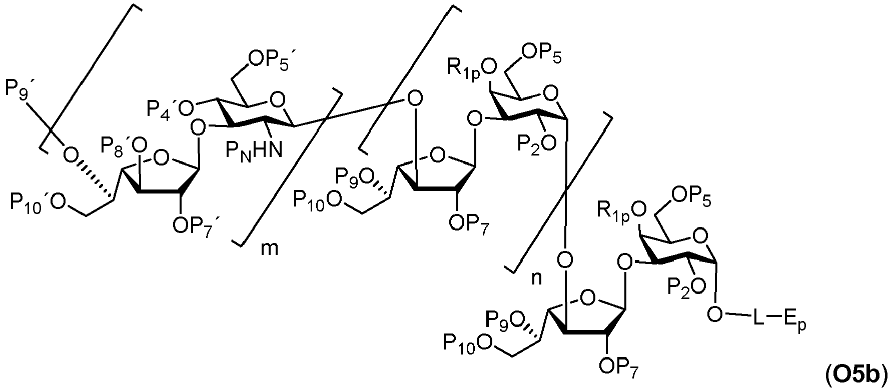 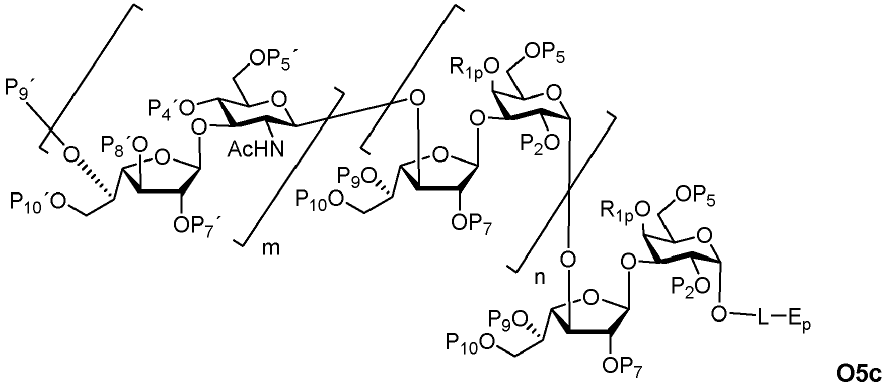 wherein, **A₆** represents activating group; **Eₚ** is protected end group,
**P_{N}, P₂, P₂', P₃', P₄, P₄', P₅, P₃', P₇, P₇', P₈, P₈', P₉, P₉', P₁₀, P₁₀', P₁₁, P₁₂, P₁₃** and **P₁₄** represent protecting groups, and **L, R₁ₚ, m**, and n have the same meanings as defined above.

In formulae **(O1b), (O1c), (O1d), (O2a), (O2b), (O3a), (O3b)**, **(O3c), (O3d), (O3e), (O3f), (O4a), (O4b), (O5a), (O5b)** and **(O5c),** preferably the linker -L- represents **-L^{a}-, -L^{a}-L^{e}- -L^{a}-L^{b}-L^{e}-,** or **-L^{a}-L^{d}-L^{e}-;**
**-L^{a}-** represents -(CH₂)ₒ-, -(CH₂-CH₂-O)ₒ-C₂H₄-, or -(CH₂-CH₂-O)ₒ-CH₂; **-L^{b}-** represents -O-;
**-L^{d}-** represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂-;
**-L^{e}-** represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂-; and
**o, q, p1** and **p2** are independently of each other an integer selected from 1, 2, 3, 4, 5, and 6, with the proviso that L is not -C₃H₆- if **-E** is -NH₂.

An especially preferred intermediate is an intermediate of formula **(O1b), (O1c), (O1d), (O2a), (O2b), (O3a), (O3b), (O3c), (O3d), (O3e), (O3f), (O4a), (O4b), (O5a), (O5b),** and **(O5c),** wherein **-L-** represents -(CH₂)ₒ- and o is an integer selected from 4, 5 and 6.

**P₂, P₂', P₃', P₄, P₄', P₅, P₅', P₇, P₈, P₉, P₁₀, P₁₁,** P**₁₂**, **P₁₃,** and **P₁₄** are suitable protecting groups for hydroxyl groups, more preferably different suitable protecting groups for hydroxyl groups capable of being removed subsequently one after another by a suitable sequence of deprotection reactions. Preferred protecting groups for hydroxyl groups are acetyl, phenyl, benzyl, isopropylidene, benzylidene, benzoyl, *p-*methoxybenzyl, *p*-methoxybenzylidene, *p*-methoxyphenyl, *p*-bromobenzyledene, *p-*nitrophenyl, allyl, acetyl, isopropyl, *p*-bromobenzyl, dimethoxytrityl, trityl, 2-naphthylmethyl, pivaloyl, triisopropylsilyl, *tert*-butyldimethylsilyl, *tert*-butyldiphenylsilyl, *tert*-butylmethoxphenylsilyl, triethylsilyl, trimethylsilyl, 2-trimethylsilylethoxymethyl, 9-fluorenylmethoxycarbonyl, benzyloxymethyl, methyloxymethyl, *tert*-butyloxymethyl, methoxyethyloxymethyl, levulinoyl.
**P_{N}** represents protecting group used for protection amine group, more preferably 2,2,2-Trichloroethyl carbonyl (Troc) or Fluorenylmethyloxycarbonyl (Fmoc).

Thus, intermediates **(O1b), (O1c), (O1d), (O2a), (O2b), (O3a), (O3b), (O3c), (O3d), (O3e), (O3f), (O4a), (O4b), (O5a), (O5b)** and **(O5c),** are especially preferred: protecting groups **P₁** and **P₆** represent phenyl, protecting groups **P₃** and **P₃'** represent 2-naphthylmethyl, protecting groups **P₂, P₄, P₄', P₅', P₁₁, P₁₂** and **P₁₄** represent benzyl, p-methoxybenzyl, protecting groups **P₂', P₅, P₇, P₇', P₈, P₈', P₉, P₁₀, P₁₀'** and **P₁₃** are benzoyl, **P₉'** is benzoyl or levulinoyl and protecting group **P₆** represents butyldimethylsilyl. **P_{N}** is 2,2,2-Trichloroethyl carbonyl (Troc). Optionally, **OP₄** and **OP₅, OP₄'** and **OP₅'** form a phenyl hemiacetal.

A further aspect of the present invention refers to a compound of formula **(I-1)** - **(I-7):** 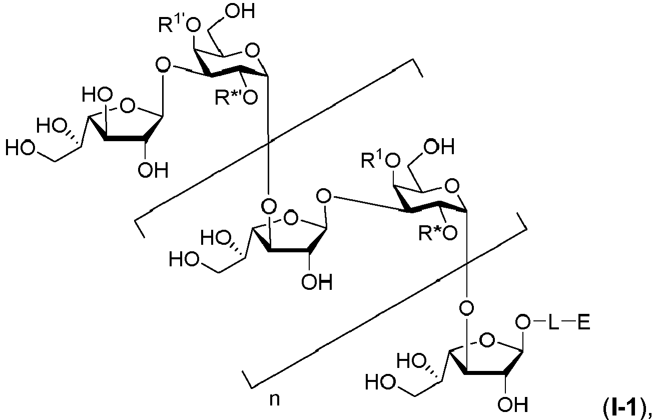 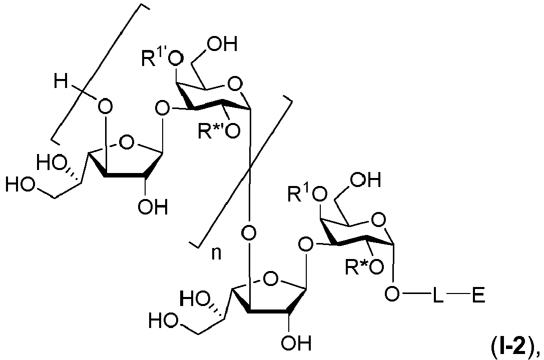 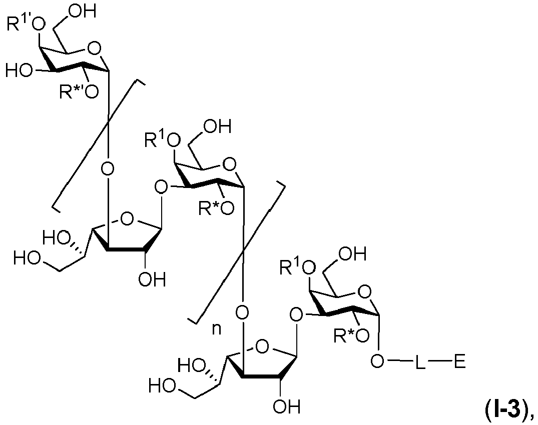 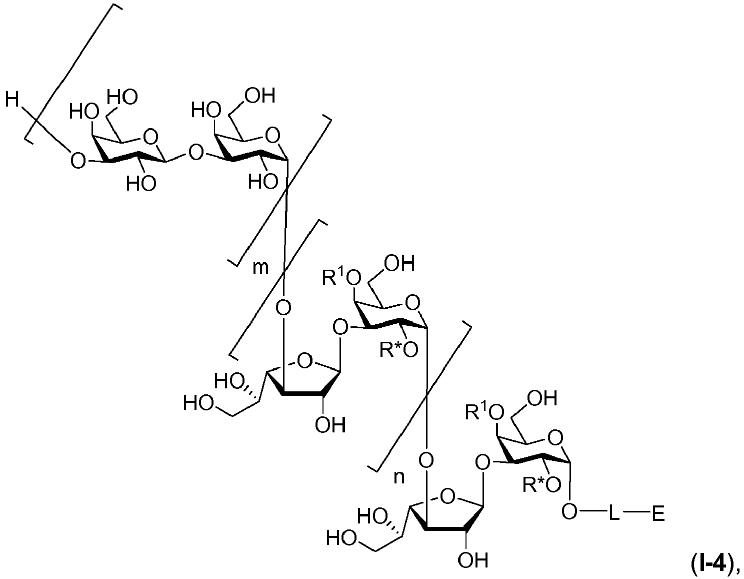 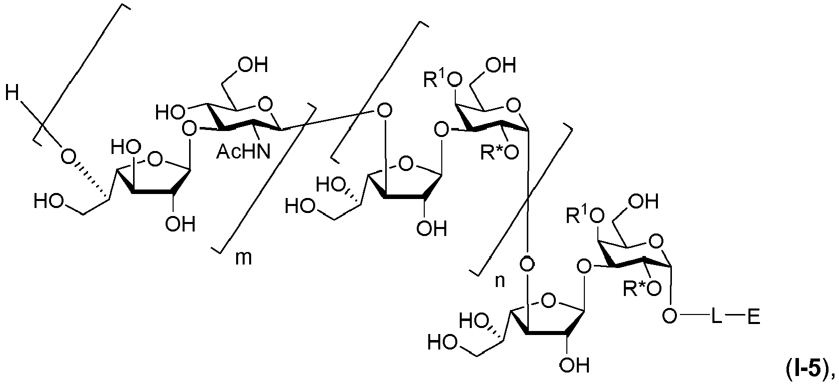 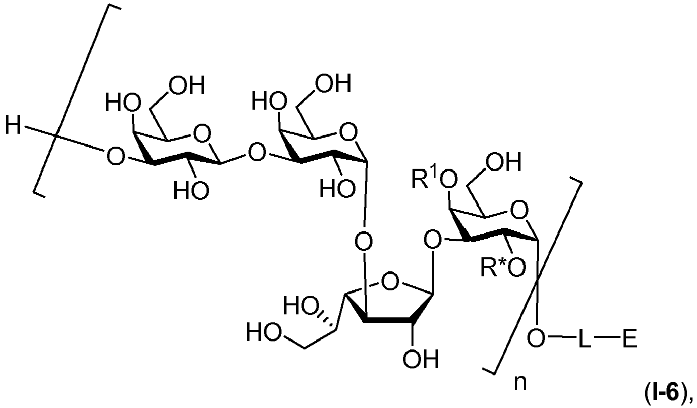 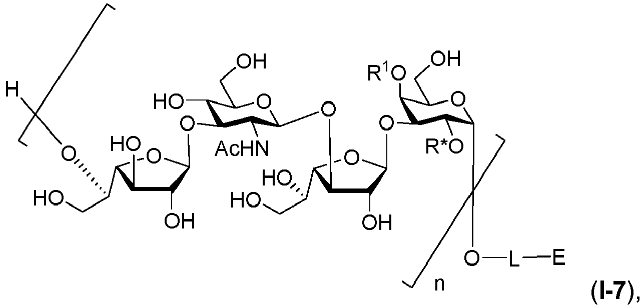 wherein m, n, L, E, R¹, R*, R1' and R*' have the meanings as defined herein.

### Glycoconjugates

Another aspect of the present invention refers to a conjugate comprising a saccharide according to the present invention. Surprisingly, said conjugate proved to be efficient as a vaccine for immunization against diseases associated with *Klebsiella pneumoniae* bacteria.

Preferred, the *Klebsiella pneumoniae* bacteria is selected from O-serotypes comprising or consisting of O1, O2, O2ac, O3, O4, O5, O7, O8, O12 and subtypes thereof and carbapenem resistant *Klebsiella pneumoniae* (CRKP). Preferred, O-serotypes O1, O2a, O2ab, O2ac, O2ae, O2aeh, O2afg, O8, and CRKP strain ST 258, more preferred O1, O2a, O2ab, O2ac, O2afg, O8, CRKP strain ST 258. Still more preferred, *Klebsiella pneumoniae* strains serotypes are O1:K1, O1:K2, O1:K7, O1:K8, O1:K10, O1:K12, O1:K16, O1:K19, O1:K21, O1:K22, O1:K27, O1:K34, O1:K42, O1:K45, O1:K55, O1:K57, O1:K62, O1:K65, O1:K66, O1:K69 and O1:K70, O2a, O2ac, and CRKP strain ST 258.

Saccharides are known by the person skilled in the art as generally TI-2 (T cell independent-2) antigens and poor immunogens. TI-2 antigens are antigens, which are recognized only by mature B cells through the cross linking of surface exposed immunoglobulin receptors. Without T cell help, no immunological memory is generated and neither isotype switching from IgM to other IgG subclasses, nor B cells affinity maturation occurs. Moreover, saccharides are known poor immunogens in humans due to the structural homology to human glycolipids and glycoproteins. Due to their poor immunogenic properties, saccharides manifest poor ability to produce both antibody production by B cells, as well as the formation of memory cells, features which are essential for the production of potent vaccines.

Therefore, to produce a potent saccharide-based vaccine, the saccharides of general formulae (I), **(I-1)** - **(I-7),** and **(II-1)-(II-17),** preferred saccharides **A-01** - **A-140, B-01** - **B-140, C-01** - **C-70, D-01** - **D-70, E-01** - **E-70, F-01** - **F-530, G-01** - **G-350, H-01** - **H-70, J-01** - **J-70, K-01** - **K-350, L-01** - **L-112, M-01** - **M-70, N-01** - **N-70, O-01** - **O-70, P-01** - **P-70** and **Q-1** - **Q-700** are conjugated to an immunogenic carrier to provide conjugates, which present increased immunogenicity in comparison with the saccharide. Hence, under the scope of the present application is covered also a conjugate of general formula (**III**) 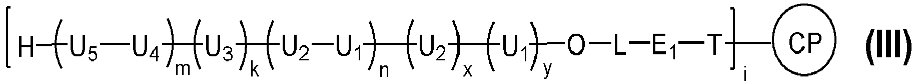 wherein
i is an integer selected from 2 to 18;
**-E₁-** represents a covalent bond, -NH-, -O-NH-, -O-, -S-, -CO-, -CH=CH-, -CONH-, -CO-NHNH-, 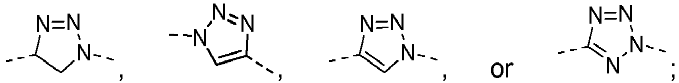
-T- represents 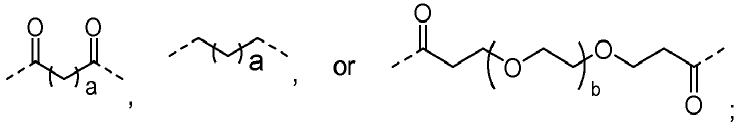
**a** represents an integer from 1 to 10;
**b** represents an integer from 1 to 4;
**CP** is a carrier protein; and
**U₁, U₂, U₃, U₄, U₅ L, m, n, k, x,** and **y** have the same meanings as defined herein.

Preferably **E₁** is a covalent bond, -NH-, -CH=CH-, -CONH-, 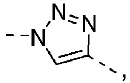 or

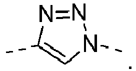

Said conjugate consists of at least one synthetic saccharide of the general formula (**I**) and an immunogenic carrier, preferred carrier proteins, to which the at least one saccharide (**I**) is covalently bound.

Surprisingly, it was found that immunization with a conjugate comprising a saccharide of general formula (**I**) covalently linked to an immunogenic carrier, preferred carrier proteins, results in the production of high titers of antibodies specific to the carbohydrate part of the saccharide of general formula (I) Said antibodies are cross-reacting with the *Klebsiella pneumoniae* serotype O1, O2, O2ac, O8 O-polysaccharide as well as carbapenem-resistant *Klebsiella pneumoniae* ST258 O-polysaccharide and present opsonophagocytosis and bactericidal activity, thus conferring protection against *Klebsiella pneumoniae.*
Preferred, the *Klebsiella pneumoniae* bacteria is selected from O-serotypes comprising or consisting of O1, O2, O2ac, O3, O4, O5, O7, O8, O12 and subtypes thereof and carbapenem resistant *Klebsiella pneumoniae* (CRKP). Preferred, O-serotypes O1, O2a, O2ab, O2ac, O2ae, O2aeh, O2afg, O8, and CRKP strain ST 258, more preferred O1, O2a, O2ab, O2ac, O2afg, O8, CRKP strain ST 258. Still more preferred, *Klebsiella pneumoniae* strains serotypes are O1:K1, O1:K2, O1:K7, O1:K8, O1:K10, O1:K12, O1:K16, O1:K19, O1:K21, O1:K22, O1:K27, O1:K34, O1:K42, O1:K45, O1:K55, O1:K57, O1:K62, O1:K65, O1:K66, O1:K69 and O1:K70, O2a, O2ac, and CRKP strain ST 258.

Vaccines containing at least one conjugate of the present invention cause fewer side effects and/or non-protective immune responses in comparison to vaccines containing isolated (and not synthesized) mixtures of saccharides obtained by non-selective cleavage of the capsular polysaccharide of *Klebsiella pneumoniae* or conjugates thereof. Moreover the inventive vaccines can be easier manufactured in accordance with the GMP regulations than the vaccines containing isolated mixtures of non-selectively cleaved capsular polysaccharides and are easier characterized, which makes stability and purity control easier as well as detection of kind and amount of impurities.

In this context the term "immunogenic carrier" is defined as a structure, which is conjugated to the saccharide to form a conjugate that presents an increased immunity in comparison with the saccharide *per se.* Thus, the conjugate (**III**) is obtained by conjugation of the saccharides of the general formulae **(I), (I-1)-(I-7), (II-1)** - **(II-17),** preferred the saccharides **A-01** - **A-140, B-01** - **B-140, C-01** - **C-70, D-01** - **D-70, E-01** - **E-70, F-01** - **F-530, G-01** - **G-350, H-01** - **H-70, J-01** - **J-70, K-01** - **K-350, L-01** - **L-112, M-01** - **M-70, N-01** - **N-70** and **O-01** - **O-70, P-01** - **P-70** and **Q-1** - **Q-700** to the immunogenic carrier has as effect the stimulation of the immune response against the saccharide of general formula (**I**) without inducing an immune response against said immunogenic carrier.

Most preferred, the conjugate (**III**) is obtained by conjugation of the saccharides selected from the group consisting of: compounds **A-01** - **A-07, A-11** - **A17, A-21** - **A-27, A-31** - **A-37, A-41** - **A-47, A-51** - **A-57, A-61** - **A-67, A-71** - **A-77, A-81** - **A-87, A-91** - **A-97, A-101** - **A-107, A-111** - **A-117, A-121** - **A-127, A-131** - **A-137, F-01, F-19, F-27, F-31, F-36, F-54, F-62, F-66, F-71, F-89, F-97, F-101, F-106, F-124, F-132, F-136, F-141, F-159, F-167, F-171, F-176, F-194, F-202, F-206, F-211, F-229, F-237, F-241, F-246, F-264, F-299, F-281, F-272, F-276, F-307, F-311, F-316, F-334, F-342, F-346, F-351, F-414, F-417, F-421, F-426, F-444, F-452, F-456, F-461, F-479, F-487, F-491, F-496, F-514, F-522, F-526, K-01, K-06, K-11, K-26, K-31, K-36, K-51, K-56, K-61, K-76, K-81, K-86, K-101, K-106, K-111, K-126, K-131, K-136, K-151, K-156, K-161, K-176, K-181, K-186, K-201, K-206, K-211, K-226, K-231, K-236, K-251, K-256, K-261, K-276, K-281, K-286, K-301, K-306, K-311, K-326, K-331, K-336, O-01, O-02, O-03, O-06, O-07, O-08, O-11, O-12, O-13, O-16, O-17, O-18, O-21, O-22, O-23, O-26, O-27, O-28, O-31, O-32, O-33, O-36, O-37, O-38, O-41, O-42, O-43, O-46, O-47, O-48, O-51, O-52, O-53, O-56, O-57, O-58, O-61, O-62, O-63, O-66, O-67, O-88, P-01** - **P-03, P-06** - **P-08, P-11** - **P-13, P-16** - **P-18, P-21** - **P-23, P-26** - **P-28, P-31** - **P-33, P-36** - **P-38, P-41** - **P-43, P-46** - **P-48, P-51** - **P-53, P-56** - **P-58, P-61** - **P-63, P-66** - **P-68, Q-1, Q-26, Q-101, Q-151, Q-251, Q-301, Q-351, Q-376, Q-451, Q-501, Q-551, Q-601** and **Q-651.**

Preferred immunogenic carriers are carrier proteins (CP) or glycosphingolipids with immunomodulatory properties. For the person skilled in the art, a carrier protein (CP) is a protein selected from the group comprising or consisting of: a diphtheria toxoid, a mutated diphtheria toxoid, a modified diphtheria toxoid, a mutated and modified diphtheria toxoid, a tetanus toxoid, a modified tetanus toxoid, a mutated tetanus toxoid, outer membrane protein (OMP), bovine serum albumin (BSA), keyhole limpet hemocyanine (KLH) or cholera toxoid (CT). The term "toxoid" as used herein refers to a bacterial toxin (usually an exotoxin), whose toxicity has been inactivated or suppressed either by chemical (formalin) or heat treatment, while other properties, typically immunogenicity, are maintained. A mutated toxoid as used herein is a recombinant bacterial toxin, which has been amended to be less toxic or even non-toxic by amending the wild-type amino acid sequence. Such a mutation could be a substitution of one or more amino acids. Such a mutated toxoid presents on its surface a functionality that can react with the functional group Y of the interconnecting molecule to provide a modified toxoid. Said functionality is known to the person skilled in the art and includes, but is not restricted to the primary amino functionality of a lysine residue that can react with activated esters, an isocyanate group or an aldehyde in presence of a reducing agent, to the carboxylate functionality of a glutamate or aspartate residue that can be activated by carbodiimides or to the thiol functionality of a cysteine residue.

Activated esters include N-(γ-maleimidobutyryloxy) sulfosuccinimide ester (sulfo-GMBS), succinimidyl (4-iodoacetyl) aminobenzoate (sulfo-SIAB), succinimidyl-3-(bromoacetamido)propionate (SBAP), disuccinimidyl glutarate (DSG), disuccinimidyl adipate (DSA), 2-pyridyldithiol-tetraoxatetradecane-*N*-hydroxysuccinimide (PEG-4-SPDP), bis-(4-nitrophenyl) adipate and bis-(4-nitrophenyl) succinate (see **Figure 3****).** Preferred activated esters are disuccinimidyl adipate (DSA), disuccinimidyl glutarate (DSG), bis-(4-nitrophenyl) adipate and bis-(4-nitrophenyl) succinate.

The cysteine residue on the carrier protein can be converted to the corresponding dehydroalanine that can be further reacted with a suitable interconnecting molecule to provide modified carrier protein having on their surface the functional group X of the interconnecting molecule.

It is especially preferred that the saccharides of general formula (**I**) are conjugated to the non-toxic mutated diphtheria toxin CRM₁₉₇ as carrier protein (CP) presenting as a functionality a primary amine functionality of a lysine residue.

CRM₁₉₇ like wild-type diphtheria toxin is a single polypeptide chain of 535 amino acids (58 kD) consisting of two subunits linked by disulfide bridges having a single amino acid substitution of glutamic acid for glycine. It is used as a carrier protein in a number of approved conjugate vaccines for diseases, such as Prevnar.

Thus, in a preferred embodiment of the present invention the carrier protein presents on its surface primary amino functionalities of lysine residues that are able to react with the functional group Y of the interconnecting molecule to provide modified carrier protein having on their surface said functional group X of the interconnecting molecule, which is able to react with the terminal amino group of the linker of the compounds of general formula (**I**).

Said functional group X of the interconnecting molecules is selected of the group comprising or consisting of maleimide, α-iodoacetyl, α-bromoacetyl, *N-*hydroxysuccinimide ester (NHS), aldehyde, imidoester, carboxylic acid, alkyl sulfonate, sulfonyl chloride, epoxide, anhydride, carbonate.

Preferred is a conjugate of general formula (**IV**) 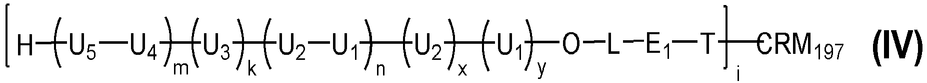 wherein
**i** is an integer selected from 2 to 18;
**-E₁-** represents a covalent bond, -NH-, -O-NH-, -S-, -CO-, -CH=CH-, -CONH-, -CO-NHNH-, 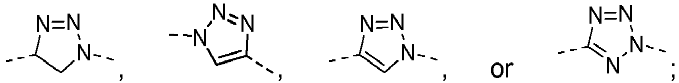
-T- represents 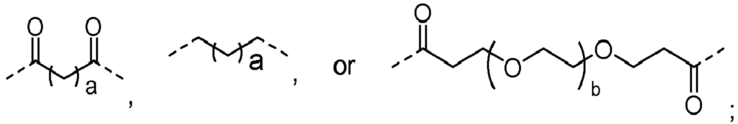
**a** represents an integer from 1 to 10;
**b** represents an integer from 1 to 4; and
**U₁, U₂, U₃, U₄, U₅, L**, **m**, **n**, **k**, **x**, and **y** have the same meanings as defined herein such as in general formula **(I).**

Preferred is a conjugate of general formula (**IV**) 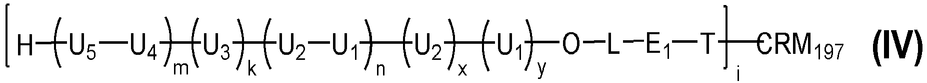 wherein
**i** is an integer selected from 2 to 18;
**-E₁-** represents a covalent bond, -NH-, -O-NH-, -S-, -CO-,
-CH=CH-, -CONH-, -CO-NHNH-, 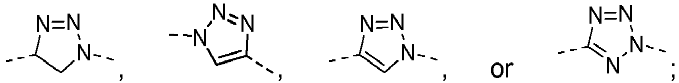
**-T-** represents 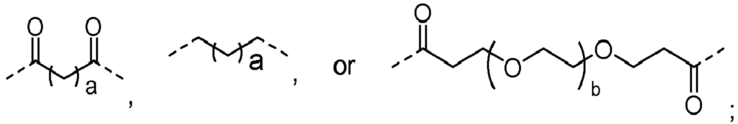
**a** represents an integer from 1 to 10;
**b** represents an integer from 1 to 4; and
when **U₂-U₁** represents 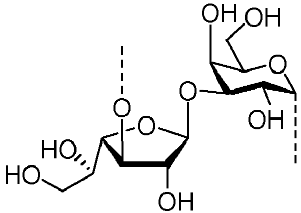
**m** cannot be 0 and **U₅-U₄** cannot be **U₂-U₁;**
**U₁, U₂, U₃, U₄, U₅, L, m, n, k, x,** and **y** have the same meanings as defined herein such as in general formula (**I**).

Preferably **E₁** is a covalent bond, -NH-, -CH=CH-, -CONH-, 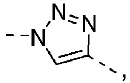 or

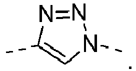

Preferred is the conjugate of the formula (**IV**) 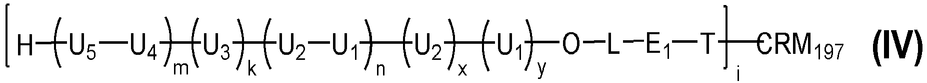 wherein
**U₁** represents 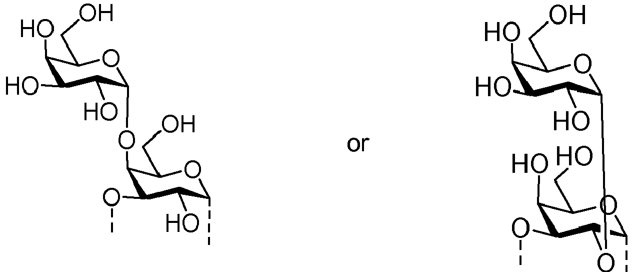
**U₂** represents 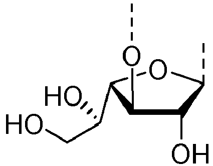
**U₃** represents 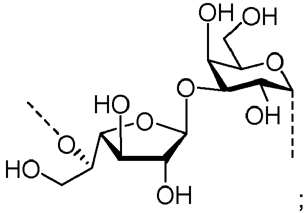
**U₅** represents a covalent bond or 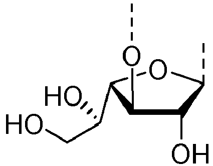
**L, E₁, i, m, n, k, x,** and **y** have the same meanings as defined herein, or anomers, hydrates, or pharmaceutically acceptable salt thereof.

Preferred is also the conjugate of the formula **(IV)** 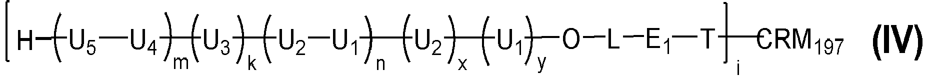 wherein
**U₁** represents 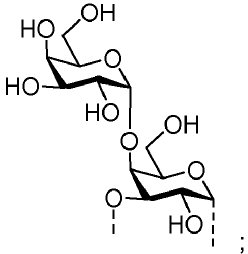
**U₂** represents 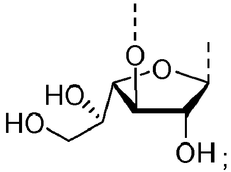
**U₄** represents 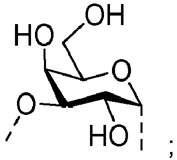
**U₅** represents a covalent bond, or 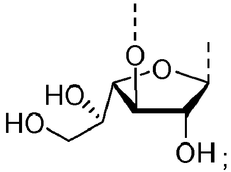
**m** is an integer from 0, or 1;
**L, E₁, i, n, k, x,** and **y** have the same meanings as defined herein.

Preferred, are synthetic saccharides of general formula (**IV**), wherein
**U₁** represents 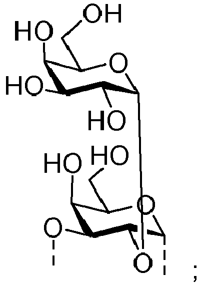
**U₂** represents 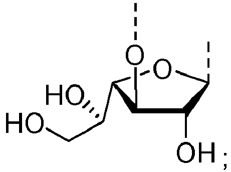
**U₄** represents 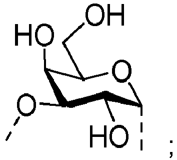
**U5** represents a covalent bond, or 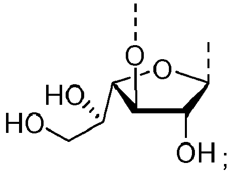
**m** is an integer selected from 0 and 1;
**L, E₁, i, n, k, x,** and **y** have the meanings as defined herein.

Preferred is the conjugate of the formula **(IV)** wherein
**U₁** represents 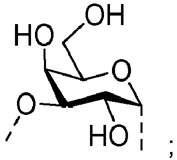
**U₂** represents 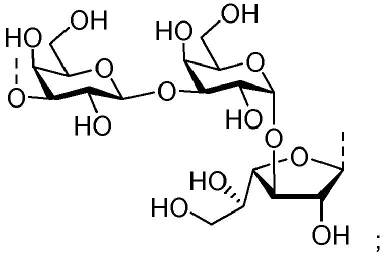
**U₄** represents 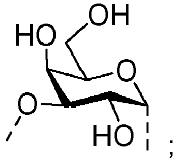
**U₅** represents a covalent bond, or 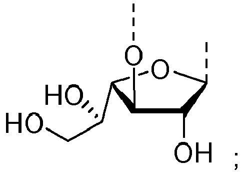
**m** is an integer from 0 or 1,
**L, E₁, i, n, k, x,** and **y** have the same meanings as defined herein.

Preferred is the conjugate of the formula **(IV),** wherein
**U₁** represents 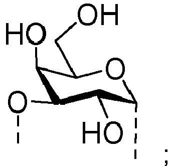
**U₂** represents 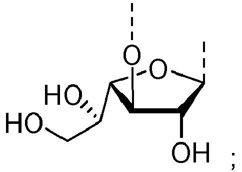
**U₄** represents 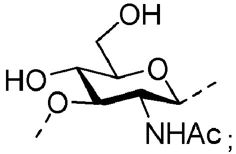
**U₅** represents a covalent bond, or 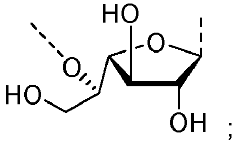
**m** is an integer from 1 to 10,
**L, E₁, i, n, k, x,** and **y** have the same meanings as defined herein.

Preferred is also the conjugate of the formula (V) 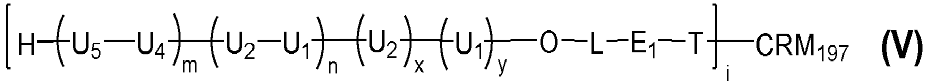 wherein
**U₁** represents 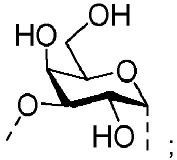
**U₂** represents 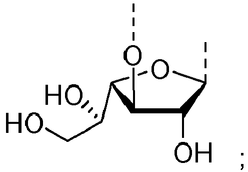
**U₄** represents 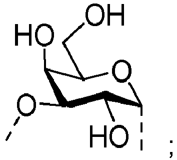
**U₅** represents a covalent bond or 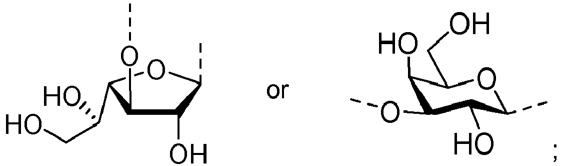
**L, E₁, i, m, n, k, x,** and **y** have the same meanings as defined herein.

Preferred is also the conjugate of the formula (V), wherein
**U₁** represents 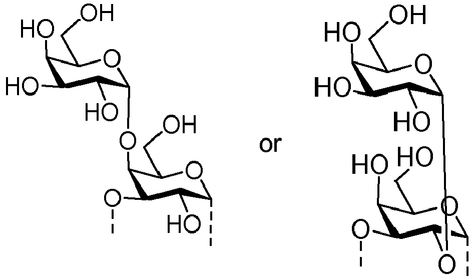
**U₂** represents 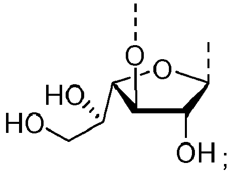
**U₄** represents 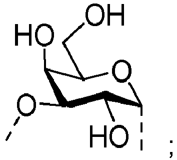
**U₅** represents a covalent bond, or 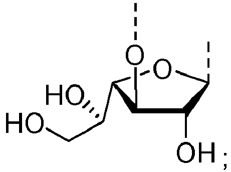
**L, E₁, i, m, n, k, x,** and **y** have the same meanings as defined herein.

Preferred is also the conjugate of the formula (**V**), wherein
**U₁** represents 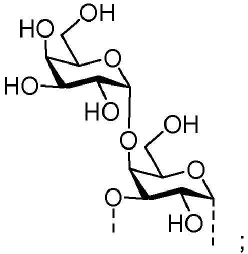
**U₂** represents 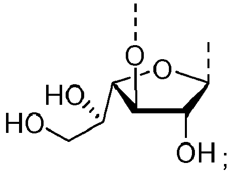
**U₄** represents 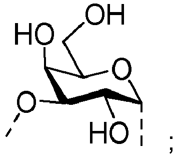
**U₅** represents a covalent bond, or 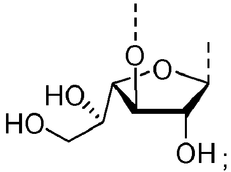
**L, E₁, i, m, n, k, x,** and **y** have the same meanings as defined herein.

Preferred is also the conjugate of the formula (**V**), wherein
**U₁** represents 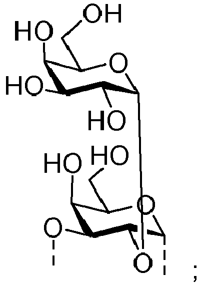
**U₂** represents 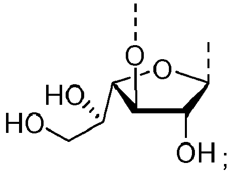
**U₄** represents 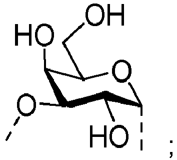
**U₅** represents a covalent bond, or 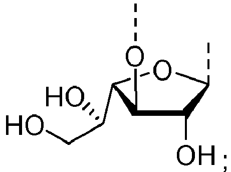
**L, E₁, i, m, n, k, x,** and **y** have the same meanings as defined herein.

Preferred is also the conjugate of the formula (**V**), wherein
**U₁** represents 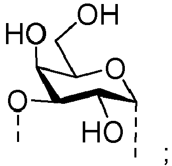
**U₂** represents 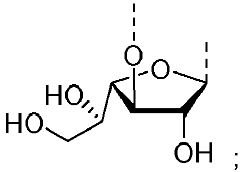
**U₄** represents 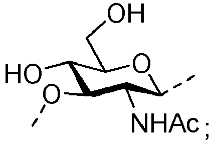
**U₅** represents a covalent bond, or 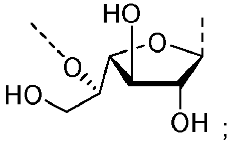
**m** is an integer from 1 to 10;
**L, E₁, i, n, k, x,** and **y** have the same meanings as defined herein.

More preferred is a conjugate of any one of the formulae **(V-1)** - **(V-17):** 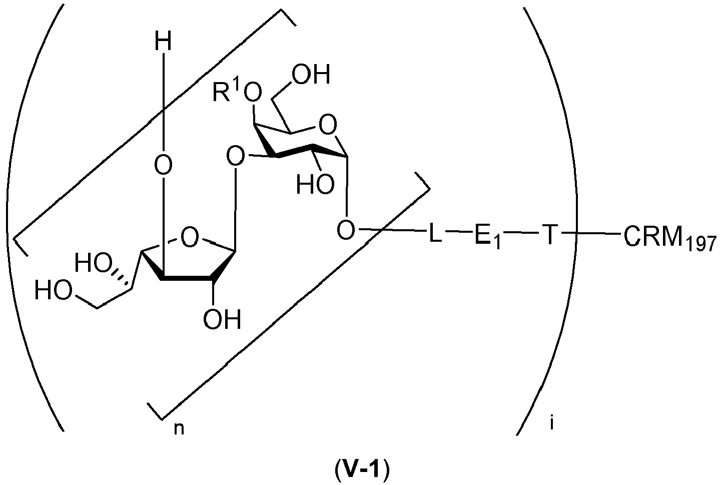 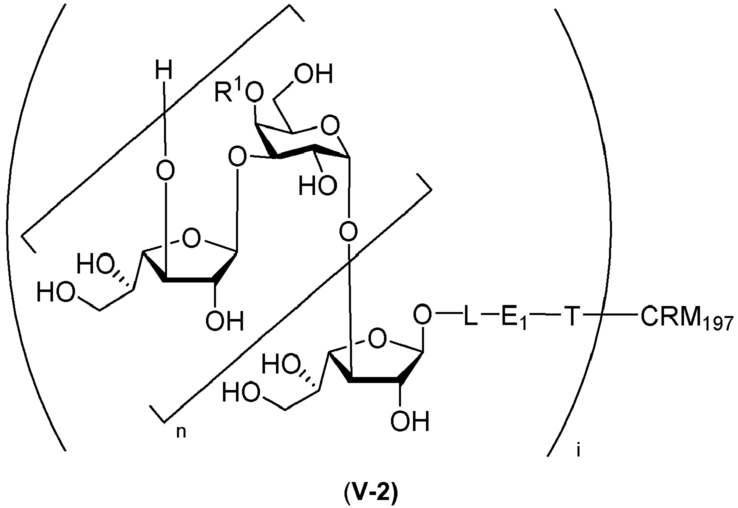 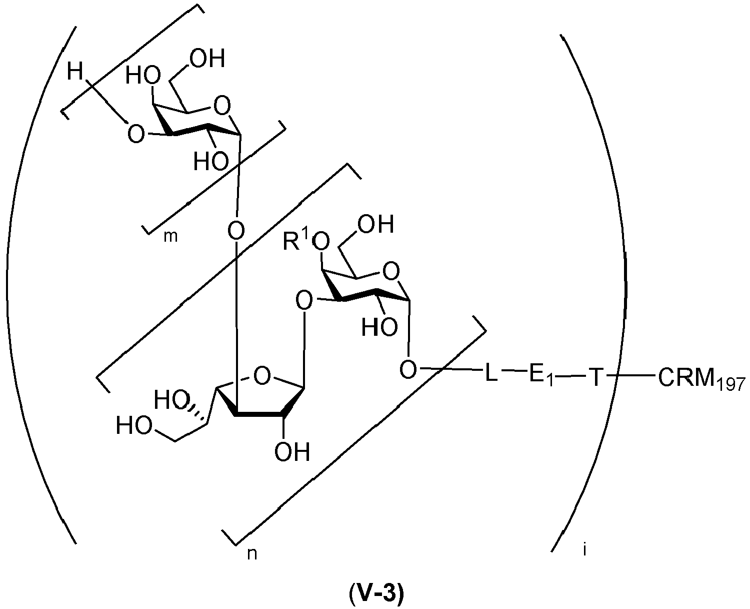 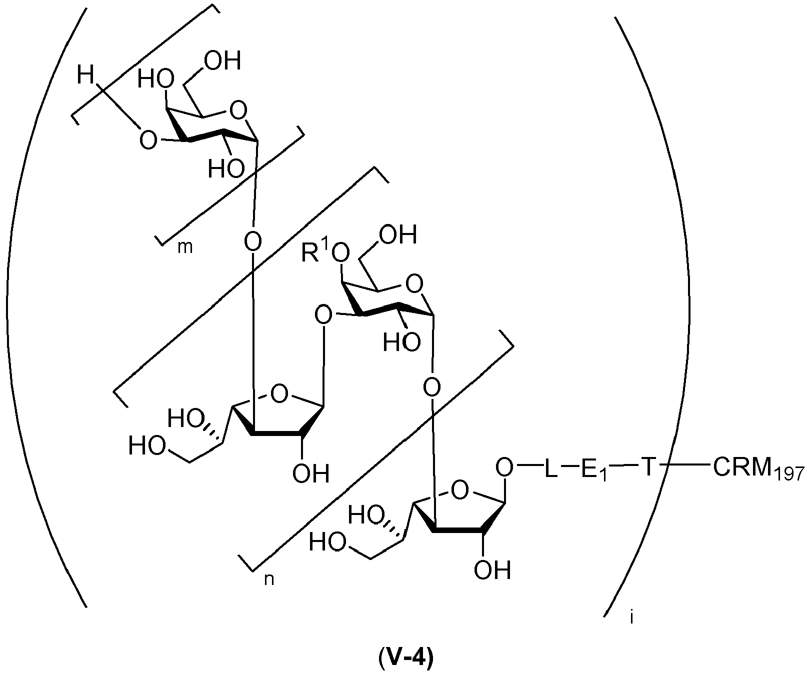 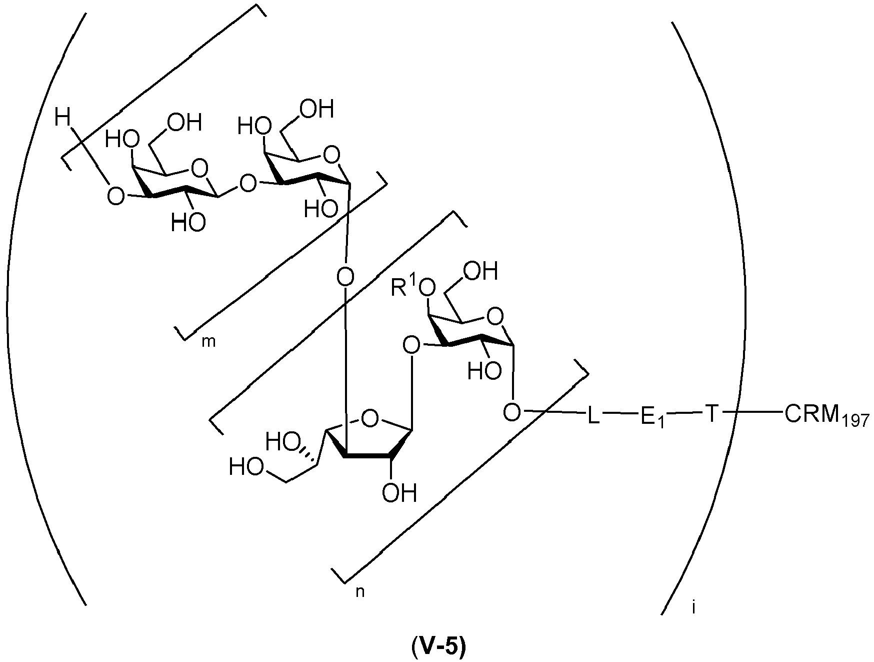 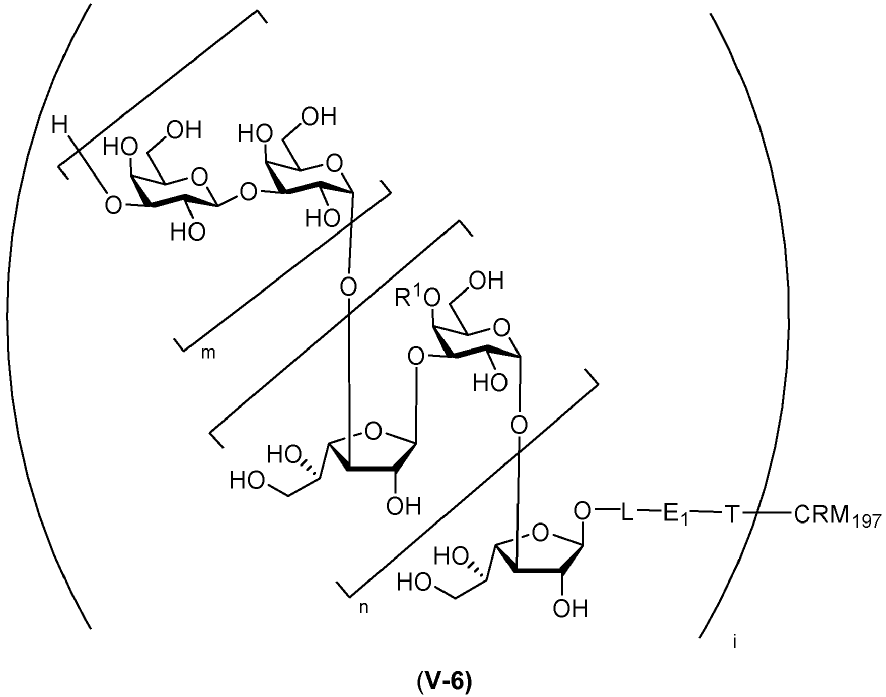 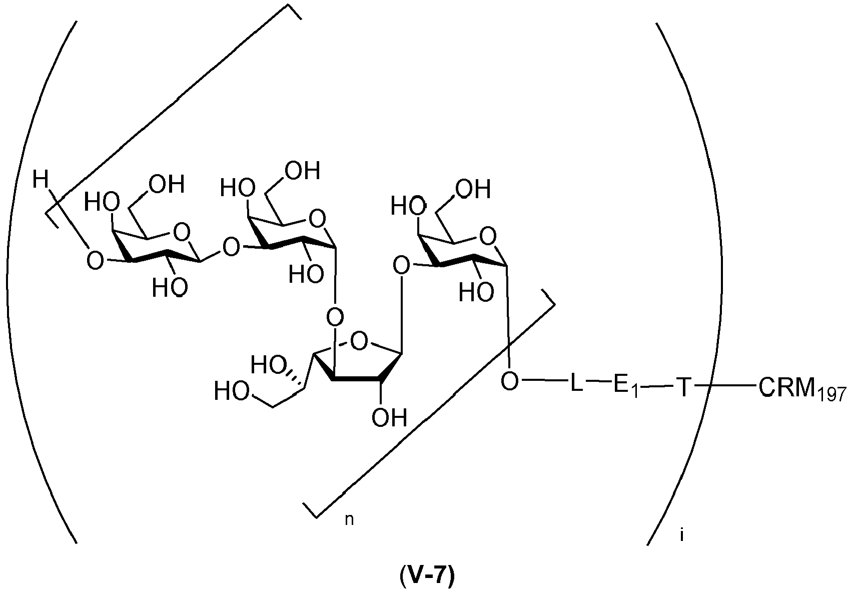 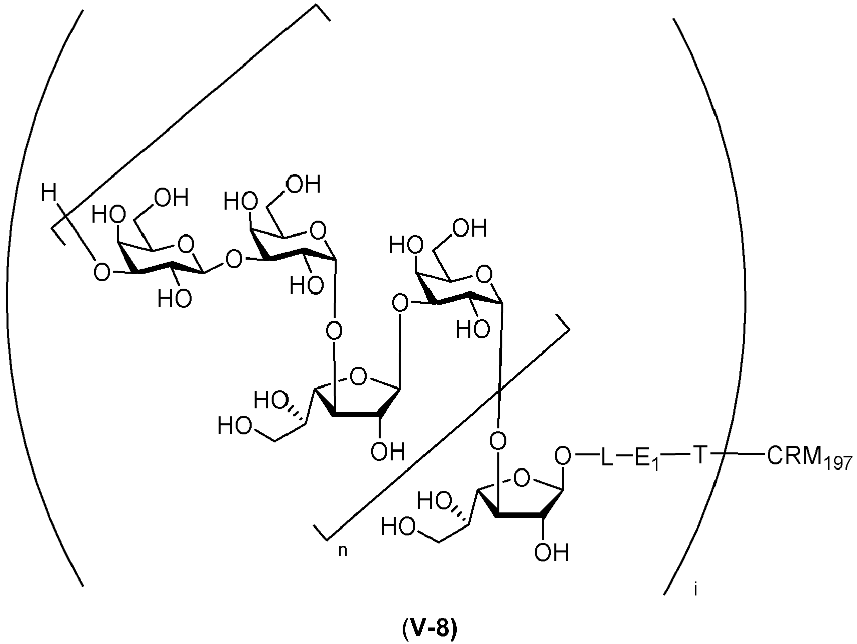 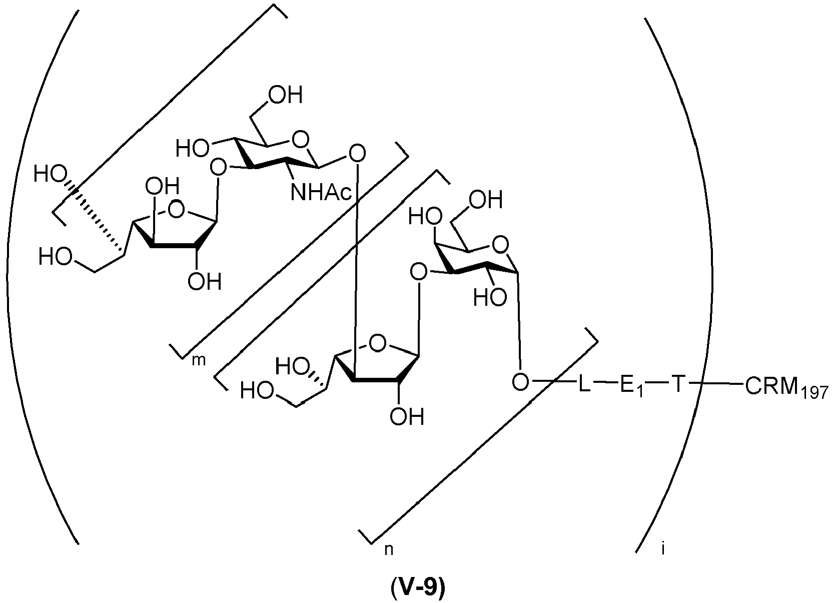 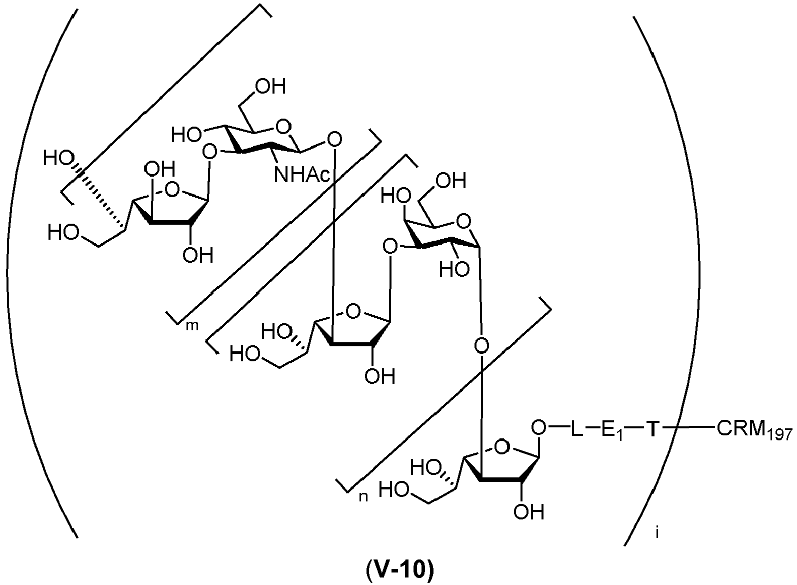 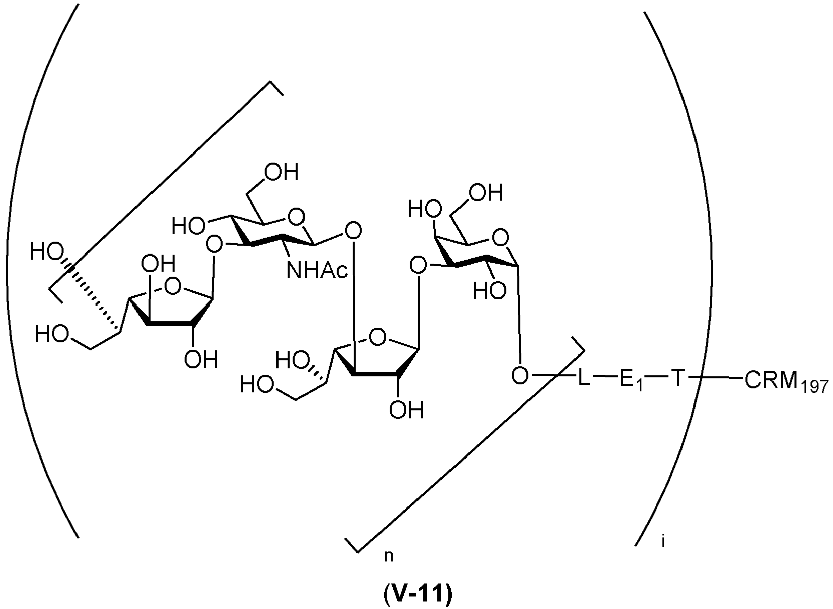 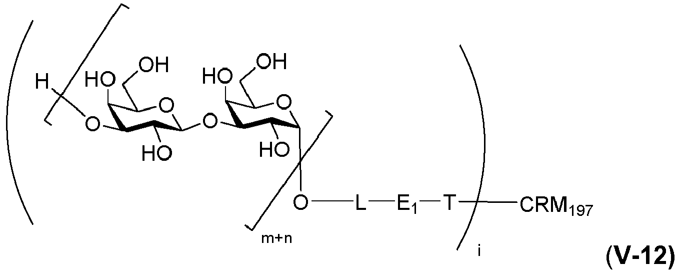 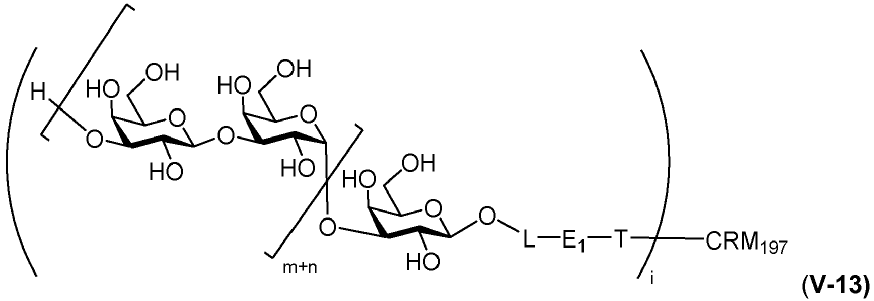 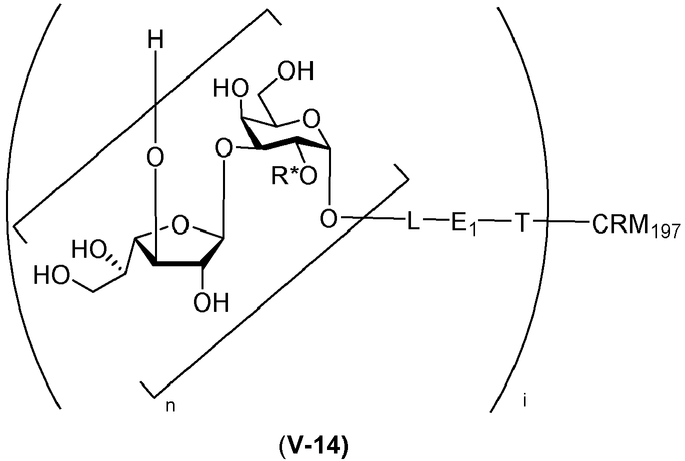 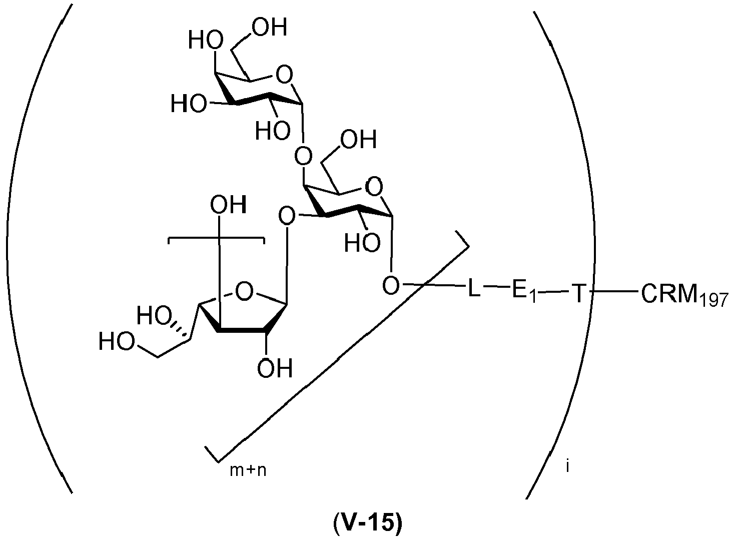 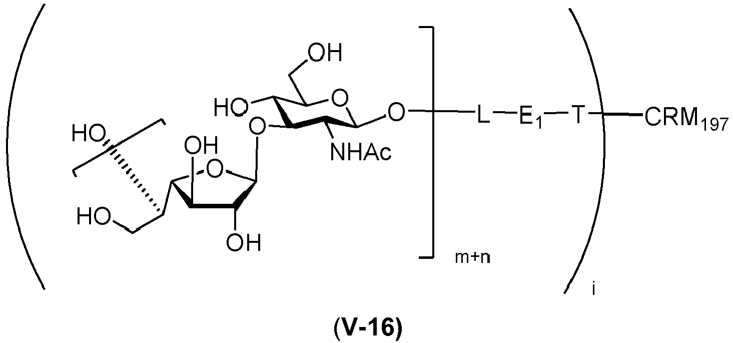 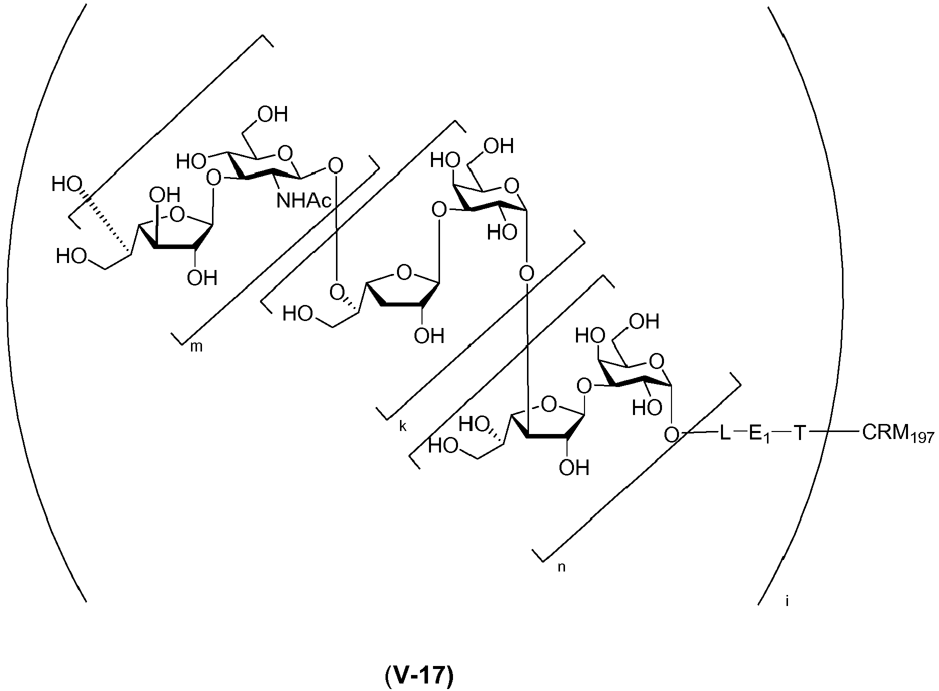 wherein
**R¹** and **R*** represent independently -H, or 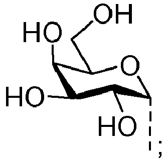
wherein **R¹** and **R*** cannot be simultaneously 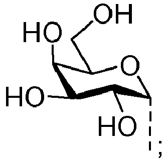 and
**L, E₁, T, i, m, k,** and **n** have the same meanings as defined above, preferably, **n** and **m** is an integer from 1 to 10.

More preferred the conjugate of any one of the formulae (**III**), (**IV**), (**V**) and (**V**-**1**) - (**V-17**), wherein **n** is an integer from 1 to 10.
More preferred the conjugate of any one of the formulae (**III**), (**IV**), (**V**) and (**V-1**) - (**V-17**), wherein **i** is selected from 4 to 10.

Preferably -T- represents 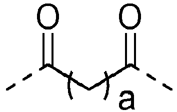 and a is an integer selected from 2, 3, 4, 5 and 6.

Thus, a conjugate of any one of general formulae (**IV**), (**V**) and (**V-1**) - (**V-17**), wherein -T- represents 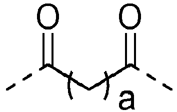 and a is an integer selected from 2, 3, 4, 5 and 6 is especially preferred.

Preferably, the linker **-L-** represents **-L^{a}-, -L^{a}-L^{e}-, -L^{a}-L^{b}-L^{e}-,** or **-L^{a}-L^{d}-L^{e}-; -L^{a}-** represents -(CH₂)ₒ-, -(CH₂-CH₂-O)ₒ-C₂H₄-, or -(CH₂-CH₂-O)ₒ-CH₂; **-L^{b}-** represents -O-;
**-L^{d}-** represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂-;
**-L^{e}-** represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂-; and
**o, q, p1** and **p2** are independently of each other an integer selected from 1, 2, 3, 4, 5, and 6

In the most preferred embodiment, **E₁** is a covalent bond, -NH-, -CH=CH-, -CONH-,

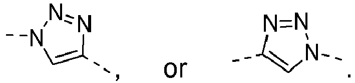

In another embodiment, said immunogenic carrier is preferably a glycosphingolipid with immunomodulatory properties, and more preferably (2*S*,3*S*,4*R*)-1-(α-D-galactopyranosyl)-2-hexacosanoylaminooctadecane-3,4-diol. The term glycosphingolipid with immunomodulatory properties, as used herein, refers to a suitable glycosphingolipid capable of stimulating the immune system's response to a target antigen, but which does not in itself confer immunity as defined above.

Glycosphingolipids as used herein are compounds containing a carbohydrate moiety α-linked to a sphingolipid. Preferably, the carbohydrate moiety is a hexopyranose and most preferably is α-D-galactopyranose. For the person skilled in the art, sphingolipids are a class of lipids containing a C18 amino alcohol connected *via* an amide bond to a fatty acid. The C18 amino alcohol is preferably mono-, di- or polysubstituted with hydroxyl groups. Especially preferred, the C18 amino alcohol is phytosphingosine. The fatty acid is preferably a monocarboxylic acid having a saturated alkyl chain of a number of carbons ranging from 16 to 28 and more preferably from 18 to 26. Glycosphingolipids with immunomodulatory properties include, but they are not restricted to (2*S*,3*S*,4*R*)-1-(α-D-galactopyranosyl)-2-hexacosanoylaminooctadecane-3,4-diol, which can stimulate natural killer (NK) activity and cytokine production by natural killer T (NKT) cells and exhibits potent antitumor activity *in vivo* (Proc. Natl Acad. Sci. USA, 1998, 95, 5690).

The conjugates of the saccharides of general formula I with a glycosphingolipid with immunomodulatory properties have the advantage of being heat stable. Additionally, they are able to produce in mice high titers of IgG1, IgG2a and IgG3 antibodies against the saccharide of general formula (I) and the O-polysaccharide of CRKP. To be suitable for conjugation, a functionality is introduced on the glycosphingolipid with immuno-modulatory properties. Said functionality is prone to react directly with the terminal amino group of the linker of the saccharides of general formula (**I**) to provide conjugates of the saccharides of general formula (**I**), or with the functional group Y of the interconnecting molecule to provide the modified glycosphingolipid with immunomodulatory properties.

Preferably, said functionality is introduced at the carbon 6 of the galactose moiety of the glycosphingolipid with immunomodulatory properties. Thus, the glycosphingolipid with immunomodulatory properties is functionalized with a functionality, which is prone of reacting with the terminal amino group of the saccharides or with the functional group Y of the interconnecting molecule. A functionality prone to react with an amino group includes, but it is not restricted to activated ester, isocyanate group, aldehyde, epoxide, imidoester, carboxylic acid, alkyl sulfonate and sulfonyl chloride. A functionality prone to react with the functional group Y of the interconnecting molecule so that to provide the modified glycosphingolipid with immunomodulatory properties presenting the functional group X of the interconnecting molecule includes, but it is not restricted to amine, alcohol, thiol, activated ester, isocyanate group, aldehyde, epoxide, vinyl, imidoester, carboxylic acid, alkyl sulfonate, sulfonyl chloride, vinyl group, alkynyl group and azido group.

Preferably, the functionality introduced at the C6 of the carbohydrate moiety of the glycosphingolipid with immunomodulatory properties is selected from the group comprising or containing an amine, a thiol, an alcohol, a carboxylic acid, a vinyl, maleimide, α-iodoacetyl, α-bromoacetyl, *N*-hydroxysuccinimide ester (NHS) and 2-pyridyldithiols.

Said functional group X of the interconnecting molecules is selected of the group comprising or consisting of: maleimide, α-iodoacetyl, α-bromoacetyl, *N*-hydroxysuccinimide ester (NHS), aldehyde, carboxylic acid, epoxyde, alkyl sulfonate, sulfonyl chloride, anhydride and carbonate.

As used herein, the term "interconnecting molecule" refers to a bifunctional molecule containing functional group X and functional group Y, wherein functional group X is capable of reacting with the terminal amino group on the linker -L- and the functional group Y is capable of reacting with a functionality present on the immunogenic carrier or on the solid support.

It was found that a conjugate comprising at least one saccharide of any one of general formulae **(I), (I-A), (I-B), (I-1)** - **(I-7), (II-1)** - **(II-17),** preferred any one of the saccharides **A-01** - **A-140, B-01** - **B-140, C-01** - **C-70, D-01** - **D-70, E-01** - **E-70, F-01** - **F-530, G-01** - **G-350, H-01** - **H-70, J-01** - **J-70, K-01** - **K-350, L-01** - **L-112, M-01** - **M-70, N-01** - **N-70, O-01** - **O-70, P-01** - **P-70** and **Q-1** - **Q-700.** and particularly a conjugate of any one of general formulae (**III**), (**IV**), (**V**) and (**V-1**) - (**V-17**), elicits a protective immune response in a human and/or animal host, and therefore is useful for prevention and/or treatment of diseases associated with *Klebsiella pneumoniae* bacteria. Thus, the conjugates comprising the saccharides of general formula (**I**) conjugated to an immunogenic carrier are useful for prevention and/or treatment of diseases associated with *Klebsiella pneumoniae* bacteria. The diseases associated with *Klebsiella pneumoniae* bacteria include pneumonia, bronchitis, meningitis, urinary tract infection, nosocomial pneumonia, intra-abdominal infections, wound infection, infection of blood, osteomyelitis, bacteremia, septicemia and ankylosing spondylitis.

Preferred, the *Klebsiella pneumoniae* bacteria is selected from O-serotypes comprising or consisting of O1, O2, O2ac, O3, O4, O5, O7, O8, O12 and subtypes thereof and carbapenem resistant *Klebsiella pneumoniae* (CRKP). Preferred, O-serotypes O1, O2a, O2ab, O2ac, O2ae, O2aeh, O2afg, O8, and CRKP strain ST 258, more preferred O1, O2a, O2ab, O2ac, O2afg, O8, CRKP strain ST 258. Still more preferred, *Klebsiella pneumoniae* strains serotypes are O1:K1, O1:K2, O1:K7, O1:K8, O1:K10, O1:K12, O1:K16, O1:K19, O1:K21, O1:K22, O1:K27, O1:K34, O1:K42, O1:K45, O1:K55, O1:K57, O1:K62, O1:K65, O1:K66, O1:K69 and O1:K70, O2a, O2ac, and CRKP strain ST 258.

### Pharmaceutical compositions

Another aspect of the present invention is directed to a pharmaceutical composition or a vaccine comprising at least one conjugate that comprises at least one saccharide of general formula (**I**) conjugated to an immunogenic carrier and/or at least one saccharide of general formula (**I**) as an active ingredient together with at least one pharmaceutically acceptable adjuvant and/or excipient. Said pharmaceutical composition can be used for raising a protective immune response in a human and/or animal host. Ideally, the pharmaceutical composition is suitable for use in humans.
Particularly said pharmaceutical composition or said vaccine elicits a protective immune response in a human and/or animal host, and therefore is useful for prevention and/or treatment of diseases associated with *Klebsiella pneumoniae* bacteria. Thus, said pharmaceutical composition or said vaccine is useful for prevention and/or treatment of diseases associated with *Klebsiella pneumoniae* bacteria. The diseases associated with *Klebsiella pneumoniae* bacteria include pneumonia, bronchitis, meningitis, urinary tract infection, nosocomial pneumonia, intra-abdominal infections, wound infection, infection of blood, osteomyelitis, bacteremia, septicemia and ankylosing spondylitis.

Preferred, said pharmaceutical composition or said vaccine is useful for prevention and/or treatment of diseases associated with *Klebsiella pneumoniae* bacteria. wherein the *Klebsiella pneumoniae* bacteria is selected from O-serotypes comprising or consisting of O1, O2, O2ac, O3, O4, O5, O7, O8, O12 and subtypes thereof and carbapenem resistant *Klebsiella pneumoniae* (CRKP). Preferred, O-serotypes O1, O2a, O2ab, O2ac, O2ae, O2aeh, O2afg, O8, and CRKP strain ST 258, more preferred O1, O2a, O2ab, O2ac, O2afg, O8, CRKP strain ST 258. Still more preferred, *Klebsiella pneumoniae* strains serotypes are O1:K1, O1:K2, O1:K7, O1:K8, O1:K10, O1:K12, O1:K16, O1:K19, O1:K21, O1:K22, O1:K27, O1:K34, O1:K42, O1:K45, O1:K55, O1:K57, O1:K62, O1:K65, O1:K66, O1:K69 and O1:K70, O2a, O2ac, and CRKP strain ST 258.

Preferred, the pharmaceutical composition or a vaccine comprises at least one saccharide of any one of general formulae **(I-1)** - **(I-7), (II-1)** - **(II-17)** and/or at least one of the conjugates comprising at least one saccharide of any one of general formulae **(I-1)** - **(I-7), (II-1)** - **(II-17)** as an active ingredient.
Particularly, the pharmaceutical composition or a vaccine comprises at least one conjugate of any one of general formulae **(III), (IV), (V)** and **(V-1)** - **(V-17),**
More preferred, the pharmaceutical composition or a vaccine comprises at least one of the saccharides **A-01** - **A-140, B-01** - **B-140, C-01** - **C-70, D-01** - **D-70, E-01** - **E-70, F-01 - F-530, G-01 - G-350, H-01 - H-70, J-01 - J-70, K-01 - K-350, L-01 - L-112, M-01 - M-70, N-01 - N-70, O-01** - **O-70,** and **P-01 - P-70.** and/or at least one of the conjugates comprising at least one of the saccharides **A-01 - A-140, B-01** - **B-140, C-01 - C-70, D-01 - D-70, E-01 - E-70, F-01 - F-530, G-01 - G-350, H-01** - **H-70, J-01 - J-70, K-01 - K-350, L-01 - L-112, M-01 - M-70, N-01 - N-70, O-01** - **O-70, P-01 - P70** and **Q-1 - Q-700.**
Most preferred, More preferred, the pharmaceutical composition or a vaccine comprises at least one of the saccharides **A-01 - A-07, A-11 - A17, A-21 - A-27, A-31 - A-37, A-41 - A-47, A-51 - A-57, A-61 - A-67, A-71 - A-77, A-81 - A-87, A-91 - A-97, A-101 - A-107, A-111 - A-117, A-121 - A-127, A-131 - A-137, F-01, F-19, F-27, F-31, F-36, F-54, F-62, F-66, F-71, F-89, F-97, F-101, F-106, F-124, F-132, F-136, F-141, F-159, F-167, F-171, F-176, F-194, F-202, F-206, F-211, F-229, F-237, F-241, F-246, F-264, F-299, F-281, F-272, F-276, F-307, F-311, F-316, F-334, F-342, F-346, F-351, F-414, F-417, F-421, F-426, F-444, F-452, F-456, F-461, F-479, F-487, F-491, F-496, F-514, F-522, F-526, K-01, K-06, K-11, K-26, K-31, K-36, K-51, K-56, K-61, K-76, K-81, K-86, K-101, K-106, K-111, K-126, K-131, K-136, K-151, K-156, K-161, K-176, K-181, K-186, K-201, K-206, K-211, K-226, K-231, K-236, K-251, K-256, K-261, K-276, K-281, K-286, K-301, K-306, K-311, K-326, K-331, K-336, O-01, O-02, O-03, O-06, O-07, O-08, O-11, 0-12, O-13, O-16, O-17, O-18, O-21, O-22, O-23, O-26, O-27, O-28, O-31, O-32, O-33, O-36, O-37, O-38, O-41, O-42, O-43, O-46, O-47, O-48, O-51, O-52, O-53, O-56, O-57, O-58, O-61, O-62, O-63, O-66, O-67, O-88, P-01 - P-03, P-06 - P-08, P-11 - P-13, P-16 - P-18, P-21 - P-23, P-26 - P-28, P-31 - P-33, P-36 - P-38, P-41 - P-43, P-46 - P-48, P-51 - P-53, P-56 - P-58, P-61 - P-63, P-66 - P-68, Q-1, Q-26, Q-101, Q-151, Q-251, Q-301, Q-351, Q-376, Q-451, Q-501, Q-551, Q-601** and **Q-651.** and/or at least one of the conjugates comprising at least one of the saccharides **A-01 - A-07, A-11 - A17, A-21 - A-27, A-31** - **A-37, A-41 - A-47, A-51 - A-57, A-61 - A-67, A-71 - A-77, A-81 - A-87, A-91 - A-97, A-101 - A-107, A-111 - A-117, A-121 - A-127, A-131 - A-137, F-01, F-19, F-27, F-31, F-36, F-54, F-62, F-66, F-71, F-89, F-97, F-101, F-106, F-124, F-132, F-136, F-141, F-159, F-167, F-171, F-176, F-194, F-202, F-206, F-211, F-229, F-237, F-241, F-246, F-264, F-299, F-281, F-272, F-276, F-307, F-311, F-316, F-334, F-342, F-346, F-351, F-414, F-417, F-421, F-426, F-444, F-452, F-456, F-461, F-479, F-487, F-491, F-496, F-514, F-522, F-526, K-01, K-06, K-11, K-26, K-31, K-36, K-51, K-56, K-61, K-76, K-81, K-86, K-101, K-106, K-111, K-126, K-131, K-136, K-151, K-156, K-161, K-176, K-181, K-186, K-201, K-206, K-211, K-226, K-231, K-236, K-251, K-256, K-261, K-276, K-281, K-286, K-301, K-306, K-311, K-326, K-331, K-336, O-01, O-02, O-03, O-06, O-07, O-08, O-11, O-12, O-13, O-16, O-17, O-18, O-21, O-22, O-23, O-26, O-27, O-28, O-31, O-32, O-33, O-36, O-37, O-38, O-41, O-42, O-43, O-46, O-47, O-48, O-51, O-52, O-53, O-56, O-57, O-58, O-61, O-62, O-63, O-66, O-67, O-88, P-01** - **P-03, P-06** - **P-08, P-11** - **P-13, P-16** - **P-18, P-21** - **P-23, P-26** - **P-28, P-31** - **P-33, P-36** - **P-38, P-41** - **P-43, P-46** - **P-48, P-51** - **P-53, P-56** - **P-58, P-61** - **P-63, P-66** - **P-68, Q-1, Q-26, Q-101, Q-151, Q-251, Q-301, Q-351, Q-376, Q-451, Q-501, Q-551, Q-601** and **Q-651.**

In another aspect of the present invention, said pharmaceutical composition or vaccine further comprises at least one of capsular polysaccharides, O-polysaccharides and/or capsular polysaccharide fragments, O-polysaccharide fragments and/or protein conjugates thereof of *Klebsiella pneumoniae* bacteria selected from the group comprising or consisting of *Klebsiella pneumoniae* serotypes O1, O2, O2a, O2ac, O3, O4, O5, O7, O8, O12 and carbapenem-resistant *Klebsiella pneumoniae* ST258.

The term "adjuvant" as used herein refers to an immunological adjuvant i.e. a material used in a vaccine composition that modifies or augments the effects of said vaccine by enhancing the immune response to a given antigen contained in the vaccine without being antigenically related to it. For the persons skilled in the art, classically recognized examples of immunological adjuvants include, but are not restricted to oil emulsions (e.g. Freund's adjuvant), saponins, aluminum or calcium salts (e.g. alum), non-ionic block polymer surfactants, and many others.

Pharmaceutical compositions are preferably in aqueous form, particularly at the point of administration, but they can also be presented in non-aqueous liquid forms or in dried forms e.g. as gelatin capsules, or as lyophilisates, etc.

Pharmaceutical compositions may include one or more preservatives, such as thiomersal or 2-phenoxyethanol. Mercury-free compositions are preferred, and preservative-free vaccines can be prepared.

Pharmaceutical compositions may include a physiological salt, such as a sodium salt e.g. to control tonicity. Sodium chloride (NaCl) is typical and may be present at between 1 and 20 mg/ml. Other salts that may be present include potassium chloride, potassium dihydrogen phosphate, disodium phosphate dehydrate, magnesium chloride, calcium chloride, etc..

Pharmaceutical compositions can have an osmolality of between 200 mOsm/kg and 400 mOsm/kg.

Pharmaceutical compositions may include compounds (with or without an insoluble metal salt) in plain water (e.g. w.f.i.), but will usually include one or more buffers. Typical buffers include: a phosphate buffer; a Tris buffer; a borate buffer; a succinate buffer; a histidine buffer (particularly with an aluminium hydroxide adjuvant); or a citrate buffer. Buffer salts will typically be included in the 5-20 mM range.

Pharmaceutical compositions typically have a pH between 5.0 and 9.5 e.g. between 6.0 and 8.0.

Pharmaceutical compositions are preferably sterile and gluten free.

Pharmaceutical compositions are suitable for administration to animal (and, in particular, human) patients, and thus include both human and veterinary uses. They may be used in a method of raising an immune response in a patient, comprising the step of administering the composition to the patient.

The pharmaceutical compositions of the present invention may be administered before a subject is exposed to a *Klebsiella pneumoniae* and/or after a subject is exposed to a *Klebsiella pneumoniae.*

Preferred, the *Klebsiella pneumoniae* bacteria is selected from O-serotypes comprising or consisting of O1, O2, O2ac, O3, O4, O5, O7, O8, O12 and subtypes thereof and carbapenem resistant *Klebsiella pneumoniae* (CRKP). Preferred, O-serotypes O1, O2a, O2ab, O2ac, O2ae, O2aeh, O2afg, O8, and CRKP strain ST 258, more preferred O1, O2a, O2ab, O2ac, O2afg, O8, CRKP strain ST 258. Still more preferred, *Klebsiella pneumoniae* strains serotypes are O1:K1, O1:K2, O1:K7, O1:K8, O1:K10, O1:K12, O1:K16, O1:K19, O1:K21, O1:K22, O1:K27, O1:K34, O1:K42, O1:K45, O1:K55, O1:K57, O1:K62, O1:K65, O1:K66, O1:K69 and O1:K70, O2a, O2ac, and CRKP strain ST 258.

In another aspect of the present invention, the present invention is directed to use of at least one conjugate that comprises at least one saccharide of general formula (**I**) conjugated to an immunogenic carrier and/or at least one saccharide of general formula (**I**) for the manufacture of said pharmaceutical composition or said vaccine for prevention and/or treatment of diseases associated with *Klebsiella pneumoniae* bacteria, particularly, diseases associated with *Klebsiella pneumoniae* bacteria is selected from the group comprising or consisting of pneumonia, bronchitis, meningitis, urinary tract infection, nosocomial pneumonia, intra-abdominal infections, wound infection, infection of blood, osteomyelitis, bacteremia, septicemia and ankylosing spondylitis.

Preferred, the *Klebsiella pneumoniae* bacteria is selected from O-serotypes comprising or consisting of O1, O2, O2ac, O3, O4, O5, O7, O8, O12 and subtypes thereof and carbapenem resistant *Klebsiella pneumoniae* (CRKP). Preferred, O-serotypes O1, O2a, O2ab, O2ac, O2ae, O2aeh, O2afg, O8, and CRKP strain ST 258, more preferred O1, O2a, O2ab, O2ac, O2afg, O8, CRKP strain ST 258. Still more preferred, *Klebsiella pneumoniae* strains serotypes are O1:K1, O1:K2, O1:K7, O1:K8, O1:K10, O1:K12, O1:K16, O1:K19, O1:K21, O1:K22, O1:K27, O1:K34, O1:K42, O1:K45, O1:K55, O1:K57, O1:K62, O1:K65, O1:K66, O1:K69 and O1:K70, O2a, O2ac, and CRKP strain ST 258.

Preferred, the present invention refers to the use of at least one saccharide of any one of general formulae **(I-1)** - **(I-7), (II-1)** - **(II-17)** and/or at least one of the conjugates comprising at least one saccharide of any one of general formulae **(I-1)** - **(I-7), (II-1)** - **(II-17)** for the manufacture of said pharmaceutical composition or said vaccine.
More preferred, the present invention refers to the use of at least one of the saccharides **A-01** - **A-140, B-01** - **B-140, C-01** - **C-70, D-01** - **D-70, E-01** - **E-70, F-01** - **F-530, G-01** - **G-350, H-01** - **H-70, J-01** - **J-70, K-01** - **K-350, L-01** - **L-112, M-01** - **M-70, N-01** - **N-70, O-01** - **O-70, P-01** - **P-70** and **Q-1** - **Q-700** and/or at least one of the conjugates comprising at least one of the saccharides **A-01** - **A-140, B-01** - **B-140, C-01** - **C-70, D-01** - **D-70, E-01** - **E-70, F-01** - **F-530, G-01** - **G-350, H-01** - **H-70, J-01** - **J-70, K-01** - **K-350, L-01** - **L-112, M-01** - **M-70, N-01** - **N-70, O-01** - **O-70** and **P-01** - **P-70** and **Q-1** - **Q-700** for the manufacture of said pharmaceutical composition or said vaccine.

Particularly, the present invention refers to the use of at least one conjugate of any one of general formulae (**III**), (**IV**), (**V**) and (**V-1**) - (**V-17**) for the manufacture of said pharmaceutical composition or said vaccine,

Pharmaceutical compositions may be prepared in unit dose form. In some embodiments a unit dose may have a volume of between 0.1-1.0 mL e.g. about 0.5 mL.

The invention also provides a delivery device (e.g. syringe, nebuliser, sprayer, inhaler, dermal patch, etc.) containing a pharmaceutical composition of the invention e.g. containing a unit dose. This device can be used to administer the composition to a vertebrate subject.

The invention also provides a sterile container (e.g. a vial) containing a pharmaceutical composition of the invention e.g. containing a unit dose.

The invention also provides a unit dose of a pharmaceutical composition of the invention.

The invention also provides a hermetically sealed container containing a pharmaceutical composition of the invention. Suitable containers include e.g. a vial.

Pharmaceutical compositions of the invention may be prepared in various forms. For example, the compositions may be prepared as injectables, either as liquid solutions or suspensions. Solid forms suitable for solution in, or suspension in, liquid vehicles prior to injection can also be prepared (e.g. a lyophilised composition or a spray-freeze dried composition). The composition may be prepared for topical administration e.g. as an ointment, cream or powder. The composition may be prepared for oral administration e.g. as a tablet or capsule, as a spray, or as a syrup (optionally flavoured). The composition may be prepared for pulmonary administration e.g. by an inhaler, using a fine powder or a spray. The composition may be prepared as a suppository. The composition may be prepared for nasal, aural or ocular administration e.g. as a spray or drops. Injectables for intramuscular administration are typical.

The pharmaceutical compositions may comprise an effective amount of an adjuvant i.e. an amount which, when administered to an individual, either in a single dose or as part of a series, is effective for enhancing the immune response to a co-administered *Klebsiella penumoniae* antigen.

Preferred, the *Klebsiella pneumoniae* is selected from O-serotypes comprising or consisting of O1, O2, O2ac, O3, O4, O5, O7, O8, O12 and subtypes thereof and carbapenem resistant *Klebsiella pneumoniae* (CRKP). Preferred, O-serotypes O1, O2a, O2ab, O2ac, O2ae, O2aeh, O2afg, O8, and CRKP strain ST 258, more preferred O1, O2a, O2ab, O2ac, O2afg, O8, CRKP strain ST 258. Still more preferred, *Klebsiella pneumoniae* strains serotypes are O1:K1, O1:K2, O1:K7, O1:K8, O1:K10, O1:K12, O1:K16, O1:K19, O1:K21, O1:K22, O1:K27, O1:K34, O1:K42, O1:K45, O1:K55, O1:K57, O1:K62, O1:K65, O1:K66, O1:K69 and O1:K70, O2a, O2ac, and CRKP strain ST 258.

This amount can vary depending upon the health and physical condition of the individual to be treated, age, the taxonomic group of individual to be treated (e.g. non-human primate, primate, etc.), the capacity of the individual's immune system to synthesize antibodies, the degree of protection desired, the formulation of the vaccine, the treating doctor's assessment of the medical situation, and other relevant factors. The amount will fall in a relatively broad range that can be determined through routine trials.

Techniques for the formulation and administration of the vaccine of the present invention may be found in "Remington's Pharmaceutical Sciences" Mack Publishing Co., Easton PA.

A therapeutically effective dosage of one conjugate according to the present invention or of one saccharide of general formula (I) refers to that amount of the compound that results in an at least a partial immunization against a disease. Toxicity and therapeutic efficacy of such compounds can be determined by standard pharmaceutical, pharmacological, and toxicological procedures in cell cultures or experimental animals. The dose ratio between toxic and therapeutic effect is the therapeutic index. The actual amount of the composition administered will be dependent on the subject being treated, on the subject's weight, the severity of the affliction, the manner of administration and the judgement of the prescribing physician.

Another aspect of the present invention is directed to a method of inducing immune response against *Klebsiella pneumoniae* in a human and/or animal host, said method comprising administering of the saccharide of general formula (I) and/or salt thereof and/or a conjugate thereof or pharmaceutical composition thereof to said human and/or animal host. A method of treating or preventing diseases caused by *Klebsiella pneumoniae,* in a human and/or animal host according to the present invention comprises administering of at least one saccharide of general formula (I) and/or salt thereof and/or a conjugate thereof or pharmaceutical composition thereof to said human and/or animal host. Preferred, the *Klebsiella pneumoniae* bacteria is selected from O-serotypes comprising or consisting of O1, O2, O2ac, O3, O4, O5, 07, 08, 012 and subtypes thereof and carbapenem resistant *Klebsiella pneumoniae* (CRKP). Preferred, O-serotypes O1, O2a, O2ab, O2ac, O2ae, O2aeh, O2afg, O8, and CRKP strain ST 258, more preferred O1, O2a, O2ab, O2ac, O2afg, O8, CRKP strain ST 258. Still more preferred, *Klebsiella pneumoniae* strains serotypes are O1:K1, O1:K2, O1:K7, O1:K8, O1:K10, O1:K12, O1:K16, O1:K19, O1:K21, O1:K22, O1:K27, O1:K34, O1:K42, O1:K45, O1:K55, O1:K57, O1:K62, O1:K65, O1:K66, O1:K69 and O1:K70, O2a, O2ac, and CRKP strain ST 258.

### Immunological assays

Yet another aspect of the present invention refers to saccharide of general formula (I) for use as marker in immunological assays for detection of antibodies against bacteria containing in their O-polysaccharide or capsular polysaccharide one of the following saccharide fragments:
→3)-β-D-Gal*f*-(1→3)-α-D-Gal*p*-(1→
→3)-α-D-Gal*p*-(1→3)-α-D-Gal*p*-(1→
[→3)-β-D-Gal*p*-(1→3)-α-D-Gal*p*-(1→]ₘ-[→3)-β-D-Gal*f*-(1→3)-α-D-Gal*p*-(1→]ₙ
[→5)-β-D-Gal*f*-(1→3)-β-D-GlcNAc-(1→]ₘ-[→3)-β-D-Gal*f*-(1→3)-α-D-Gal*p*-(1→]ₙ.

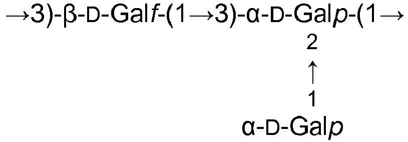

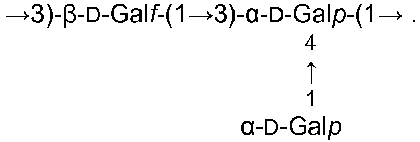

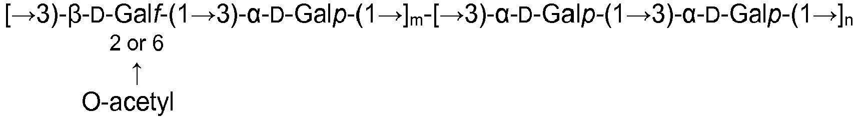

Preferred, the saccharide of general formula (**I**) is useful as marker in immunological assays for detection of antibodies against *Klebsiella pneumoniae.*
Such assays comprise, for instance, microarray and ELISA useful for detection of antibodies against *Klebsiella pneumoniae.*

The saccharides of the present invention can be easily conjugated to solid supports for providing immunological assays useful for detection of antibodies against *Klebsiella pneumoniae.*
Preferred, the *Klebsiella pneumoniae* bacteria is selected from O-serotypes comprising or consisting of O1, O2, O2ac, O3, O4, O5, O7, O8, O12 and subtypes thereof and carbapenem resistant *Klebsiella pneumoniae* (CRKP). Preferred, O-serotypes O1, O2a, O2ab, O2ac, O2ae, O2aeh, O2afg, O8, and CRKP strain ST 258, more preferred O1, O2a, O2ab, O2ac, O2afg, O8, CRKP strain ST 258. Still more preferred, *Klebsiella pneumoniae* strains serotypes are O1:K1, O1:K2, O1:K7, O1:K8, O1:K10, O1:K12, O1:K16, O1:K19, O1:K21, O1:K22, O1:K27, O1:K34, O1:K42, O1:K45, O1:K55, O1:K57, O1:K62, O1:K65, O1:K66, O1:K69 and O1:K70, O2a, O2ac, and CRKP strain ST 258.

Said solid supports present on their surface a functionality that is prone to react with the amino group of saccharides of general formula (**I**) or with the functional group Y of the interconnecting molecule to provide modified solid supports, presenting on their surface the functional group X of the interconnecting molecule that can further react with the amino group of saccharides of general formula (**I**). In an embodiment according to the present invention the solid supports are microarray slides, which present on their surface a functionality that is prone to react with the functional group Y of the interconnecting molecule to provide modified microarray slides, presenting of their surface the functional group X of the interconnecting molecule. Examples of such microarray slides include, but are not restricted to Corning® epoxide coated slides or Corning® GAPS™ II coated slides.

In a preferred embodiment the solid supports are microarray slides presenting on their surface a functionality that is prone to react with the amino group of saccharides of general formula (**I**), and more preferably an *N*-hydroxysuccinimide (NHS) activated ester. Such microarray slides are for example CodeLink® NHS slides.

### Description of the figures

**Figure 1** shows the chemical structure of the repeating unit of *Klebsiella pneumoniae* O-polysaccharide.
**Figure 2** shows the chemical structure of the repeating unit of *Klebsiella pneumoniae* O-polysaccharide
**Figure 3** provides examples of functional group X of the interconnecting molecule according to the present invention.

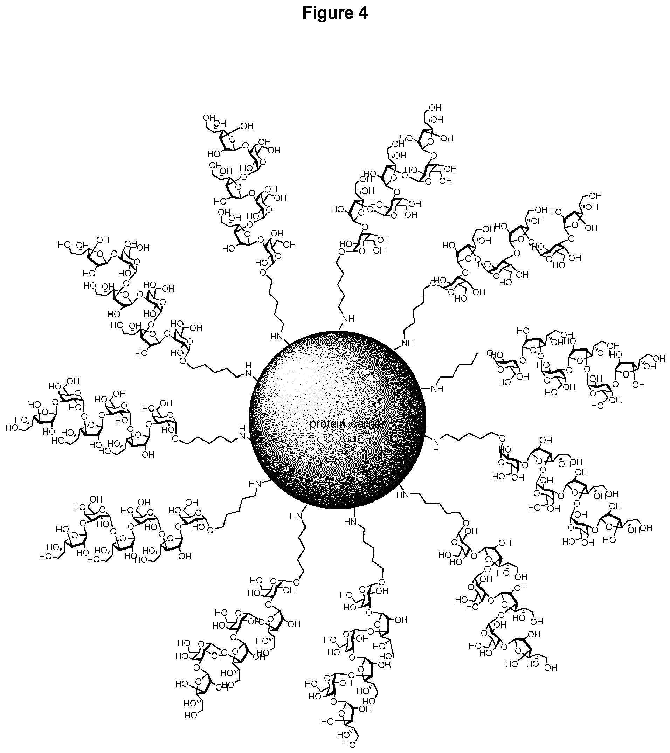

**Figure 4** presents schematically a conjugate of inventive oligosaccharides.

The following examples are included to demonstrate preferred embodiments of the invention. It should be appreciated by those skilled in the art that the techniques disclosed in the examples, which follow represent techniques discovered by the inventor to function well in the practice of the invention, and thus can be considered to constitute preferred modes for its practice. However, those skilled in the art should, in light of the present disclosure, appreciate that many changes can be made in the specific embodiments, which are disclosed and still obtain a like or similar result without departing from the spirit and scope of the invention.
Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### Examples

### A. Chemical synthesis

### General information:

Commercial grade solvents were used unless stated otherwise. Dry solvents were obtained from a Waters Dry Solvent System. Solvents for chromatography were distilled prior to use. Sensitive reactions were carried out in heat-dried glassware and under an argon atmosphere. Analytical thin layer chromatography (TLC) was performed on Kieselgel 60 F254 glass plates precoated with a 0.25 mm thickness of silica gel. Spots were visualized by staining with vanillin solution (6% (w/v) vanillin and 10% (v/v) sulfuric acid in 95% EtOH) or Hanessian's stain (5% (w/v) ammonium molybdate, 1% (w/v) cerium(II) sulfate and 10% (v/v) sulfuric acid in water). Silica column chromatography was performed on Fluka Kieselgel 60 (230-400 mesh).
¹H, ¹³C and two-dimensional NMR spectra were measured with a Varian 400-MR spectrometer at 296 K. Chemical shifts (d) are reported in parts per million (ppm) relative to the respective residual solvent peaks (CDCl₃: d 7.27 in ¹H and 77.23 in ¹³C NMR; CD₃OD: d 3.31 in ¹H and 49.15 in ¹³C NMR). The following abbreviations are used to indicate peak multiplicities: s singlet; d doublet; *dd* doublet of doublets; *t* triplet; *dt* doublet of triplets; q quartet; m multiplet. Coupling constants (J) are reported in Hertz (Hz). Optical rotation (OR) measurements were carried out with a Schmidt & Haensch UniPol L1000 polarimeter at λ = 589 nm and a concentration (c) expressed in g/100 mL in the solvent noted in parentheses. High resolution mass spectrometry (HRMS) was performed at the Free University Berlin, Mass Spectrometry Core Facility, with an Agilent 6210 ESI-TOF mass spectrometer. Infrared (IR) spectra were measured with a Perkin Elmer 100 FTIR spectrometer.

### Abbreviations

- AcOH: Acetic acid
- Alloc: Allyloxycarbonyl
- aq.: aqueous
- BH₃: borane
- BBr₃: boron tribromide
- Boc: tert-Butoxycarbonyl
- br.: broad
- CAS: CAS Registry Number (CAS = Chemical Abstracts Service)
- CHCl₃: chloroform
- cHex: cyclohexane
- d: doublet
- dd: doublet of doublets
- DCM: dichloromethane
- DEAD: diethyl azodicarboxylate
- DIPEA: N,N-diisopropyl-ethylamine
- DME: dimethoxyethane
- DMF: dimethylformamide
- DMSO: dimethylsulfoxide
- DPPA: diphenylphosphoryl azide
- EDC•HCl: N1-((ethylimino)methylene)-N3,N3-dimethylpropane-1,3-diamine hydrochloride
- ES: electrospray
- Et₂O: diethyl ether
- EtOAc: ethyl acetate
- FCS: fetal calf serum
- GSDMD: Gasdermin-D
- h: hour
- HCl: hydrochloric acid
- HEK293T: embryonic kidney fibroblast cell line
- H₂O: water
- HOBt.H₂O: 1H-benzo[d][1,2,3]triazol-1-ol hydrate
- hPBMC: human Peripheral Blood Mononuclear Cells
- IC₅₀: half maximal inhibitory concentration
- K₂CO₃: potassium carbonate
- LDH: lactate dehydrogenase
- LiAlH₄: lithium aluminium hydride
- m: multiplet
- MeCN: acetonitrile
- MeOH: methanol
- Mel: methyl iodide
- MgSO₄: magnesium sulphate
- min: minutes
- MS: mass spectrometry
- Na₂CO₃: sodium carbonate
- NaCNBH₃: sodium cyanoborohydride
- NaHCO₃: sodium hydrogencarbonate
- NaH: sodium hydride
- NaOH: sodium hydroxyde
- Na₂SO₄: sodium sulphate
- NCS: N-chlorosuccinimide
- NET: neutrophil extracellular traps
- NMR: nuclear magnetic resonance
- PBS: phosphate-buffered saline
- Pd/C: palladium on carbon
- Pd(PPh₃)₄: Tetrakis(triphenylphosphine)palladium(0)
- PMA: phorbol 12-myristate 13-acetate
- PPh₃: triphenylphosphine
- q: quartet
- rt: room temperature
- s: singlet
- sat.: saturated
- sep: septet
- t: triplet
- TFA: trifluoroacetic acid
- THF: tetrahydrofuran
- THP1: acute monocytic leukaemia cancer cell line
- TsOH: tosic acid
- Wt: weight. 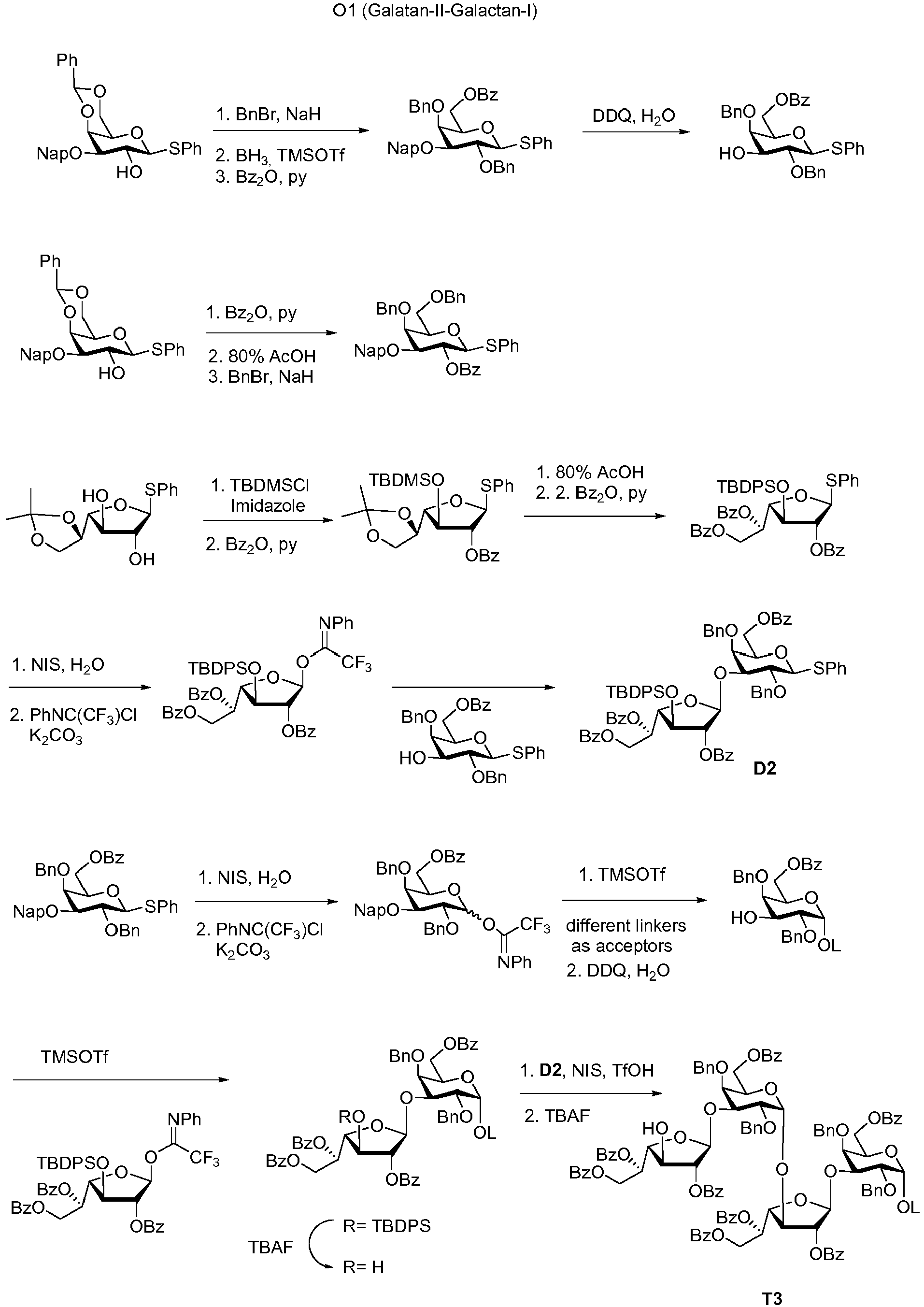 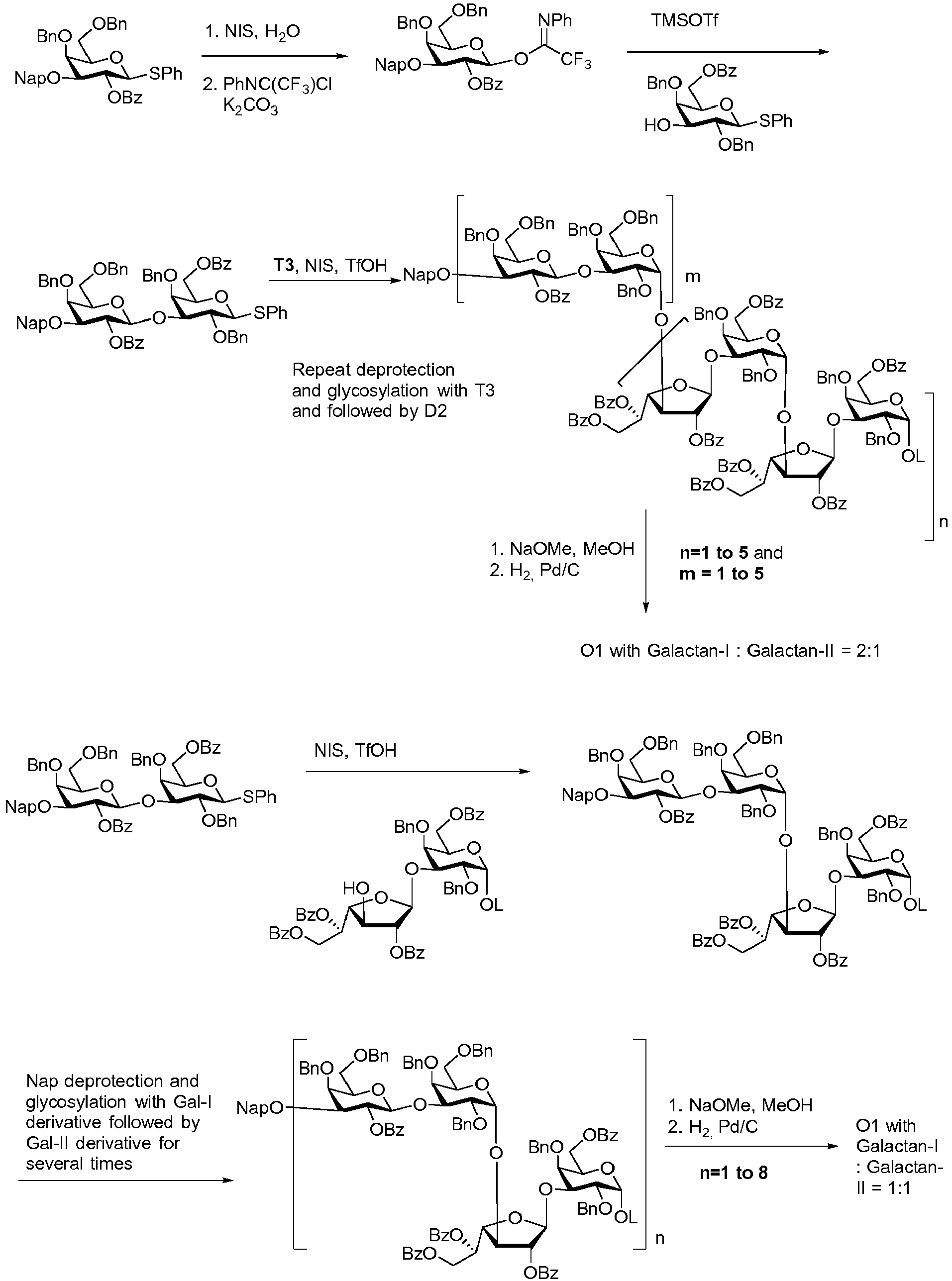 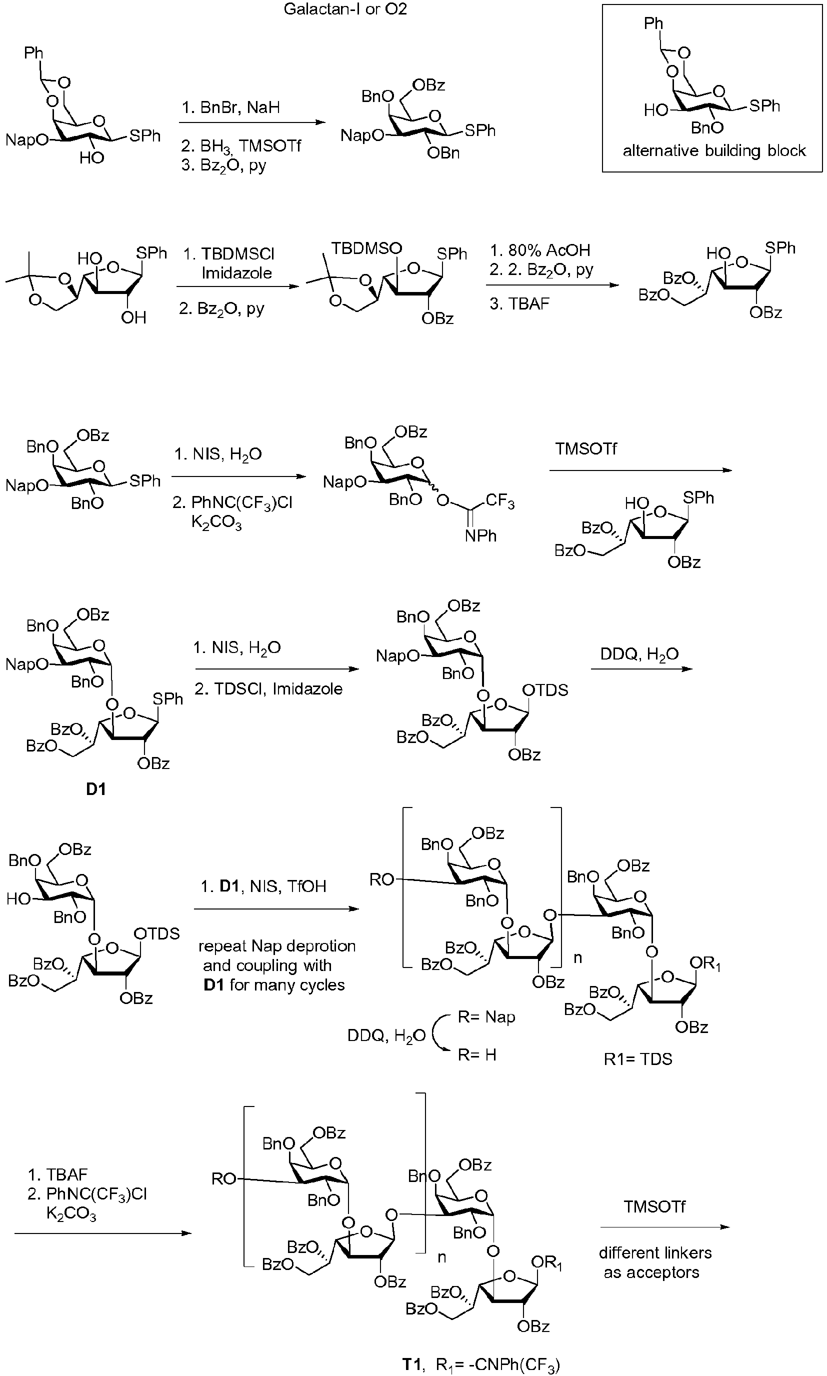 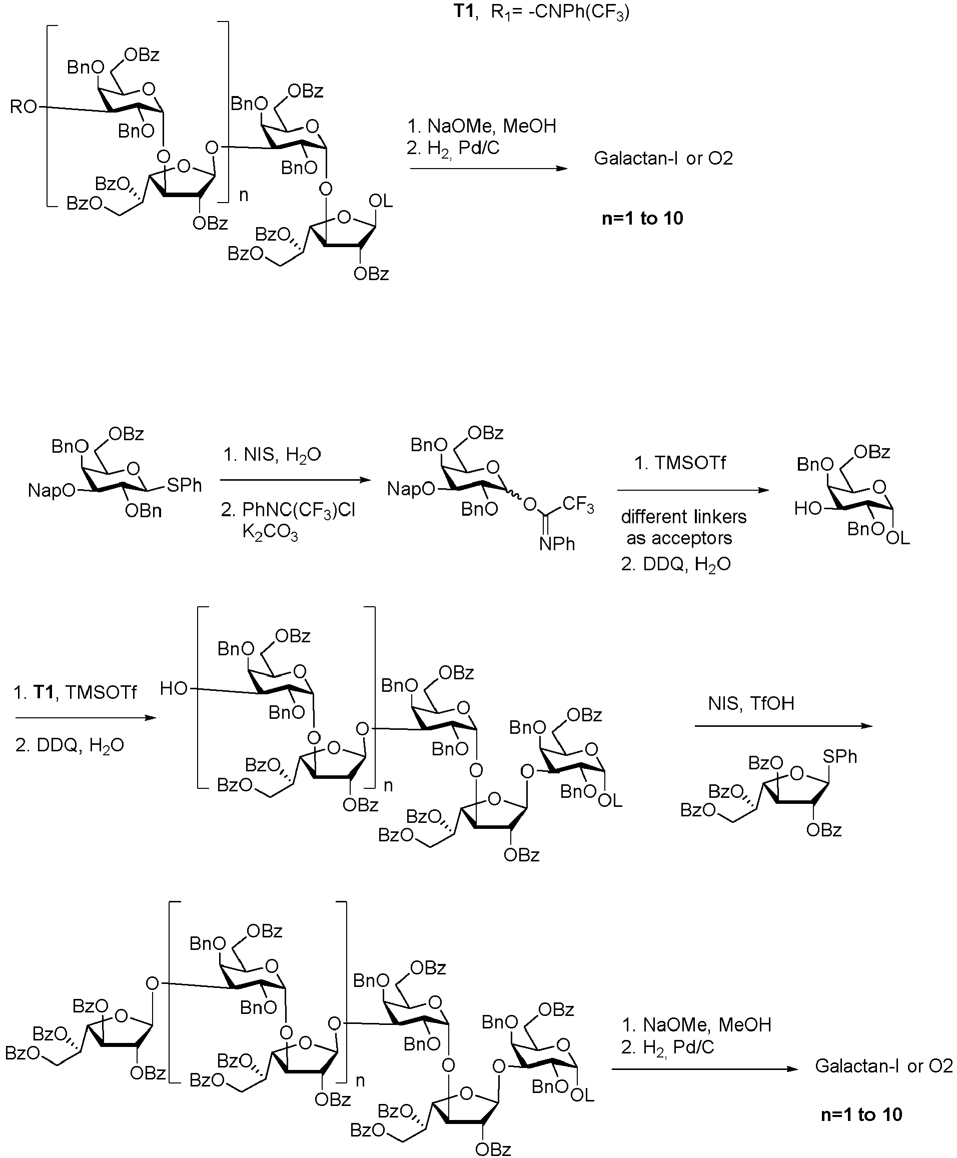 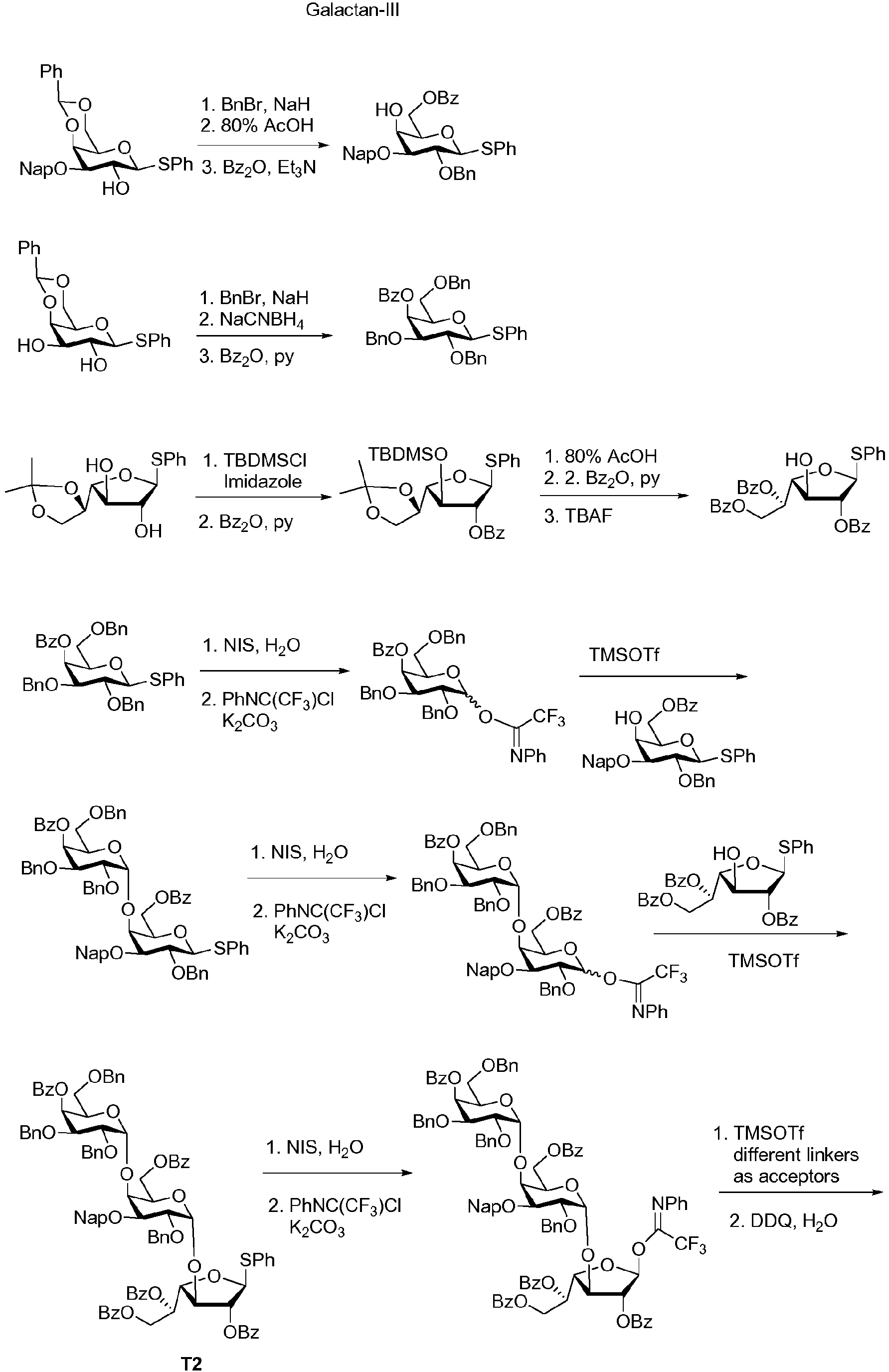 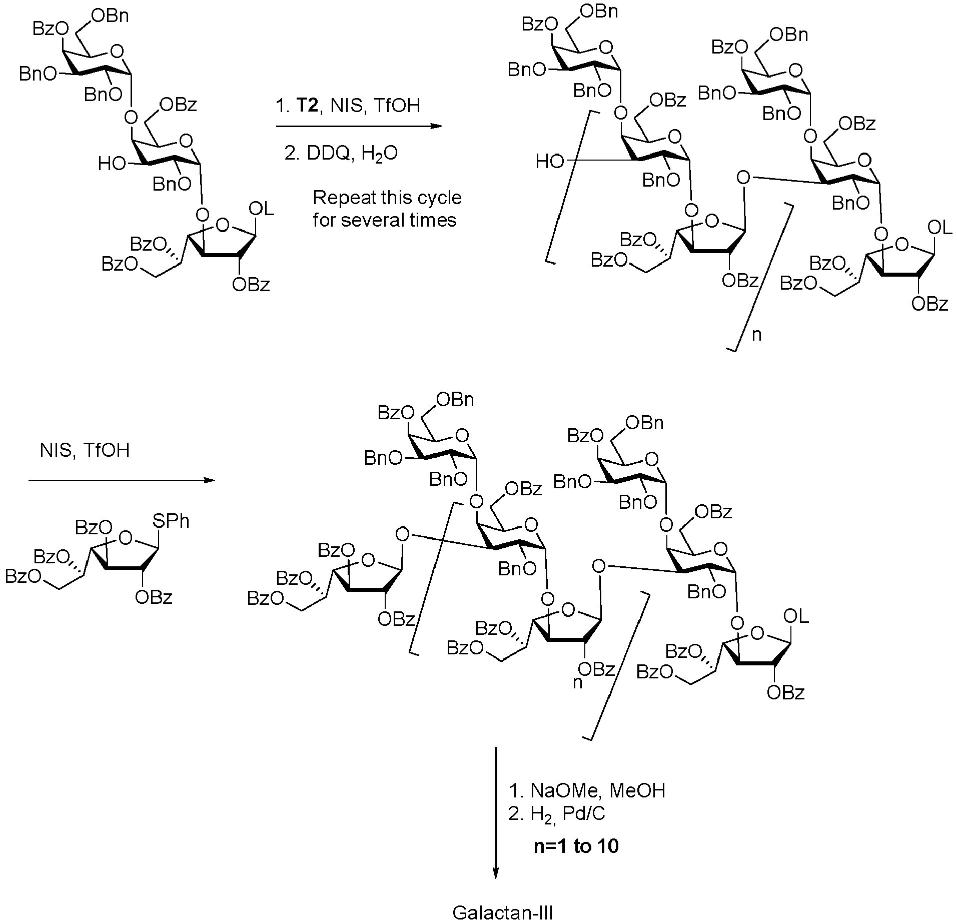
O2ac antigen synthesis

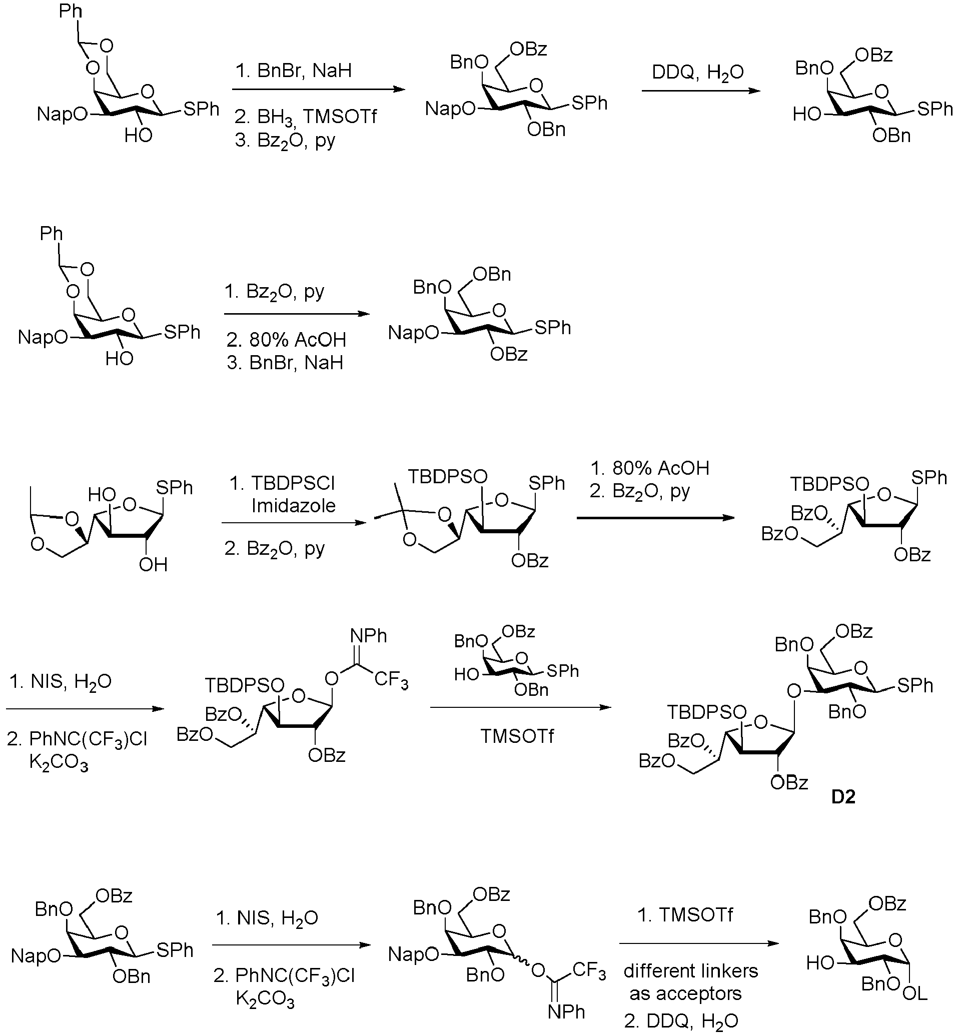

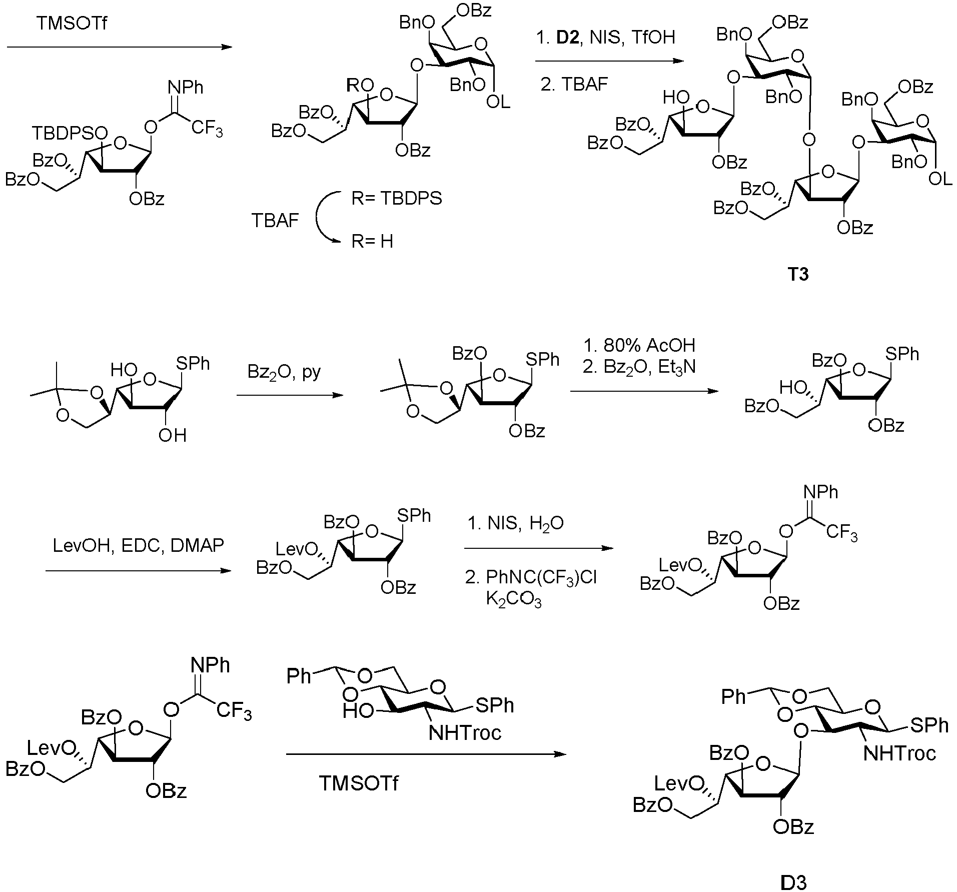

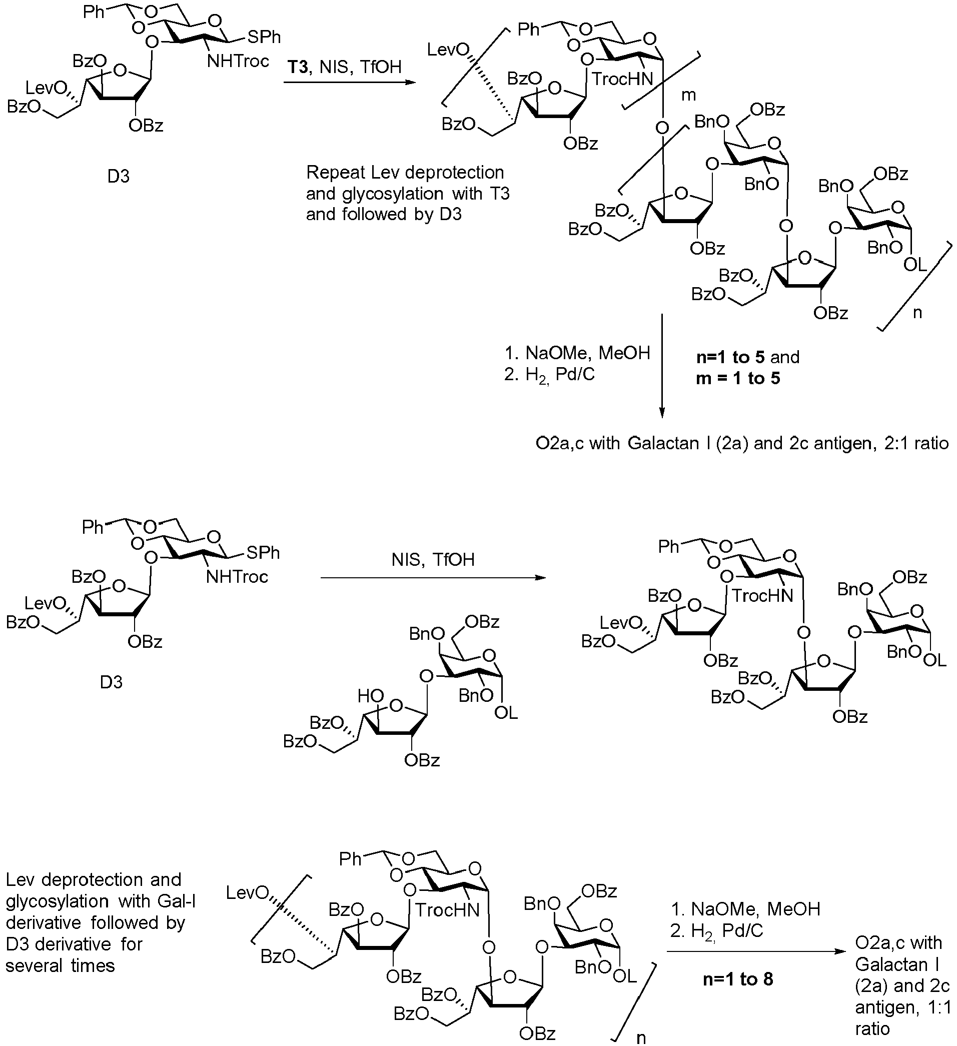

**Glycan microarray analysis:** Glycan microarray analysis was performed as described in Example B-2 using the same printed slides. Mean fluorescence intensity of spots was plotted.

## Claims

1. A saccharide of general formula (**I**) 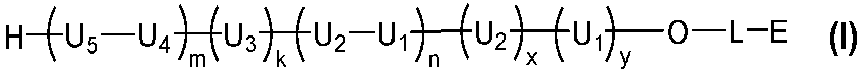 wherein
**U₁** represents 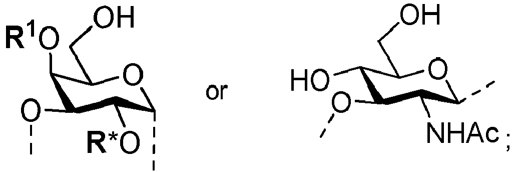
**U₂** represents 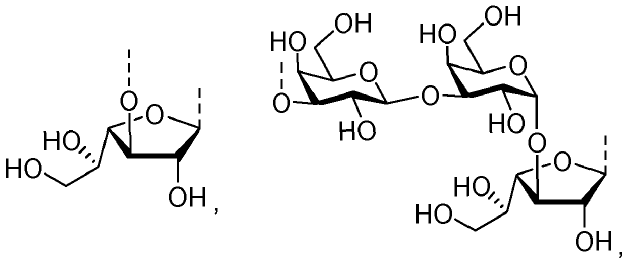 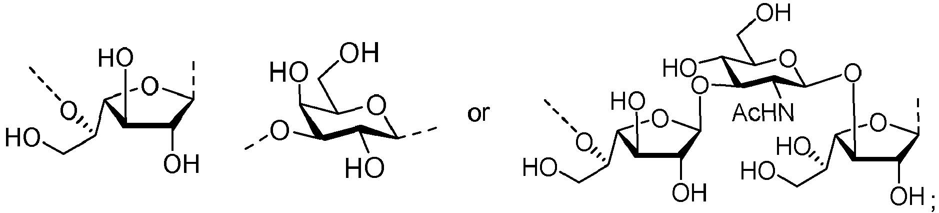
**U₃** represents 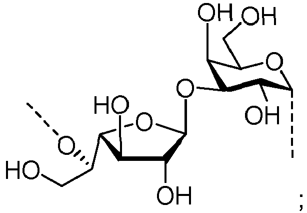
**U₄** represents 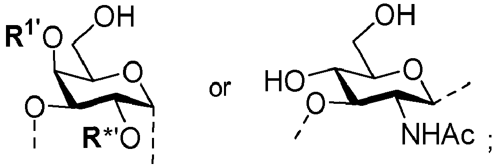
**U₅** represents a covalent bond or 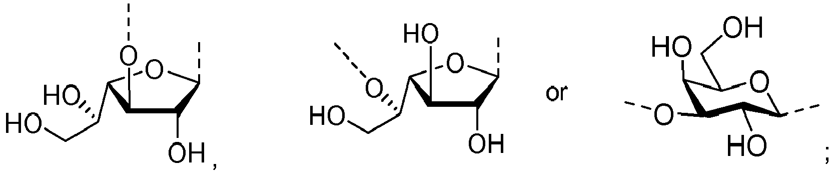
**U₆** represents 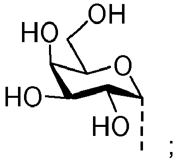
**R¹, R^{1'}**, **R*** and **R*'** represent independently from each other -H or **U₆,** wherein **R¹** and **R*** cannot be simultaneously **-U₆** and **R^{1'}** and **R*'** cannot be simultaneously **-U₆**
**L** represents a linker;
**E** represents -NH₂, -N₃, -CN, -O-NH₂, -CH=CH₂, -C=CH, -Br, -CI, -I, -CO₂R', -CONH-NH₂, -OH, -SH, or -SAc;
**R'** represents -H, -Me, -Et, 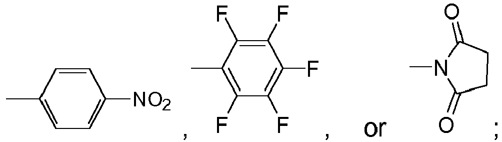
**n** is an integer from 1 to 20;
**m** is an integer from 0 to 20;
**k** is an integer selected from 0 to 10;
**x** and **y** are independently of each other the integer 0 or 1;
and when **U₁** and **U₂** are monosaccharides and **n** is 1, **m, x,** and **y** are not 0 at the same time;
or anomers, hydrates, or pharmaceutically acceptable salts thereof with the proviso that **L** is not -C₃H₆- if **-E** is -NH₂.

2. The saccharide according to claim 1, wherein
**U₁** represents 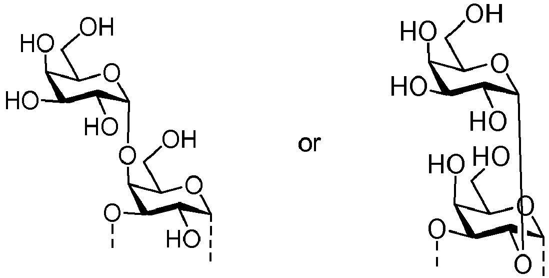
**U₂** represents 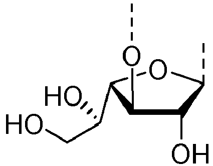
**U₃** represents 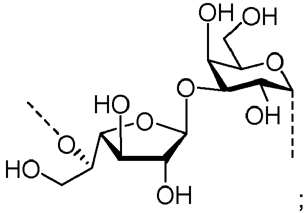
**U₅** represents a covalent bond or 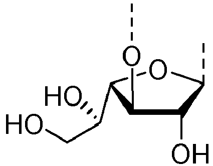
**m, n, k, x, y, L** and **E** have the meanings as defined in Claim 1.

3. The saccharide according to claim 1, wherein
**U₁** represents 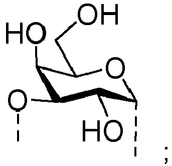
**U₂** represents 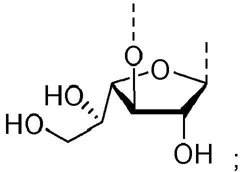
**U₄** represents 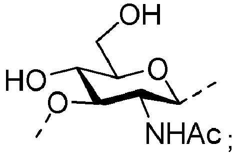
**U₅** represents a covalent bond, or 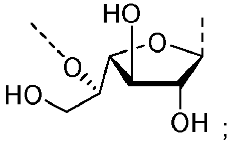
**m** is an integer from 1 to 10,
k is 0,
**n, U₃, x, y, L** and **E** have the meanings as defined in Claim 1.

4. The saccharide according to any one of the claims 1 to 3, wherein
**-L-** represents **-L^{a}-, -L^{a}-L^{e}-, -L^{a}-L^{b}-L^{e}-**, or **-L^{a}-L^{d}-L^{e}- ;**
**-L^{a}-** represents -(CH₂)ₒ-, -(CH₂-CH₂-O)ₒ-C₂H₄-, or -(CH₂-CH₂-O)ₒ-CH₂;
**-L^{b}-** represents -O-;
**-L^{d}-** represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂-;
**-L^{e}-** represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂- ; and
**o, q, p1** and **p2** are independently of each other an integer selected from 1, 2, 3, 4, 5, and 6, with the proviso that L is not -C₃H₆-.

5. The saccharide according to any one of the claims 1 to 4, wherein -O-**L-E** is selected from the group consisting of: 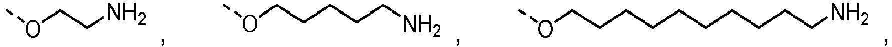 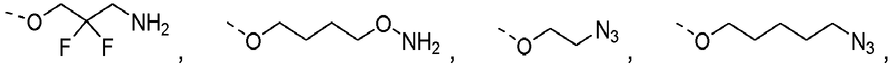 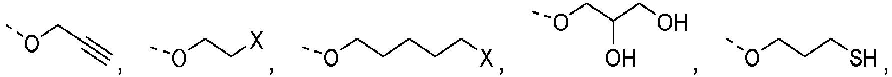 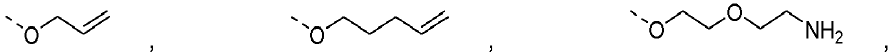 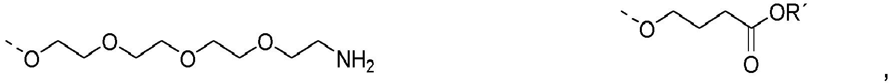 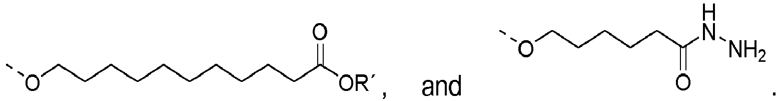 wherein R' represents -H, -Me, -Et, 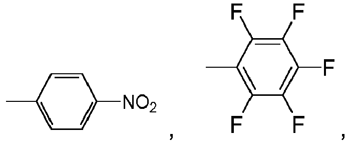 or 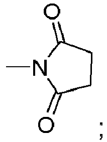 X represents -Br, -CI, -I, -CO₂H, or -SAc.

6. A conjugate of general formula (**III**) 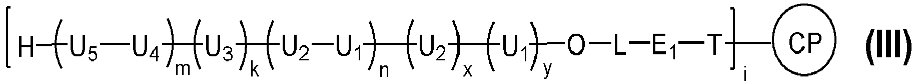 wherein
**i** is an integer selected from 2 to 18;
**-E₁-** represents a covalent bond, -NH-, -O-NH-, -O-, -S-, -CO-, -CH=CH-, -CONH-, -CO-NHNH-, 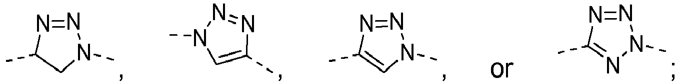
**-T-** represents 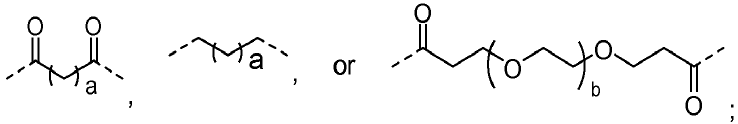
**a** represents an integer from 1 to 10;
**b** represents an integer from 1 to 4;
**CP** is a carrier protein; and
**U₁, U₂, U₃, U₄, U₅, L, m, n, k, x,** and **y** have the same meanings as defined in Claim 1.

7. The conjugate according to claim 6, wherein the conjugate has formula (**IV**) 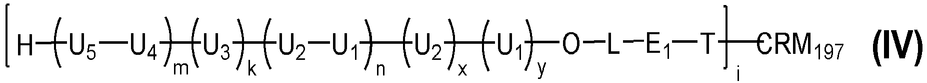 wherein
**i** is an integer selected from 2 to 18;
**-E₁-** represents a covalent bond, -NH-, -O-NH-, -O-, -S-, -CO-, -CH=CH-, -CONH-, -CO-NHNH-, 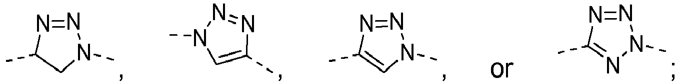
**-T-** represents 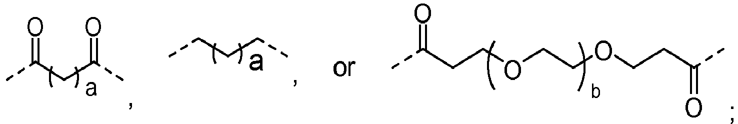
**a** represents an integer from 1 to 10;
**b** represents an integer from 1 to 4; and
**U₁**, **U₂, U₃, U₄, U₅, L, m, n, k, x,** and **y** have the same meanings as defined in Claim 1.

8. The conjugate according to claim 6 or 7, wherein the conjugate has any one of the formula (**V-1**) - (**V-17**): 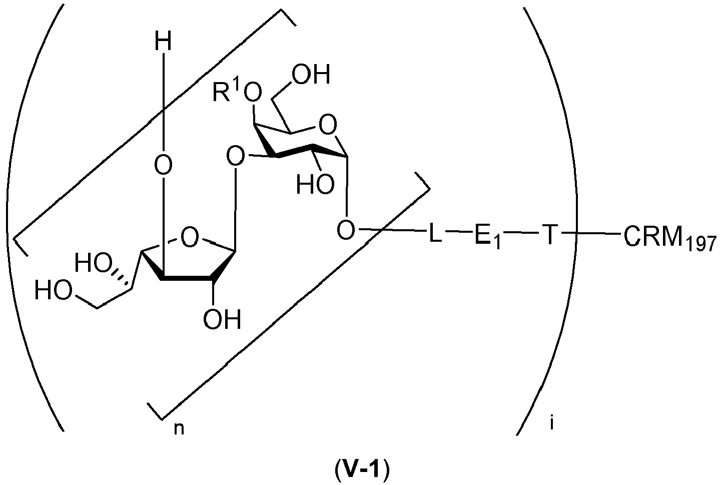 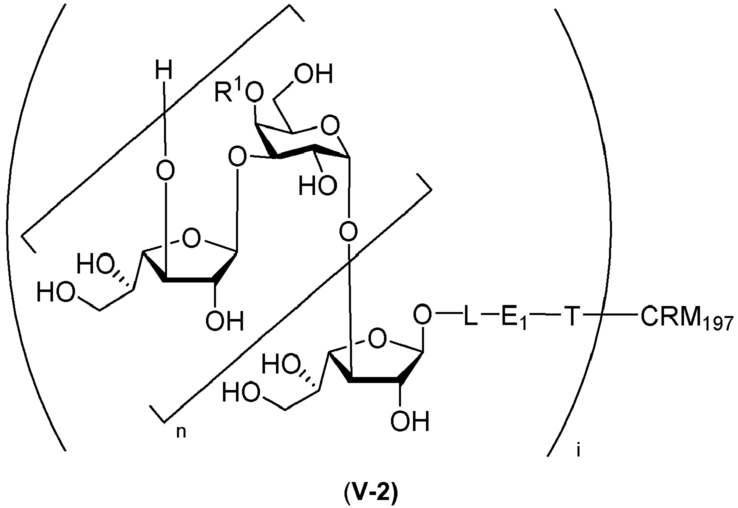 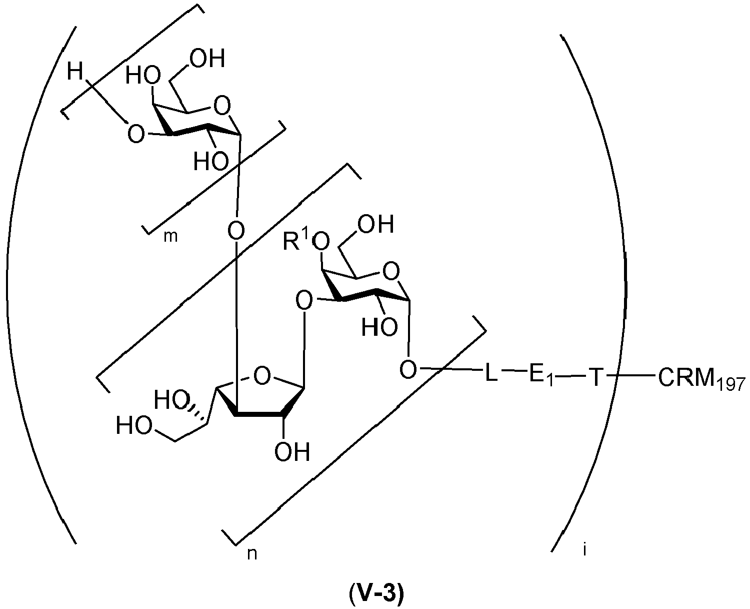 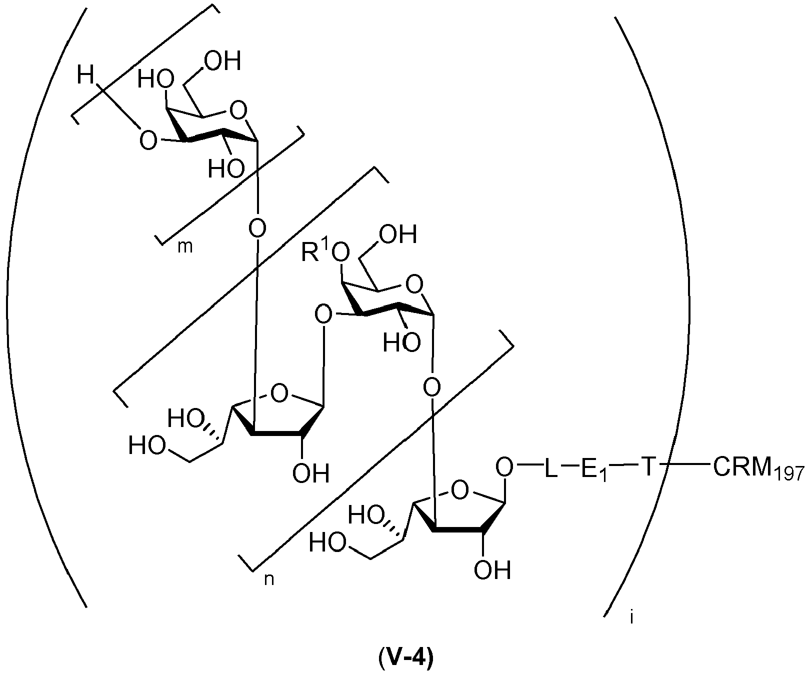 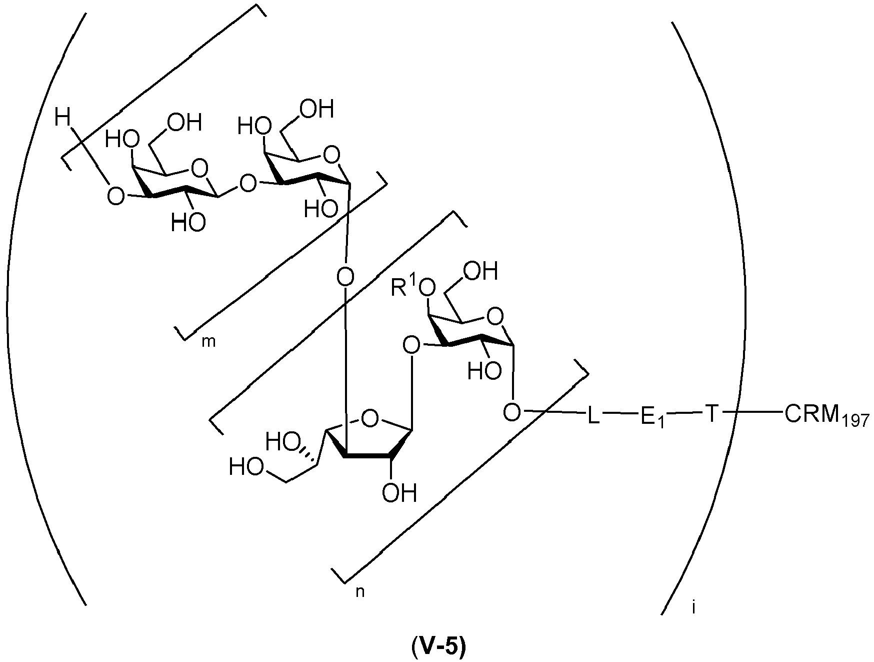 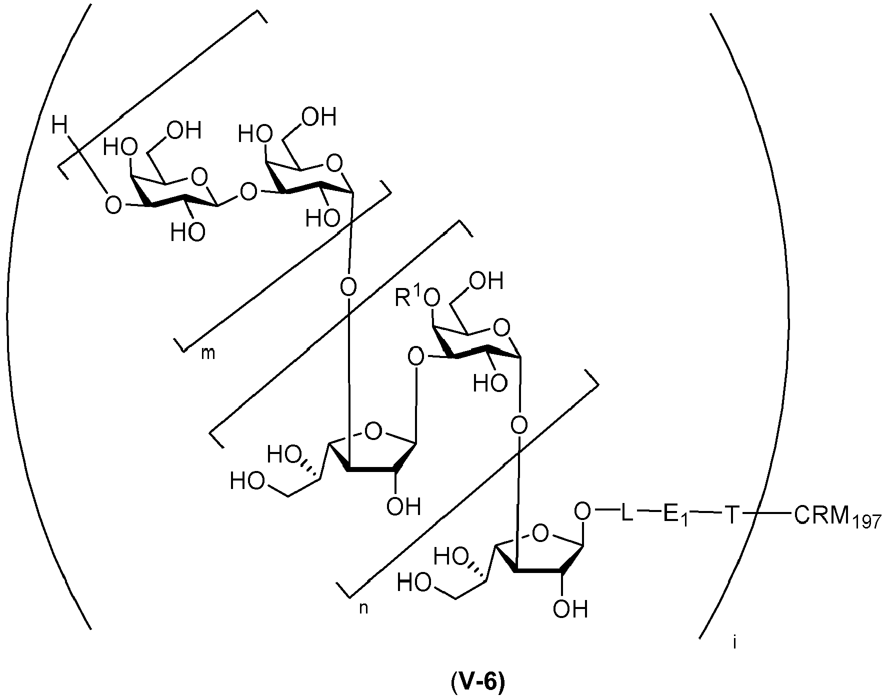 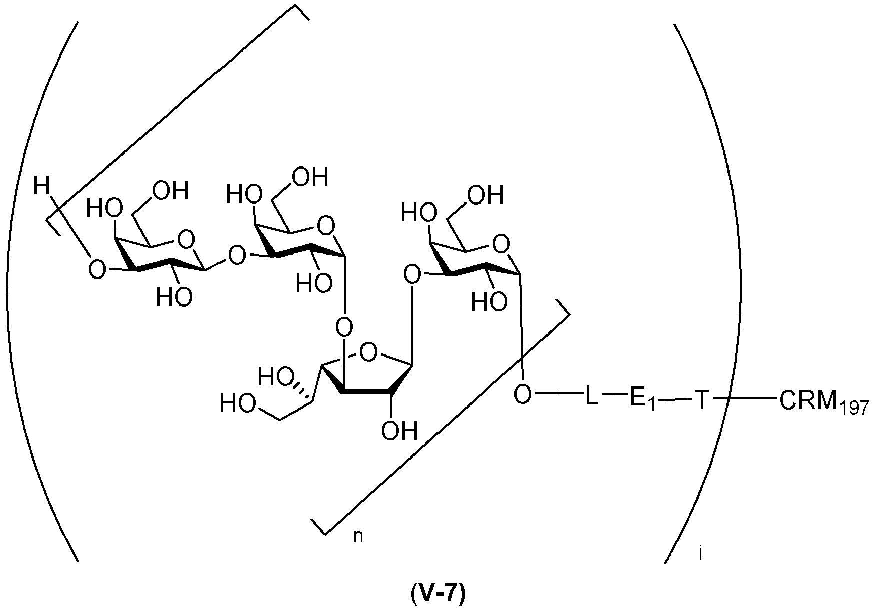 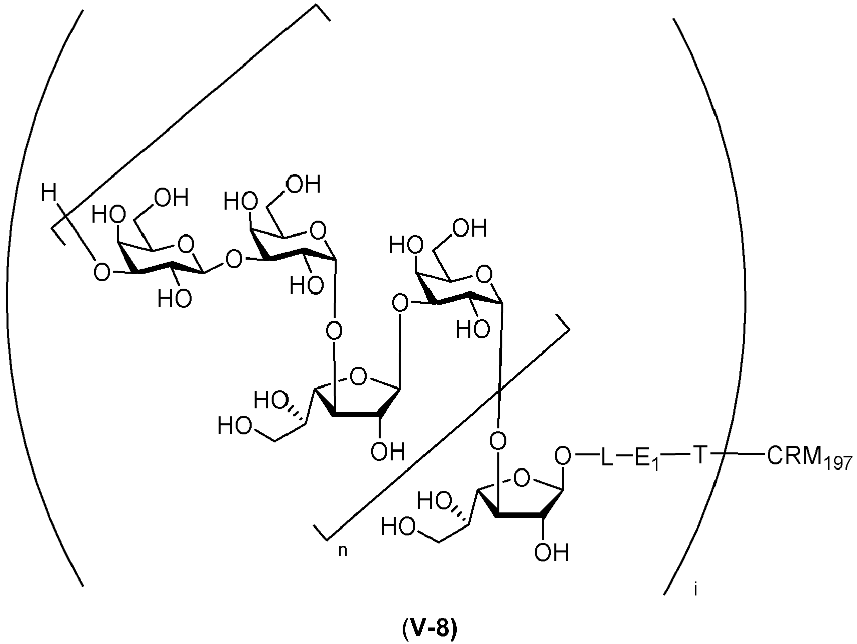 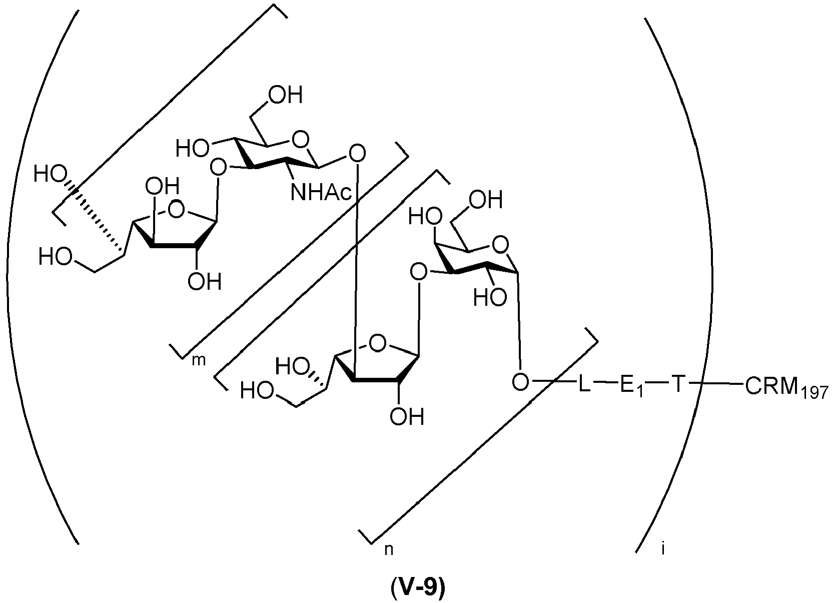 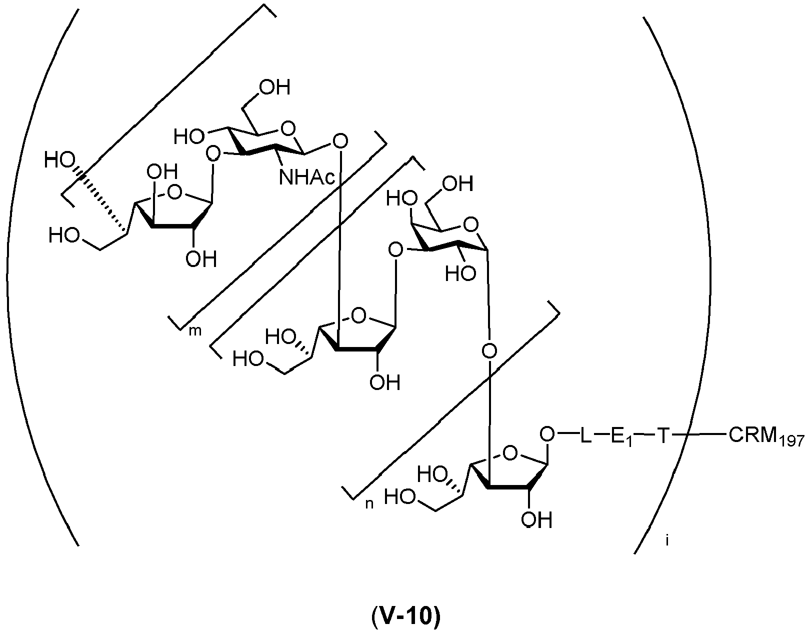 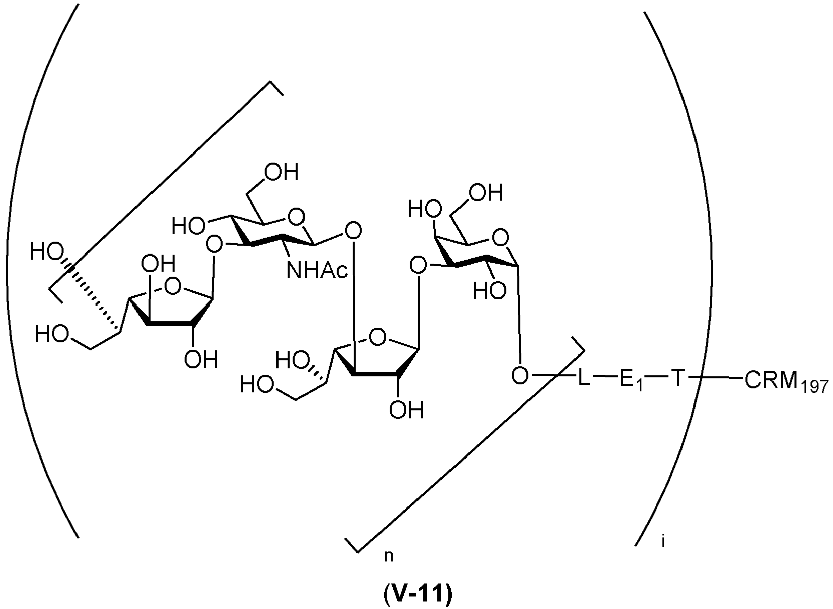 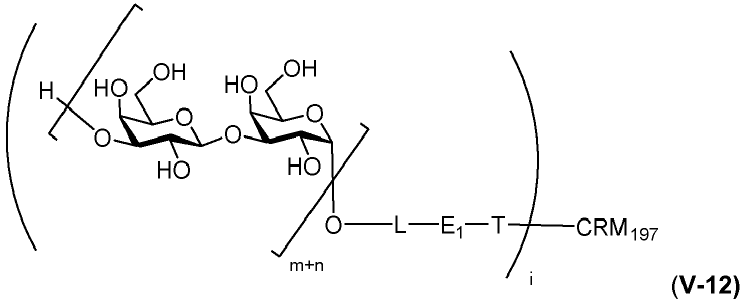 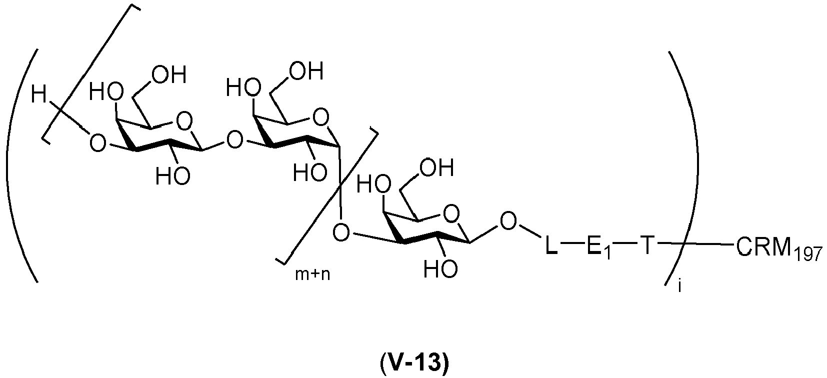 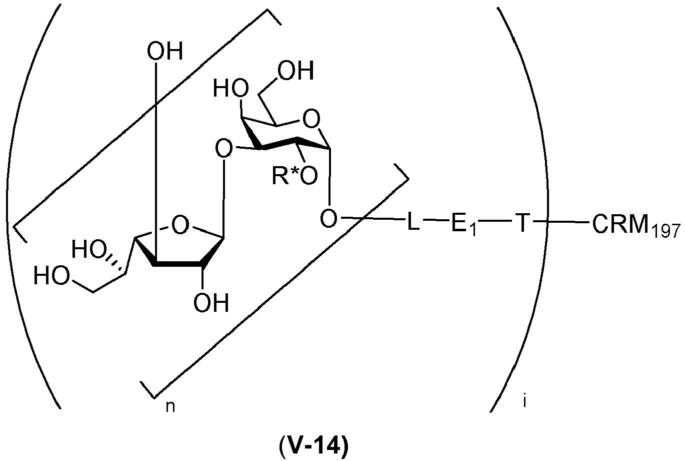 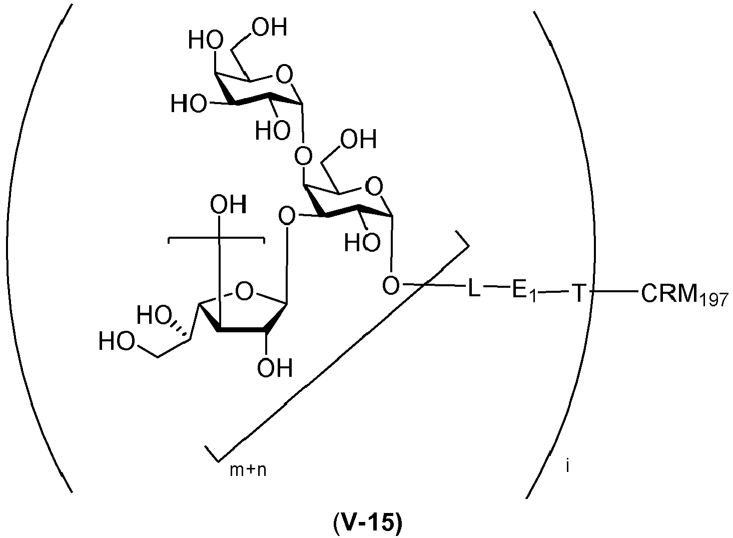 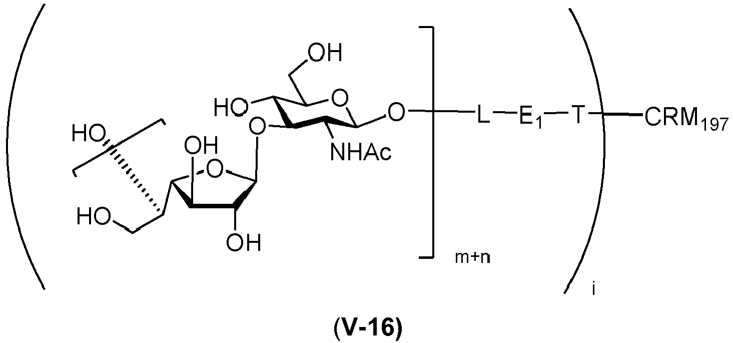 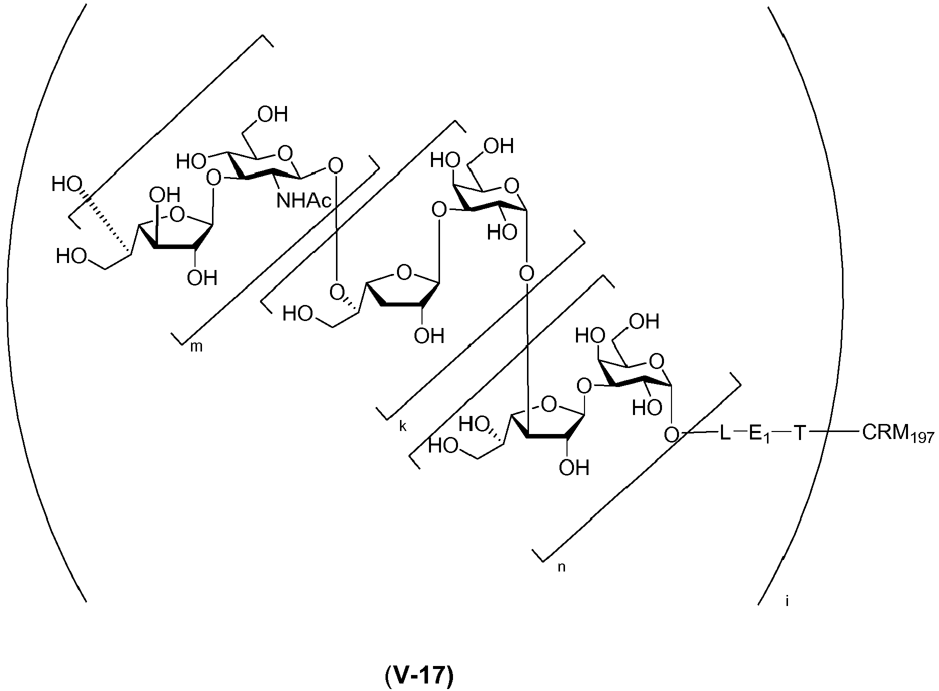 wherein
**R¹** and **R*** represent independently -H, or 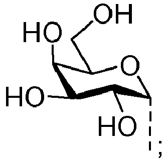
wherein **R¹** and **R*** cannot be simultaneously 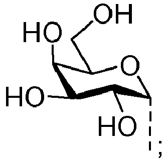 and
**L, E₁, T, *i***, **m, k** and **n** have the same meanings as defined in Claim 6.

9. The conjugate according to any one of the claims 6 - 8, wherein
**-L-** represents **-L^{a}-, -L^{a}-L^{e}- -L^{a}-L^{b}-L^{e}-**, or **-L^{a}-L^{d}-L^{e}-;**
**-L^{a}-** represents -(CH₂)ₒ-, -(CH₂-CH₂-O)ₒ-C₂H₄-, or -(CH₂-CH₂-O)ₒ-CH₂;
**-L^{b}-** represents -O-;
**-L^{d}-** represents -(CH₂)_{q}-, -(CH(OH))_{q}-, -(CF₂)_{q}-, -(CH₂-CH₂-O)_{q}-C₂H₄-, or -(CH₂-CH₂-O)_{q}-CH₂-;
-**L**^{e}- represents -(CH₂)ₚ₁-, -(CF₂)ₚ₁-, -C₂H₄-(O-CH₂-CH₂)ₚ₁-, -CH₂-(O-CH₂-CH₂)ₚ₁- or -(CH₂)ₚ₁-O-(CH₂)ₚ₂-; and
**o**, **q**, **p1** and **p2** are independently of each other an integer selected from 1, 2, 3, 4, 5, and 6.

10. A pharmaceutical composition comprising at least one saccharide according to any one of the claims 1 - 5 and/or at least one conjugate according any one of the claims 6 - 9 as an active ingredient together with at least one pharmaceutically acceptable adjuvant and/or excipient.

11. The saccharide according to any one of the claims 1 - 5, the conjugate according to any one of the claims 6 - 9, or the pharmaceutical composition according to claim 10 for use in prevention and/or treatment of disease associated with *Klebsiella pneumoniae,* wherein the disease is meningitis, urinary tract infection, nosocomial pneumonia, intra-abdominal infections, wound infection, infection of blood, osteomyelitis, bacteremia, septicemia or ankylosing spondylitis

12. The saccharide, the conjugate or the pharmaceutical composition for use according to claim 11, wherein the *Klebsiella pneumoniae* is selected from O-serotypes comprising or consisting of O1, O2a, O2ab, O2ac, O2aeh, O2afg, 08, and CRKP strain ST 258.

13. The saccharide according to any one of the claims 1 - 5 for use as a marker in immunological assays for the detection of antibodies against *Klebsiella pneumoniae.*

14. A method for synthesis of a saccharide of general formula (**I**) comprising:
**A2)** providing a disaccharide **D6** 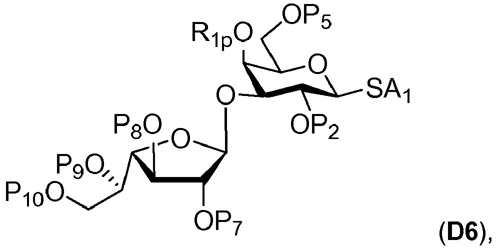 wherein **R_{1P}** is **P₄** or **U₅ₚ**;
**U₅ₚ** is 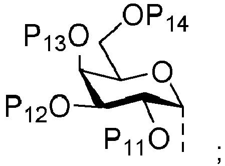
**B1')** reacting the saccharide **D6** with a saccharide **D8** 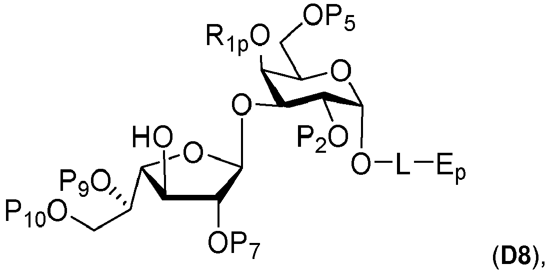 to obtain a saccharide **O2b** 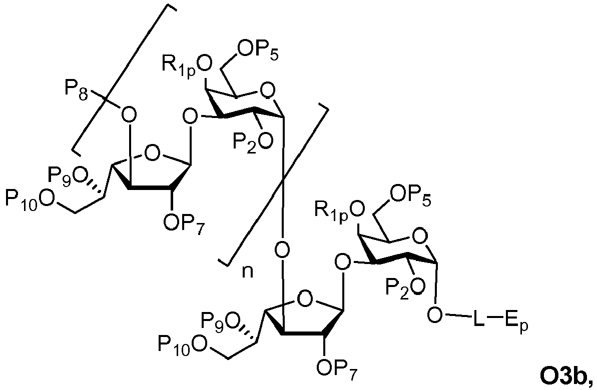 wherein n is 1;
when n is an integer from 2 to 20,
then repeating the following steps **B2')** and **B3')** for **n-1** times
**B2')** removing the protecting group P₈ of a saccharide obtained by reacting with the saccharide **D6**;
**B3')** reacting the saccharide **D6** with the saccharide obtained after the step **B2')** to obtain a saccharide **O3b** 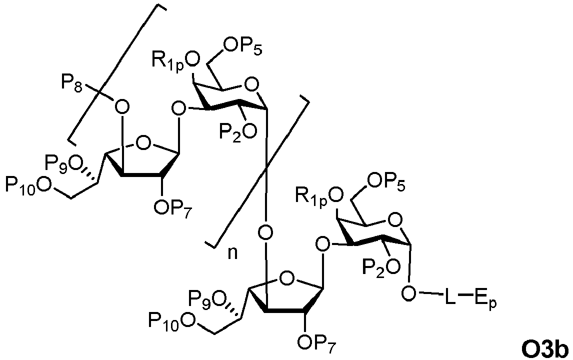 wherein n is an integer from 2 to 20,
optionally,
**E3)** removing the protecting group P₈ of the saccharide **O3b** to obtain a saccharide **O3c,** 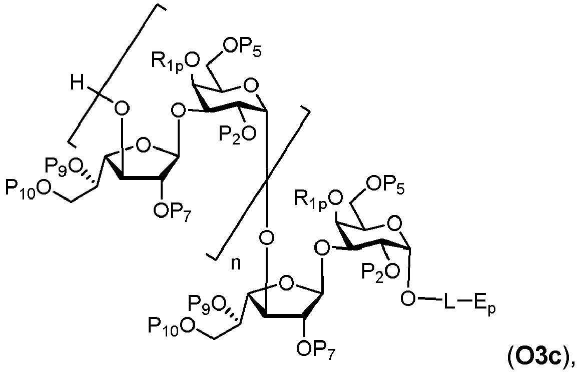 and
**E4)** reacting the saccharide **O3c** with a saccharide **M3** 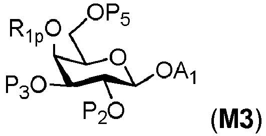 to obtain a saccharide **O4b** 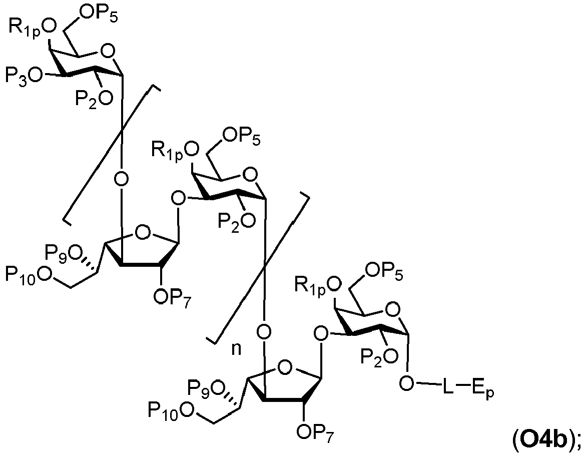 or
**E5)** reacting the saccharide **O3c** with a disaccharide **D4** 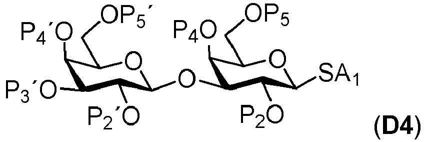 to obtain a saccharide **O5a** 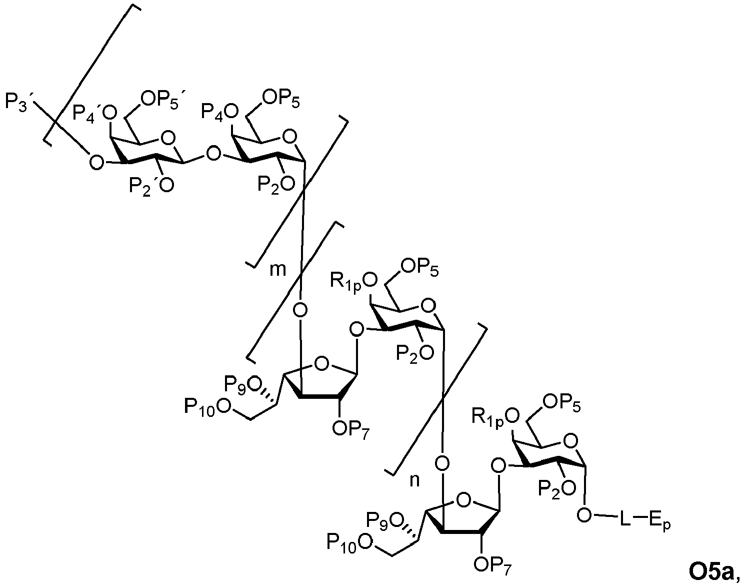 wherein **m** is 1, when **m** is an integer from 2 to 20,
then repeating the following steps **e5)** and **e6)** for **m-1** times
**e5)** removing the protecting group **P₃'** of a saccharide obtained by reacting with the monosaccharide **D4**;
**e6)** reacting the saccharide **D4** with the saccharide obtained after the step **e5)** to obtain a saccharide **O5a** 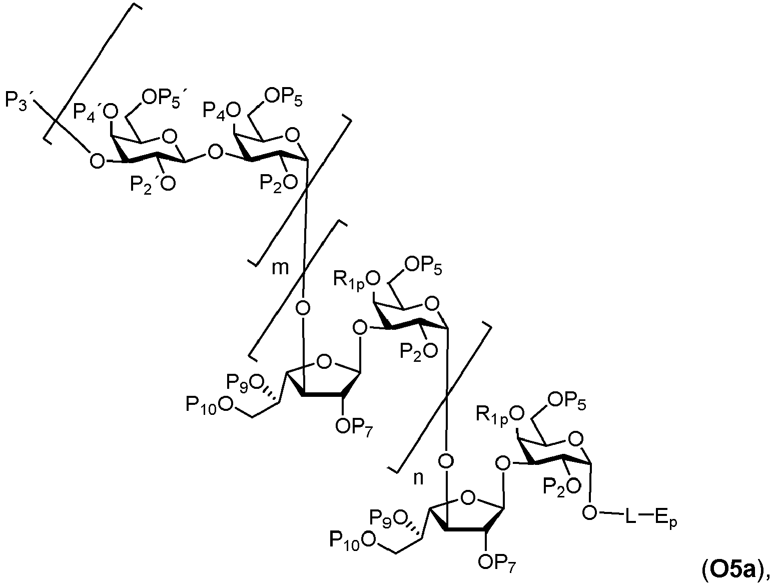 wherein **m** is an integer from 2 to 20;
or
**E6)** reacting the saccharide **O3c** with a disaccharide **D5** 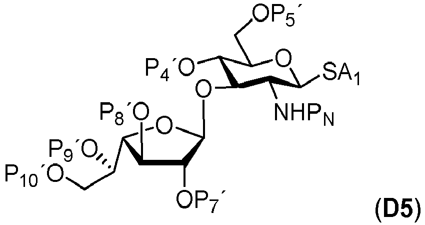 to obtain a saccharide **O5b** 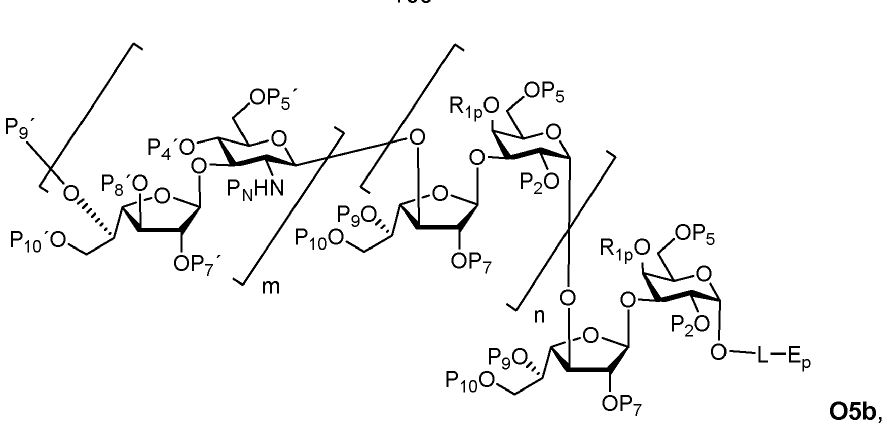 wherein **m** is 1,
when **m** is an integer from 1 to 20,
then repeating following steps **e7**) and **e8**) for **m-1** times
**e7)** removing the protecting group **P₉**' of a saccharide obtained by reacting with the monosaccharide **D5**;
**e8)** reacting the saccharide **D5** with the saccharide obtained after the step **e7**) to obtain a saccharide **O5b** 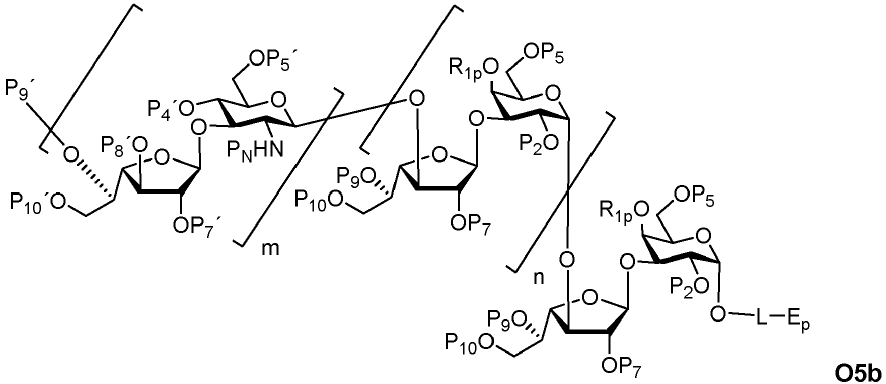 wherein **m** is an integer from 2 to 20;
**e9)** removing a protecting groups **P_{N}** and converting resulting -NH₂ groups to -NHAc groups to obtain a saccharide **O5c** 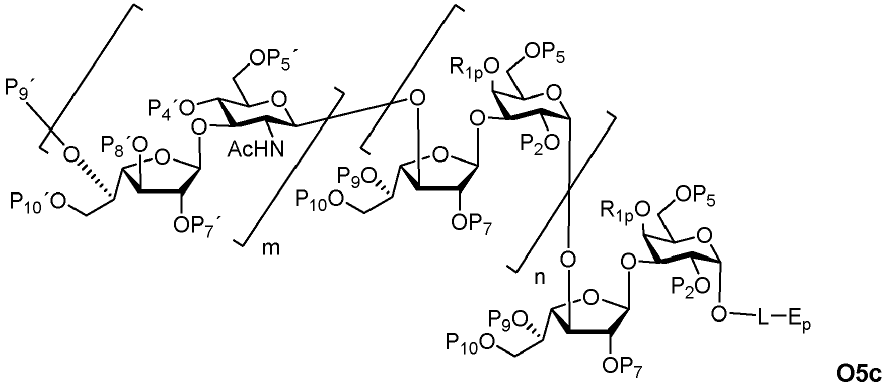
**F2)** removing all protecting groups of the saccharide **O3b**, **O4b**, **O5a** or **O5c**, to obtain a corresponding saccharide of the formula (**I-2**), (**I-3**), (**I-4**) or (**I-5**), 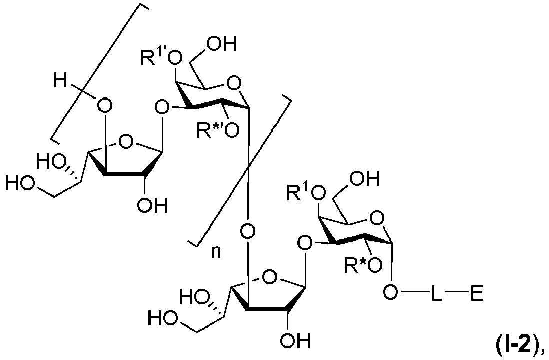 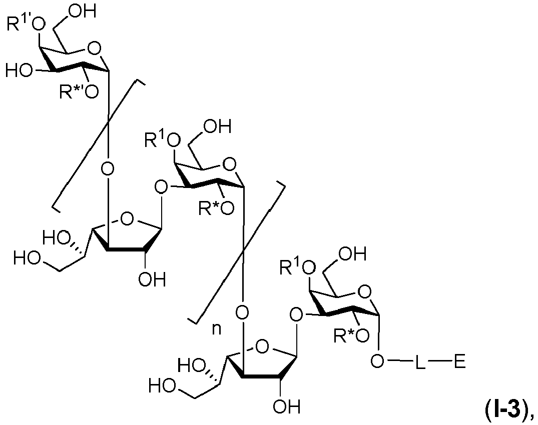 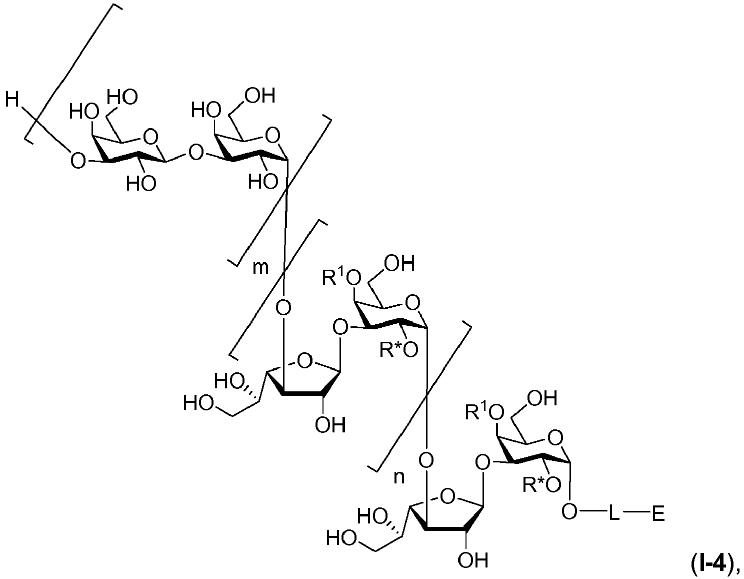 or 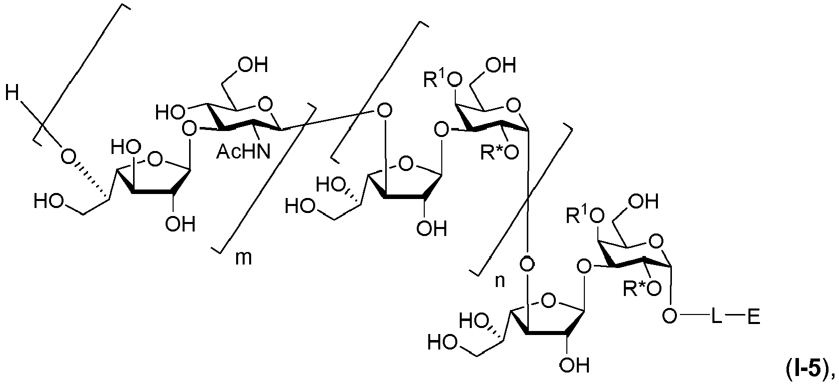 wherein **Eₚ** is a protected end group; **A₁** is an activating group; **P_{N}**, **P₁**, **P₂**, **P₂'**, **P₃**, **P₃'**, **P₄**, **P₄'**, **P₅**, **P₅'**, **P₆**, **P₇**, **P₇'**, **P₈, P₈'**, **P₉, P₉'**, **P₁₀**, **P₁₀'**, **P₁₁, P₁₂**, **P₁₃** and **P₁₄** represent protecting groups, and **L, E, R¹**, **R¹'**, **R***, **R***', **m** and **n** have the same meanings as defined in claim 1.
**OR**
a method for synthesis of a saccharide of general formula (**I**) comprising:
**A3)** providing a disaccharide **D8** 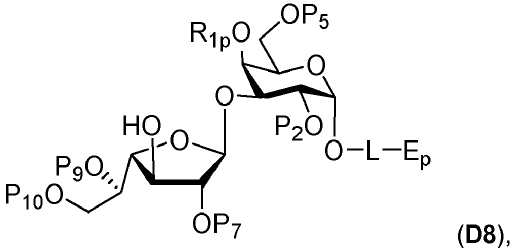 wherein **R_{1P}** is **P₄** or **U₅ₚ**; 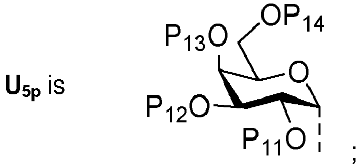
**B1")** reacting the saccharide **D8** with a saccharide **D4** 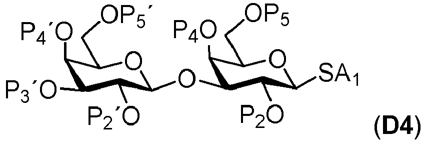 to obtain a saccharide **O3d** 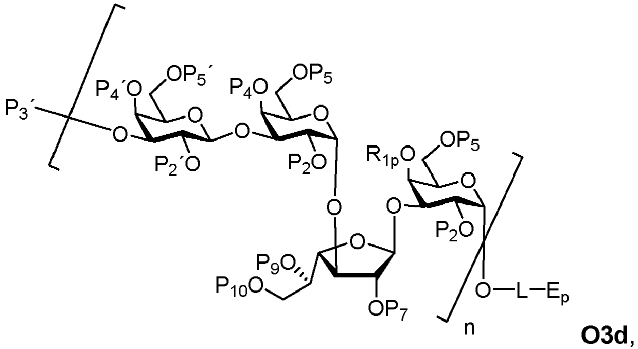 wherein n is 1,
when n is an integer from 2 to 20,
then repeating the following steps **B2")** - **B5")** for **n-1** times
**B2")** removing the protecting group **P₃'** of a saccharide obtained by reacting with the saccharide **D4**;
**B3")** reacting the saccharide **D6** with the saccharide obtained after the step B2) 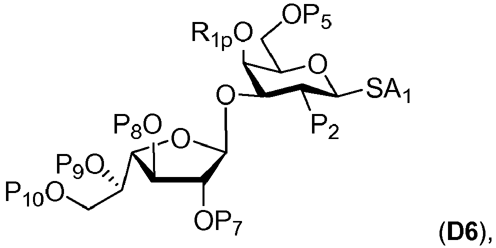
**B4")** removing the protecting group **P₈** of a saccharide obtained by reacting with the saccharide **D6**;
**B5")** reacting the saccharide **D4** with the saccharide obtained after the step B4 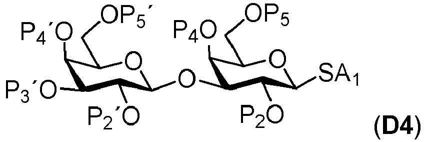 to obtain a saccharide **O3d** 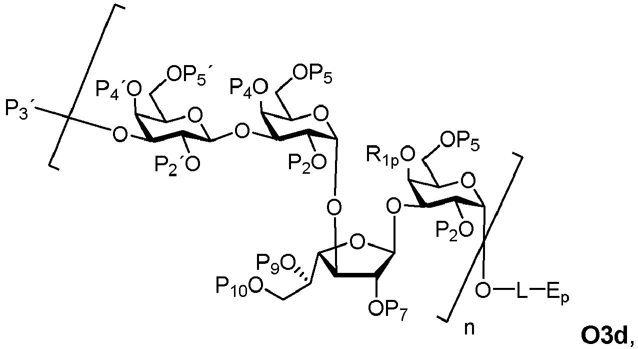 wherein n is an integer from 2 to 20,
or
**B1''')** reacting the saccharide **D8** with a saccharide **D5** 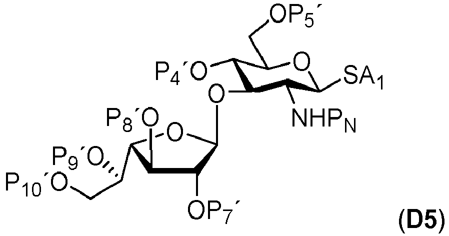 to obtain a saccharide **O3e** 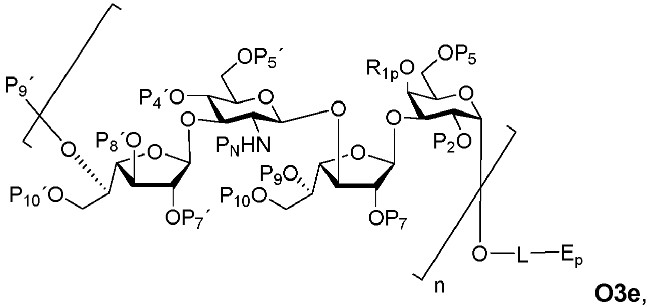 wherein n is 1,
when n is an integer from 2 to 20,
then repeating the following steps **B2''')** - **B5'''**) for **n-1** times
**B2''')** removing the protecting group **P₉'** of a saccharide obtained by reacting with the saccharide **D4**;
**B3'''**) reacting the saccharide **D6** with the saccharide obtained after the step B2) 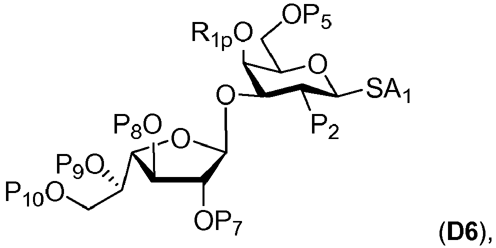
**B4''')** removing the protecting group **P₈** of a saccharide obtained by reacting with the saccharide **D6**;
**B5''')** reacting the saccharide **D5** with the saccharide obtained after the step B4 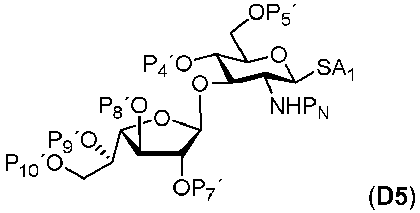 to obtain a saccharide **O3e** 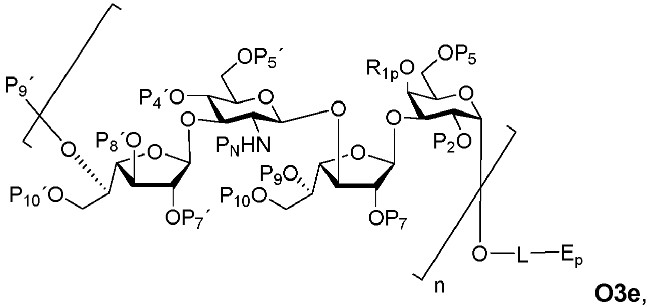 wherein n is an integer from 2 to 20,
**B6''')** removing a protecting groups **P_{N}** and converting resulting -NH₂ groups to -NHAc groups to obtain a saccharide **O3f** 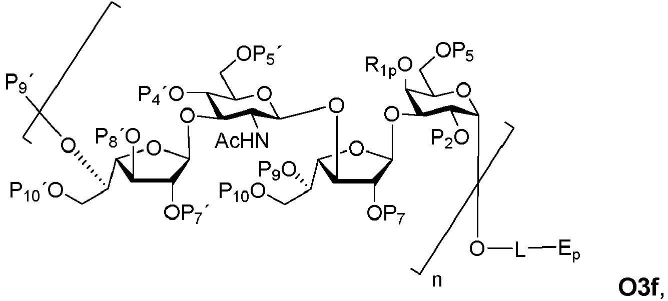
**F3)** removing all protecting groups of the saccharide **O3d** or **O3f** to obtain a corresponding saccharide of the formula **(I-6)** or **(I-7)** 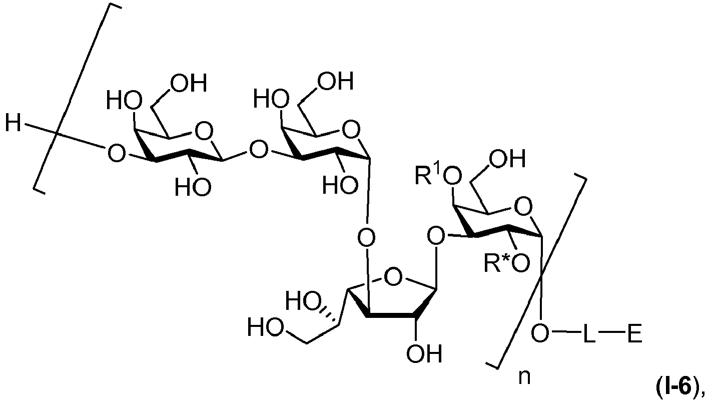 or 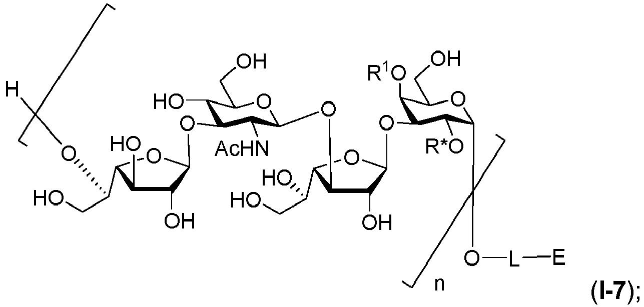 wherein **Eₚ** is a protected end group; **A₁** is an activating group; **P_{N}**, **P₂**, **P₂'**, **P₃'**, **P₄**, **P₄'**, **P₅**, **P₅'**, **P₇**, **P₇'**, **P₈, P₈'**, **P₉**, **P₉'**, **P₁₀**, **P₁₀'**, **P₁₁**, **P₁₂** , **P₁₃** and **P₁₄** represent protecting groups, and **L**, **E**, **R¹**, **R***, **m**, and **n** have the same meanings in claim 1.

15. An intermediate compound for preparing a saccharide of the general formula (**I**), wherein the intermediate compound has any one of general formulae (**O1b**), (**O1c**), (**O1d**), (**O2a**), (**O2b**), (**O3a**), (**O3b**), (**O3c**), (**O3d**), (**O3e**), (**O3f**), (**O4a**), (**O4b**), (**O5a**), (**O5b**), and (**O5c**): 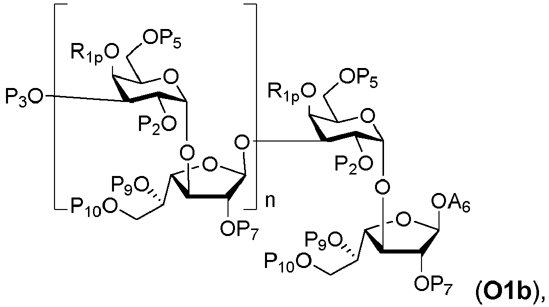 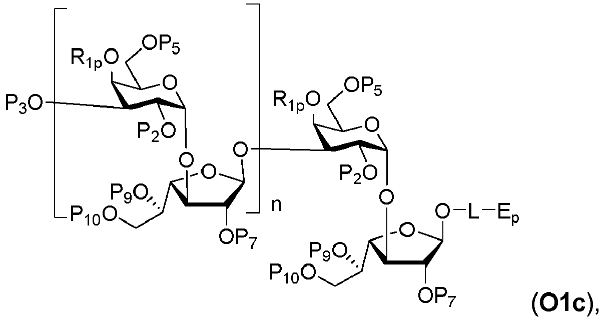 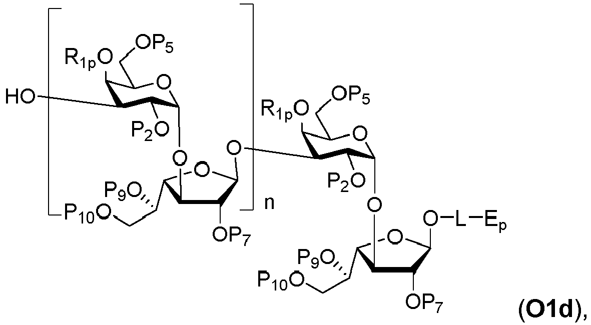 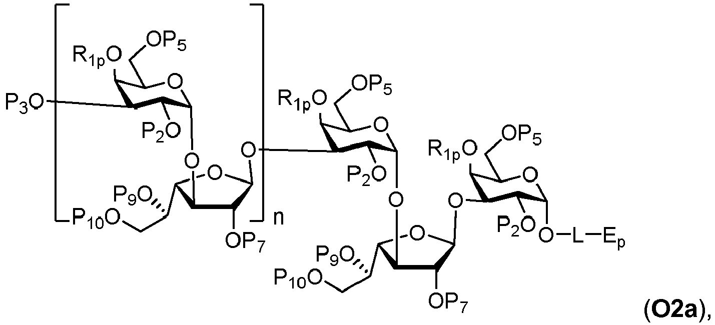 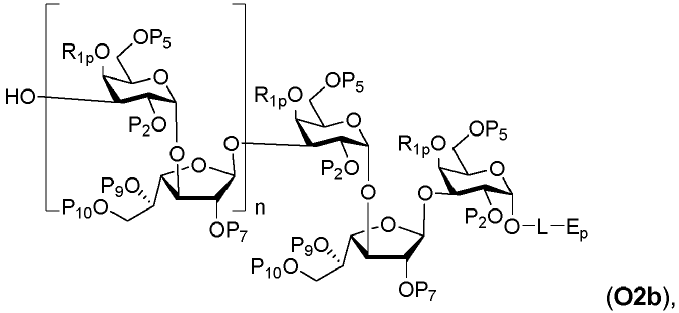 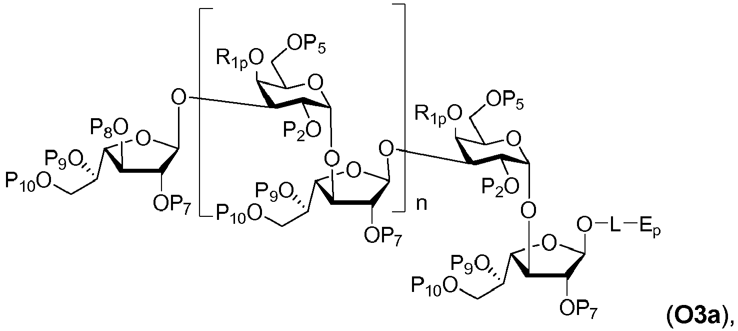 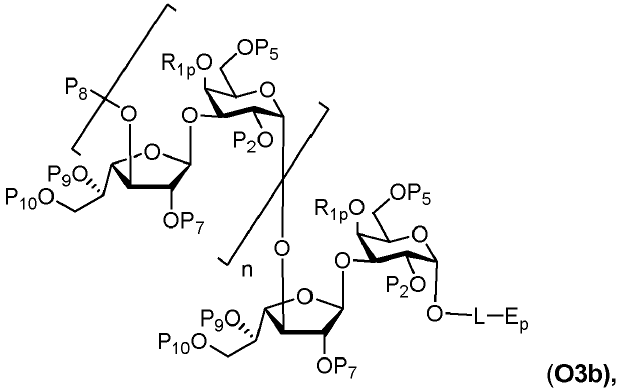 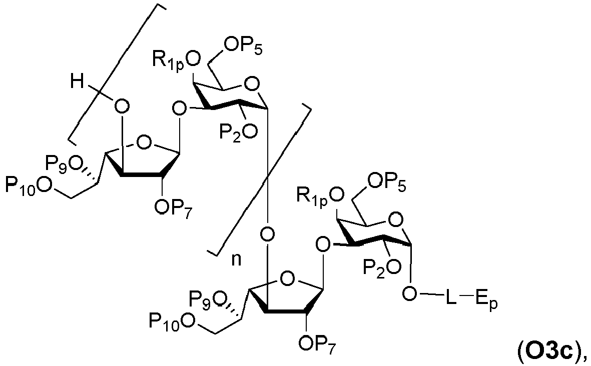 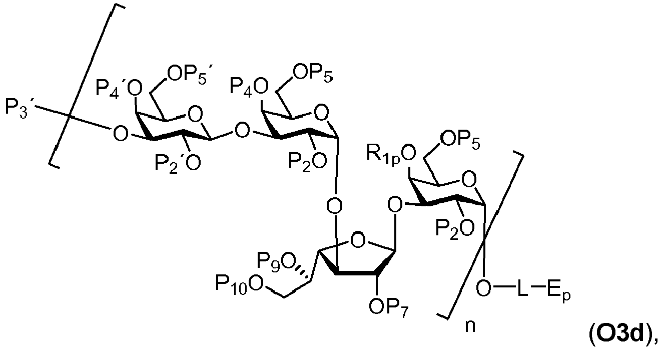 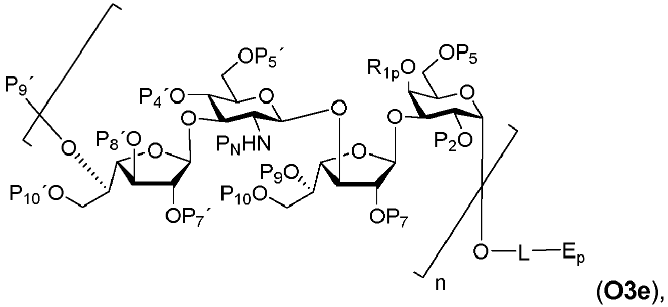 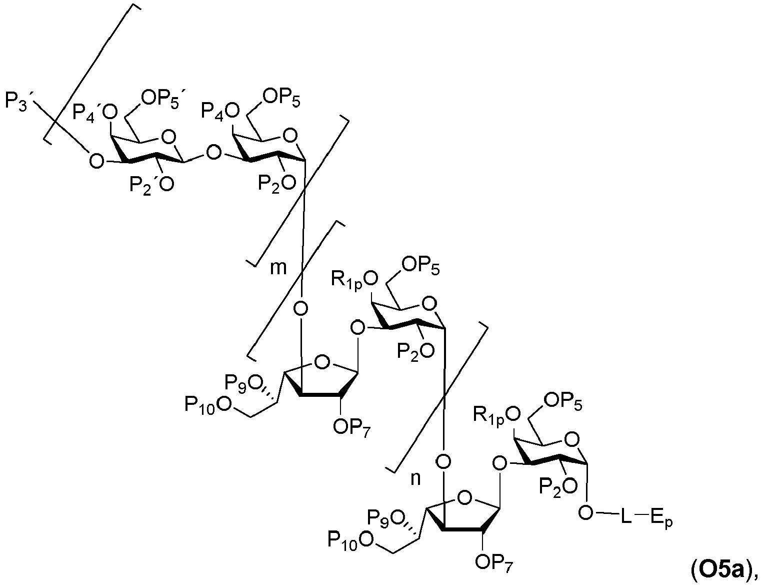 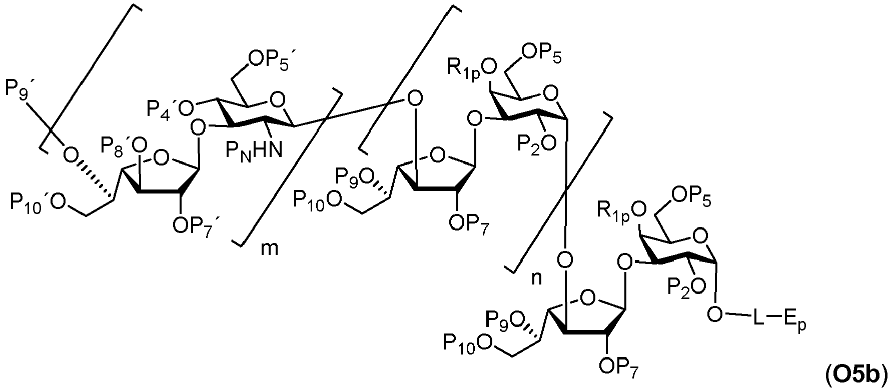 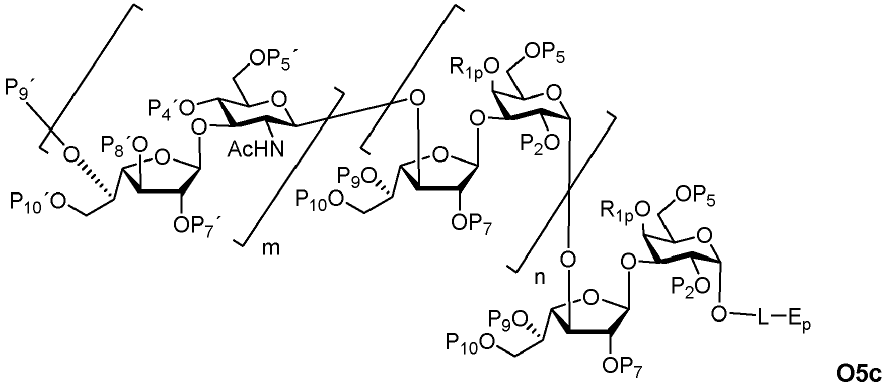 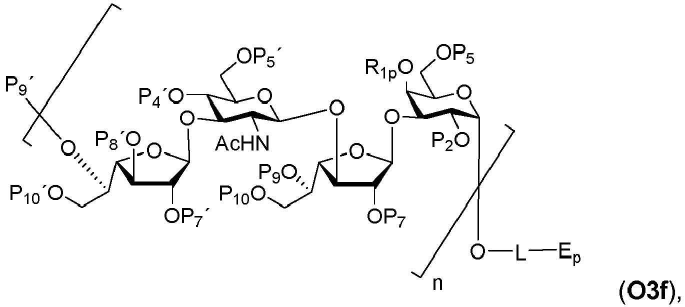 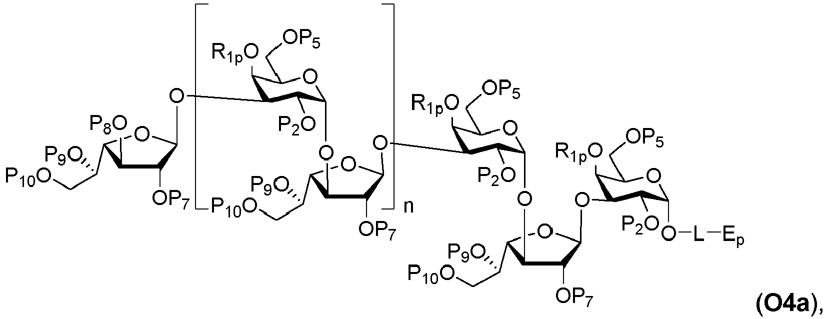 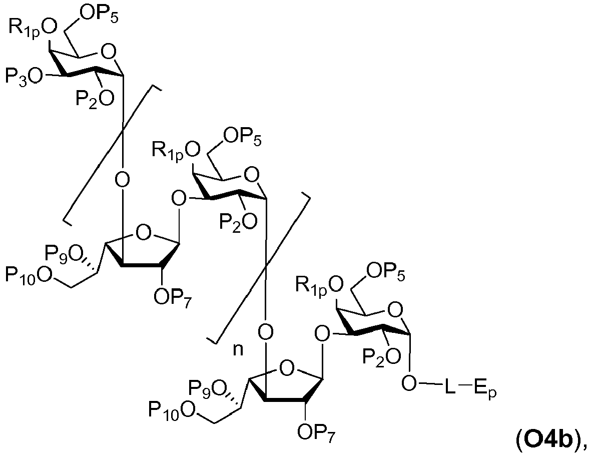 wherein **A₆** represents an activating group; **Eₚ** is protected end group, **P_{N}**, **P₂**, **P₂'**, **P₃'**, **P₄**, **P₄'**, **P₅**, **P₅'**, **P₇**, **P₇'**, **P₈, P₈', P₉, P₉'**, **P₁₀**, **P₁₀'**, **P₁₁**, **P₁₂**, **P₁₃** and **P₁₄** represent protecting groups, and **L, R₁ₚ**, **m,** and **n** have the same meanings as defined in claim 24.
